# EUROPÄISCHE PATENTSCHRIFT

(11) **EP 1 406 891 B1**
(45) Veröffentlichungstag und Bekanntmachung des Hinweises auf die Patenterteilung: **22.03.2006**
(21) Anmeldenummer: 02805269.4
(22) Anmeldetag: 21.05.2002
(51) Int. Cl.: C07D 335/06, C07D 335/16, A61K 31/382, A61P 25/28

(54) **THIOCHROMENONE GEGEN SCHMERZZUSTÄNDE UND NEURODEGENERATIVE ERKRANKUNGEN**
THIOCHROMENONES USED TO COMBAT PAINFUL CONDITIONS AND NEURODEGENERATIVE DISEASES
THIOCHROMEMONES UTILISES POUR LUTTER CONTRE DES ETATS DOULOUREUX ET DES MALADIES NEURODEGENERATIVES

(30) Priorität: 31.05.2001 DE 10126434
(43) Veröffentlichungstag der Anmeldung: 14.04.2004
(73) Patentinhaber: Bayer HealthCare AG, 51368 Leverkusen (DE)
(72) Erfinder: MEIER, Heinrich, 42115 Wuppertal (DE); ALLERHEILIGEN, Swen, 45259 Essen (DE); GERISCH, Michael, 42115 Wuppertal (DE); SCHOHE-LOOP, Rudolf, 42327 Wuppertal (DE); VOERSTE, Arnd, 50677 Köln (DE); MAULER, Frank, 51491 Overath (DE); DE VRY, Jean, Marie, Victor, 51503 Rösrath (DE); MÜLLER, Thomas, 42115 Wuppertal (DE); METHFESSEL, Christoph, 42327 Wuppertal (DE)
(86) Internationale Anmeldenummer: PCT/EP2002/005538
(87) Internationale Veröffentlichungsnummer: WO 2003/053955

(56) Entgegenhaltungen:
- PATENT ABSTRACTS OF JAPAN vol. 008, no. 232 (C-248), 25. Oktober 1984 (1984-10-25) & JP 59 112983 A (UENO SEIYAKU OUYOU KENKYUSHO:KK), 29. Juni 1984 (1984-06-29)
- DATABASE CHEMABS [Online] Chemical abstracts service, columbus, ohio, US; TSUJIKAWA, TERUAKI ET AL: "Thiochromene compounds" Database accession no. 1973:111131 XP002214131 -& JP 48 004471 B 20. Januar 1973 (1973-01-20)

## Beschreibung

Die Erfindung betrifft neue Thiochromenone und Verfahren zu ihrer Herstellung, ihre Verwendung zur Behandlung und/oder Prophylaxe von Krankheiten, insbesondere zur Behandlung und/oder Prophylaxe von Schmerzzuständen und neurodegenerativen Erkrankungen.

Die Aminosäure L-Glutamat ist der wichtigste erregende Neurotransmitter im Gehim. Glutamatrezeptoren lassen sich in in zwei grosse Klassen einteilen: 1. ionotrope Rezeptoren, die Ionenkanäle direkt steuern und 2. metabotrope Rezeptoren (mGluRs).

Metabotrope Glutamatrezeptoren sind eine heterogene Klasse von G-Proteingekoppelten Rezeptoren, die durch Glutamat aktiviert unterschiedliche Second Messenger-Kaskaden aktivieren können. Am Ende der Second Messenger-Kaskaden steht die Modulation zahlreicher intrazellulärer Vorgänge, u.a. auch die Regulation der präsynaptischen Glutamat-Ausschüttung bzw. die Regulation der postsynaptischen, ionotropen Glutamat-Rezeptoren.

Derzeit kennt man 8 verschiedene Subtypen der metabotropen Glutamatrezeptoren, die sich durch Second-Messenger Kaskade, Pharmakologie und Lokalisation im Hirn unterscheiden (zur Übersicht: *Ann. Rev. Pharmacol. Toxicol.* **1997**,*37*,205) .

Die US-A-4,221,800 und US-A-4,571,405 beschreiben die anti-allergische Wirkung von Thioxanthen-9-onen.

Die Herstellung bzw. die anti-schistosomiatische Wirkung von Thioxanthen-9-onen und 2,3-Cyclopentathiochromonen werden in US-A-3,312,598, GB 803,803, GB 804,689, GB 805,870, *Chem. Abstr. 54,* 7740d (DE 1024981), *Chem. Abstr. 62,* 11763h und *J. Med. Chem.* **1967**, *10*, 867 - 876 offenbart.

In *Liebigs Ann.* **1964**, *680,* 40-51 wird die Synthese von 2-Chloro-6,7,8,9,10,10a-hexahydrocyclohepta[b]thiochromen-11(5aH)-on beschrieben.

Aus *CAPLUS* **1984,** 610989 (JP 59112983) sind 2,3-Cyclopentathiochromone als Analgetika und Entzündungshemmer bekannt.

Die vorliegende Erfindung betrifft Verbindungen der allgemeinen Formel (I) 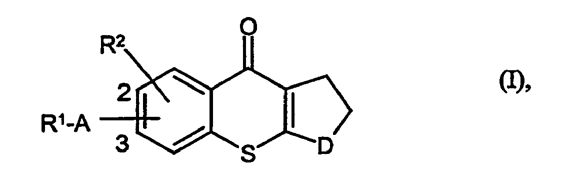 in welcher
der Rest R¹-A- an einer der Positionen 2 oder 3 des Thiochromenon-Rings sitzt,
- R¹: für (C₆-C₁₀)-Aryl oder 5- bis 10-gliedriges Heteroaryl steht, wobei Aryl und Heteroaryl gegebenenfalls gleich oder verschieden durch Reste ausgewählt aus der Gruppe Halogen, Formyl, Carbamoyl, Cyano, Hydroxy, Trifluormethoxy, Nitro, -NR³R⁴, Tetrazolyl, (C₁-C₆)-Alkoxycarbonyl sowie gegebenenfalls durch Hydroxy, Morpholinyl, (C₁-C₆)-Acyloxy oder Halogen substituiertes (C₁-C₆)-Alkyl, (C₁-C₆)-Alkoxy, (C₁-C₆)-Acyl und (C₁-C₆)-Alkylthio substituiert sind,
worin
R³ und R⁴ unabhängig voneinander Wasserstoff, (C₁-C₆)-Alkyl oder (C₁-C₆)-Acyl bedeuten, für 3- bis 12-gliedriges Carbocyclyl oder 4- bis 12-gliedriges Heterocyclyl steht, wobei Carbocyclyl und Heterocyclyl gegebenenfalls gleich oder verschieden durch Reste ausgewählt aus der Gruppe (C₁₋C₆)-Alkyl, (C₁-C₆)-Alkoxy, (C₁-C₆)-Acyl, (C₁-C₆)-Alkoxycarbonyl oder Oxo substituiert sind, und
- A: für eine Bindung oder eine Gruppe der Formel O, S, NR⁶, CO, SO, SO₂, SO₂₋O, CO-NR⁷, SO₂-NR⁸, O-SO₂, NR⁹-CO, NR¹⁰-SO₂, NR¹¹-SO₂-O, NR¹²-SO₂₋NR¹³ oder NR¹⁴-CO-NR¹⁵ steht,
worin R⁶, R⁷, R⁸, R⁹, R¹⁰, R¹¹, R¹², R¹³, R¹⁴ und R¹⁵ (C₃-C₈)-Cycloalkyl oder gegebenenfalls ungesättigtes (C₁-C₆)-Alkyl, das gegebenenfalls durch Hydroxy, Phenyl, (C₁-C₆)-Alkoxy, (C₁-C₆)-Alkoxycarbonyl oder (C₃-C₈)-Cycloalkyl substituiert ist, bedeuten, wobei Phenyl seinerseits durch Halogen oder (C₁-C₄)-Alkyl substituiert sein kann, oder
worin R⁶, R⁷, R⁹, R¹⁰, R¹¹, R¹², R¹⁴ und R¹⁵ Wasserstoff bedeuten,
oder
- R¹: für eine Gruppe der Formel R⁵-E- steht,
worin
E gegebenenfalls ungesättigtes (C₁-C₁₀)-Alkandiyl bedeutet, und
R⁵ Wasserstoff, Carbamoyl, Halogen, Hydroxy, Nitro, Trifluormethyl, Amino, Mono-(C₁-C₆)-Alkylamino, Di-(C₁-C₆)-Alkylamino, (C₁-C₆)-Alkoxy, (C₆-C₁₀)-Aryl, 5- bis 10-gliedriges Heteroaryl oder 4- bis 10-gliedriges, gegebenenfalls mit Oxo und/oder (C₁-C₆)-Alkyl substituiertes, gegebenenfalls benzokondensiertes Heterocyclyl bedeutet, wobei Aryl, Heteroaryl und Benzo ihrerseits durch Reste ausgewählt aus der Gruppe Halogen, Cyano, Trifluormethyl, Trifluormethoxy, Nitro und (C₁-C₆)-Alkyl substituiert sein können, und
A für S, NR⁶, CO, SO, SO₂, SO₂₋O, CO-NR⁷, SO₂-NR⁸, O-SO₂, NR⁹-CO, NR¹⁰-SO₂, NR¹¹-SO₂-O, NR¹²-SO₂₋NR¹³ oder NR¹⁴-CO-NR¹⁵ steht,
worin
R⁶, R⁷, R⁸, R⁹, R¹⁰, R¹¹, R¹², R¹³, R¹⁴ und R¹⁵ (C₃-C₈)-Cycloalkyl oder gegebenenfalls ungesättigtes (C₁-C₆)-Alkyl, das gegebenenfalls durchHydroxy, Phenyl, (C₁-C₆)-Alkoxy, (C₁-C₆)-Alkoxycarbonyl oder (C₃₋C₈)-Cycloalkyl substituiert ist, bedeuten, wobei Phenyl seinerseits durch Halogen oder (C₁-C₄)-Alkyl substituiert sein kann, oder worin
R⁶, R⁷, R⁹, R¹⁰, R¹¹, R¹², R¹⁴ und R¹⁵ Wasserstoff bedeuten,
oder
der Rest R¹-A- für Wasserstoff oder Amino steht,
R² für Wasserstoff, Halogen oder (C₁-C₆)-Alkyl oder (C₁-C₆)-Alkoxy steht, wobei Alkyl und Alkoxy gegebenfalls bis zu zweifach gleich oder verschieden durch Resten ausgewählt aus der Gruppe Hydroxy, (C₁-C₆)-Alkoxy, Mono- und Di-(C₁-C₆)-alkylamino substituiert sind, und
D für einen gegebenenfalls mit Fluor substituierten, zweibindigen Kohlenwasserstoffrest mit 3 bis 10 Kohlenstoffatomen steht,
und deren Salze, Hydrate und/oder Solvate,
mit der Ausnahme von 2-Chloro-6,7,8,9,10,10a-hexahydrocyclohepta[b]thiochromen-11(5aH)-on.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomere und Diastereomere lassen sich in bekannter Weise in die stereoisomer einheitlichen Bestandteile trennen.

Die erfindungsgemäßen Verbindungen können auch in Form ihrer Salze, Hydrate und/oder Solvate vorliegen.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methyl-piperidin.

Hydrate der erfindungsgemäßen Verbindungen sind stöchiometrische Zusammensetzungen der Verbindungen oder seinen Salzen mit Wasser.

Solvate der erfindungsgemäßen Verbindungen sind stöchiometrische Zusammensetzungen der Verbindungen oder seinen Salzen mit Lösungsmittel.

Im Rahmen der vorliegenden Erfindung haben die Substituenten im Allgemeinen die folgende Bedeutung:

(C₁-C₆)-Acyl steht für einen geradkettigen oder verzweigten Acylrest mit 1 bis 6 Kohlenstoffatomen. Beispielsweise seien genannt: Acetyl, Ethylcarbonyl, Propylcarbonyl, Isopropylcarbonyl, n-Butylcarbonyl, Pivaloyl, Isobutylcarbonyl, Pentylcarbonyl und Hexylcarbonyl. Bevorzugt ist ein geradkettiger oder verzweigter Acylrest mit 1 bis 4 Kohlenstoffatomen. Besonders bevorzugt sind Acetyl und Ethylcarbonyl.

(C₁-C₁₀)-Alkandiyl steht für einen geradkettigen oder verzweigten Alkandiylrest mit 1 bis 10 Kohlenstoffatomen, wobei die beiden freien Valenzen des Alkandiylrests an einem Kohlenstoffatom (geminal), an benachbarten Kohlenstoffatomen (vicinal) oder an nicht-benachbarten Kohlenstoffatomen sein können. Bevorzugt ist ein geradkettiger oder verzweigter Alkandiylrest mit 3 bis 8, besonders bevorzugt mit 3 bis 6 Kohlenstoffatomen. Beispielsweise seien genannt Methylen, Ethylen, Propylen, Propan-1,2-diyl, Propan-2,2-diyl, 2-Methyl-propan-1,3-diyl, Butan-1,3-diyl, Butan-2,4-diyl, Pentan-2,4-diyl, 2-Methyl-pentan-2,4-diyl.

(C₁-C₆)-Alkoxy steht für einen geradkettigen oder verzweigten Alkoxyrest mit 1 bis 6 Kohlenstoffatomen. Bevorzugt ist ein geradkettiger oder verzweigter Alkoxyrest mit 1 bis 4 Kohlenstoffatomen. Beispielsweise seien genannt: Methoxy, Ethoxy, n-Propoxy, Isopropoxy, tert.Butoxy, n-Pentoxy und n-Hexoxy. Besonders bevorzugt ist ein geradkettiger oder verzweigter Alkoxyrest mit 1 bis 3 Kohlenstoffatomen.

(C₁-C₆)-Alkoxycarbonyl steht für einen geradkettigen oder verzweigten Alkoxycarbonylrest mit 1 bis 6 Kohlenstoffatomen. Bevorzugt ist ein geradkettiger oder verzweigter Alkoxycarbonylrest mit 1 bis 4 Kohlenstoffatomen. Beispielsweise seien genannt: Methoxycarbonyl, Ethoxycarbonyl, n-Propoxycarbonyl, Isopropoxycarbonyl und tert.Butoxycarbonyl. Besonders bevorzugt ist ein geradkettiger oder verzweigter Alkoxycarbonylrest mit 1 bis 3 Kohlenstoffatomen.

(C₁-C₆)- und C₁-C₃)-Alkyl steht für einen geradkettigen oder verzweigten Alkylrest mit 1 bis 6 bzw. 1 bis 3 Kohlenstoffatomen. Bevorzugt ist ein geradkettiger oder verzweigter Alkylrest mit 1 bis 4, besonders bevorzugt mit 1 bis 3 Kohlenstoffatomen.

Beispielsweise seien genannt: Methyl, Ethyl, n-Propyl, Isopropyl, tert.Butyl, n-Pentyl und n-Hexyl.

(C₁-C₆)-Alkylamino steht fiir einen geradkettigen oder verzweigten Alkylaminorest mit 1 bis 6 Kohlenstoffatomen. Bevorzugt ist ein geradkettiger oder verzweigter Alkylaminorest mit 1 bis 4 Kohlenstoffatomen. Beispielsweise seien genannt: Methylamino, Ethylamino, n-Propylamino, Isopropylamino, tert.Butylamino, n-Pentylamino und n-Hexylamino. Besonders bevorzugt ist ein geradkettiger oder verzweigter Alkylaminorest mit 1 bis 3 Kohlenstoffatomen.

(C₁-C₆)-Dialkylamino steht für einen geradkettigen oder verzweigten Dialkylaminorest, wobei die Alkylreste gleich oder verschieden sein können und jeweils 1 bis 6 Kohlenstoffatome enthalten. Bevorzugt ist ein geradkettiger oder verzweigter Dialkylaminorest, wobei der Alkylrest jeweils 1 bis 4 Kohlenstoffatome enthält Beispielsweise seien genannt: Dimethylamino, Diethylamino, Di-n-propylamino, Diisopropylamino, Di-t-butylamino, Di-n-pentylamino, Di-n-hexylamino, Ethylmethylamino, Isopropylmethylamino, n-Butylethylamino, n-Hexyl-i-pentylamino, Besonders bevorzugt ist ein geradkettiger oder verzweigter Alkylaminorest mit 1 bis 3 Kohlenstoffatomen.

(C₁-C₆)-Alkylthio steht für einen geradkettigen oder verzweigten Alkylthiorest mit 1 bis 6 Kohlenstoffatomen. Bevorzugt ist ein geradkettiger oder verzweigter Alkylthiorest mit 1 bis 4 Kohlenstoffatomen. Beispielsweise seien genannt: Methylthio, Ethylthio, n-Propylthio, Isopropylthio, tert.Butylthio, n-Pentylthio und n-Hexylthio. Besonders bevorzugt ist ein geradkettiger oder verzweigter Alkylthiorest mit 1 bis 3 Kohlenstoffatomen.

(C₆-C₁₀)-Aryl steht im allgemeinen für einen aromatischen Rest mit 6 bis 10 Kohlenstoffatomen. Bevorzugte Arylreste sind Phenyl und Naphthyl.

3- bis 12-gliedriges Carbocyclyl steht für einen mono- oder polycyclischen, carbocyclischen Rest mit 3 bis 12 Ringatomen. 3- Bis 10-gliedriges, insbesondere 3- bis 8-gliedriges Carbocyclyl sind bevorzugt. Mono- oder bicyclisches Carbocyclyl ist bevorzugt. Besonders bevorzugt ist monocyclisches Carbocyclyl. Die Carbocyclyl-Reste können gesättigt oder teilweise ungesättigt sein. Gesättigte Carbocyclyl-Reste sind bevorzugt. Ebenso bevorzugt sind (C₃ - C₁₀)-Cycloalkyl, ganz besonders (C₄ - C₇)-Cycloalkyl. Beispielsweise seien genannt: Cyclopropyl, Cyclobutyl, Cyclopentyl, Cyclopentenyl, Cyclohexyl, Cycloheptyl, Norborn-1-yl, Norborn-2-yl, Norbom-7-yl, Norbom-2-en-7-yl, Cyclooctyl, Cubyl, Cyclononyl, Cyclodecyl, Decalinyl, Adamant-1-yl, Adamant-2-yl.

(C₃-C₈)-Cycloalkan-1,1-diyl steht für Cyclopropan-1,1-diyl, Cyclobutan-1,1-diyl, Cyclopentan-1,1-diyl oder Cyclohexan-1,1-diyl.

(C₃-C₈)-Cycloalkyl steht für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl.

Halogen steht für Fluor, Chlor, Brom und Jod. Bevorzugt sind Fluor, Chlor und Brom. Besonders bevorzugt sind Fluor und Chlor.

5- bis 10-gliedriges Heteroaryl steht für einen aromatischen, mono- oder bicyclischen Rest mit 5 bis 10 Ringatomen und bis zu 5 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff- oder Heteroatom gebunden sein. Beispielsweise seien genannt: Thienyl, Furyl, Pyrrolyl, Thiazolyl, Oxazolyl, Imidazolyl, Tetrazolyl, Pyridyl, Pyrimidyl, Pyridazinyl, Indolyl, Indazolyl, Benzofuranyl, Benzothiophenyl, Chinolinyl, Isochinolinyl.

4- bis 12-gliedriges oder 4- bis 10-gliedriges Heterocyclyl steht für einen mono- oder polycyclischen, heterocyclischen Rest mit 4 bis 12 bzw. 10 Ringatomen und bis zu 4, vorzugsweise bis zu 2 Heteroatomen bzw. Heterogruppen aus der Reihe N, O, S, SO, SO₂. 4- bis 8-gliedriges Heterocyclyl ist bevorzugt. Mono- oder bicyclisches Heterocyclyl ist bevorzugt. Besonders bevorzugt ist monocyclisches Carbocyclyl. Als Heteroatome sind N und O bevorzugt. Die Heterocyclyl-Reste können gesättigt oder teilweise ungesättigt sein. Gesättigte Heterocyclyl-Reste sind bevorzugt. Die Heterocyclyl-Reste können über ein Kohlenstoffatom oder ein Heteroatom gebunden sein. Besonders bevorzugt sind 5- bis 7-gliedrige, monocyclische gesättigte Heterocyclylreste mit bis zu zwei Heteroatomen aus der Reihe O, N und S. Beispielsweise seien genannt: Oxetan-3-yl, Pyrrolidin-2-yl, Pyrrolidin-3-yl, Pyrrolinyl, Tetrahydrofuranyl, Tetrahydrothienyl, Pyranyl, Piperidinyl, Piperazinyl, Thiopyranyl, Morpholinyl, Perhydroazepinyl, 1,5-Dioxa-9-azaspiro[5,5]undec-yl.

Ein zweibindiger Kohlenwasserstoffrest mit 3 bis 10 Kohlenstoffatomen steht für einen geradkettigen, verzweigten, teilweise cyclischen oder cyclischen, gesättigten oder ungesättigten organischen Rest, der 3 bis 10 Kohlenstoffatome umfasst, der über zwei Bindungen an einem oder zwei Kohlenstoffatomen des Kohlenwasserstoffrests an die Nachbaratome gebunden ist und der an den freien Valenzen, abhängig vom Sättigungs- und Cyclisierungsgrad, mit Wasserstoffatomen abgesättigt ist. Gesättigte organische Reste sind bevorzugt. Ebenso bevorzugt sind Kohlenwasserstoffreste mit 3 bis 8, besonders bevorzugt mit 3 bis 6 Kohlenstoffatomen. Der Kohlenwasserstoffrest kann aus einem geradkettigen oder verzweigten Alkandiyl-Rest bestehen, wobei zwei geminale, vicinale oder nicht benachbarte Wasserstoffatome des Alkandiyl-Rests wiederum durch einen geradkettigen oder verzweigten Alkandiyl-Rest ersetzt sein können. Beispielsweise seien genannt: geradkettiges oder verzweigtes (C₃-C₁₀)-Alkandiyl, (C₃-C₁₀)-Cycloalkandiyl sowie insgesamt 3 bis 10 Kohlenstoffatome umfassendes Mono- oder Di-alkylcycloalkandiyl, Cycloalkyl-alkandiyl, (Ylo-alkyl)cycloalkyl, (Ylo-cycloalkyl)alkyl sowie [(Ylo-alkyl)cycloalkyl]alkyl. Beispielsweise seien genannt: Propylen, Butylen, Pentylen, Butan-1,2-diyl, 2-Ethyl-propan-1,3-diyl, 2-Methyl-ethan-1,2-diyl, 2-Methyl-propan-1,2-diyl, 2-Methyl-butan-1,3-diyl, Cyclobutan-1,1-diyl, Cyclopentan-1,1-diyl, Cyclohexan-1,1-diyl, Cyclohexan-1,2-diyl, Cyclohexan-1,3-diyl, 4,4-Dimethyl-cyclohexan-1,1-diyl, 4-*tert*Butyl- cyclohexan-1,1-diyl, 2-Cyclohexyl-propan-1,3-diyl, 1-Ylomethyl-cyclobutyl, 1-Ylomethyl-cyclohexyl, 1-(2-Yloethyl)cyclohexyl, 2-(2-Yloethyl)cyclohexyl, [1-(Ylomethyl)cyclobut-1-yl]methyl, [1-(Ylomethyl)cyclohex-1-yl]methyl.

Oxo steht für ein doppelt gebundenes Sauerstoffatom.

Wenn Reste in den erfindungsgemäßen Verbindungen gegebenenfalls substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt.

Wenn Reste in den erfindungsgemäßen Verbindungen gegebenenfalls ungesättigt sind, enthält der Rest, soweit nicht anders spezifiziert, eine oder mehrere Doppel- oder Dreifachbindungen, welche gegebenenfalls konjugiert oder cumuliert vorliegen. Doppelbindungen sind bevorzugt. Besonders bevorzugt ist eine Doppelbindung.

Eine Ausführungsform der Erfindung betrifft Verbindungen der allgemeinen Formel (I),
in welcher der Rest R¹-A- an der Position 3, der Rest R² an der Position 2 des Thiochromenon-Rings sitzt und R¹, A, R² und D die oben angegebene Bedeutung haben.

Eine weitere Ausführungsform der Erfindung betrifft Verbindungen der allgemeinen Formel (I),
in welcher
der Rest R¹-A- an der Position 3 des Thiochromenon-Rings sitzt,
- R¹: für (C₆-C₁₀)-Aryl oder 5- bis 10-gliedriges Heteroaryl steht, wobei Aryl und Heteroaryl gegebenenfalls gleich oder verschieden bis zu zweifach durch Reste ausgewählt aus der Gruppe Halogen, Formyl, Cyano, Hydroxy, Hydroxymethyl, (C₁-C₆)-Alkyl, substituiert sind,
für 4- bis 10-gliedriges Heterocyclyl steht, wobei Heterocyclyl gegebenenfalls gleich oder verschieden durch Reste ausgewählt aus der Gruppe (C₁-C₆)-Alkyl, (C₁-C₆)-Alkoxy, (C₁-C₆)-Alkoxycarbonyl oder Oxo substituiert sind,
- A: für eine Bindung oder eine Gruppe der Formel NR⁶, CO-NR⁷, SO₂-NR⁸ oder NR⁹-CO, steht,
worin
R⁶, R⁷, R⁸ und R⁹ Wasserstoff oder gegebenenfalls ungesättigtes (C₁-C₆)-Alkyl, das gegebenenfalls bis zu zweifach gleich oder verschieden durch Hydroxy oder Methoxy substituiert ist, bedeuten, oder worin
R⁶, R⁷ und R⁹ Wasserstoff bedeuten,
- R²: für Wasserstoff steht, und
- D: für eine Gruppe der Formel (CH₂)ₘ-CR¹⁶R¹⁷-(CH₂)ₙ steht,
worin die Gesamtzahl der Kohlenstoffatome 3 bis 10 beträgt,
m und n gleich oder verschieden sind und eine natürliche Zahl aus der Reihe 0 bis 6 bedeuten,
und
R¹⁶ und R¹⁷ gleich oder verschieden sind und Wasserstoff oder (C₁-C₆)-Alkyl, das gegebenenfalls gleich oder verschieden mit (C₃-C₅)-Cycloalkyl oder Halogen substituiert ist, bedeuten,
oder
CR¹⁶R¹⁷ (C₃-C₆)-Cycloalkan-1,1-diyl bedeutet,
und deren Salze, Hydrate und/oder Solvate.

Eine weitere Ausführungsform der Erfindung betrifft Verbindungen der allgemeinen Formel (I),
in welcher
der Rest R¹-A- an der Position 3 des Thiochromenon-Rings sitzt,
- R¹: für Phenyl oder 5- bis 6-gliedriges Heteroaryl steht, wobei Phenyl und Heteroaryl gegebenenfalls gleich oder verschieden bis zu zweifach durch Reste ausgewählt aus der Gruppe Halogen, Cyano, (C₁-C₃)-Alkyl, substituiert sind,
für 5- bis 7-gliedriges Heterocyclyl steht, wobei Heterocyclyl gegebenenfalls gleich oder verschieden durch Reste ausgewählt aus der Gruppe (C₁-C₃)-Alkyl oder Oxo substituiert sind,
A für eine Bindung oder eine Gruppe der Formel NR⁶, SO₂-NR⁸ oder NR⁹-CO, steht,
worin
R⁶, R⁸ und R⁹ Wasserstoff oder gegebenenfalls ungesättigtes (C₁-C₃)-Alkyl, das gegebenenfalls bis zu zweifach gleich oder verschieden durch Hydroxy oder Methoxy substituiert ist, bedeuten, oder
worin
R⁶ und R⁹ Wasserstoff bedeuten,
R² für Wasserstoff steht, und
- D: für eine Gruppe der Formel (CH₂)ₘ-CR¹⁶R¹⁷-(CH₂)ₙ steht,
worin
die Gesamtzahl der Kohlenstoffatome 3 bis 6 beträgt,
m und n gleich oder verschieden sind und eine natürliche Zahl aus der Reihe 0 bis 2 bedeuten,
und
R¹⁶ und R¹⁷ gleich oder verschieden sind und Wasserstoff oder (C₁-C₃)-Alkyl, bedeuten,
oder
CR¹⁶R¹⁷ (C₃-C₆)-Cycloalkan-1',1-diyl bedeutet,
und deren Salze, Hydrate und/oder Solvate.

Eine weitere Ausführungsform der Erfindung betrifft Verbindungen der allgemeinen Formel (I),
in welcher
der Rest R¹-A- an der Position 3 des Thiochromenon-Rings sitzt,
R¹, A und R² die oben angegebene Bedeutung haben, und
- D: für 2-Methyl-propan-1,2-diyl steht.

Eine weitere Ausführungsform der Erfindung betrifft Verbindungen der allgemeinen Formel (I),
in welcher
der Rest R¹-A- an der Position 3 des Thiochromenon-Rings sitzt,
- A: für CO-NR⁷, SO₂-NR⁸ oder NR⁹-CO steht
worin
R⁷, R⁸und R⁹ die oben angegebene Bedeutung haben,
R² für Wasserstoff steht, und
R¹ und D die oben angegebene Bedeutung haben.

Die Erfindung betrifft weiterhin Verfahren zur Herstellung der Verbindungen der Formel (I).

### Bei Verfahren

### [A] setzt man Verbindungen der allgemeinen Formel (II)

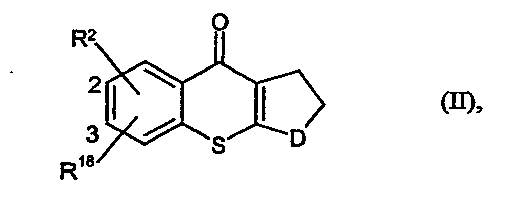

in welcher
- R¹⁸: an einer der Positionen 2 oder 3 des Thiochromenon-Rings sitzt,
- R² und D: die oben angegebene Bedeutung aufweisen, und
- R¹⁸: für Brom oder Chlor steht,
mit Verbindungen der allgemeinen Formel (III)

R¹⁹-BR²⁰R²¹ (III),

in welcher
- R¹⁹: für (C₆-C₁₀)-Aryl oder 5- bis 10-gliedriges Heteroaryl steht, wobei Aryl und Heteroaryl gegebenenfalls gleich oder verschieden durch Reste ausgewählt aus der Gruppe Halogen, Formyl, Carbamoyl, Cyano, Hydroxy, Trifluoromethoxy, Nitro, -NR³R⁴, (C₁-C₆)-Alkoxy carbonyl sowie gegebenenfalls durch Hydroxy, Morpholinyl, (C₁-C₆)-Acyloxy oder Halogen substituiertes (C₁-C₆)-Alkyl, (C₁-C₆)-Alkoxy, (C₁-C₆)-Acyl und (C₁-C₆)-Alkylthio substituiert sind,
worin
- R³ und R⁴: unabhängig voneinander Wasserstoff, (C₁-C₆)-Alkyl oder (C₁-C₆)-Acyl bedeuten, und
- R²⁰ und R²¹: für Hydroxy steht,
oder
- BR²⁰R²¹: für 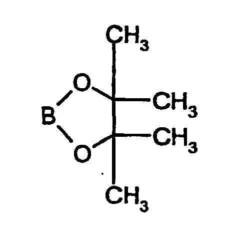 steht,
zu Verbindungen der allgemeinen Formel (Ia) 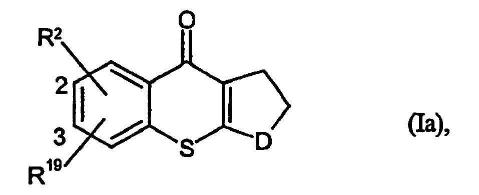 in welcher
- R¹⁹: an einer der Positionen 2 oder 3 des Thiochromenon-Rings sitzt, und
- R², R¹⁹ und D: die oben angegebene Bedeutung aufweisen, in inerten Lösungsmitteln unter üblichen Reaktionsbedingungen in Gegenwart eines Katalysators um (siehe beispielsweise: J. Tsuji, Palladium Reagents and Catalysts, J. Wiley & Sons, 1995; Suzuki-Kupplung, Übersicht: N. Miyaura, A. Suzuki, *Chem. Rev.* **1995,** *95*, 2457-2483; H. Gröger, *J. Prakt. Chem.* **2000**, *342*, 334-339).

Verfahren [A] wird bevorzugt unter Suzuki-Reaktionsbedingungen mit dafür üblichen Palladium-Katalysatoren durchgeführt, besonders bevorzugt sind Katalysatoren wie z.B. Dichlorbis(triphenylphosphin)palladium, Tetrakistriphenylphosphinpalladium(0), Palladium(II)acetat oder Bis-(diphenylphosphanferrocenyl)-palladium-(II)-chlorid.

Suzuki-Reaktionen werden mit üblichen Zusatzreagenzien wie Kaliumacetat, Cäsium-, Kalium- oder Natriumcarbonat, Bariumhydroxid, Kalium-tert.-butylat, Cäsiumfluorid oder Kaliumphosphat durchgeführt, besonders bevorzugt sind Zusatzreagenzien wie z.B. Kaliumacetat und/oder wässrige Natriumcarbonatlösung.

Suzuki-Reaktionen werden in inerten Lösungsmitteln durchgeführt, die sich unter den Reaktionsbedingungen nicht verändern, hierzu gehören Ether wie Dioxan, Tetrahydrofuran oder 1,2-Dimethoxyethan, Kohlenwasserstoffe wie Benzol, Xylol oder Toluol, oder andere Lösemittel wie Nitrobenzol, Dimethylformamid, Dimethylacetamid, Dimethylsulfoxid oder N-Methylpyrrolidon, besonders bevorzugt sind Lösungsmittel wie z.B. Dimethylformamid, Dimethylacetamid, Dimethylsulfoxid oder 1,2-Dimethoxyethan.

Verfahren [A] wird bevorzugt in einem Temperaturbereich von Raumtemperatur bis 130°C bei Normaldruck durchgeführt.

Die Verbindungen (III) sind kommerziell erhältlich oder können nach bekannten Methoden hergestellt werden oder können für die Reaktion *in situ,* wie unten beschrieben, hergestellt werden.

Die Biaryl-Synthesen, die über *in situ* hergestellte Boronate erfolgen, werden unter üblichen Reaktionsbedingungen in Gegenwart eines Katalysators, bevorzugt in Gegenwart eines Übergangsmetallkatalysators, insbesondere in Gegenwart eines Palladiumkatalysators (siehe beispielsweise A. Giroux, Y. Han, P. Prasit, *Tetrahedr. Lett.* **1997**, *38*, 3841-44; T. Ishiyama, M. Murata, N. Miyaura, *J*. *Org. Chem.* **1995**, *60*, 7508-10.), bevorzugt in Dimethylformamid oder Dimethylsulfoxid als Lösungsmittel, hergestellt. Als Übergangsmetallkatalysatoren finden bevorzugt Palladium(0)- oder Palladium (II)-Verbindungen, insbesondere Bis-(diphenylphosphanferrocenyl)-palladium-(II)-chlorid, Verwendung. Die Reaktion erfolgt insbesondere bei einer Temperatur von 70°C bis 110°C in Gegenwart von Basen, bevorzugt Kaliumacetat und/oder wässrige Natriumcarbonatlösung.

Neben Suzuki-Reaktionen finden auch Arylkupplungsreaktion mit Organozinn-(Stille-Kupplung) oder substituierten Olefinen (Heck-Reaktion) unter den dafür üblichen Reaktionsbedingungen in Gegenwart eines Katalysators, bevorzugt in Gegenwart eines Übergangsmetallkatalysators, insbesondere in Gegenwart eines Palladiumkatalysators (siehe beispielsweise J. Tsuji, Palladium Reagents and Catalysts, J. Wiley & Sons, 1995) bevorzugt in Dimethylformamid, N-Methylpyrrolidon, 1,2-Dimethoxyethan oder Toluol als Lösungsmittel bei einer Temperatur von 60-140°C Verwendung. Als Übergangsmetallkatalysatoren finden bevorzugt Palladium(0)- oder Palladium (II)-Verbindungen, insbesondere Bis(triphenylphosphan)palladium(II)chlorid, Palladium(II)acetat oder Tetrakis-(triphenylphosphan)-paUadium(0), Verwendung. Bei der Verwendung von Zinn-organischen Verbindungen (Stille-Kupplung, Übersicht: V. Farina, V. Krishnamurthy, W.J. Scott in: The Stille Reaction, J. Wiley and Sons, New York; 1998) erfolgt die Reaktion insbesondere bei einer Temperatur von 110°C bis 130°C. Bei der Verwendung von Olefinen (Heck-Reaktion, Übersicht: I. P. Beletskaya, A. V. Cheprakov: The Heck Reaction as a Sharpening Stone of Palladium Catalysis, *Chem. Rev.* **2000,** *100*, 3009-3066.) erfolgt die Reaktion insbesondere bei einer Temperatur von 80-100°C in Gegenwart einer Base, bevorzugt Triethylamin oder wässriger Natriumhydrogencarbonat-Lösung, sowie in Gegenwart eines Tetraalkylammonium- oder phosphoniumsalzes, bevorzugt Tetrabutylammoniumchlorid oder -bromid.

Die Verbindungen der allgemeinen Formel (II) lassen sich beispielsweise in Analogie zum Verfahren [M] aus den entsprechenden Edukten herstellen.

Die Verbindungen der allgemeinen Formel (III) sind bekannt oder lassen sich nach bekannten Verfahren herstellen.

### Bei Verfahren

### [B] setzt man Verbindungen der allgemeinen Formel (II) mit Verbindungen der allgemeinen Formel (IV)

R²²-H (IV),

in welcher
- R²²: für einen über N gebundenen 4- bis 12-gliedriges Heterocyclyl steht, wobei Heterocyclyl mindestens ein Stickstoffatom mit einer freien Valenz enthält und gegebenenfalls gleich oder verschieden durch Reste ausgewählt aus der Gruppe (C₁-C₆)-Alkyl, (C₁-C₆)-Alkoxy, (C₁₋C₆)-Acyl, (C₁-C₆)-Alkoxycarbonyl oder Oxo substituiert ist,
zu Verbindungen der allgemeinen Formel (Ib) 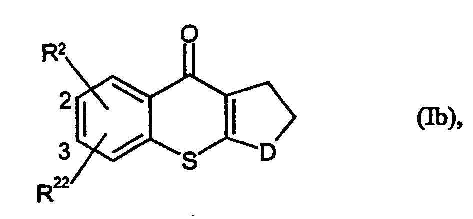 in welcher
- R²²: an einer der Positionen 2 oder 3 des Thiochromenon-Rings sitzt, und
- R¹, R²² und D: die oben angegebene Bedeutung aufweisen,
in inerten Lösungsmitteln unter den für solche Reaktionen üblichen Bedingungen um.

### Bei Verfahren

### [C] setzt man Verbindungen der allgemeinen Formel (II) mit Verbindungen der allgemeinen Formel (V)

R¹-G-H (V),

in welcher
- R¹: die oben angegebene Bedeutung aufweist, und
- G: für O, S oder NR⁶ steht, worin R⁶ die oben angegebene Bedeutung aufweist,
zu Verbindungen der allgemeinen Formel (Ic) 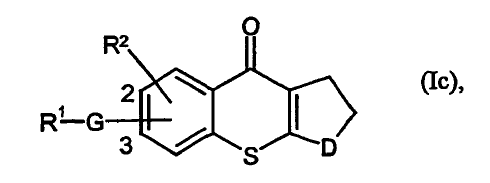 in welcher
- R¹-G-: an einer der Positionen 2 oder 3 des Thiochromenon-Rings sitzt, und
- R¹, R², D und G: die oben angegebene Bedeutung aufweisen,
in inerten Lösungsmitteln unter üblichen Reaktionsbedingungen in Gegenwart eines Katalysators um.

Die Verfahren [B] und [C] werden bevorzugt unter Palladium- (S.L. Buchwald, et al., *J. Am. Chem. Soc.* **1999**, *121*, 4369-4378; J.F. Hartwig, et al., *J. Org. Chem.* **1999**, *64*, 5575-5580; Buchwald Aminierungen: J.P. Wolfe, S.L. Buchwald, *J.* Org. *Chem.* **2000**, *65*, 1144-1157; J.P. Wolfe, H. Tomori, J.P. Sadighi, J. Yin, S.L. Buchwald, J. *Org. Chem.* **2000**, *65,* 1158-1174) oder Cu(I)-Katalyse (J. Lindley, *Tetrahedron* **1984**, *40*, 1433) durchgeführt, besonders bevorzugt sind Katalysatoren wie z.B. Tris(dibenzylidenaceton)di-palladium(O), Palladiumacetat oder Kupfer(I)iodid.

Die Verfahren [B] und [C] werden gegebenenfalls unter Zugabe von Basen durchgeführt, wie beispielsweise Alkalicarbonate wie Cäsiumcarbonat, Natrium- oder Kaliumcarbonat, oder Natrium- oder Kaliummethanolat, oder Natrium- oder Kaliumethanolat, Natrium- oder Kalium-tert.-butylat, oder andere Basen wie Kaliumphosphat, DBU, Pyridin, Triethylamin oder Diisopropylethylamin, besonders bevorzugt sind Basen wie z.B. Kaliumphosphat, Natrium-tert.-butylat, Kaliumcarbonat, Cäsiumcarbonat, Triethylamin, Diisopropylethylamin oder Pyridin.

Wird die Reaktion unter Pd-Katalyse durchgeführt, werden die Verfahren [B] und [C] gegebenenfalls unter Zugabe von Liganden durchgeführt, besonders bevorzugt sind Liganden wie z.B. 2-(Di-t-butylphosphino)biphenyl, Triphenylphosphin, [5-(Diphenylphosphino)-9,9-dimethyl-9H-xanthen-4-yl](diphenyl)phosphin, 1,1'-Biphenyl-2-yl(dicyclohexyl)phosphin oder (+/-)-2,2-Bis(diphenylphosphino)-1,1-binaphthyl.

Die Verfahren [B] und [C] werden in inerten Lösungsmitteln durchgeführt, die sich unter den Reaktionsbedingungen nicht verändern, hierzu gehören Ether wie Dioxan, Tetrahydrofuran oder 1,2-Dimethoxyethan, Kohlenwasserstoffe wie Benzol, Xylol oder Toluol, oder andere Lösemittel wie Dimethylformamid, Dimethylacetamid, Pyridin oder N-Methylpyrrolidon, besonders bevorzugt sind Lösungsmittel wie z.B. Toluol, Xylol, Dimethylformamid, Dimethylacetamid oder Pyridin.

Die Verfahren [B] und [C] werden bevorzugt in einem Temperaturbereich von Raumtemperatur bis zum Rückfluss der Lösungsmittel bei Normaldruck durchgeführt.

Die Verbindungen der allgemeinen Formeln (IV) und (V) sind bekannt oder lassen sich nach bekannten Verfahren herstellen.

### Bei Verfahren

### [D] setzt man Verbindungen der allgemeinen Formel (VI)

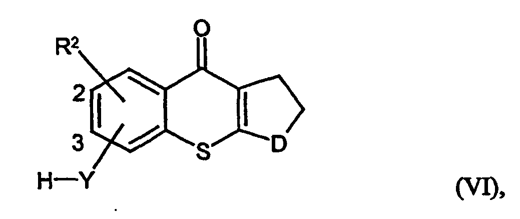

in welcher
H-Y- an einer der Positionen 2 oder 3 des Thiochromenon-Rings sitzt,
R² und D die oben angegebene Bedeutung aufweisen, und
- Y: für O, S oder NR⁶ steht, worin R⁶ die oben angegebene Bedeutung hat,
mit Verbindungen der allgemeinen Formel (VII)

R⁵-E-X¹ (VII),

in welcher
R⁵ und E die oben angegebene Bedeutung aufweisen, und
- X¹: für eine Abgangsgruppe, bevorzugt für Mesylat, Tosylat oder Halogen, besonders bevorzugt für Brom oder Jod, steht,
zu Verbindungen der allgemeinen Formel (Id) 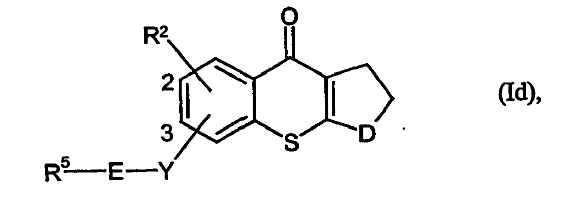 in welcher
R⁵-E-Y- an einer der Positionen 2 oder 3 des Thiochromenon-Rings sitzt, und
R², R⁵, D, E und Y die oben angegebene Bedeutung aufweisen,
in inerten Lösungsmitteln, die sich unter den Reaktionsbedingungen nicht verändern, hierzu gehören Halogenkohlenwasserstoffe wie Methylenchlorid, Trichlormethan oder 1,2-Dichlorethan, Ether wie Dioxan, Tetrahydrofuran oder 1,2-Dimethoxyethan, oder andere Lösemittel wie Aceton, Dimethylformamid, Dimethylacetamid, 2-Butanon oder Acetonitril, bevorzugt Tetrahydrofuran, Methylenchlorid, Aceton, 2-Butanon, Acetonitril, Dimethylformamid oder 1,2-Dimethoxyethan, in Gegenwart einer Base, wie beispielsweise Alkalicarbonate wie Cäsiumcarbonat, Natrium- oder Kaliumcarbonat, oder Natrium- oder Kaliummethanolat, oder Natrium- oder Kaliumethanolat oder Kalium-tert.-butylat, oder andere Basen wie Natriumhydrid, DBU, bevorzugt Kalium-tert.-butylat, Cäsiumcarbonat, DBU, Natriumhydrid, Kaliumcarbonat oder Natriumcarbonat, gegebenenfalls in Gegenwart von Kaliumiodid, bevorzugt in einem Temperaturbereich von Raumtemperatur bis zum Rückfluss der Lösungsmittel bei Normaldruck, um.

Die Verbindungen der allgemeinen Formel (VI) lassen sich beispielsweise in Analogie zum Verfahren [M] oder nach dem für die Herstellung der Verbindungen der allgemeinen Formel (VIII) beschriebenen Verfahren aus den entsprechenden Edukten herstellen.

Die Verbindungen der allgemeinen Formel (VII) sind bekannt oder lassen sich nach bekannten Verfahren herstellen.

### Bei Verfahren

### [E] setzt man Verbindungen der allgemeinen Formel (VIII)

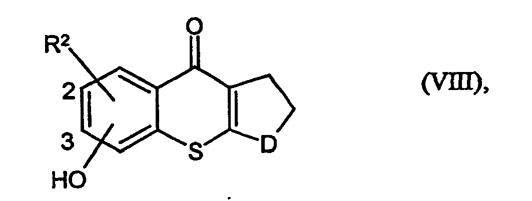

in welcher
Hydroxy an einer der Positionen 2 oder 3 des Thiochromenon-Rings sitzt, und
R² und D die oben angegebene Bedeutung aufweisen,
mit Verbindungen der allgemeinen Formel (IX)

R¹⁹-X² (IX),

in welcher
- R¹⁹: die oben angegebene Bedeutung hat,
- X²: für Halogen, bevorzugt Brom steht,
zu Verbindungen der allgemeinen Formel (Ie) 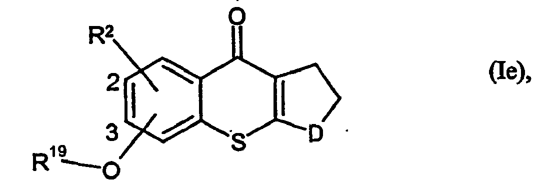 in welcher
R¹⁹-O- an einer der Positionen 2 oder 3 des Thiochromenon-Rings sitzt, und
R², R¹⁹ und D die oben angegebene Bedeutung aufweisen,
in inerten Lösungsmitteln, die sich unter den Reaktionsbedingungen nicht verändern, hierzu gehören Lösemittel wie Dimethylformamid, Dimethylacetamid, N-Methylpyrrolidon, Pyridin oder Hexamethylphosphorsäuretriamid, bevorzugt Dimethylformamid, Dimethylacetamid, Pyridin oder N-Methylpyrrolidon, in Gegenwart eines Katalysators, bevorzugt unter Kupferkatalyse, besonders bevorzugt sind Cu(I)- Katalysatoren wie z.B. Kupfer(I)iodid, gegebenenfalls in Gegenwart einer Base, wie beispielsweise Alkalicarbonate wie Cäsiumcarbonat, Natrium- oder Kaliumcarbonat, oder Natrium- oder Kaliummethanolat, oder Natrium- oder Kaliumethanolat oder Kalium-tert.butylat, oder andere Basen wie DBU, Pyridin oder Picolin, bevorzugt sind Kaliumcarbonat oder Pyridin, bevorzugt in einem Temperaturbereich von 80°C bis zum Rückfluss der Lösungsmittel bei Normaldruck, um.
Zur Herstellung der Verbindungen der allgemeinen Formel (VIII) setzt man Verbindungen der allgemeinen Formel (If) 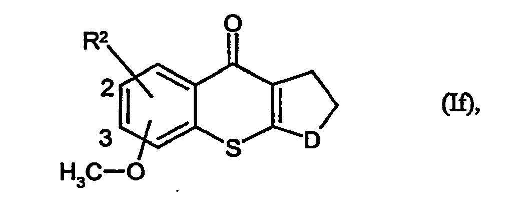 in welcher
Methoxy an einer der Positionen 2 oder 3 des Thiochromenon-Rings sitzt,
und
R² und D die oben angegebene Bedeutung aufweisen,
mit Bromwasserstoffsäure in Eisessig, bevorzugt in einem Temperaturbereich von 80°C bis zum Rückfluss der Lösungsmittel bei Normaldruck, um.

Zur Herstellung von Verbindungen der Formel (VIII) können auch die Verbindungen der allgemeinen Formel (1f) in einem inerten Lösungsmitttel, bevorzugt Dichlormethan mit Bortrichlorid oder Bortribromid, gegebenenfalls in Gegenwart eines Phasentransferkatalysators, bevorzugt quartäre Ammoniumsalze, besonders bevorzugt Tetrabutylammoniumbromid oder -jodid, bevorzugt in einem Temperaturbereich von minus 20°C bis zum Rückfluss der Lösungsmittel bei Normaldruck, um.

Die Verbindungen der allgemeinen Formel (If) lassen sich beispielsweise in Analogie zum Verfahren [M] aus den entsprechenden Edukten herstellen.

Die Verbindungen der allgemeinen Formel (IX) sind bekannt oder lassen sich nach bekannten Verfahren herstellen.

### Bei Verfahren

### [F] setzt man Verbindungen der allgemeinen Formel (IX)

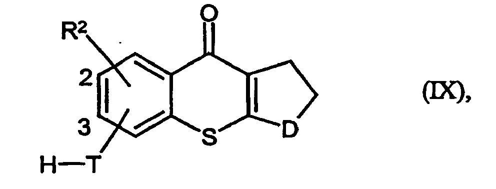

in welcher
H-T- an einer der Positionen 2 oder 3 des Thiochromenon-Rings sitzt
R² und D die oben angegebene Bedeutung aufweisen, und
- T: für O oder NR²³ steht, worin R²³ die für R⁶ angegebene Bedeutung hat,
mit Verbindungen der allgemeinen Formel (X) 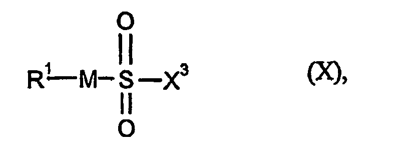 in welcher
- R¹: die oben angegebene Bedeutung aufweist,
- M: für eine Bindung oder NR²⁴ steht, worin R²⁴ die für R⁶ angegebene Bedeutung hat,
- X³: für Halogen, bevorzugt Brom oder Chlor, steht,
zu Verbindungen der allgemeinen Formel (Ig) 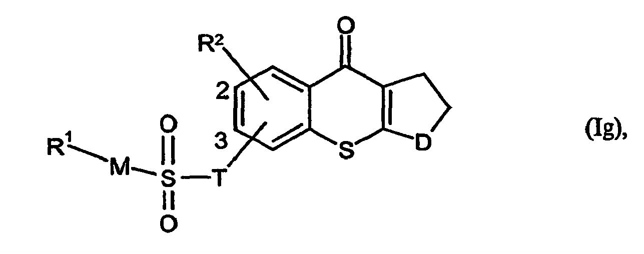 in welcher
R¹-M-SO₂-T- an einer der Positionen 2 oder 3 des Thiochromenon-Rings sitzt, und
R¹, R², D, M und T die oben angegebene Bedeutung aufweisen,
in inerten Lösungsmitteln oder Lösungsmittelgemischen mit Wasser, die sich unter den Reaktionsbedingungen nicht verändern, hierzu gehören Halogenkohlenwasserstoffe wie Methylenchlorid, Trichlormethan oder 1,2-Dichlorethan, Ether wie Diethylether, Dioxan, Tetrahydrofuran oder 1,2-Dimethoxyethan, Kohlenwasserstoffe wie Benzol, Xylol oder Toluol, oder andere Lösemittel wie Aceton, Dimethylformamid, 2-Butanon, Acetonitril oder Pyridin. Ebenso ist es möglich, Gemische der genannten Lösemittel, gegebenenfalls auch mit Wasser, einzusetzen. Gemische mit Wasser sind z. B. Methylenchlorid/Wasser oder Dioxan/Wasser, bevorzugt sind Methylenchlorid, Methylenchlorid/Wasser, Dioxan, Dioxan/Wasser oder Tetrahydrofuran, gegebenenfalls in Gegenwart eines Phasentransferkatalysators, bevorzugt quartäre Ammoniumsalze, besonders bevorzugt Tetrabutylammoniumchlorid oder Tetrabutylammoniumbromid, gegebenenfalls in Gegenwart einer Base, wie beispielsweise Alkalihydroxide wie Natrium- oder Kaliumhydroxid, oder Alkalicarbonate wie Cäsiumcarbonat, Natrium- oder Kaliumcarbonat, oder Natrium- oder Kaliummethanolat, oder Natrium- oder Kaliumethanolat oder Kalium-tert.butylat, oder andere Basen wie Natriumhydrid, DBU, Triethylamin, Diisopropylethylamin oder Pyridin, bevorzugt sind Pyridin oder Natriumhydroxid, bevorzugt in einem Temperaturbereich von Raumtemperatur bis zum Rückfluss der Lösungsmittel bei Normaldruck, um.

Die Verbindungen der allgemeinen Formel (IX) sind in Analogie zu den Verbindungen der allgemeinen Formel (VI) herstellbar.

Die Verbindungen der allgemeinen Formel (X) sind bekannt oder lassen sich nach bekannten Verfahren herstellen.

### In Verfahren

### [G] setzt man Verbindungen der allgemeinen Formel (XI)

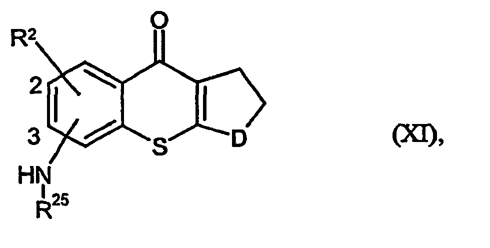

in welcher
R²⁵-NH- an einer der Positionen 2 oder 3 des Thiochromenon-Rings sitzt,
R² und D die oben angegebene Bedeutung aufweisen, und
R²⁵ die oben angegebene Bedeutung von R⁶ hat,
mit Verbindungen der allgemeinen Formel (XII)

R¹-L-X³ (XII),

in welcher
- R¹: die oben angegebene Bedeutung aufweist,
- X³: die oben angegebene Bedeutung hat,
- L: für CO, SO₂ oder NR¹²SO₂ oder NR¹⁴CO steht, worin R¹² und R¹⁴ die oben angegebene Bedeutung haben,
zu Verbindungen der allgemeinen Formel (Ih) 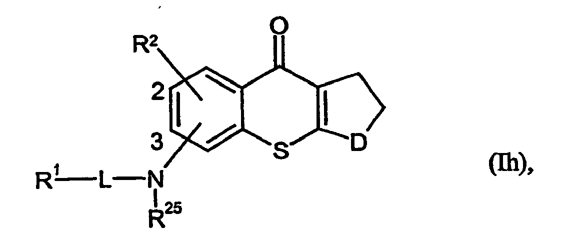 in welcher
R¹-L-NR²⁵ - an einer der Positionen 2 oder 3 des Thiochromenon-Rings sitzt,
und
R¹, R², R²⁵, D und L die oben angegebene Bedeutung aufweisen,
in inerten Lösungsmitteln, die sich unter den Reaktionsbedingungen nicht verändern, hierzu gehören Halogenkohlenwasserstoffe wie Methylenchlorid, Trichlormethan oder 1,2-Dichlorethan, Ether wie Diethylether, Dioxan, Tetrahydrofuran oder 1,2-Dimethoxyethan, oder andere Lösemittel wie Dimethylformamid, Dimethylacetamid, Acetonitril oder Pyridin, bevorzugt Tetrahydrofuran, Pyridin, Chloroform oder Methylenchlorid, in Gegenwart einer Base, wie beispielsweise Alkalicarbonate wie Cäsiumcarbonat, Natrium- oder Kaliumcarbonat, oder Kalium-tert.butylat, oder andere Basen wie Natriumhydrid, DBU, Triethylamin oder Düsopropylethylamin, bevorzugt Triethylamin, Diisopropylethylamin, Cäsiumcarbonat, Kaliumcarbonat, Natriumcarbonat, Pyridin und/oder DMAP, bevorzugt in einem Temperaturbereich von 0°C bis zum Rückfluss der Lösungsmittel bei Normaldruck, um.

Die Verbindungen der allgemeinen Formel (XI) sind in Analogie zu den Verbindungen der allgemeinen Formel (VI) herstellbar.

Die Verbindungen der allgemeinen Formel (XII) sind bekannt oder lassen sich nach bekannten Verfahren herstellen.

### In Verfahren

### [H] setzt man Verbindungen der allgemeinen Formel (Ii).

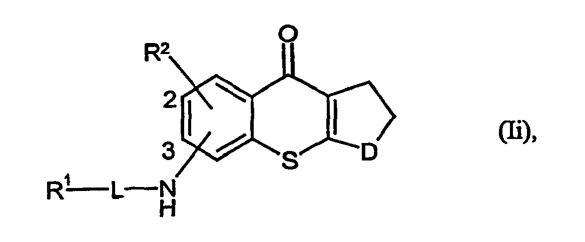

in welcher
R¹-L-NH- an einer der Positionen 2 oder 3 des Thiochromenon-Rings sitzt,
und worin
R¹, R², D und L die oben angegebene Bedeutung aufweisen,
mit Verbindungen der allgemeinen Formel (XIII)

R²⁶-X¹ (XIII),

in welcher
- R²⁶: für (C₃-C₈)-Cycloalkyl oder gegebenenfalls ungesättigtes (C₁-C₆)-Alkyl, das gegebenenfalls durch Hydroxy, Phenyl, (C₁-C₆)-Alkoxy, (C₁-C₆)-Alkoxycarbonyl oder (C₃-C₈)-Cycloalkyl substituiert ist, steht, wobei Phenyl seinerseits durch Halogen oder (C₁-C₄)-Alkyl substituiert sein kann,
- X¹: die oben angegebene Bedeutung hat,
zu Verbindungen der allgemeinen Formel (Ij) 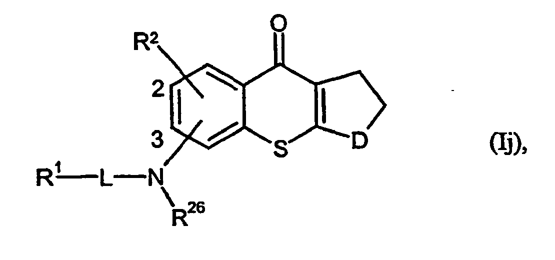 in welcher
R¹-L-NR²⁶- an einer der Positionen 2 oder 3 des Thiochromenon-Rings sitzt, und
R¹, R², R²⁶, D und L die oben angegebene Bedeutung aufweisen,
in inerten Lösungsmitteln, die sich unter den Reaktionsbedingungen nicht verändern, hierzu gehören Halogenkohlenwasserstoffe wie Methylenchlorid, Trichlonnethan, Trichlorethan oder 1,2-Dichlorethan, Ether wie Diethylether, Dioxan, Tetrahydrofuran oder 1,2-Dimethoxyethan, oder andere Lösemittel wie Aceton, Dimethylformamid, Dimethylacetamid, N-Methylpyrrolidon, 2-Butanon oder Acetonitril, bevorzugt Tetrahydrofuran, Dimethylformamid, 2-Butanon oder Aceton, in Gegenwart einer Base, wie beispielsweise Alkalicarbonate wie Cäsiumcarbonat, Natrium- oder Kaliumcarbonat, oder Natrium- oder Kaliummethanolat, oder Natrium- oder Kaliumethanolat oder Kalium-tert.butylat, oder andere Basen wie Natriumhydrid, DBU, Triethylamin oder Diisopropylethylamin, bevorzugt Natriumhydrid, DBU, Kalium- tert.-butylat oder Kaliumcarbonat, mit gegebenenfalls einem Phasentransferkatalysator, bevorzugt Tetrabutylammoniumhydrogensulfat, bevorzugt in einem Temperaturbereich von Raumtemperatur bis 120°C bei Normaldruck, um.

Die Verbindungen der allgemeinen Formel (Ij) lassen sich in Analogie zum Verfahren [G] aus den entsprechenden Edukten herstellen.

Die Verbindungen der allgemeinen Formel (XIII) sind bekannt oder lassen sich nach bekannten Verfahren herstellen.

### Bei Verfahren

### [I] setzt man Verbindungen der allgemeinen Formel (XIV)

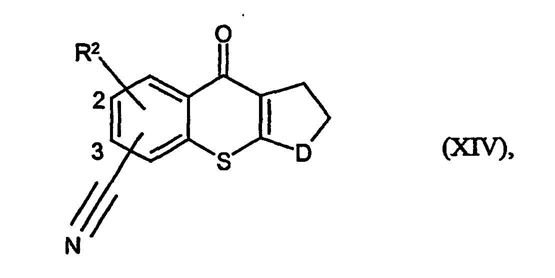

in welcher
Cyano an einer der Positionen 2 oder 3 des Thiochromenon-Rings sitzt, und
R² und D die oben angegebene Bedeutung aufweisen,
mit einem Azid, vorzugsweise Natriumazid, zu Verbindungen der allgemeinen Formel (Ik) 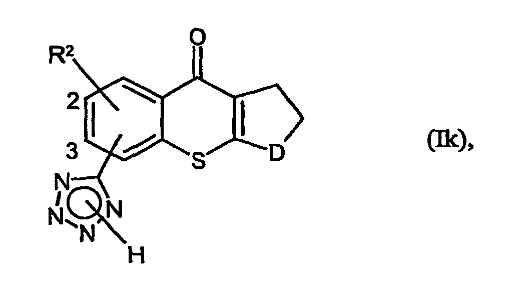 in welcher
der Tetrazol-Rest an einer der Positionen 2 oder 3 des Thiochromenon-Rings sitzt, und
R² und D die oben angegebene Bedeutung aufweisen,
in inerten Lösungsmitteln, bevorzugt Toluol oder Xylol, in Gegenwart einer Säure, bevorzugt Triethylammoniumhydrochlorid, bevorzugt in einem Temperaturbereich von 80°C bis zum Rückfluss der Lösungsmittel bei Normaldruck, um.

Gegebenenfalls setzt man Verbindungen der Formel (Ik) in einem zweiten Schritt mit Verbindungen der allgemeinen Formel (XV)

R²⁷-X³ (XV),

in welcher
- R²⁷: für (C₁-C₆)-Alkyl oder (C₁-C₆)-Acyloxymethyl steht, und
- X³: die oben angegebene Bedeutung hat,
zu Verbindungen der allgemeinen Formel (II) 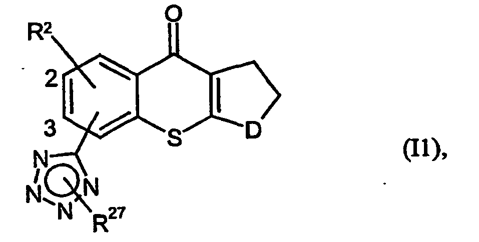 in welcher
der Heterocyclus an einer der Positionen 2 oder 3 des Thiochromenon-Rings sitzt, und
R³, R²⁷ und D die oben angegebene Bedeutung aufweisen,
in inerten Lösungsmitteln, die sich unter den Reaktionsbedingungen nicht verändern, hierzu gehören Halogenkohlenwasserstoffe wie Methylenchlorid, Trichlormethan, Trichlorethan oder 1,2-Dichlorethan, Ether wie Diethylether, Dioxan, Tetrahydrofuran oder 1,2-Dimethoxyethan, oder andere Lösemittel wie Aceton, Dimethylformamid, Dimethylacetamid, 2-Butanon oder Acetonitril, bevorzugt Tetrahydrofuran, Methylenchlorid, Aceton, 2-Butanon oder Dimethylformamid, in Gegenwart einer Base, wie beispielsweise Alkalicarbonate wie Cäsiumcarbonat, Natrium- oder Kaliumcarbonat, oder Natrium- oder Kaliummethanolat, oder Natrium- oder Kaliumethanolat oder Kalium-tert.butylat, oder andere Basen wie Natriumhydrid, DBU, Triethylamin oder Diisopropylethylamin, bevorzugt Natriumhydrid oder DBU, bevorzugt in einem Temperaturbereich von 0°C bis zum Rückfluss der Lösungsmittel bei Normaldruck, um.

Verfahren [I] findet auch für entsprechende Substituentenvariationen Verwendung.

Zur Herstellung der Verbindungen der allgemeinen Formel (XIV) setzt man beispielsweise Verbindungen der allgemeinen Formel (II) mit Zn(II)cyanid in Gegenwart eines Katalysators in einem geeigneten Lösungsmittel, hierzu gehören Ether wie Dioxan, 1,2-Dimethoxyethan oder Diethylenglykoldimethylether, oder andere Lösemittel wie Dimethylformamid, Dimethylacetamid, N-Methylpyrrolidon oder Acetonitril, besonders bevorzugt sind Dimethylformamid oder Dimethylacetamid, bevorzugt in einem Temperaturbereich von 60°C bis 160°C bei Normaldruck, um.

Die Reaktion wird bevorzugt unter Palladiumkatalyse (D.M. Tschaen, et al., Synth. Commun. 1994, 24, 887; F. Jin, N.P. Confalone, Tetrahedron Lett. 2000, 41, 3271) durchgeführt, besonders bevorzugt sind Katalysatoren wie z.B. Tetrakis(triphenylphosphin)palladium(O) oder Tris(dibenzylidenaceton)dipalladium in Gegenwart von 1,1'-[Bis(diphenylphosphino)ferrocen] und Zink.

Die Verbindungen der allgemeinen Formel (XV) sind bekannt oder lassen sich nach bekannten Verfahren herstellen.

### Bei Verfahren

### [J] setzt man Verbindungen der allgemeinen Formel (XIV) im ersten Schritt mit Hydroxylamin und anschließend mit Chlorameisensäureestern, bevorzugt Chlorameisensäure-2-ethylhexylester in zwei weiteren Stufen zu Verbindungen der allgemeinen Formel (Im)

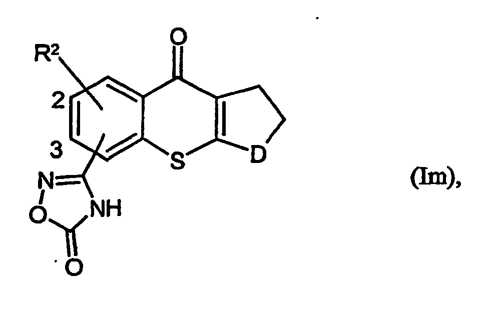

in welcher
der Heterocyclus an einer der Positionen 2 oder 3 des Thiochromenon-Rings sitzt, und
R² und D die oben angegebene Bedeutung aufweisen,
um.

Der erste Reaktionsschritt wird in inerten Lösungsmitteln, die sich unter den Reaktionsbedingungen nicht verändern, hierzu gehören Ether wie Diethylether, Dioxan, Tetrahydrofuran, 1,2-Dimethoxyethan oder Diethylenglykoldimethylether, Kohlenwasserstoffe wie Benzol, Xylol oder Toluol, oder andere Lösemittel wie Dimethylformamid, Dimethylacetamid, Dimethylsulfoxid, Pyridin oder Hexamethylphosphorsäuretriamid, bevorzugt Dimethylsulfoxid, bevorzugt in einem Temperaturbereich von Raumtemperatur bis 100°C bei Normaldruck, durchgeführt.

Der zweite Reaktionsschritt wird in inerten Lösungsmitteln, die sich unter den Reaktionsbedingungen nicht verändern, hierzu gehören Halogenkohlenwasserstoffe wie Methylenchlorid, Trichlormethan, 1,2-Dichlorethan oder Trichlorethylen, Ether wie Diethylether, Dioxan, Tetrahydrofuran, Glykoldimethylether oder Diethylenglykoldimethylether, oder andere Lösemittel wie Dimethylformamid, Dimethylacetamid, 1,2-Dimethoxyethan, Pyridin oder N-Methylpyrrolidin, bevorzugt Dimethylformamid, gegebenenfalls in Gegenwart einer Base, wie beispielsweise Alkalicarbonate wie Cäsiumcarbonat, Natrium- oder Kaliumcarbonat, oder andere Basen wie Natriumhydrid, DBU, Triethylamin, Diisopropylethylamin oder Pyridin, bevorzugt Pyridin, bevorzugt in einem Temperaturbereich von 0°C bis Raumtemperatur bei Normaldruck, durchgeführt.

Der dritte Reaktionsschritt wird in inerten Lösungsmitteln, bevorzugt Xylol, bevorzugt in einem Temperaturbereich von 100°C bis zum Rückfluss der Lösungsmittel bei Normaldruck, durchgeführt.

Verfahren [J] findet auch für entsprechende Substituentenvariationen Verwendung.

### Bei Verfahren

### [K] setzt man Verbindungen der allgemeinen Formel (XIV) zunächst mit einer geeigneten Säure zu Verbindungen der allgemeinen Formel (XVI)

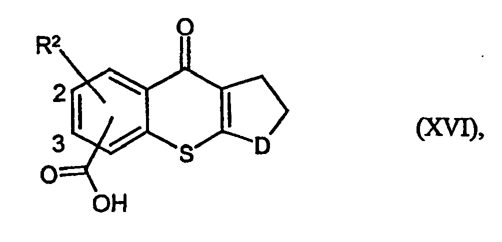

in welcher
Carboxyl an einer der Positionen 2 oder 3 des Thiochromenon-Rings sitzt, und
R² und D die oben angegebene Bedeutung aufweisen,
bevorzugt in einem Temperaturbereich von Raumtemperatur bis zum Rückfluss der Lösungsmittel bei Normaldruck, um.

Als Säuren eignen sich im allgemeinen Trifluoressigsäure, Schwefelsäure, Chlorwasserstoff, Bromwasserstoff und Essigsäure oder deren Gemisch gegebenenfalls unter Zusatz von Wasser. Besonders bevorzugt wird Schwefelsäure eingesetzt.

Anschließend setzt man die Verbindungen der allgemeinen Formel (XVI) mit Verbindungen der allgemeinen Formel (XVII) 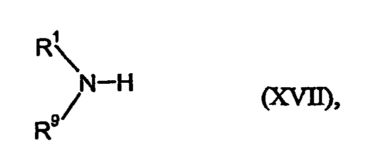 in welcher
R¹ und R⁹ die oben angegebene Bedeutung aufweisen,
zu Verbindungen der allgemeinen Formel (In) 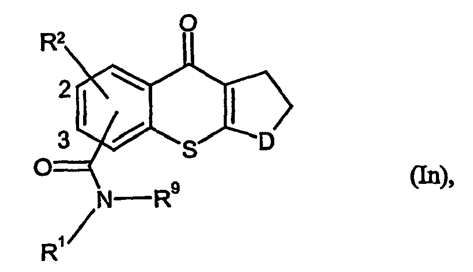 in welcher
R¹R⁹N-CO- an einer der Positionen 2 oder 3 des Thiochromenon-Rings sitzt, und
R¹, R², R⁹ und D die oben angegebene Bedeutung aufweisen,
um.

Bei der Umsetzung mit Verbindungen der allgemeinen Formel (XVM) sind Dichlormethan, Tetrahydrofuran oder Dimethylformamid bevorzugte Lösungsmittel. Die Reaktion erfolgt insbesondere bei Raumtemperatur. Als Hilfsstoffe für diese Reaktion werden bevorzugt fibliche Kondensationsmittel eingesetzt, wie Carbodiimide z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodümid-N'-propyloxymethyl-Polystyrol (PS-Carbodiimid) oder Carbonylverbindungen wie Carbonyldiimidazol, oder 1,2-Oxazoliwnverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri-(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluorophosphat (BOP). Als Basen werden Alkalicarbonate z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin eingesetzt. Besonders bevorzugt ist die Kombination von N-Cyclohexylcarbodiimid-N'-propyloxymethyl-Polystyrol (PS-Carbodiimid) und 1-Hydroxybenztriazol (HOBt) sowie die Kombination von Benzotriazol-1-yloxytris(dimethylamino)phosphonium-hexafluorophosphat (BOP) und Triethylamin.

Die Verbindungen der allgemeinen Formel (XVII) sind bekannt oder lassen sich nach bekannten Verfahren herstellen.

### Bei Verfahren

### [L] setzt man Verbindungen der allgemeinen Formel (XI) mit Verbindungen der allgemeinen Formel (XVIII)

R¹-N=C=O (XVIII),

in welcher
- R¹: die oben angegebene Bedeutung aufweist,
zu Verbindungen der allgemeinen Formel (Io) 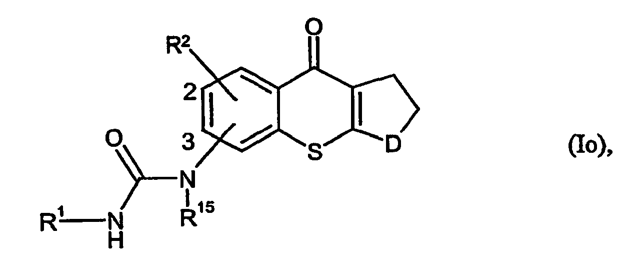 in welcher
R¹-NH-CO-NR¹⁵- an einer der Positionen 2 oder 3 des Thiochromenon-Rings sitzt, und
R¹, R², R¹⁵ und D die oben angegebene Bedeutung aufweisen,
in inerten Lösungsmitteln, die sich unter den Reaktionsbedingungen nicht verändern, hierzu gehören Halogenkohlenwasserstoffe wie Methylenchlorid, Trichlormethan oder 1,2-Dichlorethan, Ether wie Diethylether, Dioxan, Tetrahydrofuran oder Glykoldimethylether, Kohlenwasserstoffe wie Benzol, Xylol oder Toluol, oder andere Lösemittel wie Aceton, Dimethylformamid, Dimethylacetamid, 2-Butanon, Acetonitril oder Pyridin, bevorzugt Tetrahydrofuran, Pyridin oder Methylenchlorid, gegebenenfalls in Gegenwart einer Base, wie Alkalicarbonate wie Cäsiumcarbonat, Natrium- oder Kaliumcarbonat, oder Kalium-tert.butylat, oder andere Basen wie Natriumhydrid, DBU, Triethylamin, Pyridin oder Diisopropylethylamin, bevorzugt Triethylamin, Pyridin oder Diisopropylethylamin, bevorzugt in einem Temperaturbereich von Raumtemperatur bis zum Rückfluss der Lösungsmittel bei Normaldruck, um.

Die Verbindungen der allgemeinen Formel (XVIII) sind bekannt oder lassen sich nach bekannten Verfahren herstellen.

### Bei Verfahren

### [M] setzt man Verbindungen der allgemeinen Formel (XIX)

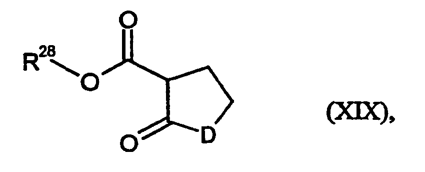

in welcher
- R²⁸: für (C₁-C₆)-Alkyl oder Benzyl, bevorzugt Ethyl oder Methyl, steht,
und
- D: die oben angegebene Bedeutung hat,
mit Verbindungen der allgemeinen Formel (XX) 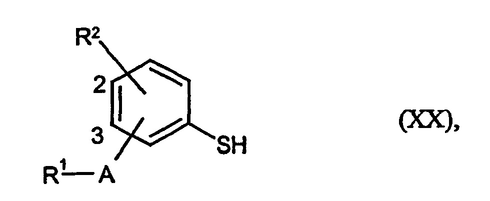 in welcher
R¹, R² und A die oben angegeben Bedeutung aufweisen,
in Polyphosphorsäure zu Verbindungen der allgemeinen Formel (I) 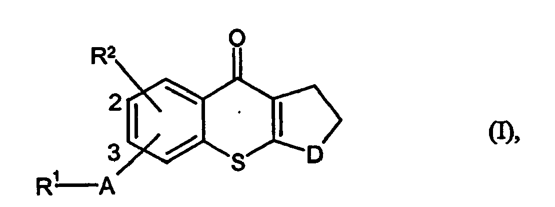 in welcher
R¹-A- an einer der Positionen 2 oder 3 des Thiochromenon-Rings sitzt, und
R¹, R², A und D die oben angegebene Bedeutung aufweisen,
bevorzugt in einem Temperaturbereich von 70°C bis 110°C bei Normaldruck, um.

Es werden bevorzugt solche Verbindungen der Formel (XX) eingesetzt, die unter den verwendeten Reaktionsbedingungen stabil sind.

Die cyclischen Ketoester (XIX) sind bekannt oder lassen sich durch allgemein bekannte Verfahren herstellen, z.B. durch Claisenkondensation der cyclischen Ketone mit Carbonsäuredialkylestern (z.B. S. J. Rhoads et al., *Tetrahedron* **1963**, *19*, 1625), oder mit Oxalsäuredialkylestern gefolgt von Decarbonylierung (z.B. L. Re, H. Schinz, *Heiv. Chim. Acta* **1958**, *41*, 1695).

Die cyclischen Ketone sind bekannt oder lassen sich durch allgemein bekannte Verfahren herstellen, z.B. aus den entsprechenden Cyclo-2-alkenonen bzw. 1,3-Diketonen.

Die Verbindungen und (XX) sind bekannt oder lassen sich nach bekannten Methoden herstellen. An einem Schwefel substituierte Dimercaptobenzole lassen sich beispielsweise nach J. Campbell et al. *J. Org. Chem.* **1964**, *29*, 1830-1833; Rumpf et al., *Bull. Soc. Chim. Fr.* **1940**, *7*, 632 darstellen. 3-Sulfonyl-thiophenole lassen sich beispielsweise nach Melloni et al., J. *Chem. Soc., Perkin Trans.* **1972**, *1*, 218; Borwell et al. 1953, *75*, 6019 darstellen. 3-Mercaptobenzolsulfonsäuren lassen sich nach H. Kawai et al., *Chem. Pharm. Bull.* **1991**, *39*,1422-1425 darstellen.

### Bei Verfahren

### [N] setzt man Verbindungen der allgemeinen Formel (Ip)

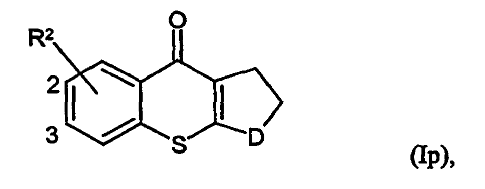

in welcher
R² und D die oben angegebene Bedeutung aufweisen,
mit Verbindungen der allgemeinen Formel (XXI) 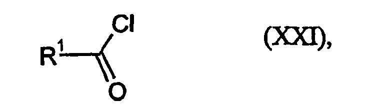 in welcher
- R¹: die oben angegebene Bedeutung hat,
zu Verbindungen der allgemeinen Formel (Iq) 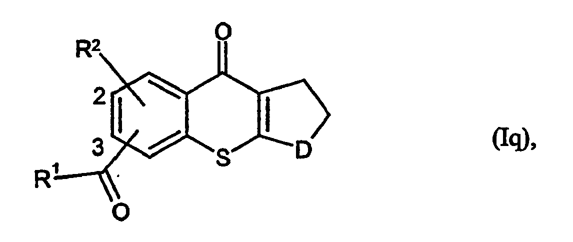 in welcher
R¹CO- an einer der Positionen 2 oder 3 des Thiochromenon-Rings sitzt, und
R¹, R² und D die oben angegebene Bedeutung aufweisen,
in inerten Lösungsmitteln, die sich unter den Reaktionsbedingungen nicht verändern, hierzu gehören Halogenkohlenwasserstoffe wie Tetrachlorethan, 1,2-Dichlorethan oder Chlorbenzol, oder andere Lösemittel wie Dimethylformamid, bevorzugt Dimethylformamid, bevorzugt in Gegenwart von Aluminiumtrichlorid, bevorzugt unter Friedel-Crafts-Acylierungs-Bedingungen analog den in der Literatur (O. Diouf et al., Eur. *J. Med. Chem.* **1995**, *30,* 715-719; S. Yous et al., *J. Org. Chem.* **1994**, *59,* 1574-1576) beschriebenen Bedingungen, um.

Die entsprechenden Methylketone [R¹ ist Methyl in (Ip)] können außerdem aus den Verbindungen der allgemeinen Formel (II) bevorzugt mittels Heck- oder Stille-Reaktion nach literaturbekannten Methoden (W. Cabri et al., *Tetrahedr. Lett.* **1991**, *32*, 1753-1756; M. Kosugi et al., *Bull. Chem. Soc. Jpn.* **1987***, 60,* 767-768.) erhalten werden. Die Methylketone können gegebenenfalls nach Bromierung der Methylgruppe unter dafür üblichen Standardbedingungen weiter derivatisiert werden.

Die Verbindungen der allgemeinen Formel (Ip) lassen sich beispielsweise in Analogie zum Verfahren [M] aus den entsprechenden Edukten herstellen.

Die Verbindungen der allgemeinen Formel (XXIII) sind bekannt oder lassen sich nach bekannten Verfahren herstellen.

### Bei Verfahren

### [O] setzt man Verbindungen der allgemeinen Formel (Ir)

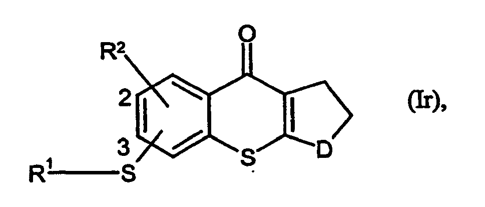

in welcher
R¹-S- an einer der Positionen 2 oder 3 des Thiochromenon-Rings sitzt, und
R¹, R² und D die oben angegebene Bedeutung aufweisen,
mit Oxidationsmitteln, wie z. B. m-Chlorperbenzoesäure, zu Verbindungen der allgemeinen Formel (Is) 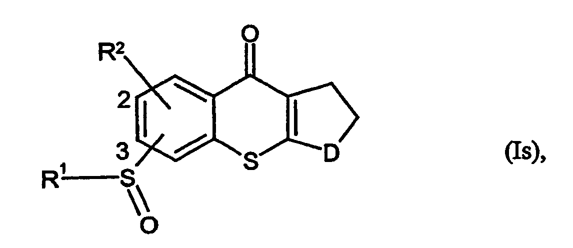 in welcher
R¹-SO- an einer der Positionen 2 oder 3 des Thiochromenon-Rings sitzt, und
R¹, R² und D die oben angegebene Bedeutung aufweisen,
beispielsweise nach den in der Literatur (P. Margaretha et al., *Helv. Chim. Acta* **1979**, *62*, 1978-1979; P. Bendazzoli et al., *Tetrahedr. Lett.* **1993**, *34*, 2975-2978.) beschriebenen Bedingungen, um.

Die Verbindungen der allgemeinen Formel (Ir) lassen sich beispielsweise in Analogie zum Verfahren [M] aus den entsprechenden Edukten herstellen.

### Bei Verfahren

### [P] setzt man Verbindungen der allgemeinen Formel (XXII)

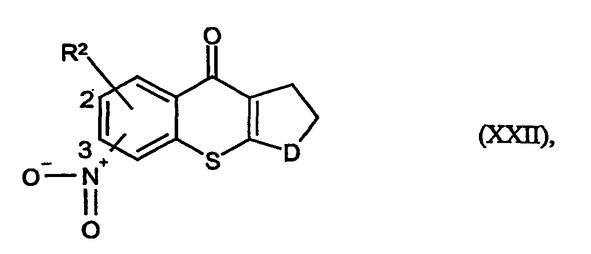

in welcher
Nitro an einer der Positionen 2 oder 3 des Thiochromenon-Rings sitzt, und
R² und D die oben angegebene Bedeutung aufweisen,
mit den entsprechenden Sulfinsäure-Salzen zu Verbindungen der allgemeinen Formel (It) 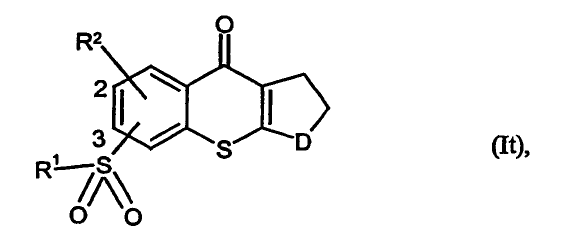 in welcher
R¹-SO₂- an einer der Positionen 2 oder 3 des Thiochromenon-Rings sitzt, und
R¹, R² und D die oben angegebene Bedeutung aufweisen,
bevorzugt nach den in der Literatur (W. Fischer et al., *Helv. Chim. Acta* **1985,** *68*, 854-59.) beschriebenen Bedingungen, um.

Die Verbindungen der allgemeinen Formel (XXII) lassen sich beispielsweise in Analogie zum Verfahren [M] aus den entsprechenden Edukten herstellen.

### Bei Verfahren

### [Q] überführt man Verbindungen der allgemeinen Formel (Iu)

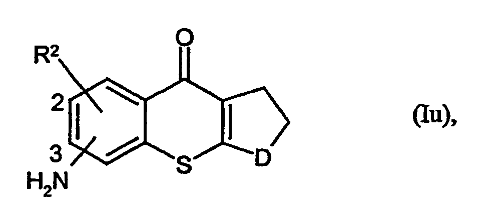

in welcher
Amino an einer der Positionen 2 oder 3 des Thiochromenon-Rings sitzt, und
R² und D die oben angegebene Bedeutung aufweisen,
nach Azotierung in die entsprechenden Sulfonsäuren und setzt diese anschließend mit Verbindungen der allgemeinen Formel (XXIII)

R¹-U-H (XXIII),

in welcher
- R¹: die oben angegebene Bedeutung hat, und
- U: für O oder NR¹⁰ steht, worin R¹⁰ die oben angegebene Bedeutung hat,
zu Verbindungen der allgemeinen Formel (Iv) 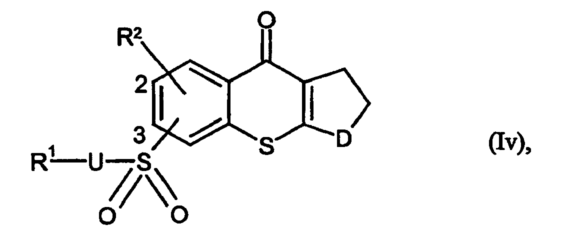 in welcher
R¹-U-SO₂- an einer der Positionen 2 oder 3 des Thiochromenon-Rings sitzt, und
R¹, R², D und U die oben angegebene Bedeutung aufweisen,
um.

Der erste Reaktionsschritt wird bevorzugt nach den in der Literatur (Meerwein et al., *Chem. Ber.* **1957,** *90,* 841-51; Polak et al., *Recl. Trav. Chim. Pays-Bas* **1910,** *29,* 423.) beschriebenen Bedingungen durchgeführt.

In der zweiten Stufe können nach den dem Fachmann bekannten Methoden durch Veresterung oder Aminierung die Sulfonsäuren, gegebenenfalls über ihre Chloride, in die entsprechenden Sulfonate oder Sulfonamide überführt werden.

Die Herstellung der Verbindungen der allgemeinen Formel (It) erfolgt wie für die Verbindungen der allgemeinen Formel (XI) beschrieben.

Die Verbindungen der allgemeinen Formel (XXIII) sind bekannt oder lassen sich nach bekannten Verfahren herstellen.

Amine in Seitenketten können auch durch reduktive Aminierung entsprechender Aldehyde mit entsprechenden Aminen dargestellt werden. Beispielsweise sei angeführt:

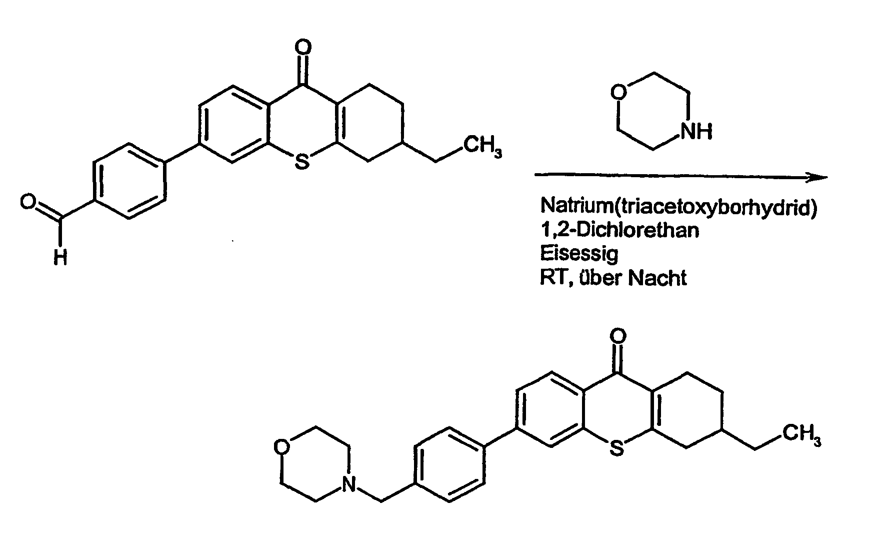

Die oben beschriebenen Verfahren können durch die folgenden Formelschemata beispielhaft erläutert werden:

Verfahren [A], [B] und [C]:

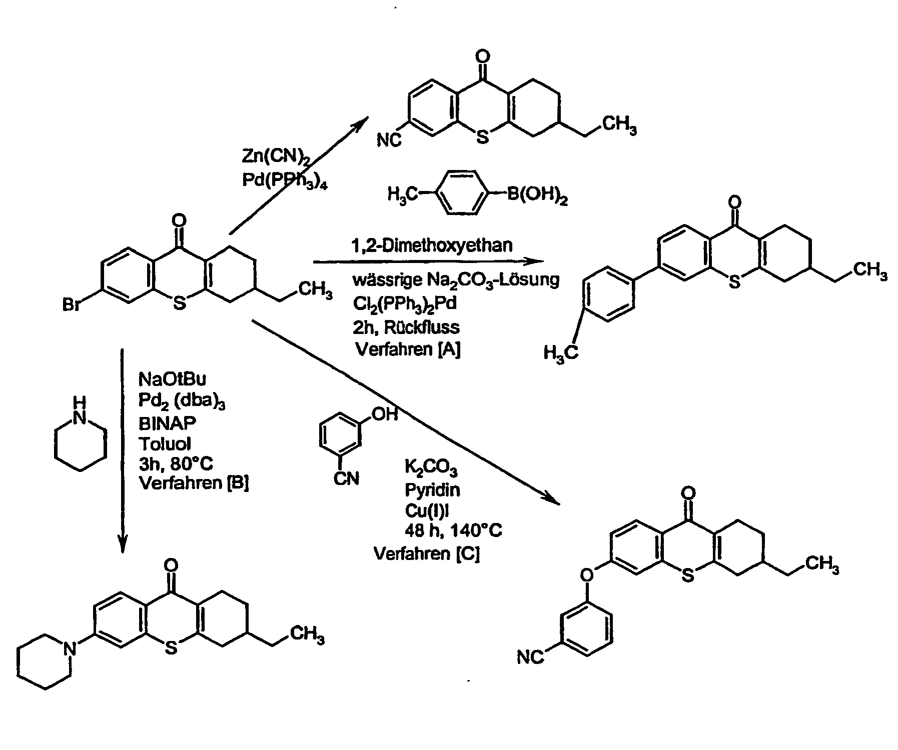

Verfahren [D]und [F]:

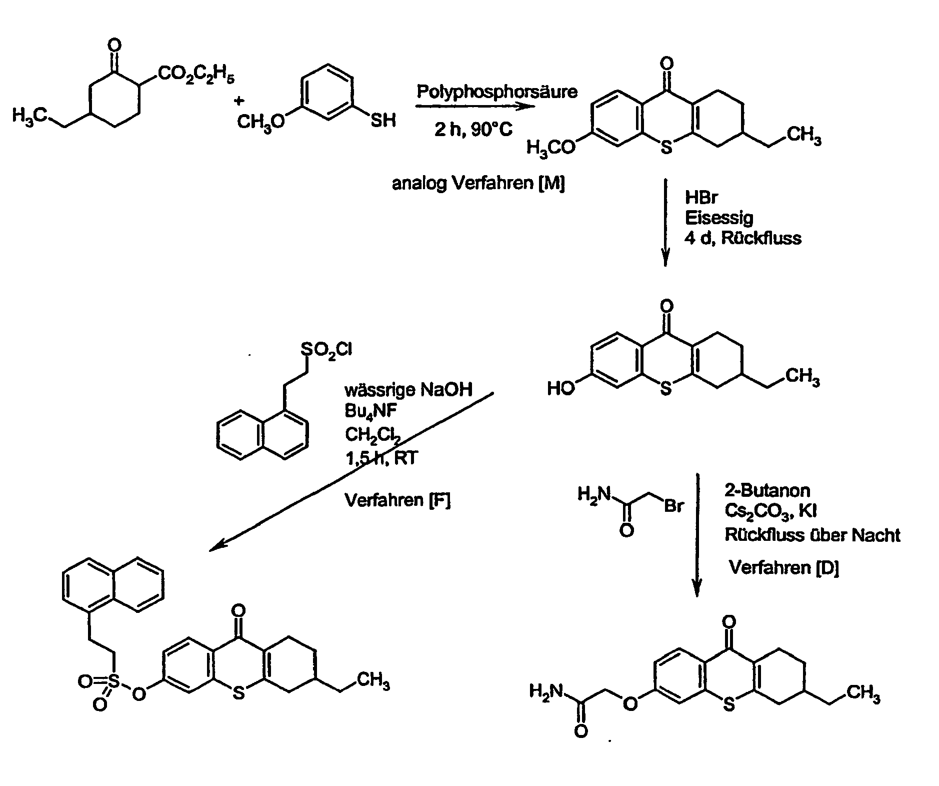

Verfahren [G] und [H]:

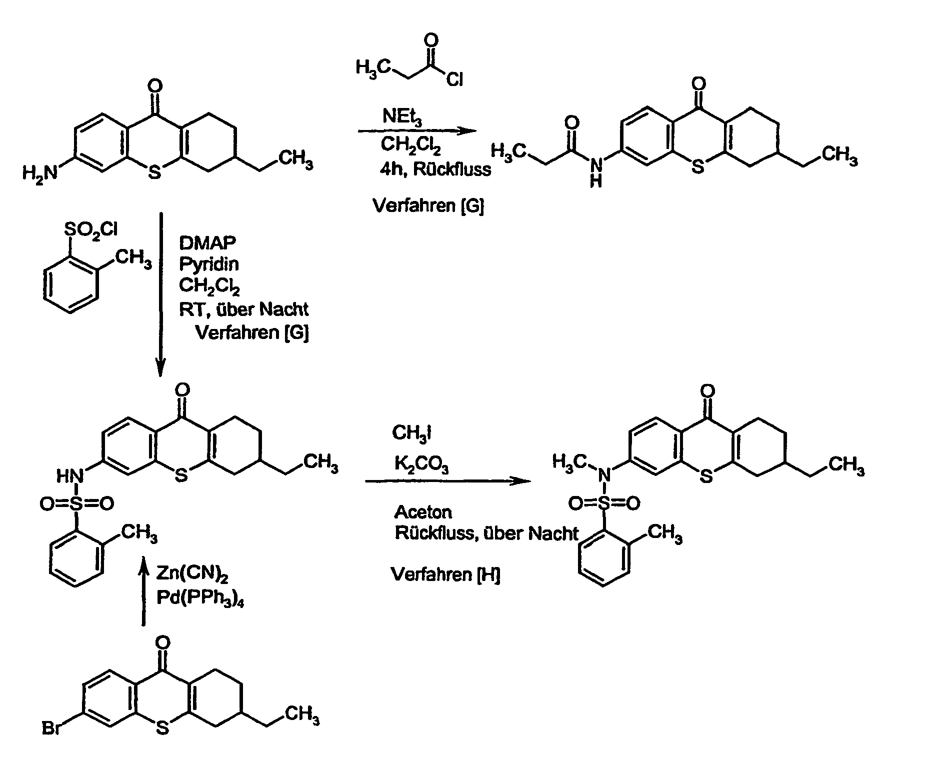

Verfahren [I]:

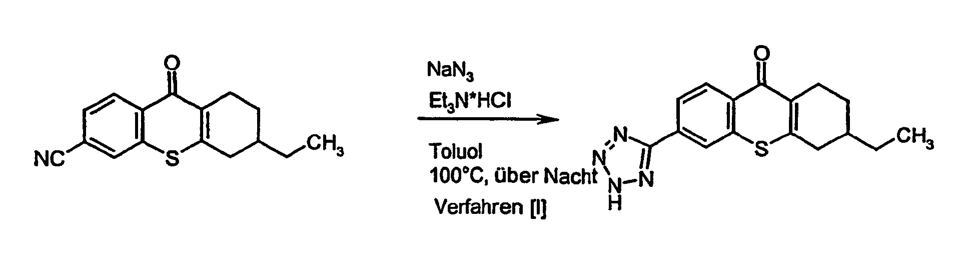

Verfahren [J] und [K]:

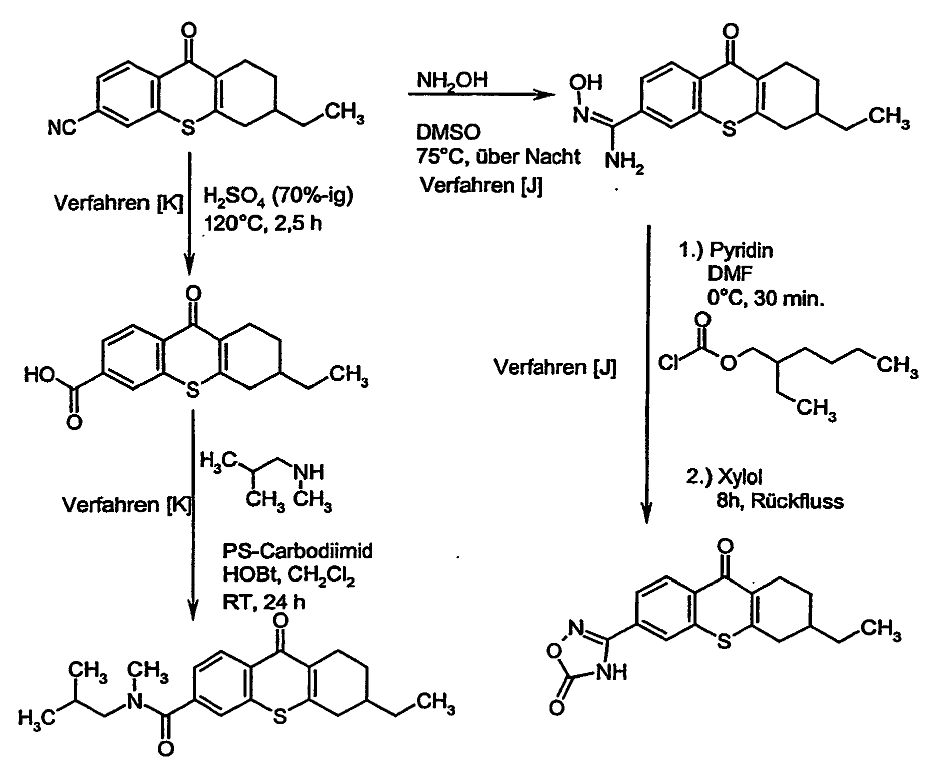

Die erfindungsgemäßen Verbindungen der allgemeinen Formel (I) eignen sich zur Verwendung als Medikamente in der Behandlung von Menschen und Tieren.

Die erfindungsgemäßen Verbindungen zeigen ein nicht vorhersehbares, wertvolles pharmakologisches Wirkspektrum.

Sie zeichnen sich als Antagonisten am mGluR1-Rezeptor aus.

Die erfindungsgemäßen Verbindungen können aufgrund ihrer pharmakologischen Eigenschaften allein oder in Kombination mit anderen Arzneimitteln zur Behandlung und/oder Prävention von neuronalen Schädigungen oder Erkrankungen, die mit einer Entgleisung der physiologischen oder pathophysiologischen Zustände des glutamatergen Systems im zentralen und peripheren Nervensystem in Verbindung gebracht werden, eingesetzt werden.

Zur Behandlung und/oder Prävention von neuronalen Schädigungen beispielsweise verursacht durch ischämischen, thromb- und/oder thrombembolischen, und hämorrhagischen Schlaganfall, Zuständen nach direkten und indirekten Verletzungen im Bereich des Gehirnes und des Schädels. Ferner zur Behandlung und/oder Prävention von cerebralen Ischämien nach sämtlichen operativen Eingriffen am Gehirn oder peripheren Organen bzw. Körperteilen und damit einhergehenden oder vorausgehenden Zuständen krankhafter bzw. allergischer Natur, die primär und/oder sekundär zu einer neuronalen Schädigung führen können.

Gleichfalls eignen sich die erfindungsgemäßen Verbindungen auch zur Therapie von primären und/oder sekundären krankhaften Zuständen des Gehirnes, beispielsweise während oder nach cerebralen Vasospasmen, Hypoxie und/oder Anoxie nicht vorher genannter Genese, perinataler Asphyxie, Autoimmunerkrankungen, Stoffwechsel- und Organerkrankungen, die mit einer Schädigung des Gehirnes einhergehen können sowie Schädigungen des Gehirnes infolge primärer Gehimerkrankungen beispielsweise Krampfleiden und athero- und/oder arterioslderetischer Veränderungen. Zur Behandlung chronischer oder psychiatrischer Leiden wie beispielsweise Depression, neurodegenerativer Erkrankungen wie beispielsweise Alzheimersche, Parkinsonsche oder Huntingtonsche Erkrankung, Multiple Sklerose, amyotrophische laterale Sklerose, Neurodegeneration durch akute und/oder chronische virale oder bakterielle Infektionen und Multiinfarktdemenz.

Darüberhinaus können sie als Arzneimittel eingesetzt werden zur Behandlung von Demenzen unterschiedlichen Ursprungs, Hirnleistungsstörungen im Alter, Gedächnisstörungen, Rückenmarksverletzungen, Schmerzzuständen, Angstzuständen unterschiedlichen Ursprungs, medikamentös bedingtem Parkinson-Syndrom, Psychosen (wie zum Beispiel Schizophrenie), Hirnödem, neuronalen Schädigungen nach Hypoglykämie, Emesis, Übelkeit, Obesitas, Suchterkrankungen und Entzugserscheiningen, ZNS-vermittelten Krämpfen, Sedation sowie Bewegungsstörungen.

Außerdem können die erfindungsgemäßen Verbindungen der allgemeinen Formel (I) verwendet werden zur Förderung der neuronalen Regeneration in der post-akuten Phase cerebraler Verletzungen oder chronischer Erkrankungen des Nervensystems.

Die erfindungsgemäßen Verbindungen können allein oder in Kombination mit anderen Arzneimitteln eingesetzt werden zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994) sowie neurodegenerativen Erkrankungen, insbesondere zur Behandlung von Krebs-induzierten Schmerzen und chronischen neuropathischen Schmerzen, wie zum Beispiel, bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Substanzen auch zur Therapie von primär akuten Schmerzen jeglicher Genese und von daraus resultierenden sekundären Schmerzzuständen, sowie zur Therapie chronifizierter, ehemals akuter Schmerzzustände.

Bevorzugt werden sie als Arzneimittel eingesetzt zur Behandlung und/oder Prophylaxe von Schmerzzuständen und neurodegenerativen Erkrankungen.

Die Modulation von Substanzen am metabotropen Glutamatrezeptor (direkte oder indirekte Beeinflussung der Kopplungseffizienz des Glutamat-Rezeptors an G-Proteinen) kann an primären Körnerzellkulturen aus dem Kleinhirn überprüft werden. Elektrophysiologische Messungen an diesen Zellkulturen im "cell attached"-Modus zeigen, dass L-Typ-Ca²⁺-Kanäle in dieser Präparation durch mGluR1-Glutamat-Rezeptoren aktiviert werden *(J. Neurosci.* **1995**, *15,* 135), wohingegen sie durch Gruppe II Rezeptoren blockiert werden *J. Neurosci.* **1994,** *14*, 7067-7076). Durch entsprechende experimentelle Anordnung kann die modulatorische Wirkung von pharmakologischen Prüfsubstanzen an Glutamatrezeptoren kontrolliert werden. Eine detaillierte Überprüfung der Subtypspezifität unter kontrollierten Bedingungen kann an *Xenopus*-Oocyten durch Injektion der entsprechenden mGluR-Subtyp-DNA erfolgen (WO 92/10583).

Die *in vitro*-Wirkung der erfindungsgemäßen Verbindungen an mGluR1-Rezeptoren kann mit folgenden biologischen Assays gezeigt werden:

### 1. Bestimmung der Aktivität am mGluR1-Rezeptor

mGluR1-Rezeptor-exprimierende CHO-Zellen werden in 96 Loch Platten (Greiner, Frickenhausen, Deutschland) ausgesät (20.000 Zellen/Vertiefung) und in UltraCHO Medium (Bio-Whittaker, Walkersville, Maryland, 10 % fetales Kälber Serum) für einen Tag kultiviert. Die zu prüfenden Substanzen werden initial in einer Konzentration von 10⁻²M in 100 % DMSO gelöst und anschließend auf die gewünschte Konzentration mit dem Kulturmedium verdünnt. Nach einmaligem Waschen mit Zellkulturmedium werden die Zellen mit 1mM Glutamat und gleichzeitig mit der zu prüfenden Substanz für 4h bei 37° C inkubiert. Anschließend wird das Medium abgesaugt und die Zellen mit 75µl Triton-Luciferase Puffer lysiert (1 % Triton X 100, 10 % Glycerin, 2 mM DTT, 25 mM Na2HPO4, 25 mM TRIS), (530 µM ATP, 470 µM Luciferin, 270 µM CoA, 20 mM Tricine, 2.67 mM MgSO4, 33.3 mM DTT, 0.1 mM EDTA, pH 7.8). Die Lumineszenz wird nach einer Minute mittels einer Kamera gemessen (Hamamatsu, Japan). Zellen, welche allein mit Glutamat inkubiert wurden, zeigen einen deutlichen Anstieg der Lumineszenz im Vergleich zu Kontrollen, Antagonisten am mGluR1-Rezeptor reduzieren diese konzentrationsabhängig bis unter die Ausgangs-Luminiszenz.

Die folgenden Beispiele zeigten im oben genannten Test die folgende Wirkung:

**Tabelle 1:**

| Beispiel | IC₅₀ [nM] |
|---|---|
| 1-3 | 9 |
| 1-10 | 97 |
| 1-27 | 23 |
| 1-29 | 21 |
| 1-31 | 31 |
| 1-34 | 81 |
| 3-21 | 22 |
| 3-22 | 13 |
| 3-23 | 28 |
| 3-49 | 35 |
| 3-55 | 35 |
| 4-08 | 25 |

Die Eignung der erfindungsgemäßen Verbindungen zur Behandlung von Schmerzzuständen, insbesondere neuropathischen Schmerzzuständen, kann in folgenden Tiermodellen gezeigt werden:

### 2. Axotomie von Ischiasverzweigungen bei der Ratte (Chronisches Schmerzmodell)

Unter Pentobarbital-Anästhesie wird die Trifurkation eines Ischias-Nerven freipräpariert, der Peroneus- sowie der Tibialiszweig werden axotomiert nachdem die Nerven proximal der Axotomiestelle ligiert wurden. Kontrolltiere erhalten eine Scheinoperation. Nach der Operation entwickeln die axotomierten Tiere eine chronische mechanische Allodynie und thermische Hyperalgesie.

Die mechanische Allodynie wird mit Hilfe eines Druckaufnehmers im Vergleich zu scheinoperierten Tieren getestet (elektronisches von Frey Anästhesiometer, IITC Inc.-Life Science Instruments, Woodland Hills, CA, USA).

Die thermische Hyperalgesie lässt sich durch Messung der Latenzzeit bestimmen, innerhalb derer eine Ratte eine Pfote aus dem Bereich einer Strahlungshitzequelle entzieht (Plantartest, Ugo Basile (Mailand)).

Die Substanzapplikation erfolgt zu unterschiedlichen Zeitpunkten vor der Schmerztestung über unterschiedlichen Applikationsrouten (i.v., i.p., p.o., i.t., i.c.v., transdermal).

### 3. Ligatur vom Ischias-Nerv bei der Ratte nach Bennett und Xie, 1988 (Chronisches Schmerz-Modell)

Bennett und Xie: A peripheral mononeuropathy in the rat that produces disorders of pain sensation like those seen in man. *Pain* **1988**, *33*, 87-107.

Unter Pentobarbital-Anästhesie wird der Ischias-Nerv unilateral frei-präpariert und ligiert (4 Ligaturen etwa 1 mm von einander entfernt, proximal zu der Trifurkation des Nerven). Kontrolltiere erhalten eine Scheinoperation. Nach der Operation entwickeln die ligierte Tiere eine chronische mechanische und thermische Hyperalgesie. Die Hyperalgesie wird mit Hilfe eines Druckaufnehmers (mechanische Hyperalgesie; elektronisches von Frey Anästhesiometer, IITC Inc.-Life Science Instruments, Woodland Hills, CA, USA) oder einer Infrarotquelle (thermische Hyperalgesie; Plantar Test, Hugo Basile Inc., Comerio, Italy) im Vergleich zu scheinoperierten Tieren getestet.

Die Substanzapplikation erfolgt zu unterschiedlichen Zeitpunkten vor der Schmerztestung über unterschiedlichen Applikationsrouten (i.v., i.p., p.o., i.t., i.c.v., transdermal).

Die Eignung der erfindungsgemäßen Verbindungen zur Behandlung von Schmerzzuständen, insbesondere Entzündungs-bedingten Schmerzzuständen, kann in folgendem Tiermodell gezeigt werden:

### 4. Modell für akuten inflammatorischen Schmerz (Carrageenin-Modell) in Ratten.

Dies Verfahren, das analgetische Wirkung in Ratten prüft, die unter inflammatorischem Schmerz leiden, folgt der Beschreibung von Winter et al. (*Proc*. *Soc*. *Exp*. *Biol*. *Med,* **1962**, *111*, 544-547.

Ratten wird subplantar in die rechte Hinterpfote eine Suspension von Carrageenin injiziert (0,75 mg je Pfote in 0,05 ml physiologischer Kochsalzlösung). Zwei Stunden später werden die Ratten nacheinander thermisch und taktil sowohl an der nicht-entzündeten wie auch an der entzündeten Hinterpfote stimuliert.

Der Apparat zur thermischen Stimulation (Ugo Basile, Ref.: 7371) besteht aus 6 individuellen, auf einer erhöhten Glasplatte plazierten Plexiglas-Boxen (17x11x13 cm). Eine Ratte wird wird für 10 min zur Habituation in die Box gesetzt. Dann wird eine bewegliche Infrarotquelle (Setting 20) unter der nicht-entzündeten und der entzündeten Hinterpfote fokussiert und die Latenzzeiten bis zum Fortziehen der Pfote automatisch aufgezeichnet. Das Wegziehen der Pfote unterbricht die reflektierte Strahlung und schaltet damit automatisch Zählwerk und Lichtquelle ab. Zur Vermeidung von Gewebeschäden wird der Test nach 45 s beendet, auch wenn keine Pfotenwegziehreaktion registriert wird.

Zur taktilen Stimulation wird das Tier in eine Plexiglasbox (17x11x13 cm), deren Boden aus einem Maschengitter besteht, gesetzt . Die Spitze eines elektronischen Von-Frey-Filaments (Bioseb, Modell 1610) wird mit steigendem Druck gegen die nicht-entzündete und die entzündete Hinterpfote gedrückt und diejenige Kraft automatisch aufgezeichnet, die benötigt wird, um ein Wegziehen der Pfote zu bewirken.

Die Testung wird dreimal durchgeführt und die mittlere Kraft je Pfote als Ergebnis für jedes Tier berechnet.

12 Ratten werden je Gruppe untersucht: Männliche Wistar (Han) Ratten, 180- 220 g. Der Test wird blind durchgeführt

Morphin (8 mg/kg i.p.) und Acetylsalicylsäure (256 mg/kg i.p.), unter gleichen experimentellen Bedingungen appliziert, dienen als Referenzsubstanzen.

Daten-Analyse erfolgt durch Vergleich der behandelten Gruppen mit den entsprechenden Kontrollen mittels des nicht-gepaarten Student's Test.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

- abs.: absolut
- Ac: Acetyl
- acac: Acetylacetonyl
- AIBN: α,α'-Azobis(isobutyronitril)
- Aloc: Allyloxycarbonyl
- aq.: wässrig
- 9-BBN: 9-Borabicyclo[3.3.1]nonan
- Bn: Benzyl
- Boc: tert.-Butoxycarbonyl
- Bom: Benzyloxymethyl
- BOP: Benzotriazol-1-yloxy-tris(dimethylamino)phosphonium-Hexafluorophosphat
- Bu: Butyl
- Bz: Benzoyl
- CAN: Cerammoniumnitrat
- Cbz: Benzyloxycarbonyl
- CDI: *N*,*N*'-Carbonyldiimidazol
- CH: Cyclohexan
- Cp: Cyclopentadienyl
- CSA: 10-Camphersulfonsäure
- Dabco: 1,4-Diazabicyclo[2.2.2]octan
- DAST: Diethylaminoschwefeltrifluorid
- DBN: 1,5-Diazabicyclo[4.3.0]non-5-en
- DBU: 1,8-Diazabicyclo[5.4.0]undec-7-en
- DC: Dünnschichtchromatographie
- DCC: *N,N'*-Dicyclohexylcarbodiimid
- DCE: 1,2-Dichlorethan
- DCI: direkte chemische Ionisation (bei MS)
- DCM: Dichlormethan
- DDQ: 2,3-Dichlor-5,6-dicyano-1,4-benzochinon
- DEAD: Azodicarbonsäurediethylester
- d.e.: Diastereomerenüberschuss
- dest.: destilliert
- DHP: 3,4-Dihydro-2*H*-pyran
- DIAD: Azodicarbonsäurediisopropylester
- DIBAH: Diisobutylaluminiumhydrid
- DIC: Diisopropylcarbodiimid
- DIEA: *N*,*N-*Diisopropylethylamin
- DMA: *N*,*N*-Dimethylacetamid
- DMAP: 4-*N*,*N*-Dimethylaminopyridin
- DME: 1,2-Dimethoxyethan
- DMF: *N*,*N-*Dimethylformamid
- DMPU: *N,N'*-Dimethylpropylenharnstoff
- DMSO: Dimethylsulfoxid
- DNPH: 2,4-Dinitrophenylhydrazin
- DPPA: Diphenylphosphorylazid
- EDC: *N*'-(3-Dimethylaminopropyl)-*N*-ethylcarbodiimid x HCl
- e.e.: Enantiomerenüberschuss
- EE: Ethylacetat (Essigsäureethylester)
- EI: Elektronenstoß-Ionisation (bei MS)
- eq: Äquivalent(e)
- ESI: Elektrospray-Ionisation (bei MS)
- Et: Ethyl
- fl.: flüssig
- Fmoc: Fluorenyhnethoxycarbonyl
- Fp.: Schmelzpunkt
- Fr.: Fraktion
- GC: Gaschromatographie
- ges.: gesättigt
- HATU: *O*-(7-Azabenzotnazol-1-yl)-*N*,*N*,*N*,*N*-tetramethyluronium-Hexafluorphosphat
- HBTU: *O*-(Benzotriazol-1-yl)-*N*,*N*,*N*,*N*'-tetramethyluronium-Hexafluorphosphat
- HMDS: 1,1,1,3,3,3-Hexamethyldisilazan
- HMPA o. HMPT: Hexamethylphosphorsäuretriamid
- HOBt: 1-Hydroxy-1H-benzotriazol x H₂O
- HOSu: *N*-Hydroxysuccinimid
- HPLC: Hochdruck-, Hochleistungsflüssigchromatographie
- Im: Imidazol-1-yl
- IR: Infrarotspektroskopie
- konz.: konzentriert
- Kp.: Siedepunkt
- krist.: kristallin / kristallisiert
- LAH: Lithiumaluminiumhydrid
- LC-MS: Flüssigchromatographie-gekoppelte Massenspektroskopie
- LDA: Lithium-*N,N*-diisopropylamid
- LiHMDS: Lithium-*N,N*-bistrimethylsilylamid
- Lit.: Literatur(stelle)
- Lsg.: Lösung
- m: meta
- mCPBA: meta-Chlorperbenzoesäure
- Me: Methyl
- MEK: Methylethylketon
- MEM: Methoxyethoxymethyl
- MG: Molekulargewicht
- MOM: Methoxymethyl
- MPLC: Mitteldruckflüssigchromatographie
- Ms: Methansulfonyl (Mesyl)
- MS: Massenspektroskopie
- MTBE: Methyl-tert.butylether
- NBS: *N*-Bromsuccinimid
- NCS: *N*-Chlorsuccinimid
- Nd.: Niederschlag
- NIS: *N*-Iodsuccinimid
- NMM: *N*-Methylmorpholin
- NMO: . *N*-Methylmorpholin-N-oxid
- NMR: Kernresonanzspektroskopie
- o: ortho
- p: para
- p.A.: pro analysi
- PCC: Pyridiniumchlorochromat
- PDC: Pyridiniumdichromat
- Pfp: Pentafluorphenyl
- Ph: Phenyl
- Piv: Pivaloyl
- PMB: p-Methoxybenzyl
- PNB: p-Nitrobenzyl
- PPA: Polyphosphorsäure
- PPTS: Pyridinium-p-toluolsulfonat
- Pr: Propyl
- PS: Polystyrol (-Harz)
- py: Pyridin
- PyBOP: Benzotriazol-1-yloxy-tris(pyrrolidino)phosphonium-Hexafluorophosphat
- RF: Rückfluss
- R_{f}: Retentionsindex (bei DC)
- RP: reverse phase (bei HPLC)
- RT: Raumtemperatur
- Rₜ: Retentionszeit (bei HPLC)
- SEM: 2-(Trimethylsilyl)ethoxymethyl
- subl.: sublimiert
- TBAF: Tetrabutylammoniumfluorid
- TBAI: Tetrabutylammoniumiodid
- TBDMS: tert.-Butyldimethylsilyl
- TBDPS: tert.-Butyldiphenylsilyl
- TBTU: *O*-(Benzotriazol-1-yl)-*N*,*N*,*N*,*N*'-tetramethyluronium-Tetrafluoroborat
- TEA: Triethylamin
- techn.: technisch
- Teoc: 2-(Trimethylsilyl)ethoxycarbonyl
- TES: Triethylsilyl
- Tf: Trifluormethansulfonyl
- TFA: Trifluoressigsäure
- TFAA: Trifluoracetanhydrid
- TfOH: Trifluormethansulfonsäure
- THF: Tetrahydrofuran
- THP: Tetrahydropyranyl
- TIPS: Triisopropylsilyl
- titr.: titriert
- TMEDA: *N, N, N; N*'-Tetramethylethylendiamin
- TMOF: Trimethylorthoformiat
- TMS: Trimethylsilyl
- TPP: Triphenylphosphin
- TPPO: Triphenylphosphinoxid
- Trt: Trityl
- Ts: p-Toluolsulfonyl (Tosyl)
- TsOH: p-Toluolsulfonsäure
- v/v: Volumen-zu-Volumen-Verhältnis (einer Lösung)
- verd.: verdünnt
- vgl.: vergleiche
- Vol.: Volumen
- w/w: Gewicht-zu-Gewicht-Verhältnis (einer Lösung)
- wässrig: wässrig
- Z: Benzyloxycarbonyl
- Zers.: Zersetzung

### Ausgangsverbindungen I

Die Bestimmung der Retentionszeit von Ausgangsverbindungen und Herstellungsbeispielen mit HPLC erfolgte unter folgenden Bedingungen.

### HPLC-Methode:

Säule: Chromasil C18 60*2; Injektionsvolumen 1.00 µl; Fluss: 0,75 ml/min; Eluent: A= 5 ml HClO₄/l H₂O, B= CH₃CN; Gradient [t(min): A/B]: 0,5: 98/2; 4,5: 10/90; 6,5: 10/90; 6,7: 98/2; 7,5: 98:2.

Präparative HPLC Methode: Reverse Phase, ACN/Wasser-Gradient; Säule: GROM-SIL 120 ODS-4 HE 10 µm, 250* 30 mm

### Beispiel I-1

### 6- Brom-3-ethyl-1 ,2,3 ,4-tetrahydro-9H-thioxanthen-9-on

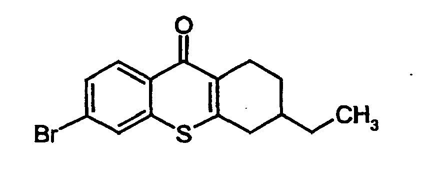

Die Reaktion wird in einer Apparatur mit mechanischem Rührer unter Argon durchgeführt, Abgase werden durch Chlorlauge abgeleitet.

110 g Polyphosphorsäure werden unter starkem Argonstrom vorgelegt, 15 min bei RT unter starkem Argonstrom verrührt, der Reaktionskolben anschließend für ca. 5 min mit einem Heißluftfön erhitzt, bis sich eine niedrigere Viskosität zeigt. Man lässt wieder abkühlen, gibt mit Einwegspritzen 10,0 g (51,3 mmol) 3-Bromthiophenol und 11,2 g (56,4 mmol) 4-Ethyl-2-oxocyclohexylcarbonsäureethylester zu und rührt einige Minuten bei Normaltemperatur nach. Die honigartige Reaktionsmischung verfärbt sich beige und wird auf etwa 90°C erwärmt, anschließend 2h bei dieser Temperatur nachgerührt.

Zur Aufarbeitung lässt man auf Normaltemperatur abkühlen, versetzt zunächst mit etwa 300 g Eis und 50 ml Wasser, nach 10 min zusätzlich mit 300 ml Dichormethan und verrührt 20 min bei Normaltemperatur. Nach Phasentrennung wird die wässrige Phase mit Dichlormethan extrahiert (3x 330 ml). Die organischen Phasen werden zweimal mit je 200 ml 1N Natronlauge und einmal mit 100 ml gesättigter Kochsalzlösung gewaschen, über Magnesiumsulfat getrocknet und am Rotationsverdampfer eingeengt.

Die so erhaltenen 17,8 g Rohprodukt werden aus jeweils etwa 150 ml Petroleumbenzin p.A. (60°-80°) zur Abtrennung des 8-Brom-Regioisomers mehrmals umkristallisiert.

Man erhält so 5,55 g (34 %) der Zielverbindung.
MS (EI+): 322 (M);
¹H-NMR (400 MHz, CDCl₃): δ = 0.99 (t, J= 7.5Hz; 3H); 1.33-1.46 (m, 3H); 1.66-1.76 (m, 1H); 2.01-2.07 (m, 1H); 2.37-2.55 (m, 2H); 2.68-2.73 (m, 1H); 2.88-2.93 (m, 1H); 7.55-7.57 (dd, J=8.5Hz, 2Hz; 1H); 7.66 (d, J=2Hz, 1H); 8.34 (d, J=8.5Hz; 1H).

In Analogie zur Vorschrift des Beispiels I-1 wurden herstellt:

### Beispiel I-2

### 3-Brom-6,7,8,9,10,11-hexahydro-12H-cycloocta[b]thiochromen-12-on

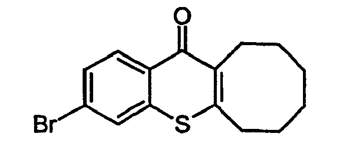

MS (EI+): 322 (M);
¹H-NMR (200 MHz, DMSO): δ = 1.29-1.63 (m, 6H); 1.70-1.83 (m, 2H); 2.82-2.90 (m, 4H); 7.71-7.76 (dd, J=8.5Hz, 2Hz, 1H); 8.17 (d, J=2Hz; 1H); 8.19-8.24 (d, J=8.5Hz; 1H).

### Beispiel I-3

### 6-Brom-3,3-dimethyl-1,2,3,4-tetrahydro-9H-thioxanthen-9-on

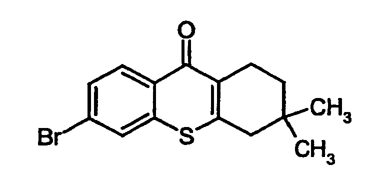

MS (EI+): 322 (M);
¹H-NMR (300 MHz, CDCl₃): δ = 1.03 (s, 6H); 1.62 (t, J=6.5Hz; 2H); 2.46 (s, 2H); 2.70 (m, 2H); 7.55-7.58 (dd, J=8.5, 2Hz; 1H); 7.67 (d, J=2Hz; 1H); 8.35 (d, J=8.5Hz; 1B).

### Beispiel I-4

### 6-Brom-3-spirobutyl-1,2,3,4-tetrahydro-9H-thioxanthen-9-on

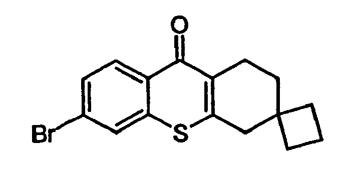

MS (EI+): 334 (M);
¹H-NMR (300 MHz, CDCl₃): δ = 1.82-1.96 (m, 8H); 2.67-2.73 (m, 4H); 7.53-7.59 (dd, J=8.5, 2Hz; 1H); 7.67 (d, J=2Hz; 1H); 8.33 (d, J=8.5Hz; 1H).

### Beispiel I-5

### 3-Ethyl-9-oxo-2,3,4,9-tetrahydro-1H-thioxanthen-6-carbonitril

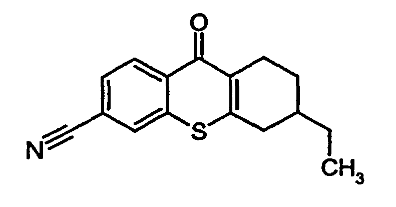

2,00 g (5,02 mmol) 6-Brom-3-ethyl-1,2,3,4-tetrahydro-9H-thioxanthen-9-on (Beispiel I-1), 0,62 g (5,27 mmol) Zinkcyanid und 0,58 g (0,50 mmol) Tetrakis(triphenylphosphin)palladium(0) werden unter Argon in 20 ml DMF gelöst und über Nacht auf 80°C erwärmt.

Zur Aufarbeitung wird nach Abkühlen auf Normaltemperatur mit 15 ml 25-%iger Ammoniaklösung und 25 ml Wasser versetzt, der Niederschlag abfiltriert und mit 75 ml Wasser gewaschen. Trocknen im Vakuum und Umkristallisation aus Cyclohexan/Ethylacetat (3:2) ergibt 1,28 g (95 %) der Zielverbindung.
MS (CI+): 287 (M+NH₄), 270 (M+H);
¹H-NMR (200 MHz, DMSO): δ = 0.94 (t, J=7Hz; 3H); 1.24-1.46 (m, 3H); 1.57-1.77 (m, 1H); 1.89-2.04 (m, 1H); 2.30-2.56 (m, 2H); 2.67-2.85 (m, 2H); 7.91-7.96 (dd, J=8.5Hz, 1.5Hz; 1H); 8.38-8.42 (d, J=8.5Hz; 1H); 8.49 (d, J=1Hz; 1H).

### Beispiel I-6

### 3-Ethyl-6-hydroxy-1,2,3,4-tetrahydro-9H-thioxanthen-9-on

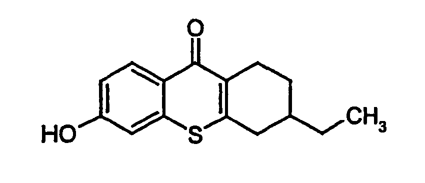

1,00 g (3,64 mmol) 3-Ethyl-6-methoxy-1,2,3,4-tetrahydro-9H-thioxanthen-9-on (Beispiel 1-1) werden in 20 ml Essigsäure vorgelegt, 8 ml 48-%ige Bromwasserstoffsäure zugegeben und das Gemisch über Nacht und drei weitere Tage bei Rückflusstemperatur gerührt, bis das Edukt vollständig umgesetzt ist.

Zur Aufarbeitung wird am Rotationsverdampfer weitgehend eingeengt, der verbleibende Rest auf Wasser gegeben und das Gemisch dreimal mit Ethylacetat extrahiert. Die vereinigten organischen Phasen werden getrocknet und eingeengt. Das so erhaltene Rohprodukt wird auf Kieselgel aufgezogen und über etwa 150 g Kieselgel beginnend mit Cyclohexan, dann mit einem Cyclohexan/Ethylacetat-Gradienten 20:1 bis 5:1 flashchromatographiert. Die Produktfraktionen werden aus Cyclohexan/Ethylacetat 5:1 umkristallisiert.

Man erhält so 655 mg (69 %) der Zielverbindung.
MS (CI+): 261 (M+H);
¹H-NMR (300 MHz, DMSO): δ = 0.93 (t, J=7.5Hz; 3H); 1.25-1.41 (m, 3H); 1.56-1.68 (m, 1H); 1.88-1.96 (m, 1H); 2.26-2.43 (m, 2H); 2.62-2.75 (m, 2H); 6.95 (m, 2H); 7.05-7.32 (m, b, 1H); 8.16-8.19 (m, 1H).

### Beispiel 1-7

### 3-Ethyl-N'-hydroxy-9-oxo-2,3,4,9-tetrahydro-1H-thioxanthen-6-carboximidamid

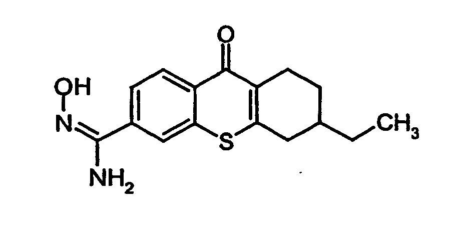

Unter Argon werden 129 mg (1,86 mmol) Hydroxylammoniumchlorid in 1 ml DMSO vorgelegt, 0,26 ml (1,86 mmol) Triethylamin zugegeben und das Gemisch etwa 5 min. nachgerührt. Man filtriert vom unlöslichen Feststoff ab, der mit Tetrahydrofuran nachgewaschen wird. Das Filtrat wird am Rotationsverdampfer vom THF befreit, zur verbleibenden DMSO-Lösung 100 mg (0,37 mmol) 3-Ethyl-9-oxo-2,3,4,9-tetrahydro-1H-thioxanthen-6-carbonitril (Beispiel I-5) in DMSO gelöst zugetropft und das Reaktionsgemisch bei 75°C über Nacht gerührt.

Zur Aufarbeitung wird die Reaktionsmischung mit 3 ml DMSO/2 ml Acetonitril verdünnt und ohne weitere Behandlung durch präparative HPLC gereinigt. Man erhält so 85,3 mg (76 %) der Zielverbindung.
MS (EI+): 302 (M);
¹H-NMR (200 MHz, DMSO): δ = 0.95 (t, J=7Hz; 3H); 1.24-1.46 (m, 3H); 1.56-1.76 (m, 1H); 1.88-2.03 (m, 1H); 2.28-2.43 (m, 2H); 2.71 (m, 1H); 2.80 (m, 1H); 6.04 (s, 2H); 7.84-7.89 (dd, J=8.5Hz, 1.5Hz, 1H); 7.99 (s, 1H); 8.26 (d, J=8.5Hz; 1H); 10.03 (s, 1H).

In Analogie zur Vorschrift des Beispiels I-7 wurden hergestellt:

### Beispiel I-8

### N'-Hydroxy-12-oxo-6,8,9,10,11,12-hexahydro-7H-cycIoocta[b]thiochromen-3-carboximidamid

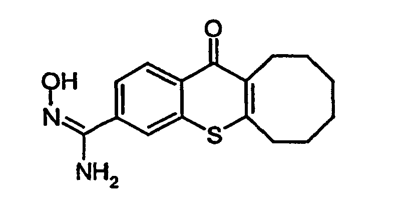

MS (EI+): 302 (M);
¹H-NMR (200 MHz, DMSO): δ = 1.32-1.50 (m, 4H); 1.54-1.63 (m, 2H); 1.73-1.82 (m, 2H); 2.85-2.91 (m, 4H); 6.04 (s, 2H); 7.86-7.89 (dd, J=8.5Hz, 1.5Hz, 1H); 8.02 (d, J=1.5Hz; 1H); 8.29 (d, J=8.5Hz; 1H); 10.32 (s, 1H).

### Beispiel I-9

### N'-Hydroxy-3,3-dimethyl-9-oxo-2,3,4,9-tetrahydro-1H-thioxanthen-6-carboximidamid

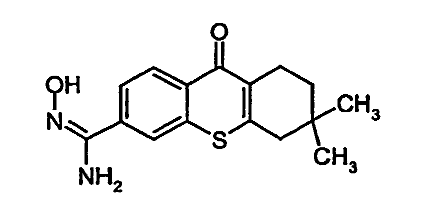

MS (CI+): 303 (M+H);
¹H-NMR (300 MHz, DMSO): δ = 0.99 (s, 6H); 1.58 (t, J=6.5Hz; 2H); 2.53-2.59 (m, 4H); 6.03 (s, 2H); 7.85-7.89 (dd, J=8.5Hz, 1.5Hz, 1H); 8.00 (d, J=1.5Hz; 1H); 8.28 (d, J=8.5Hz; 1H); 10.03 (s, 1H).

### Beispiel I-10

### 6-Chlor-3-ethyl-7-fluor-6-1,2,3,4-tetrahydro-9H-thioxanthen-9-on

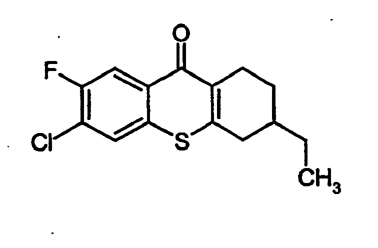

Unter Argon werden 60 g Polyphosphorsäure 10 min auf 110°C erhitzt. Nach Abkühlen werden 5,00 g (29,8 mmol) 3-Chlor-4-fluorphenylthiol und 6,50 g (32,8 mmol) 4-Ethyl-2-oxo-cyclohexancarbonsäureethylester zugesetzt und es wird 3,5 h bei 90°C Ölbadtemperatur gerührt. Das Reaktionsgemisch wird mit 200 g Eis und 200 ml Dichlormethan versetzt und nach Auftauen werden die Phasen getrennt. Die wässrige Phase wird noch zweimal mit Dichlormethan extrahiert und die vereinigten organischen Phasen werden zweimal mit Natronlauge (1 N) und einmal mit gesättigter wässriger Natriumchlorid-Lösung gewaschen. Nach Trocknen über Natriumsulfat und Filtration wird das Lösungsmittel unter schwachem Vakuum destillativ entfernt. Der Hauptteil des reinen Produktes wird durch Verrühren des erhaltenen Feststoffes in Cyclohexan erhalten. Chromatographie des Filtrates an Kieselgel (0,04-0,063 nm) mit Cyclohexan/Essigsäureethylester 40:1/30:1/20:1/10:1 als Laufmittel liefert ein Gemisch, das anschließend durch präparative HPLC gereinigt wird.
Ausbeute: 3,114 g (35,2 % der Theorie)
R_{f} (Cyclohexan/Essigsäureethylester 5:1)= 0,65
MS (EI): 297 (M + H)
HPLC, Retentionszeit= 5,69 min (LC-MS)
¹H-NMR (200 MHz, CDCl₃): δ = 0,99 (t, 3 H), 1,32-1,51 (m, 3 H), 1,59-1,82 (m, 1 H), 1,97-2,13 (m, 1 H), 2,32-2,60 (m, 2 H), 2,62-2,80 (m, 1 H), 2,81-2,99 (m, 1 H), 7,58 (d, 1 H), 8,24 (d, 1 H).

### Beispiel I-11

### 3-Ethyl-9-oxo-2,3,4,9-tetrahydro-1H-thioxanthen-6-carbonsäure

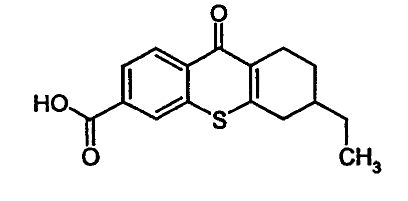

0,281 g (1,05 mmol) 3-Ethyl-9-oxo-2,3,4,9-tetrahydro-1H-thioxanthen-6-carbonitril (Beispiel I-5) wird 2,5 h bei 120°C in 5,5 ml Schwefelsäure (70 % in Wasser) gerührt. Nach Abkühlen wird die Lösung in 2 ml Eiswasser gegeben, der resultierende Niederschlag zunächst abfiltriert und dann in Natriumhydrogencarbonat (10 %-ige wässrige Lösung) aufgenommen. Nach Ansäuern mit Schwefelsäure (70 % in Wasser) fällt die Carbonsäure als weißer Feststoff aus.
Ausbeute: 262,8 mg (87,2 % der Theorie)
R_{f} (CHCl₃ : MeOH : AcOH: H₂O = 120:30:3:3)= 0,85
MS (EI): 288 (M)
HPLC, Retentionszeit= 4,64 min
¹H-NMR (200 MHz, CDCl₃): δ = 0,82 (t, 3 H), 1,14-1,35 (m, 3 H), 1,43-1,65 (m, 1 H), 1,78-1,95 (m, 1 H), 2,18-2,44 (m, 2 H), 2,50-2,81 (m, 2 H), 7,87 (dd, 1H), 8,05 (d, 1 H), 8,32 (d, 1 H), 12,63 (br. s, 1H).

### Beispiel I-12

### (4,4-Diethyl-2-oxocyclohexyl)-carbonsäureethylester

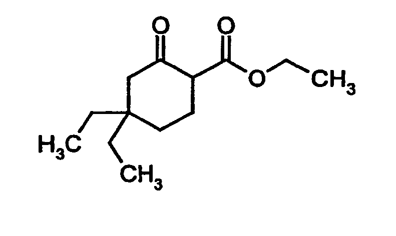

Zu 2.1 g (12.5 mmol) 5,5-Diethylcyclohexan-1,3-dion (Kon, G..A. R., Linstaed, R. P., J. Chem. Soc., (1925), 127, 819) werden unter Argon in Chloroform 634 mg (4.62 mmol) Phosphortrichlorid zugetropft. Das Gemisch wird für 3h zum Rückfluss erhitzt. Man engt ein, nimmt den Rückstand in eiskaltem Wasser auf, extrahiert dreimal mit Ether, trocknet die vereinigten organischen Phasen und engt erneut im Vakuum ein.

Das erhaltene Rohprodukt wird in 50 ml wasserfreiem Tetrahydrofuran gelöst, mit 1 g Palladium auf Kohle (5 % Palladium) versetzt und bei Normaldruck über Nacht hydriert. Man arbeitet durch Filtration und Einengen auf, und setzt wegen unvollständigem Umsatz erneut zur Hydrierung in 50 ml wasserfreiem Tetrahydrofuran mit 1.6 g Pd/C (5 % Pd) bei Normaldruck über Nacht an.

Dieser Vorgang wird noch ein weiteres mal wiederholt.

Man erhält so 1.90 g (98 %) 3,3-Diethylcyclohexanon, laut GC-MS als 97 %iges Produkt, das so weiter umgesetzt wird.

Unter Argon werden 985 mg (ca. 25 mmol) Natriumhydrid (60-%ig) im Reaktionskolben vorgelegt, mit Petrolether entölt, und mit 15 ml Diethylcarbonat versetzt. Zur resultierenden Suspension werden sodann ein Tropfen Ethanol zugegeben und langsam die oben erhaltenen 1.90 g (12.3 mmol) 3,3-Diethylcyclohexanon gelöst in etwa 10 ml Diethylcarbonat zugetropft. Man rührt über Nacht bei Normaltemperatur, wobei eine langsame Wasserstoffentwicklung zu beobachten ist.

Zur Aufarbeitung wird die Reaktionsmischung langsam mit 30 g Eis und etwa 100 ml Wasser versetzt., dann mit etwa 6 ml konzentrierter Essigsäure pH 5 eingestellt.

Die erhaltene Lösung wird dreimal mit Diethylether extrahiert. Die vereinigten organischen Phasen werden zweimal mit kalter gesättigter Natriumhydrogencarbonatlösung sowie einmal mit gesättigter Kochsalzlösung gewaschen und über Magnesiumsulfat getrocknet. Anschließend engt man am Rotationsverdampfer im Vakuum bis auf etwa 5 ml ein.

Kugelrohrdestillation ergibt 1.37 g (49 %) der Zielverbindung in hinreichender Reinheit für die Folgestufen.
¹H-NMR (200 MHz, CDCl₃): die Verbindung liegt weitgehend enolisiert vor: δ = 0,7-0,95 (m, 6 H), 1,13-1,5 (m, 9 H), 2,04 (s, breit, 2H), 2,1-2,3 (m, 2H), 4,12-4,28 (m, 2H), 12,20 (s, breit, 1H).

### Beispiel I-13

### (6- Oxo-spiro[3.5]non-7-yl)-carbonsäureethylester

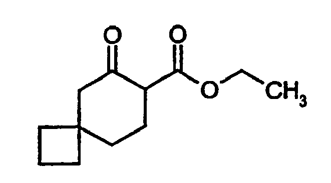

5.00 g (36.7 mmol) Spiro[3.5]non-7-en-6-on (Paulsen, H. et al., Angew. Chem., Int. Ed. (1999), 38, 3373) werden in 100 ml Ethylenglykoldimethylether vorgelegt und mit etwa 300 mg Pd/C (5 % Pd) versetzt Man hydriert bei Normaldruck über Nacht.

Zur Aufarbeitung wird über Kieselgur vom Katalysator abfiltriert und im Vakuum eingeengt.

Man erhält so 5.21 g (91-%ig nach GC-Analyse, entspricht 93 % Produktausbeute) Spiro[3.5]nonan-6-on.

Zu einer Suspension aus 2.89 g (60-%ig, ca. 72 mmol) Natriumhydrid in Diethylcarbonat wird unter Argon ein Drittel von 5.00 g (36.2 mmol) Spiro[3.5]nonan-6-on gelöst in Diethylcarbonat getropft. Erst nach Zugabe von wenig THF/Pentan zur Aktivierung des Natriumhydrids setzt eine geringe aber stetige Wasserstoffentwicklung ein. Langsame portionsweise Zugabe des restlichen Ketons erfolgt über den Tag verteilt. Danach wird über Nacht bei Normaltemperatur weitergerührt.

Zur Aufarbeitung wird die Reaktionsmischung langsam mit 20 g Eis und etwa 100 ml Wasser versetzt. Zunächst bildet sich ein zäher Schleim, der mit der Zeit langsam in Lösung geht. Mit ca. 10 ml konzentrierter Essigsäure wird ein pH von 5 eingestellt.

Die erhaltene Lösung wird dreimal mit Diethylether extrahiert. Die vereinigten organischen Phasen werden zweimal mit kalter gesättigter Natriumhydrogencarbonatlösung sowie einmal mit gesättigter Kochsalzlösung gewaschen und über Magnesiumsulfat getrocknet. Nach Einengen am Rotationsverdampfer bleiben etwa 70 ml einer Flüssigkeit zurück.

Zur Reinigung wird durch Destillation bei 50-60°C und 70-80 mbar zunächst überschüssiges Diethylcarbonat abdestilliert.

Der Rückstand wird über eine Kurzwegbrücke mit Vigreux-Einsatz bei etwa 0.25 mbar und 60-70°C destilliert.

Man erhält so 3.71g (49 %) der Zielverbindung in hinreichender Reinheit für die Folgestufen.
¹H-NMR (200 MHz, DMSO-d₆): die Verbindung liegt weitgehend enolisiert vor: δ = 1,11-1,29 (m, 3 H), 1,50-2,05 (m, 8 H), 2,17 (t, breit, J=6Hz, 2H), 2,31 (s, breit, 2H), 4,0-4,25 (m, 2H), 12,11 (s, breit, 1H).

### Beispiel I-14

### (2- Oxo-spiro[5.5]undec-3-yl)-carbonsäureethylester

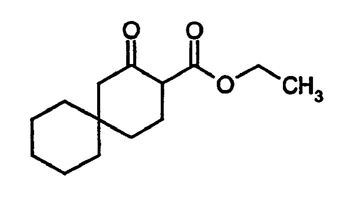

Unter Argon werden 1.11 g (27.7 mmol) Natriumhydrid (60-%ig) im Kolben vorgelegt, mit Petrolether entölt und mit 15 ml Diethylcarbonat versetzt. Zur resultierenden Suspension wird ein Tropfen Ethanol zugegeben und langsam 2.30 g (13.8 mmol) Spiro[5.5]undecan-2-on (De Jongh, H.A.P. and Wynberg, K., Tetrahedron (1964), 20, 2553) in etwa 10 ml Diethylcarbonat gelöst zugetropft. Man rührt über Nacht bei Normaltemperatur, wobei eine langsame Wasserstoffentwicklung zu beobachten ist.

Zur Aufarbeitung wird die Reaktionsmischung langsam mit 30 g Eis und etwa 100 ml Wasser versetzt, dann mit etwa 6 ml konzentrierter Essigsäure pH 5 eingestellt

Die erhaltene Lösung wird dreimal mit Diethylether extrahiert. Die vereinigten organischen Phasen werden zweimal mit kalter gesättigter Natriumhydrogencarbonatlösung sowie einmal mit gesättigter Kochsalzlösung gewaschen und über Magnesiumsulfat getrocknet. Anschließend engt man am Rotationsverdampfer im Vakuum bis auf etwa 5 ml ein.

Kugelrohrdestillation ergibt 2.43 g (74 %) der Zielverbindung.
¹H-NMR (200 MHz, CDCl₃): die Verbindung liegt weitgehend enolisiert vor: δ = 1,2-1,6 (m, 15 H), 2,11 (s, breit, 2H), 2,13-2,28 (m, 2H), 4,11-4,29 (m, 2H), 12,19 (s, breit, 1H).

In Analogie zur Vorschrift des Beispiels I-1 werden hergestellt:

### Beispiel I-15

### 6-Brom-3,3-diethyl-1,2,3,4-tetrahydro-9H-thioxanthen-9-on

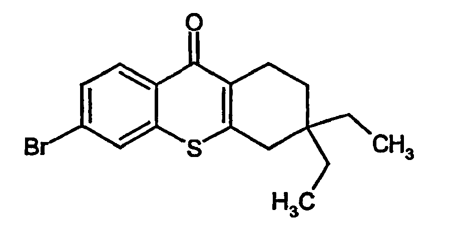

MS (EI+): 350 (M);
¹H-NMR (300 MHz, CDCl₃): δ = 0.85 (t, 6H); 1.37 (mc, 4H); 1.64 (t, 2H); 2.45 (s, br, 2H); 2.65 (t, br, 2H); 7.56 (dd, 1H); 7.67 (d, 1H); 8.35 (d, 1H).

### Beispiel I-16

### 6-Brom-3-spirohexyl-1,2,3,4-tetrahydro-9H-thioxanthen-9-on

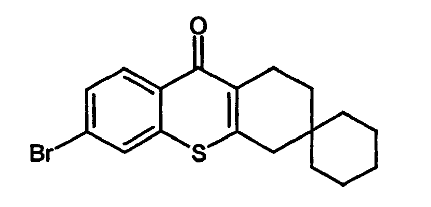

MS (CI+): 363 (M+H);
¹H-NMR (300 MHz, CDCl₃): δ = 1.3 - 1.6 (m, 10H); 1.70 (t, 2H); 2.51 (s, br, 2H); 2.66 (t, br, 2H); 7.56 (dd, 1H); 7.66 (d, 1H); 8.34 (d, 1H).

In Analogie zur Vorschrift des Beispiels I-5 werden hergestellt:

### Beispiel I-17

### 3,3-Dimethyl-9-oxo-2,3,4,9-tetrahydro-1H-thioxanthen-6-carbonitril

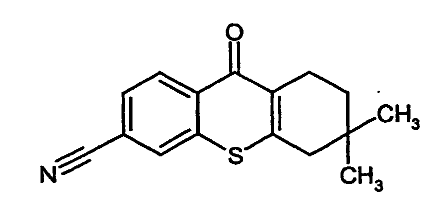

MS (CI+): 270 (M+H);
¹H-NMR (300 MHz, DMSO): δ = 0.99 (s, 6H); 1.58 (t, 2H); 2.45 - 2.6 (m, 4H); 7.94 (dd, 1H); 8.42 (d, 1H); 8.50 (d, 1H).

### Beispiel I-18

### 3,3-Diethyl-9-oxo-2,3,4,9-tetrahydro-1H-thioxanthen-6-carbonitril

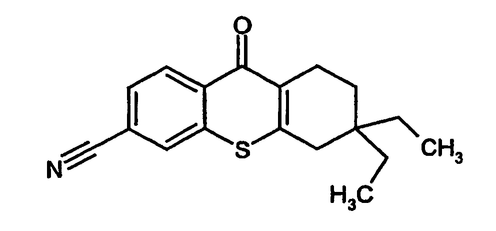

MS (CI+): 298 (M+H);
¹H-NMR (300 MHz, CDCl₃): δ = 0.86 (t, 6H); 1.38 (mc, 4H); 1.66 (t,2H); 2.49 (s, br, 2H); 2.67 (t, br, 2H); 7.67 (dd, 1H); 7.83 (d, 1H); 8.58 (d, 1H).

### Beispiel I-19

### 9-Oxo-3-spirobutyl-2,3,4,9-tetrahydro-1H-thioxanthen-6-carbonitril

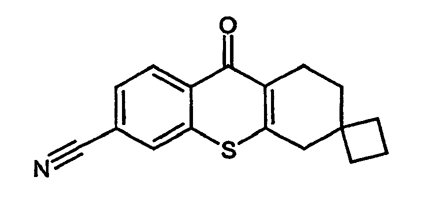

MS (CI+): 282 (M+H);
¹H-NMR (300 MHz, CDCl₃): δ = 1.80 - 2.05 (m, 8H); 2.72 (tt, 2H); 2.76 (s, br, 2H); 7.68 (dd, 1H); 7.84 (d, 1H); 8.57 (d, 1H).

### Beispiel I-20

### 9-Oxo-3-spirohexyl-2,3,4,9-tetrahydro-1H-thioxanthen-6-carbonitril

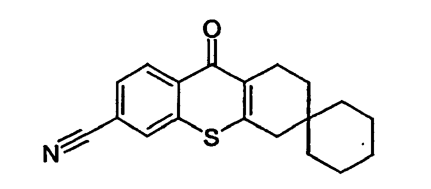

MS (EI+): 309 (M);
¹H-NMR (300 MHz, CDCl₃): δ =1.35 - 1.6 (m, 10H); 1.72 (t, 2H); 2.55 (s, br, 2H); 2.69 (t, br, 2H); 7.67 (dd, 1H); 7.83 (d, 1H); 8.58 (d, 1H).

### Beispiel I-21

### 12-Oxo-6,7,8,9,10,11-hexahydro-12H-cycloocta[b]thiochromen-3-carbonitril

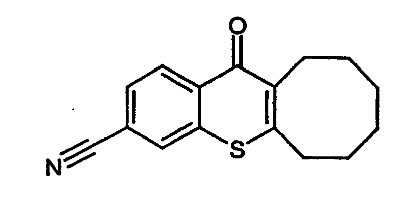

Das Produkt wird durch Kristallisation aus Aceton gereinigt.
MS (CI+): 270 (M+H);
¹H-NMR (300 MHz, DMSO): δ = 1.30 - 1.52 (m, 4H); 1.53 - 1.65 (m, 2H); 1.70 - 1.85 (m, 2H); 2.90 (m, 4H); 7.94 (dd, 1H); 8.43 (d, 1H); 8.50 (d, 1H).

In Analogie zur Vorschrift des Beispiels I-11 werden hergestellt:

### Beispiel I-22

### 3,3-Dimethyl-9-oxo-2,3,4,9-tetrahydro-1H-thioxanthen-6-carbonsäure

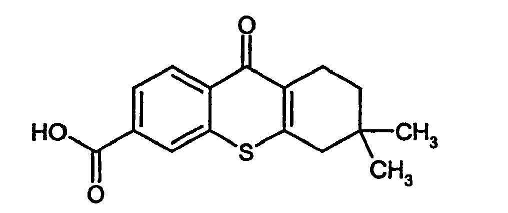

MS (ESI+): 289 (M+H);
HPLC: Rₜ= 4.57 min;
¹H-NMR (200 MHz, DMSO): δ = 0.99 (s, 6H); 1.58 (t, 2H); 2.45 - 2.65 (m, 4H); 8.02 (dd, 1H); 8.29 (d, 1H); 8.41 (d, 1H); 13.58 (s, br, 1H).

### Beispiel 1-23

### 12-Oxo-6,7,8,9,10,11-hexahydro-12H-cycloocta[b]thiochromen-3-carbonsäure

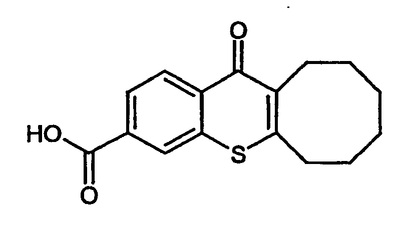

Unter Argonatmosphäre werden 200 mg (0.74 mmol) 12-Oxo-6,7,8,9,10,11-hexahydro-12H-cycloocta[b]thiochromen-3-carbonitril in 10 ml 1,2-Ethandiol mit 98 mg (1.48 mmol) Kaliumhydroxidpulver versetzt und das Gemisch über Nacht bei 120°C gerührt. Nach Abkühlen gibt man wenig Wasser zu und säuert dann mit 1N Salzsäure auf pH 2 an. Der ausgefallene Niederschlag wird abfiltriert und mit Wasser gewaschen.
Man erhält so 160 mg (75%) der Zielverbindung.
HPLC: Rₜ= 4.38 min;
¹H-NMR (300 MHz, THF): δ = 1.40-1.60 (m, 4H); 1.68 (mc, 2H); 1.70 - 1.85 (mc, 2H); 2.92 (mc, 4H); 8.04 (dd, 1H); 8.26 (d, 1H); 8.48 (d, 1H); 11 -13 (br, 1H).

### Beispiel I-24

### 6-(Cyanomethyl)-3,3-dimethyl-1,2,3,4-tetrahydro-9H-thioxanthen-9-on

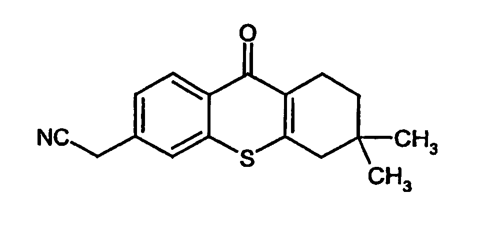

300 mg (1.02 mmol) 6-(Chlormethyl)-3,3-dimethyl-1,2,3,4-tetrahydro-9H-thioxanthen-9-on, 80.1 mg (1.23 mmol) Kaliumcyanid und 13.5 mg (0.05 mmol) 18-Krone-6 werden unter Argon in 1.00 ml N,N-Dimethylformamid zusammengegeben und anschließend über Nacht bei 40°C Badtemperatur gerührt. Zur Aufarbeitung wird das Gemisch mit Magnesiumsulfat getrocknet. Das Solvens wird im Vakuum eingeengt und der Rückstand chromatografiert (präparative HPLC, Acetonitril/Wasser 50:50 - 95:5). Man erhält so 105mg (36%) der Zielverbindung.
MS (ESI+): 284 (M+H);
HPLC: Rₜ= 4.76 min;
¹H-NMR (300 MHz, DMSO): δ = 0.99 (s, 6H); 1.58 (t, 2H); 2.45 - 2.65 (m, 4H); 4.21 (s, 2H); 7.53 (d, 1H); 7.73 (s, 1H); 8.34 (d, 1H).

### Beispiel I-25

### (3,3-Dimethyl-9-oxo-2,3,4,9-tetrahydro-1H-thioxanthen-6-yl)essigsäure

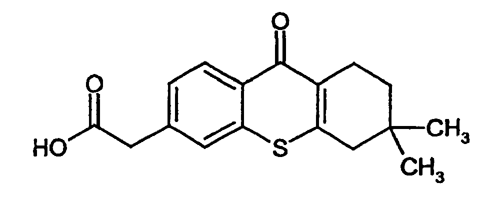

109 mg (0.38 mmol) 6-(Cyanomethyl)-3,3-dimethyl-1,2,3,4-tetrahydro-9H-thioxanthen-9-on werden in 70%iger Schwefelsäure suspendiert und unter Argon bei 120°C Badtemperatur gerührt. Nach vollständiger Umsetzung (etwa 3 Stunden) lässt man abkühlen und gibt den Ansatz auf Eiswasser. Der ausgefallene Feststoff wird abgesaugt, mit Wasser gewaschen und im Vakuum getrocknet. Man erhält so 109mg (93%) der Zielverbindung.
MS (ESI+): 303 (M+H);
HPLC: Rₜ= 4.47 min;
¹H-NMR (200 MHz, DMSO): δ = 0.99 (s, 6H); 1.57 (t, 2H); 2.4 - 2.65 (m, 4H); 3.74 (s, 2H); 7.46 (dd, 1H); 7.65 (s, br, 1H); 8.26 (d, 1H); 12.1-12.9 (s, br, 1H).

### Ausführungsbeispiele 1

In Analogie zur Vorschrift des Beispiels I-1 wurden herstellt:

### Beispiel 1-1

### 3-Ethyl-6-methoxy-1,2,3,4-tetrahydro-9H-thioxanthen-9-on

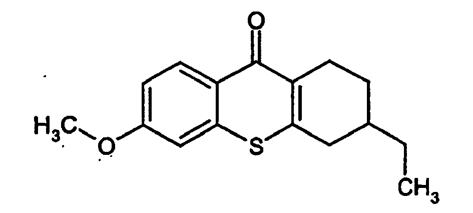

MS (EI+): 274 (M);
¹H-NMR (400 MHz, CDCl₃): δ = 0.99 (t, J=7.5Hz; 3H); 1.34-1.44 (m, 3H); 1.64-1.72 (m, 1H); 1.97-2.04 (m, 1H); 2.33-2.40 (m, 1H); 2.44-2.54 (m, 1H); 2.63-2.69 (m, 1H); 2.86-2.93 (m, 1H); 3.88 (s, 3H); 6.88 (d, J=2.5; 1H); 6.99-7.03 (dd, J=9Hz, 2.5Hz; 1H); 8.41 (d, J=9Hz; 1H).

### Beispiel 1-2

### 3-Ethyl-6-methyl-1,2,3,4-tetrahydro-9H-thioxanthen-9-on

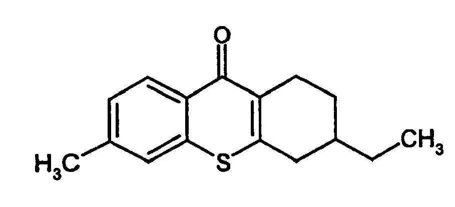

MS (EI+): 258 (M);
¹H-NMR (300 MHz, CDCl₃): δ = 0.99 (t, J=7.5Hz; 3H); 1.33-1.47 (m, 3H); 1.64-1.76 (m, 1H); 1.98-2.07 (m, 1H); 2.34-2.42 (m, 1H); 2.44 (s, 3H); 2.46-2.56 (m, 1H); 2.66-2.74 (m, 1H); 2.86-2.96 (m, 1H); 7.25-7.29 (m, 2H); 8.38 (d, J=9Hz; 1H).

### Beispiel 1-3

### 3-Ethyl-6-(1H-tetrazol-5-yl)-1,2,3,4-tetrahydro-9H-thioxanthen-9-on

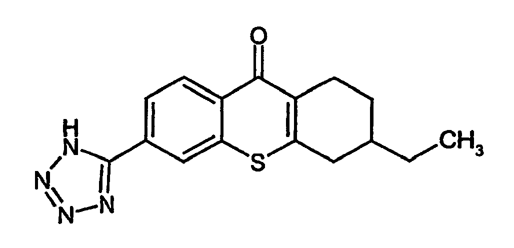

0,50 g (1,86 mmol) 3-Ethyl-9-oxo-2,3,4,9-tetrahydro-1H-thioxanthen-6-carbonitril (Beispiel I-5), 0,51 g (3,71 mmol) Triethylammoniumhydrochlorid und 0,24 g (3,71 mmol) Natriumazid werden in Toluol auf 100°C erhitzt und über Nacht bei dieser Temperatur gerührt.

Nach DC-Kontrolle auf Vollständigkeit des Umsatzes lässt man abkühlen und gibt die Reaktionsmischung auf 100 ml Wasser/20 ml Toluol, rührt intensiv 10 min, säuert mit 5N Salzsäure bis etwa pH 3 an, rührt weitere 10 min nach, saugt den ausgefallenen Feststoff ab und wäscht ihn zweimal mit Wasser. Das verbleibende Rohprodukt wird getrocknet und aus Cyclohexan/Ethylacetat ca. 1:40 umkristallisiert. Man erhält so 290 mg (50 %) der Zielverbindung.
MS (CI+): 313 (M+H);
¹H-NMR (300 MHz, DMSO): δ = 0.95 (t, J=7.5Hz; 3H); 1.30-1.43 (m, 3H); 1.60-1.74 (m, 1H); 1.92-2.02 (m, 1H); 2.33-2.47 (m, 2H); 2.72-2.82 (m, 2H); 3.00-3.60 (m, b, 1H); 8.14-8.18 (dd, J=8.5Hz, 1.5Hz; 1H); 8.39 (d, J=1.5Hz; 1H), 8.47-8.50 (d, J=8.5Hz; 1H).

In Analogie zur Vorschrift des Beispiels 1-3 wurden hergestellt:

### Beispiel 1-4

### 3-(1H-Tetraazol-5-yl)-6,7,8,9,10,11-hexahydro-12H-cycloocta[b]thiochromen-12-on

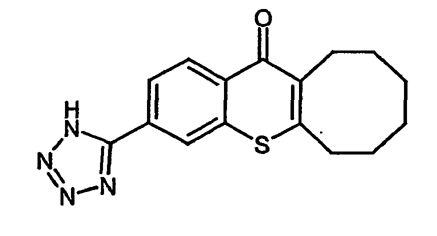

MS (EI+): 312 (M);
¹H-NMR (200 MHz, DMSO): δ = 1.30-1.43 (m, 4H); 1.54-1.66 (m, 2H); 1.73-1.84 (m, 2H); 2.83-2.94 (m, 4H); 3.12-3.68 (m, b, 1H); 8.15-8.20 (dd, J=8.5Hz, 1.5Hz; 1H); 8.43 (s, 1H), 8.49-8.53 (d, *J*=8.5Hz; 1*H*).

### Beispiel 1-5

### 3,3-Dimethyl-6-(1H-tetrazol-5-yl)-1,2,3,4-tetrahydro-9H-thioxanthen-9-on

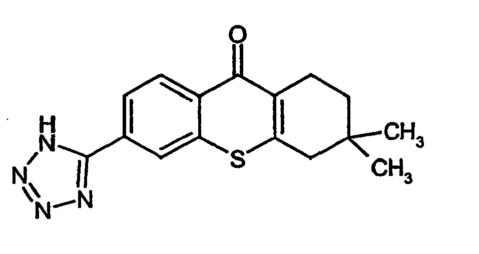

MS (CI+): 313 (M+H);
¹H-NMR (200 MHz, DMSO): δ = 1.00 (s, 6H); 1.59 (t, J=6.5Hz; 2H); 2.47-2.62 (m, 4H); 3.08-3.52 (s, b, 1H); 8.15-8.20 (dd, J=8.5Hz, 1.5Hz, 1H); 8.40 (s, 1H); 8.50 (d, J=8.5Hz; 1H).

### Beispiel 1-6

### 3-Spirobutyl-6-(1H-tetrazol-5-yl)-1,2,3,4-tetrabydro-9H-thioxanthen-9-on

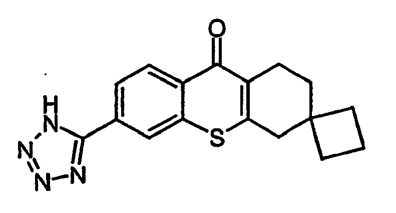

MS (ESI): 325 (M+H);
¹H-NMR (200 MHz, DMSO): δ = 1.79-1.99 (m, 8H); 2.56-2.63 (t, J=6Hz;2H); 2.84 (s, 2H); 3.12-3.60 (s, b, 1H); 8.14-8.19 (dd, J=8.5Hz, 1.5Hz; 1H); 8.41 (d, J=1.5Hz; 1H); 8.47-8.51 (d, J=8.5Hz;1H).

### Beispiel 1-7

### 3-Spirohexyl-6-(1H-tetrazol-5-yl)-1,2,3,4-tetrahydro-9H-thioxanthen-9-on

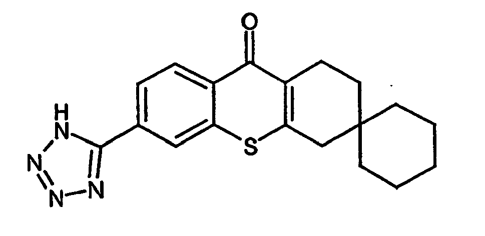

MS (EI+): 352 (M);
¹H-NMR (300 MHz, DMSO): δ = 1.33-1.48 (m, 10H); 1.67 (t, J=6.5Hz;2H); 2.56 (t, J=6.5Hz; 2H); 2.61 (s, 2H); 3.17-3.45 (s, b, 1H); 8.15-8.19 (dd, J=8.5Hz, 1.5Hz; 1H); 8.40 (d, J=1.5Hz; 1H); 8.48-8.51 (d, J=8.5Hz;1H).

### Beispiel 1-8

### 3-Ethyl-6-[3-(1H-tetrazol-5-yl)phenyl]-1,2,3,4-tetrahydro-9H-thioxanthen-9-on

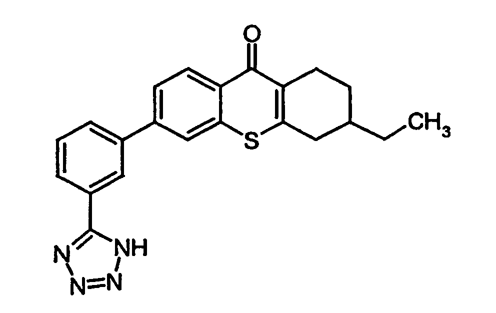

MS (ESI): 389 (M+H);
¹H-NMR (300 MHz, DMSO): δ = 0.96 (t, J=7.5Hz; 3H); 1.31-1.44 (m, 3H); 1.62-1.74 (m, 1H); 1.93-2.02 (m, 1H); 2.34-2.53 (m, 2H); 2.72-2.82 (m, 2H); 3.23-3.40 (s, b, 1H); 7.73-7.79 (t, J=8Hz; 1H); 7.95-7.98 (dd, J=8.5Hz, 1.5Hz; 1H); 8.05 (d, J=8Hz; 1H); 8.12 (d, J=8Hz; 1H); 8.19 (d, J=1.5Hz; 1H); 8.42-8.46 (m, 2H).

### Beispiel 1-9

### 3-Ethyl-6-(2-methyl-2H-tetrazol-5-yl)-1,2,3,4-tetrahydro-9H-thioxanthen-9-on

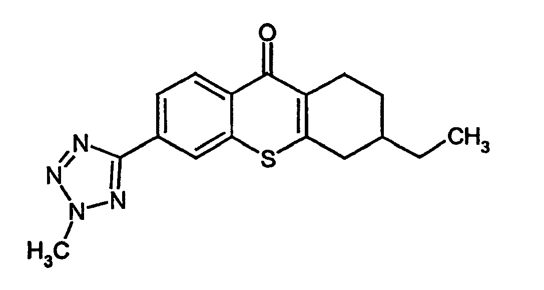

Unter Argon werden 91,0 mg (0,29 mmol) 3-Ethyl-6-(1H-tetrazol-5-yl)-1,2,3,4-tetrahydro-9H-thioxanthen-9-on (Beispiel 1-3) in 5 ml DMF gelöst und 95 mg (0,29 mmol) Cäsiumcarbonat zugegeben. Man erwärmt für 1h auf 60°C, kühlt wieder auf Normaltemperatur ab, gibt 0,03 ml (0,45 mmol) Iodmethan zu und lässt bei Normaltemperatur über Nacht rühren. Zur Aufarbeitung wird der Ansatz mit etwa 10 ml Wasser und Ethylacetat versetzt, 5 min verrührt und auf 50 ml Wasser gegeben. Man extrahiert noch dreimal mit Ethylacetat, trocknet die vereinigten organischen Phasen und engt am Rotationsverdampfer ein.

Das so erhaltene Rohprodukt wird durch präparative HPLC aufgetrennt und gereinigt.

Man erhält so 54,9mg (58 %) der Zielverbindung.
MS (EI+): 326 (M);
¹H-NMR (300 MHz, CDCl₃): δ = 1.00 (t, J=7.5Hz; 3H); 1.38-1.49 (m, 3H); 1.68-1.79 (m, 1H); 2.01-2.10 (m, 1H); 2.40-2.60 (m, 2H); 2.72-2.79 (m, 1H); 2.91-2.97 (m, 1H); 4.44 (s, 3H); 8.17-8.21 (dd, J=8.5, 1.5Hz; 1H); 8.33 (d, J=1.5Hz; 1H); 8.60 (d, J=8.5Hz; 1H).

Als Nebenprodukt werden isoliert:
5,2 mg (5,5 %) 3-Ethyl-6-(1-methyl-1*H*-tetrazol-5-yl)-1,2,3,4-tetrahydro-9H-thioxanthen-9-on
¹H-NMR (400 MHz, CDCl₃): δ = 1.01 (t, J=7.5Hz; 3H); 1.38-1.49 (m, 3H); 1.70-1.79 (m, 1H); 2.03-2.11 (m, 1H); 2.42-2.61 (m, 2H); 2.73-2.80 (m, 1H); 2.91-2.97 (m, 1H); 4.25 (s, 3H); 7.75-7.77 (dd, J=8.5, 1.5Hz; 1H); 8.01 (d, J=1.5Hz; 1H); 8.67 (d, J=8.5Hz; 1H).

### Beispiel 1-10

### 3-Ethyl-6-(2-thienyl)-1,2,3,4-tetrahydro-9H-thioxanthen-9-on

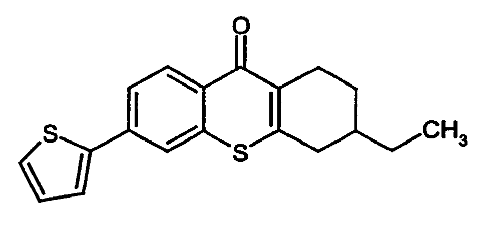

Man legt 100 mg (0,31 mmol) 6-Brom-3-ethyl-1,2,3,4-tetrahydro-9H-thioxanthen-9-on (Beispiel I-1) in 2,0 ml 1,2-Dimethoxyethan vor, gibt 47,5 mg (0,37 mmol) Thiophen-2-boronsäure, 0,34 ml (0,68 mmol) 2M wässrige Natriumcarbonatlösung und 10 mg Dichlorbis(triphenylphosphin)palladium-(II) zu und erhitzt für etwa 2h bis zur vollständigen Umsetzung zum Rückfluss.

Nach Abkühlen wird das Reaktionsgemisch über eine Kartusche mit 1g Kieselgel filtriert, das Eluat eingeengt und über präparative HPLC getrennt.

Nach Trocknen der Produktfraktionen im Vakuum erhält man 76,8 mg (76 %) der Zielverbindung.
MS (CI+): 327 (M+H);
¹H-NMR (200 MHz, CDCl₃): δ = 1.00 (t, J=7.5Hz; 3H); 1.35-1.51 (m, 3H); 1.62-1.81 (m, 1H); 1.98-2.11 (m, 1H); 2.34-2.61 (m, 2H); 2.66-2.79 (m, 1H); 2.84-3.00 (m, 1H); 7.11-7.16 (dd, J=5Hz, 3.5Hz; 1H); 7.38-7.41 (dd, J=5Hz, 1Hz; 1H); 7.45-7.47 (dd, J=3.5Hz, 1Hz; 1H); 7.67-7.72 (m, 2H); 8.47-8.51 (m, 1H).

In Analogie zur Vorschrift des Beispiel 1-10 wurden herstellt:

### Beispiel 1-11

### 3-(2-Thienyl)-6,7,8,9,10,11-hexahydro-12H-cycloocta[b]thiochromen-12-on

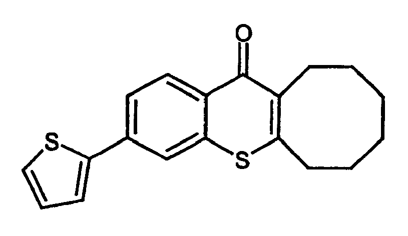

MS (CI+): 327 (M+H);
¹H-NMR (300 MHz, CDCl₃): δ = 1.40-1.55 (m, 4H); 1.69-1.77 (m, 2H); 1.81-1.89 (m, 2H); 2.84-2.88 (m, 2H); 2.93-2.97 (m, 2H); 7.12-7.15 (dd, J=5Hz, 4Hz; 1H); 7.39-7.40 (dd, J=5Hz, 1Hz; 1H); 7.46-7.47 (dd, J=3.5Hz, 1Hz; 1H); 7.70-7.74 (m, 2H); 8.49-8.52 (m, 1H).

### Beispiel 1-12

### 3,3-Dimethyl-6-(2-thienyl)-1,2,3,4-tetrahydro-9H-thioxanthen-9-on

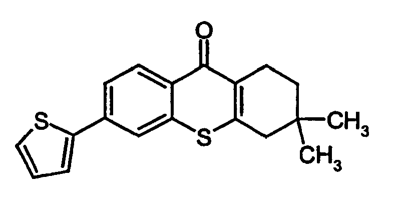

MS (EI+): 326 (M);
¹H-NMR (200 MHz, CDCl₃): δ = 1.05 (s, 6H); 1.64 (t, J=6.5Hz; 2H); 2.48 (s, 2H); 2.73 (t, J=6.5Hz; 2H); 7.11-7.16 (dd, J=5Hz, 3.5Hz; 1H); 7.38-7.41 (dd, J=5Hz, 1Hz; 1H); 7.45-7.48 (dd, J=3.5Hz, 1Hz; 1H); 7.68-7.74 (m, 2H); 8.48-8.52 (m, 1H).

### Beispiel 1-13

### 3-Spirobutyl-6-(2-thienyl)-1,2,3,4-tetrahydro-9H-thioxanthen-9-on

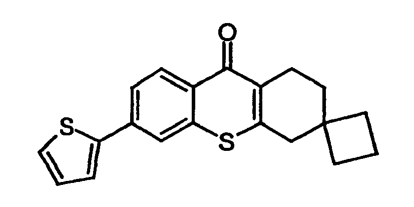

MS (CI+): 339 (M+H);
¹H-NMR (300 MHz, CDCl₃): δ = 1.80-2.00 (m, 8H); 2.68-2.81 (m, 4H); 7.12-7.15 (dd, J=5Hz, 3.5Hz; 1H); 7.38-7.40 (dd, J=5Hz, 1Hz; 1H); 7.45-7.47 (m, 1H); 7.68-7.71 (m, 2H); 8.47-8.50 (m, 1H).

### Beispiel 1-14

### 3-Ethyl-6-(4-methyl-2-thienyl)-1,2,3,4-tetrahydro-9H-thioxanthen-9-on

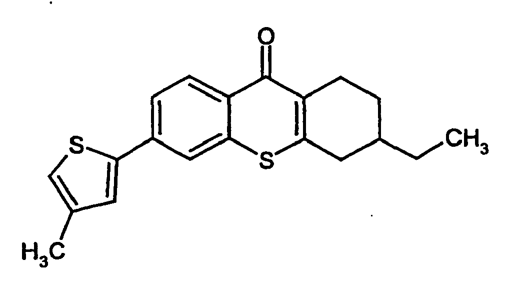

MS (CI+): 341 (M+H);
¹H-NMR (200 MHz, CDCl₃): δ = 1.00 (t, J=7.5Hz; 3H); 1.34-1.50 (m, 3H); 1.61-1.79 (m, 1H); 1.98-2.12 (m, 1H); 2.31 (s, 3H); 2.38-2.63 (m, 2H); 2.65-2.78 (m, 1H); 2.85-2.99 (m, 1H); 6.97 (s, 1H); 7.28 (m, 1H); 7.64-7.69 (m, 2H); 8.45-8.49 (m, 1H).

### Beispiel 1-15

### 6-(5-Acetyl-2-thienyl)-3-ethyl-1,2,3,4-tetrahydro-9H-thioxanthen-9-on

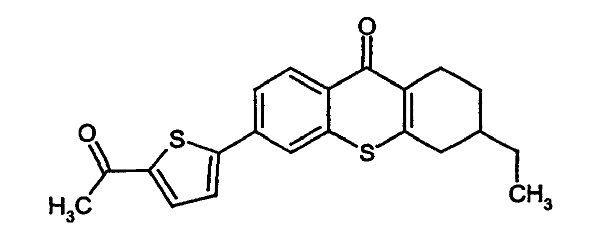

MS (ESI): 369 (M+H);
¹H-NMR (300 MHz, CDCl₃): δ = 1.00 (t, J=7.5Hz; 3H); 1.35-1.49 (m, 3H); 1.67-1.77 (m, 1H); 2.01-2.10 (m, 1H); 2.37-2.54 (m, 2H); 2.59 (s, 3H); 2.69-2.78 (m, 1H); 2.88-2.97 (m, 1H); 7.45 (d, J=4Hz; 1H); 7.69-7.75 (m, 3H); 8.52 (d, J=8.5Hz; 1H).

### Beispiel 1-16

### 3-Ethyl-6-(3-thienyl)-1,2,3,4-tetrahydro-9H-thioxanthen-9-on

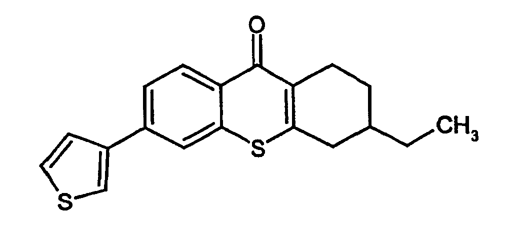

MS (CI+): 327 (M+H);
¹H-NMR (300 MHz, CDCl₃): δ = 1.00 (t, J=7.5Hz; 3H); 1.37-1.48 (m, 3H); 1.66-1.78 (m, 1H); 2.00-2.09 (m, 1H); 2.37-2.58 (m, 2H); 2.67-2.76 (m, 1H); 2.88-2.98 (m, 1H); 7.42-7.47 (m, 2H); 7.62 (m, 1H); 7.68-7.71 (m, 2H); 8.51 (m, 1H).

### Beispiel 1-17

### 3-Ethyl-6-phenyl-1,2,3,4-tetrahydro-9H-thioxanthen-9-on

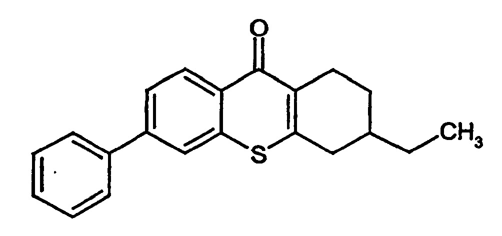

MS (CI+): 321 (M+H);
¹H-NMR (200 MHz, CDCl₃): δ = 1.00 (t, J=7.5Hz; 3H); 1.33-1.51 (m, 3H); 1.62-1.80 (m, 1H); 1.99-2.12 (m, 1H); 2.35-2.64 (m, 2H); 2.68-2.79 (m, 1H); 2.87-3.02 (m, 1H); 7.41-7.53 (m, 3H); 7.64-7.73 (m, 4H); 8.53-8.58 (m, 1H).

### Beispiel 1-18

### 6-Phenyl-3-spirobutyl-1,2,3,4-tetrahydro-9H-thioxanthen-9-on

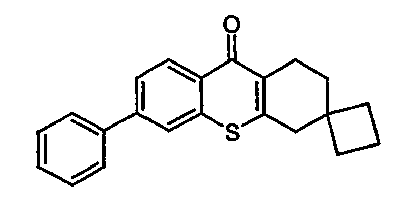

MS (CI+): 333 (M+H);
¹H-NMR (300 MHz, CDCl₃): δ = 1.80-2.04 (m, 8H); 2.72-2.76 (m, 4H); 7.39-7.52 (m, 3H); 7.63-7.71 (m, 4H); 8.54-8.57 (m, 1H).

### Beispiel 1-19

### 3-Ethyl-6-(4-methylphenyl)-1,2,3,4-tetrahydro-9H-thioxanthen-9-on

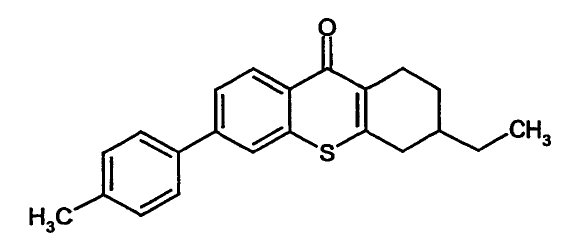

MS (CI+): 335 (M+H);
¹H-NMR (300 MHz, CDCl₃): δ = 1.00 (t, J=7.5Hz; 3H); 1.35-1.48 (m, 3H); 1.66-1.78 (m, 1H); 2.00-2.09 (m, 1H); 2.42 (s, 3H); 2.43-2.59 (m, 2H); 2.68-2.77 (m, 1H); 2.88-2.98 (m, 1H); 7.28-7.30 (d, J=8Hz; 2H); 7.54-7.57 (d, J=8Hz; 2H); 7.67-7.70 (m, 2H); 8.52-8.55 (m, 1H).

### Beispiel 1-20

### N-[3-(3-Ethyl-9-oxo-2,3,4,9-tetrahydro-1H-thioxanthen-6-yl)phenyl]acetamid

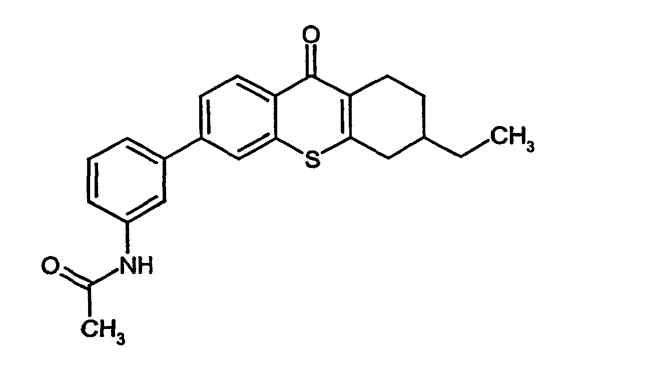

MS (CI+): 378 (M+H);
¹H-NMR (300 MHz, CDCl₃): δ = 1.00 (t, J=7.5Hz; 3H); 1.36-1.48 (m, 3H); 1.66-1.77 (m, 1H); 2.00-2.09 (m, 1H); 2.24 (s, 3H); 2.37-2.58 (m, 2H); 2.68-2.76 (m, 1H); 2.88-2.98 (m, 1H); 7.36-7.58 (m, 4H); 7.64-7.69 (m, 2H); 7.84 (s, 1H); 8.53 (m, 1H).

### Beispiel 1-21

### 3-Ethyl-6-(4-methoxyphenyl)-1,2,3,4-tetrahydro-9H-thioxanthen-9-on

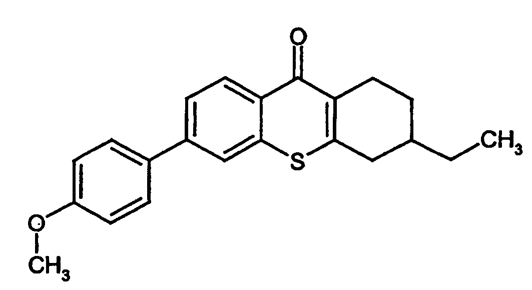

MS (CI+): 351 (M+H);
¹H-NMR (200 MHz, CDCl₃):δ = 1.00 (t, J=7.5Hz; 3H); 1.35-1.51 (m, 3H); 1.62-1.79 (m, 1H); 1.98-2.11 (m, 1H); 2.34-2.62 (m, 2H); 2.67-2.79 (m, 1H); 2.86-3.01 (m, 1H); 3.87 (s, 3H); 6.99-7.03 (m, 2H); 7.57-7.69 (m, 4H); 8.50-8.55 (m, 1H).

### Beispiel 1-22

### 6-(4-Methoxyphenyl)-3-spirobutyl-1,2,3,4-tetrahydro-9H-thioxanthen-9-on

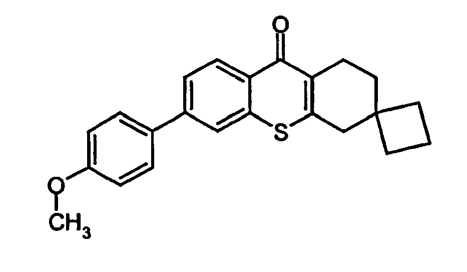

MS (CI+): 363 (M+H);
¹H-NMR (300 MHz, CDCl₃): δ = 1.80-2.03 (m, 8H); 2.71-2.76 (m, 4H); 3.87 (s, 3H); 7.00-7.03 (m, 2H); 7.58-7.68 (m, 4H); 8.50-8.53 (m, 1H).

### Beispiel 1-23

### 3-Ethyl-6-(3-nitrophenyl)-1,2,3,4-tetrahydro-9H-thioxanthen-9-on-12-on

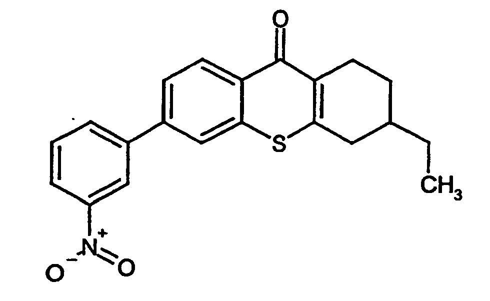

MS (CI+): 366 (M+H);
¹H-NMR (300 MHz, CDCl₃): δ = 1.01 (t, J=7.5Hz; 3H); 1.37-1.50 (m, 3H); 1.68-1.79 (m, 1H); 2.02-2.11 (m, 1H); 2.40-2.61 (m, 2H); 2.71-2.79 (m, 1H); 2.89-2.99 (m, 1H); 7.65-7.75 (m, 3H); 7.97-8.01 (m, 1H); 8.27-8.30 (m, 1H); 8.52 (m, 1H); 8.60-8.63 (m, 1H).

### Beispiel 1-24

### 6-(4-Chlorphenyl)-3-ethyl-1,2,3,4-tetrahydro-9H-thioxanthen-9-on

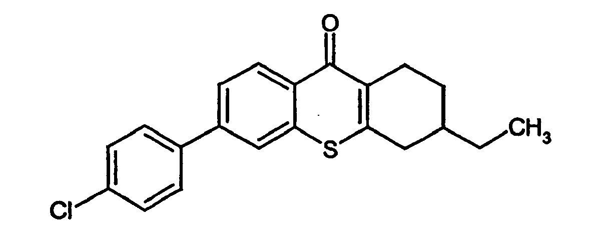

MS (CI+): 355 (M+H);
¹H-NMR (300 MHz, CDCl₃): δ = 1.00 (t, J=7.5Hz; 3H); 1.37-1.49 (m, 3H); 1.66-1.78 (m, 1H); 2.01-2.10 (m, 1H); 2.38-2.59 (m, 2H); 2.69-2.77 (m, 1H); 2.89-2.98 (m, 1H); 7.43-7.48 (m, 2H); 7.56-7.61 (m, 2H); 7.63-7.67 (m, 2H); 8.54-8.57 (m, 1H).

### Beispiel 1-25

### 3-Ethyl-6-[4-(hydroxymethyl)phenyl]-1,2,3,4-tetrahydro-9H-thioxanthen-9-on

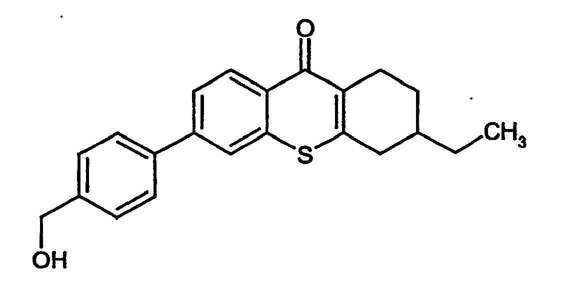

MS (CI+): 351 (M+H);
¹H-NMR (300 MHz, CDCl₃): δ = 0.95 (t, J=7.5Hz; 3H); 1.28-1.43 (m, 3H); 1.60-1.72 (m, 1H); 1.92-2.01 (m, 1H); 2.32-2.47 (m, 2H); 2.70-2.78 (m, 2H); 4.57 (d, J=5.5Hz; 2H); 5.29 (t, J=5.5Hz; 1H); 7.46 (d, J=8Hz; 2H); 7.80 (d, J=8.5Hz; 2H); 7.85-7.89 (dd, J=8.5Hz, 1.5Hz; 1H); 8.07 (d, J=1.5Hz; 1H); 8.37 (m, 1H).

### Beispiel 1-26

### 4-(3-Ethyl-9-oxo-2,3,4,9-tetrahydro-1H-thioxanthen-6-yl)benzonitril

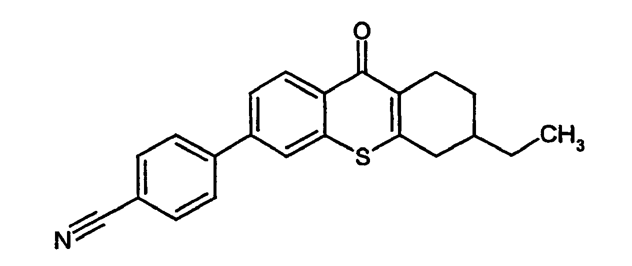

MS (EI+): 345 (M);
¹H-NMR (200 MHz, CDCl₃): δ = 1.00 (t, J=7.5Hz; 3H); 1.36-1.52 (m, 3H); 1.63-1.82 (m, 1H); 1.99-2.13 (m, 1H); 2.36-2.64 (m, 2H); 2.68-2.81 (m, 1H); 2.87-3.02 (m, 1H); 7.65-7.82 (m, 6H); 8.57-8.61 (m, 1H).

### Beispiel 1-27

### 3-(3,3-Dimethyl-9-oxo-2,3,4,9-tetrahydro-1H-thioxanthen-6-yl)benzonitril

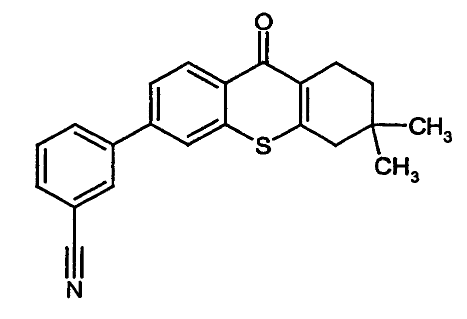

MS (EI+): 345 (M);
¹H-NMR (200 MHz, CDCl₃): δ = 1.06 (s, 6H); 1.65 (t, J=6.5Hz; 2H); 2.51 (s, 2H); 2.15 (t, J=6.5Hz; 2H); 7.57-7.74 (m, 4H); 7.85-7.93 (m, 2H); 8.58-8.62 (m, 1H).

### Beispiel 1-28

### 3-(9-Oxo-3-spirobutyl-2,3,4,9-tetrahydro-1H-thioxanthen-6-yl)benzonitril

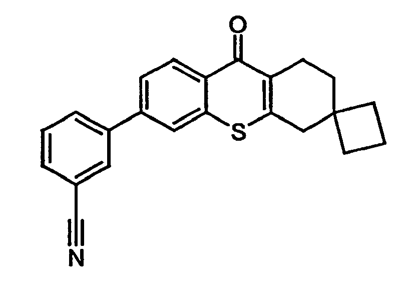

MS (CI+): 358 (M+H);
¹H-NMR (300 MHz, CDCl₃): δ = 1.81-2.01 (m, 8H); 2.72-2.78 (m, 4H); 7.58-7.73 (m, 4H); 7.86-7.90 (m, 1H); 7.93 (s, 1H); 8.59 (d, J=8.5Hz; 1H).

### Beispiel 1-29

### 3-(3-Ethyl-9-oxo-2,3,4,9-tetrahydro-1H-thioxanthen-6-yl)benzonitril

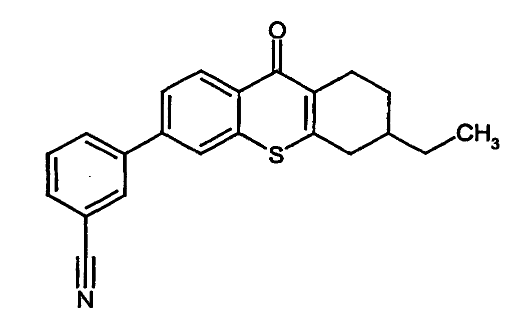

MS (CI+): 346 (M+H);
¹H-NMR (200 MHz, CDCl₃): δ = 1.01 (t, J=7.5Hz; 3H); 1.34-1.52 (m, 3H); 1.64-1.82 (m, 1H); 2.01-2.13 (m, 1H); 2.36-2.63 (m, 2H); 2.68-2.81 (m, 1H); 2.87-3.02 (m, 1H); 7.57-7.73 (m, 4H); 7.86-7.93 (m, 2H); 8.57-8.62 (m, 1H).

### Beispiel 1-30

### 3-Ethyl-6-(3-pyridinyl)-1,2,3,4-tetrahydro-9H-thioxanthen-9-on

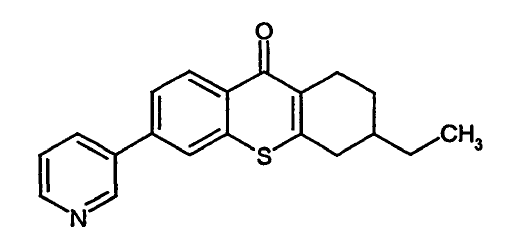

MS (DCI+): 322 (M+H);
¹H-NMR (200 MHz, CDCl₃): δ = 1.01 (t, J=7.5Hz; 3H); 1.36-1.52 (m, 3H); 1.65-1.81 (m, 1H); 1.99-2.13 (m, 1H); 2.36-2.64 (m, 2H); 2.68-2.82 (m, 1H); 2.87-3.02 (m, 1H); 7.39-7.45 (m, 1H); 7.66-7.70 (m, 2H); 7.92-7.98 (m, 1H); 8.58-8.69 (m, 2H); 8.92 (m, 1H).

### Beispiel 1-31

### 3,3-Dimethyl-6-(3-pyridinyl)-1,2,3,4-tetrahydro-9H-thioxanthen-9-on

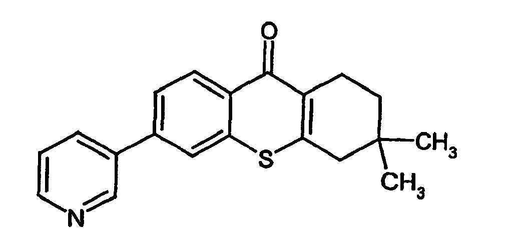

MS (CI+): 322 (M+H);
¹H-NMR (300 MHz, CDCl₃): δ = 1.06 (s, 6H); 1.65 (t, J=6.5Hz; 2H); 2.50 (s, 2H); 2.75 (t, J=6.5Hz; 2H); 7.40-7.45 (m, 1H); 7.67-7.69 (m, 2H); 7.93-7.97 (m, 1H); 8.61 (dd, J=8Hz, 1Hz; 1H); 8.67 (dd, J=4.5Hz, 1.5Hz; 1H); 8.90 (m, 1H).

### Beispiel 1-32

### 6-(3-Pyridinyl)-3-spirobutyl-1,2,3,4-tetrahydro-9H-thioxanthen-9-on

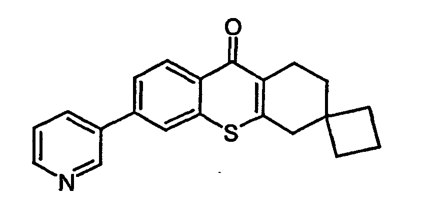

MS (CI+): 334 (M+H);
¹H-NMR (300 MHz, CDCl₃): δ = 1.81-2.02 (m, 8H); 2.70-2.77 (m, 4H); 7.40-7.45 (m, 1H); 7.66-7.70 (m, 2H); 7.92-7.96 (m, 1H); 8.59 (d, J=8.5Hz; 1H); 8.66-8.68 (dd, J=4.5Hz, 1.5Hz; 1H); 8.91 (d, J=2Hz; 1H).

### Beispiel 1-33

### 3-Ethyl-6-(4-pyridinyl)-1,2,3,4-tetrahydro-9H-thioxanthen-9-on

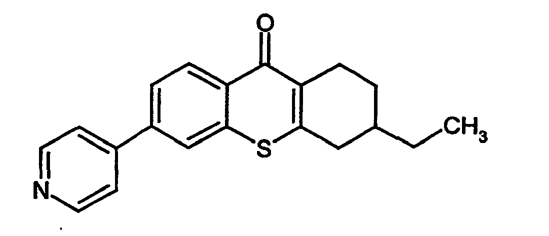

MS (CI+): 322 (M+H);
¹H-NMR (300 MHz, CDCl₃): δ = 1.01 (t, J=7.5Hz; 3H); 1.38-1.49 (m, 3H); 1.68-1.79 (m, 1H); 2.02-2.11 (m, 1H); 2.39-2.59 (m, 2H); 2.71-2.78 (m, 1H); 2.89-2.98 (m, 1H); 7.55-7.58 (m, 2H); 7.70-7.75 (m, 2H); 8.59-8.16 (d, J=8.5Hz; 1H); 8.72 (m, 2H).

### Beispiel 1-34

### 3-(3-Ethyl-9-oxo-2,3,4,9-tetrahydro-1H-thioxanthen-6-yl)-1,2,4-oxadiazol-5(4I-1)-on

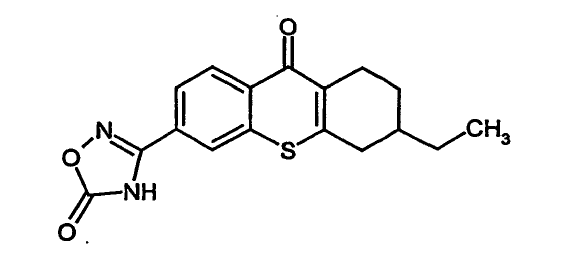

Unter Argon werden 100 mg (0,33 mmol) 3-Ethyl-N'-hydroxy-9-oxo-2,3,4,9-tetrahydro-1H-thioxanthen-6-carboximidamid (Beispiel I-7) und 0,03 ml (0,36 mmol) Pyridin in 2 ml DMF gelöst. Man kühlt die Lösung auf etwa 0°C, tropft 0,06 ml (0,33 mmol) Chlorameisensäure-2-ethylhexylester zu und rührt 30 min bei etwa 0°C nach. Anschließend gibt man den Ansatz auf etwa 50 ml Wasser gegeben, extrahiert dreimal mit je 50 ml Ethylacetat, vereinigt und trocknet die organischen Phasen und engt am Rotationsverdampfer ein.

Der verbleibende Rückstand wird in Xylol aufgenommen und 8h zum Sieden erhitzt.

Die nach Stehen bei Normaltemperatur über Nacht gebildete Suspension wird mit 20 ml Diethylether versetzt, das Ganze 5 min verrührt und dann der Feststoff abfiltriert, intensiv mit etwa 30 ml Diethylether gewaschen und getrocknet. Man erhält so 68,2 mg (63 %) der Zielverbindung.
MS (ESI): 329 (M+H);
¹H-NMR (300 MHz, DMSO): δ = 0.95 (t, J=7.5Hz; 3H); 1.34-1.44 (m, 3H); 1.61-1.73 (m, 1H); 1.92-2.01 (m, 1H); 2.33-2.46 (m, 2H); 2.70-2.82 (m, 2H); 7.93 (m, 1H); 8.17 (m, 1H); 8.44 (m, 1H); 13.15 (s, b, 1H).

In Analogie zur Vorschrift des Beispiels 1-34 wurden hergestellt:

### Beispiel 1-35

### 3-(12-Oxo-6,8,9,10,11,12-hexahydro-7H-cycloocta[b]thiochromen-3-yl)-1,2,4-oxadiazol-5(4H)-on

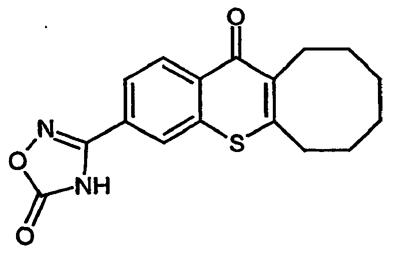

MS (ESI): 329 (M+H);
¹H-NMR (300 MHz, DMSO): δ = 1.34-1.49 (m, 4H); 1.54-1.64 (m, 2H); 1.74-1.82 (m, 2H); 2.86-2.93 (m, 4H); 3.25-3.36 (s, b, 1H); 7.93-7.97 (dd, J=8.5Hz, 1.5Hz, 1H); 8.19 (d, J=1.5Hz; 1H); 8.46 (d, J=8.5Hz; 1H).

### Beispiel 1-36

### 3-(3,3-Dimethyl-9-oxo-2,3,4,9-tetrahydro-1H-thioxanthen-6-yl)-1,2,4-oxadiazol-5(4H)-on

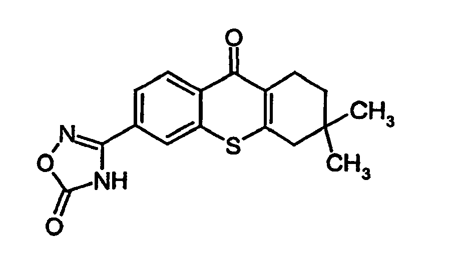

MS (ESI): 329 (M+H);
¹H-NMR (200 MHz, DMSO): δ = 1.00 (s, 6H); 1.59 (t, J=6.5Hz; 2H); 2.49-2.62 (m, 3H); 7.92-7.97 (dd, J=8.5Hz, 1.5Hz, 1H); 8.17 (s, 1H); 8.45 (d, J=8.5Hz; 1H), 13.15 (s, b, 1H).

### Beispiel 1-37

### 2-[(3-Ethyl-9-oxo-2,3,4,9-tetrahydro-1H-thioxanthen-6-yl)oxy]acetamid

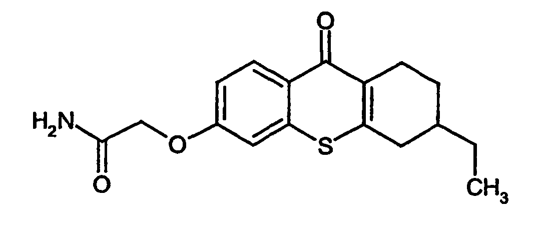

Unter Argon werden in 3 ml 2-Butanon 50 mg (0,19 mmol) 3-Ethyl-6-hydroxy-1,2,3,4-tetrahydro-9H-thioxanthen-9-on (Beispiel I-6), 53 mg (0,38 mmol) Bromacetamid, 188 mg (0,58 mmol) Cäsiumcarbonat und etwa 3 mg Kaliumiodid zusammengeben und das resultierende Reaktionsgemisch über Nacht zum Rückfluss erhitzt.

Zur Aufarbeitung wird der Ansatz mit 30 ml Ethylacetat versetzt, vom unlöslichen Feststoff abgetrennt, das Filtrat einrotiert und über präparative HPLC gereinigt.

Man erhält so 37,7mg (62 %) der Zielverbindung.
MS (ESI): 318 (M+H);
¹H-NMR (300 MHz, CDCl₃): δ = 0.99 (m, 3H); 1.34-1.46 (m, 3H); 1.72-1.78 (m, 1H); 1.97-2.07 (m, 1H); 2.33-2.56 (m, 2H); 2.64-2.73 (m, 1H); 2.84-2.94 (m, 1H); 4.58 (d, 2H); 5.70 (s, 1H); 6.50 (s, 1H); 6.93 (m, 1H); 7.05 (m, 1H); 8.46 (m, 1H).

In Analogie zur Vorschrift des Beispiels 1-37 wurden hergestellt:

### Beispiel 1-38

### [(3-Ethyl-9-oxo-2,3,4,9-tetrahydro-1H-thioxanthen-6-yl)-oxy]-acetonitril

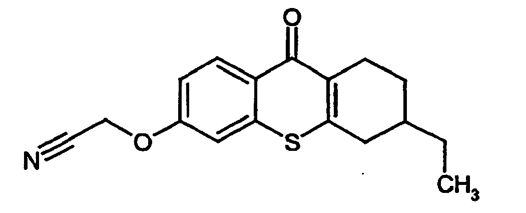

MS (CI+): 300 (M+H);
¹H-NMR (200 MHz, CDCl₃): δ = 0.99 (t, J=7.5Hz; 3H); 1.34-1.50 (m, 3H); 1.62-1.76 (m, 1H); 1.96-2.09 (m, 1H); 2.30-2.58 (m, 2H); 2.63-2.74 (m, 1H); 2.83-2.97 (m, 1H); 4.86 (s, 2H); 7.00 (d, J=2.5Hz; 1H); 7.06-7.12 (dd, J=9Hz, 2.5Hz; 1H); 8.49 (d, J=9Hz; 1H).

### Beispiel 1-39

### 6-Ethoxy-3-ethyl-1,2,3,4-tetrahydro-9H-thioxanthen-9-on

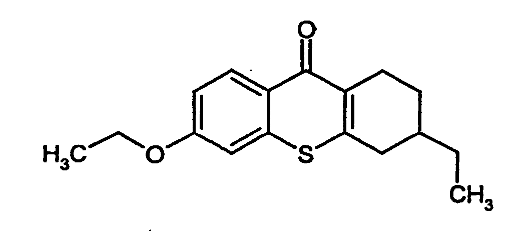

MS (CI+): 289 (M+H);
¹H-NMR (300 MHz, CDCl₃): δ = 0.99 (t, J=7.5Hz; 3H); 1.34-1.48 (m, 6H); 1.66-1.74 (m, 1H); 1.98-2.07 (m, 1H); 2.32-2.56 (m, 2H); 2.63-2.71 (m, 1H); 2.86-2.96 (m, 1H); 4.08-4.13 (quart., J=7Hz; 2H); 6.88 (d, J=2.5Hz; 1H); 6.99-7.03 (dd, J=9Hz, 2.5Hz; 1H); 8.39-8.43 (d, J=9Hz; 1H).

### Beispiel 1-40

### 3-Ethyl-9-oxo-2,3,4,9-tetrahydro-1H-thioxanthen-6-yl methansulfonat

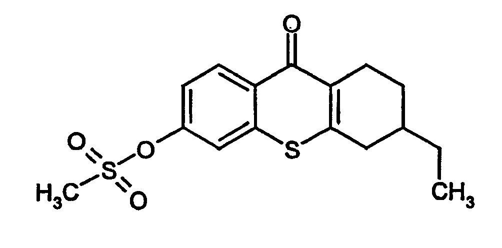

Unter Argon werden 70 mg (0,27 mmol) 3-Ethyl-6-hydroxy-1,2,3,4-tetrahydro-9H-thioxanthen-9-on (Beispiel I-6) in Dichlormethan vorgelegt, zunächst 43 mg (0,13 mmol) Tetra-n-butylammoniumbromid und 120 mg 45-prozentige Natronlauge, nach 10 min 34 mg (0,30 mmol) Methansulfonsäurechlorid zugegeben. Nach 1,5h Rühren bei Normaltemperatur werden zur Aufarbeitung 0,5 ml Puffer pH7 zugegeben, das resultierende Gemisch über eine Kartusche mit 1 g Extrelut/Kieselgel gesaugt, die Kartusche mit Ethylacetat nachgewaschen und das Eluat eingeengt.

Reinigung des Rohproduktes durch präparative HPLC ergibt 62,9 mg (69 %) der Zielverbindung.
MS (CI+): 339 (M+H);
¹H-NMR (300 MHz, CDCl₃): δ = 0.99 (t, J=7.5Hz; 3H); 1.36-1.48 (m, 3H); 1.64-1.77 (m, 1H); 2.00-2.09 (m, 1H); 2.36-2.57 (m, 2H); 2.67-2.76 (m, 1H); 2.86-2.95 (m, 1H); 3.22 (s, 3H); 7.32-7.36 (dd, J=9Hz, 2.5Hz; 1H); 7.48 (d, J=2.5Hz; 1H); 8.54-8.57 (d, J=9Hz; 1H).

In Analogie zur Vorschrift des Beispiels 1-40 wurden hergestellt:

### Beispiel 1-41

### 3-Ethyl-9-oxo-2,3,4,9-tetrahydro-1H-thioxanthen-6-yl 4,4,4-trifluor-1-butansulfonat

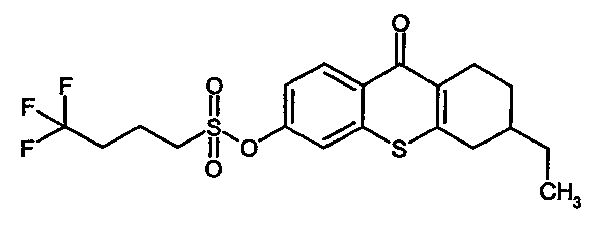

MS (CI+): 435 (M+H);
¹H-NMR (300 MHz, CDCl₃): δ = 0.99 (t, J=7.5Hz; 3H); 1.36-1.48 (m, 3H); 1.64-1.77 (m, 1H); 2.00-2.09 (m, 1H); 2.26-2.58 (m, 6H); 2.68-2.76 (m, 1H); 2.86-2.96 (m, 1H); 3.41 (t, J=7Hz; 2H); 7.30-7.33 (dd, J=9Hz, 2.5Hz; 1H); 7.45 (d, J=2.5Hz; 1H); 8.53-8.56 (d, J=9Hz; 1H).

### Beispiel 1-42

### 3-[(3-Ethyl-9-oxo-2,3,4,9-tetrahydro-1H-thioxanthen-6-yl)oxy]benzonitril

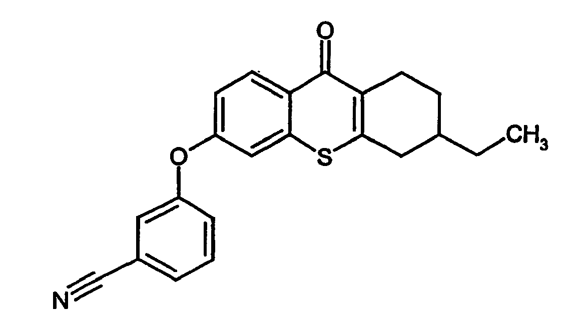

Unter Argon werden 250 mg (0,77 mmol) 6-Brom-3-ethyl-1,2,3,4-tetrahydro-9H-thioxanthen-9-on (Beispiel I-1), 276 mg (2,32 mmol) 3-Cyanophenol und 214 mg (1,55 mmol) Kaliumcarbonat in Pyridin vorgelegt und kurz auf 140°C erhitzt. Man lässt wieder leicht abkühlen und gibt 147 mg (0,77 mmol) Kupfer(I)jodid zu. Anschließend wird der Ansatz 48h bei etwa 140°C gerührt.

Zur Aufarbeitung wird das Pyridin am Rotationsverdampfer entfernt, dazu der zunächst verbleibende Rückstand noch zweimal mit Toluol aufgenommen und erneut eingeengt. Der Rückstand wird in Ethylacetat aufgenommen, das Ganze mit 5N Salzsäure extrahiert, mit Natriumhydrogencarbonatlösung und Wasser gewaschen. Nach Trocknen über Magnesiumsulfat und Einengen wird das ölige Rohprodukt auf 0,5 g Kieselgel gezogen und säulenchromatographisch an etwa 40 g Kieselgel mit einem Laufmittelgradienten Cyclohexan/Ethylacetat 20:1 bis 2:1 gereinigt. Man erhält so 95,3 mg (34,1%) der Zielverbindung.
MS (CI+): 362 (M+H);
¹H-NMR (300 MHz, CDCl₃): δ = 0.99 (t, J=7.5Hz; 3H); 1.34-1.47 (m, 3H); 1.64-1.76 (m, 1H); 1.98-2.07 (m, 1H); 2.34-2.57 (m, 2H); 2.64-2.73 (m, 1H); 2.86-2.96 (m, 1H); 7.03 (d, J=2Hz; 2H); 7.08-7.11 (m, 1H); 7.28-7.37 (m, 1H); 7.47-7.54 (m, 2H); 8.50-8.53 (m, 1H).

In Analogie zur Vorschrift des Beispiels 1-42 wurden hergestellt:

### Beispiel 1-43

### 3-[(3,3-Dimethyl-9-oxo-2,3,4,9-tetrahydro-1H-thioxanthen-6-yl)oxy]benzonitril

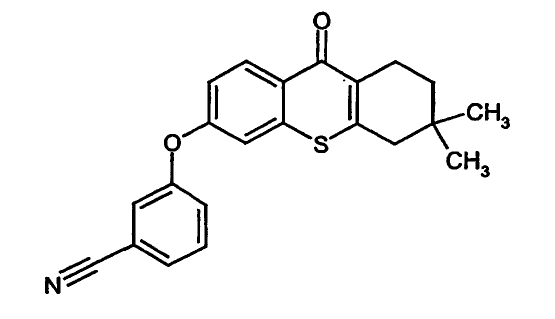

MS (EI+): 361 (M);
¹H-NMR (300 MHz, CDCl₃): δ = 1.04 (s, 6H); 1.63 (t, J=6.5Hz; 2H); 2.45 (s, 2H); 2.71 (t, J=6.5Hz; 2H); 7.03 (d, J 2.5Hz; 1H); 7.08-7.12 (dd, J=9Hz, 2.5Hz; 1H); 7.29-7.35 (m, 2H); 7.47-7.52 (m, 2H); 8.52 (d, J=9Hz; 1H).

In Analogie zur Vorschrift des Beispiels 1-3 werden hergestellt:

### Beispiel 1-44

### 3,3-Diethyl-6-(1H-tetrazol-5-yl)-1,2,3,4-tetrahydro-9H-thioxanthen-9-on

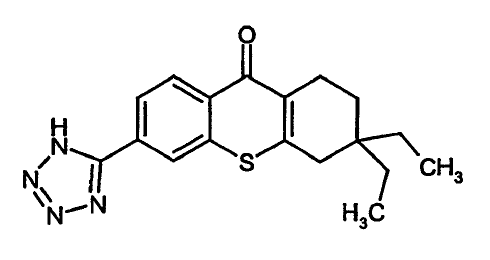

HPLC: Rₜ= 4.95 min;
¹H-NMR (400 MHz, DMSO): δ = 0.82 (t, 6H); 1.33 (mc, 4H); 1.60 (t; 2H); 2.45 - 2.60 (m, 4H); 3.1-3.6 (s, b, 1H); 8.17 (dd, 1H); 8.40 (s, 1H); 8.49 (d, 1H).

In Analogie zur Vorschrift des Beispiel 1-10 werden hergestellt:

### Beispiel 1-45

### 3,3-Diethyl-6-(2-thienyl)-1,2,3,4-tetrahydro-9H-thioxanthen-9-on

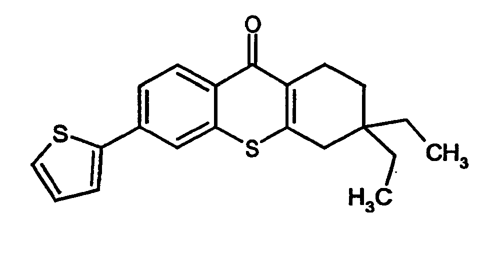

HPLC: Rₜ = 7.05 min;
MS (ESI+): 355 (M+H);
¹H-NMR (300 MHz, CDCl₃): δ = 0.83 (t, 6H); 1.2-1.5 (m, 4H); 1.65 (t, 2H); 2.7 (s, br, 2H); 2.67 (t, 2H); 7.14 (dd, 1H); 7.40 (dd, 1H); 7.46 (dd, 1H); 7.69 (s, 1H); 7.71 (dd, 1H); 8.49 (d, 1H).

### Beispiel 1-46

### 6-(2-Thienyl)-3-spirohexyl-1,2,3,4-tetrahydro-9H-thioxanthen-9-on

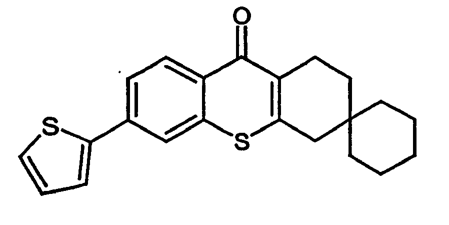

MS (EI+): 366 (M);
HPLC: Rₜ= 6.55 min;
¹H-NMR (200 MHz, CDCl₃): δ = 1.3 - 1.6 (m, 10H); 1.71 (t, 2H); 2.53 (s, br, 2H); 2.69 (t, br, 2H); 7.14 (dd, 1H); 7.40 (dd, 1H); 7.46 (dd, 1H); 7.65 - 7.75 (m, 2H); 8.49 (d, 1H).

### Beispiel 1-47

### 6-(3-Cyanophenyl)-3,3-diethyl-1,2,3,4-tetrahydro-9H-thioxanthen-9-on

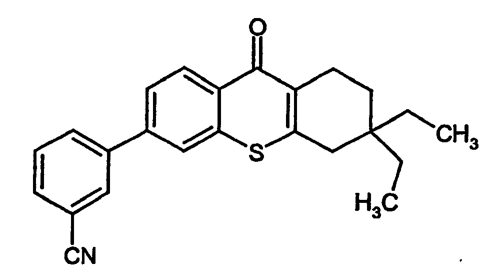

MS (ESI+): 374 (M+H);
HPLC: Rₜ = 5.59 min;
¹H-NMR (300 MHz, CDCl₃): δ = 0.87 (t, 6H); 1.23 - 1.52 (m, 4 H); 1.67 (t, 2H); 2.50 (s, br, 1H); 2.69 (t, 2H); 7.55 - 7.75 (m, 4H); 7.88 (dt, 1H); 7.90 - 7.95 (m, 1H); 8.60 (d, 1H).

### Beispiel 1-48

### 6-(3-Cyanophenyl)-3-spirohexyl-1,2,3,4-tetrahydro-9H-thioxanthen-9-on

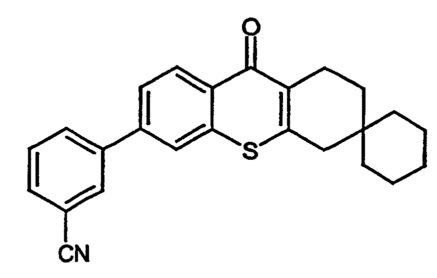

MS (EI+): 385 (M);
HPLC: Rₜ= 6.11 min;
¹H-NMR (200 MHz, CDCl₃): δ = 1.35 - 1.6 (m, 10H); 1.73 (t, 2H); 2.56 (s, br, 2H); 2.71 (t, br, 2H); 7.55 - 7.76 (m, 4H); 7.83 - 7.96 (dd, 2H); 8.59 (dd, 1H).

### Beispiel 1-49

### 6-(Hydroxymethyl)-3,3-dimethyl-1,2,3,4-tetrahydro-9H-thioxanthen-9-on

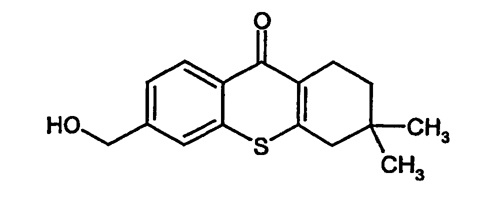

Zu einer Lösung von 500 mg (1.73 mmol) 3,3-Dimethyl-9-oxo-2,3,4,9-tetrahydro-1H-thioxanthen-6-carbonsäure und 844 mg (1.91 mmol) Benzotriazolyloxy-tris(dimethylamino)-phosphonium-hexafluorophosphat in 8.5 ml Tetrahydrofuran gibt man unter Argon 269 mg (1.19 mmol) Diisopropylethylamin und nach 5 min Rühren bei Normaltemperataur Natriumborhydrid. Nach 80 min wird das Lösungsmittel im Vakuum entfernt, der Rückstand in Diethylether aufgenommen und die organische Phase nacheinander mit 1N Salzsäure, gesättigter Natriumhydrogen-carbonatlösung und gesättigter Kochsalzlösung gewaschen, über Magnesiumsulfat getrocknet und im Vakuum eingeengt. Durch Chromatographie an Kieselgel (Laufmittel Cyclohexan/Essigsäureethylester-Gradient 5:1 - 2:1) erhält man 302 mg (63%) der Zielverbindung.
MS (ESI+): 275 (M+H);
HPLC: Rₜ = 4.47 min;
¹H-NMR (300 MHz, CDCl₃): δ =1.04 (s, 6H); 1.63 (t, 2H); 1.94 (s, br, 1H); 2.47 (s, br, 2H); 2.72 (t, 2H); 7.41 (d, br, 1H); 7.53 (s, br,1H); 8.46 (d,1H).

### Beispiel 1-50

### 6-(Chlormethyl)-3,3-dimethyl-1,2,3,4-tetrahydro-9H-thioxanthen-9-on

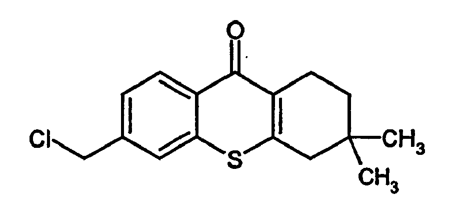

1.00 g (3.64 mmol) 6-(Hydroxymethyl)-3,3-dimethyl-1,2,3,4-tetrahydro-9H-thioxanthen-9-on und 1.91 g (7.29 mmol) Triphenylphosphin werden in 7.2 ml Dichlormethan vorgelegt und die Mischung in einem Trockeneis/Acetonbad auf minus 10°C gekühlt. Dann gibt man zu der sich bildenden farblosen Suspension 1.12 g (7.29 mmol) Tetrachlorkohlenstoff und lässt die Reaktion über Nacht rühren und dabei auftauen. Man verdünnt mit Dichlormethan, wäscht mit gesättigter Natriumhydrogencarbonatlösung, Wasser, sowie mit gesättigter Natriumchoridlösung und trocknet über Magnesiumsulfat. Das Solvens wird im Vakuum entfernt, der Rückstand an Kieselgel adsorbiert und über Kieselgel filtriert (Laufmittel: Cyclohexan/Essigsäureethylester 5:1).
Man erhält so 1.02 g (95%) der Zielverbindung.
MS (ESI+): 293 (M+H);
HPLC: Rₜ= 5.35 min;
¹H-NMR (300 MHz, CDCl₃): δ = 0.99 (s, 6H); 1.63 (t, 2H); 2.48 (s, 2H); 2.72 (t, 2H); 4.64 (s, 2H); 7.47 (dd, 1H); 7.53 (s, br, 1H); 8.49 (d, 1H).

### Beispiel 1-51

### 2-(3,3-Dimethyl-9-oxo-2,3,4,9-tetrahydro-1H-thioxanthen-6-yl)-N-isobutyl-N-methyl-essigsäureamid

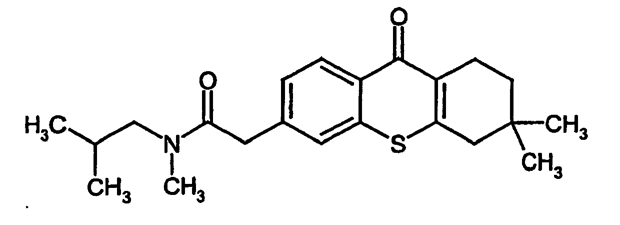

Zu einer Lösung von 50mg (0.17 mmol) (3,3-Dimethyl-9-oxo-2,3,4,9-tetrahydro-1H-thioxanthen-6-yl)essigsäure und 43 mg (0.33 mmol) Diisopropylethylamin gibt man unter Eiskühlung 26 mg (0.18 mmol) 1-Hydroxy-1H-benzotriazol-Hydrat und danach 36 mg (0.18 mmol) N-(3-Dimethylaminopropyl)-N'-ethylcarbodiimid-Hydrochlorid. Man lässt die Reaktion 15 min nachrühren und gibt dann 16 mg (0.18 mmol) N-Isobutyl-N-methylamin hinzu. Die Mischung wird 19h gerührt wobei man das Gemisch auf Normaltemperatur kommen lässt. Zur Aufarbeitung wird der Ansatz mit Dichlormethan verdünnt, mit Wasser sowie gesättigter Natriumchoridlösung gewaschen und über Magnesiumsulfat getrocknet. Entfernen des Lösungsmittels im Vakuum und Chromatografie (präparative HPLC, Acetonitril/Wasser 30:70 - 95:5) liefert 54 mg (88%) der Zielverbindung.
MS (ESI+): 372 (M+H);
HPLC: Rₜ = 4.95 min;
¹H-NMR (400 MHz, CDCl₃): δ = 0.87, 0.90 (2 d, zusammen 6H); 1,03 (s, 6H); 1,62 (t, 2H) 1,96 (mc, 1H), 2,46 (s, 2H), 2,71 (t, 2H); 2.96, 2.97 (2 s, zusammen 3H); 3.11, 3.23 (2 d, zusammen 2H), 3.79, 3.80 (2 s, zusammen 2H); 7.30 - 7.40 (2 dd, zusammen 1H); 7.43, 7.44 (2 s , zusammen 1H); 8.45, 8.45 (2 d, zusammen 1H).

### Ausgangsverbindungen II

### HPLC-Methoden:

- Methode A:: Eluent:A = 0.5 % HClO₄ in Wasser, B = Acetonitril; Gradient: 0.5 min 98 % A, 2 % B, 4.5 min 10%A, 90% B, 6.7 min 98% A,2 % B; Fluss: 0.75 ml/min; Säulentemperatur: 30
- °C; UV-Detektion:: 210 nm; Säule: Kromasil C18 (60*2 mm)
- Methode C:: Eluent: A = 0.1 % Ameisensäure in Acetonitril, B = 0.1 % Ameisensäure in Wasser; Gradient: 0 min 10 % A, 90 % B, 4.0 min 90 % A, 10 % B, 6.1 min 10 %A, 90 % B, 7.50 min 10 % A, 90 % B; Fluss: 0.5 ml/min 0.00 - 6.10 min, .0 ml/min 6.10 - 7.5 min; Säulentemperatur: 40°C;- UV-Detektion: 208-400 nm; Säule: Symmetry C18 (50*2.1 mm), 3.5 µm
- Methode D:: Eluent:A = Acetonitril, B = 0.3 g 30 % HCl in 11 Wasser; Gradient: 3 min 90 % B, 10 % A, Fluss 0.9 ml/min, 6 min 90 % A, 10 % B, Fluss 1.2 ml/min, Säulentemperatur: 50°C; Säule: Symmetry C18 (150*2. mm); andere Parameter siehe Methode A

### Beispiel II-1

### 6-(Benzyloxy)-3-ethyl-1,2,3,4-tetrahydro-9H-thioxanthen-9-on

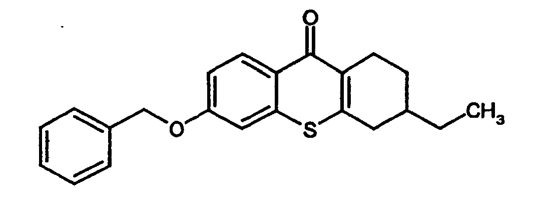

60 mg (0.23 mmol) 3-Ethyl-6-hydroxy-1,2,3,4-tetrahydro-9H-thioxanthen-9-on, 39.4 mg (0.23 mmol) Benzylbromid und 35 mg (0.25 mmol) gepulvertes Kaliumcarbonat werden in 2 ml Acetonitril unter Argon bei RT über Nacht zum Rückfluss erhitzt. Nach Einengen wird mit Dichlormethan versetzen, mit verdünnter Salzsäure und verdünnter Natronlauge gewaschen und die organische Phase über Magnesiumsulfat getrocknet. Nach Einengen erhält man das Rohprodukt als Öl, das im Hochvakuum von Lösemittelresten befreit wird. Nach Anreiben mit wenig Isopropanol erhält man einen Feststoff, der an der Luft getrocknet wird; Ausbeute 37,3 mg (46 %), Schmp. 94°C
MS (ESI): 351 (M+H)⁺
HPLC: 97,4 %, Retentionszeit 6.06 min (Methode A)

In Analogie zur Vorschrift des Beispiels II-1 wurden hergestellt:

### Beispiel II-2

### 3-Ethyl-6-(2-phenylethoxy)-1,2,3,4-tetrahydro-9H-thioxanthen-9-on

Ausbeute: 42 %, Schmp. 105°C, HPLC: 100 %, MS (ESI): 365 (M+H)⁺

### Beispiel II-3

### 3-{[(3-Ethyl-9-oxo-2,3,4,9-tetrahydro-1H-thioxanthen-6-yl)oxy]methyl} benzonitril

Ausbeute: 72 %, Schmp. 158°C, HPLC: 100 %, MS (ESI): 375 (M)⁺

### Beispiel II-4a und Beispiel II-4b

### N-(3-Ethyl-9-oxo-2,3,4,9-tetrahydro-1H-thioxanthen-6-yl)acetamid und 6-Amino-3-ethyl-1,2,3,4-tetrahydro-9H-thioxanthen-9-on

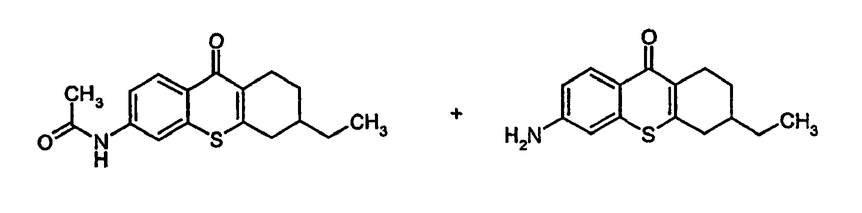

15 ml Polyphosphorsäure, 1.11 g (6.6 mmol) N-(3-Mercaptophenyl)acetamid und 1.45 g (7.3 mmol) Ethyl 4-ethyl-2-oxocyclohexancarboxylat werden unter Argon 30 Minuten auf 90°C erhitzt. Nach Abkühlen auf Raumtemperatur wird mit 100 ml Eiswasser versetzt und 30 Minuten gerührt. Extraktive Aufarbeitung mit Ethylacetat und Waschen der organischen Phase mit 1 N Natronlauge sowie gesättigter Kochsalzlösung ergibt nach Trocknen über Natriumsulfat und Einengen ein Rohprodukt, das durch säulenchromatographische Trennung (Kieselgel, Cyclohexan/Ethylacetat-Gemische) gereinigt wird. Man erhält 135 mg (7 %) N-(3-Ethyl-9-oxo-2,3,4,9-tetrahydro-1H-thioxanthen-6-yl)acetamid als Feststoff, Schmp. 243°C.
HPLC: 99 %, Retentionszeit 4.61 min (Methode A),
MS (ESI): 302 (M+H)⁺

Als weitere Fraktion werden 77 mg (4.5 %) 6-Amino-3-ethyl-1,2,3,4-tetrahydro-9H-thioxanthen-9-on als farbloser Feststoff, Schmp. 187°C, erhalten.
HPLC: 100 %, Retentionszeit 4.51 min (Methode A),
MS (ESI): 260 (M+H)⁺

In Analogie zur Vorschrift der Beispiele II-4a und II-4b wurden hergestellt:

### Beispiel II-5

### 6-Amino-3,3-dimethyl-1,2,3,4-tetrahydro-9H-thioxanthen-9-on

Die Titelverbindung wird aus 3-Amino-thiophenol und 4,4-Dimethyl-2-oxocyclohexan-carbonsäure-ethylester in Polyphosphorsäure (43 %) erhalten.
Schmp. 210°C.
HPLC: 100 %, Retentionszeit 4.44 min (Methode A),
MS (EI pos): 259 (M)⁺

### Beispiel II-6

### N-(3-Ethyl-9-oxo-2,3,4,9-tetrahydro-1H-thioxanthen-6-yl)benzolsulfonamid

Die Titelverbindung wird analog zu Beispiel 2-11 aus Benzolsulfonsäurechlorid und 6-Amino-3-ethyl-1,2,3,4-tetrahydro-9H-thioxanthen-9-on erhalten.
Ausbeute: 76 % farblose Kristalle.
Schmp. 264°C
HPLC: 100 %, Retentionszeit 5.20 min (Methode A),
MS (ESI): 400 (M+H)⁺

### Ausführungsbeispiele 2

### Beispiel 2-1

### 2-{4-[(3-Ethyl-9-oxo-2,3,4,9-tetrahydro-1H-thioxanthen-6-yl)oxy]butyl}-1,2-benzisothiazol-3(2H)-on 1,1-dioxid

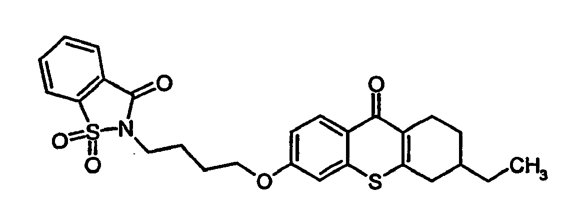

Unter Argon werden 60 mg (0.23 mmol) 3-Ethyl-6-hydroxy-1,2,3,4-tetrahydro-9H-thioxanthen-9-on in 1 ml Tetrahydrofuran gelöst, mit 0.34 ml einer 1M Lösung von Kaliumtertiärbutylat in Tetrahydrofuran versetzt und 30 min bei Raumtemperatur geschüttelt. Es werden 110 mg (0.35 mmol) 2-(4-Bromobutyl)-1,2-benzisothiazol-3(2H)-on 1,1-dioxid zugegeben; die Lösung wird über Nacht bei 65°C geschüttelt.

Nach Abkühlen auf Raumtemperatur werden ca 130 mg PS-Thiophenol (Fa. Argonaut, 1.4 mmol/g) zugegeben und 30 min bei Raumtemperatur geschüttelt. Die Lösung wird filtriert, der Feststoff mit 0.5 ml Dimethylformamid nachgewaschen und das Filtrat wird im Hochvakuum eingeengt.

Die Reinigung erfolgt durch präparative HPLC (Reverse Phase, Acetonitril/Wasser-Gemische).
Ausbeute 22.4 mg (20 %)
MS (ESI+): 498 (M+H)⁺

Auf gleiche Weise werden die in der folgenden Tabelle aufgeführten Verbindungen erhalten:

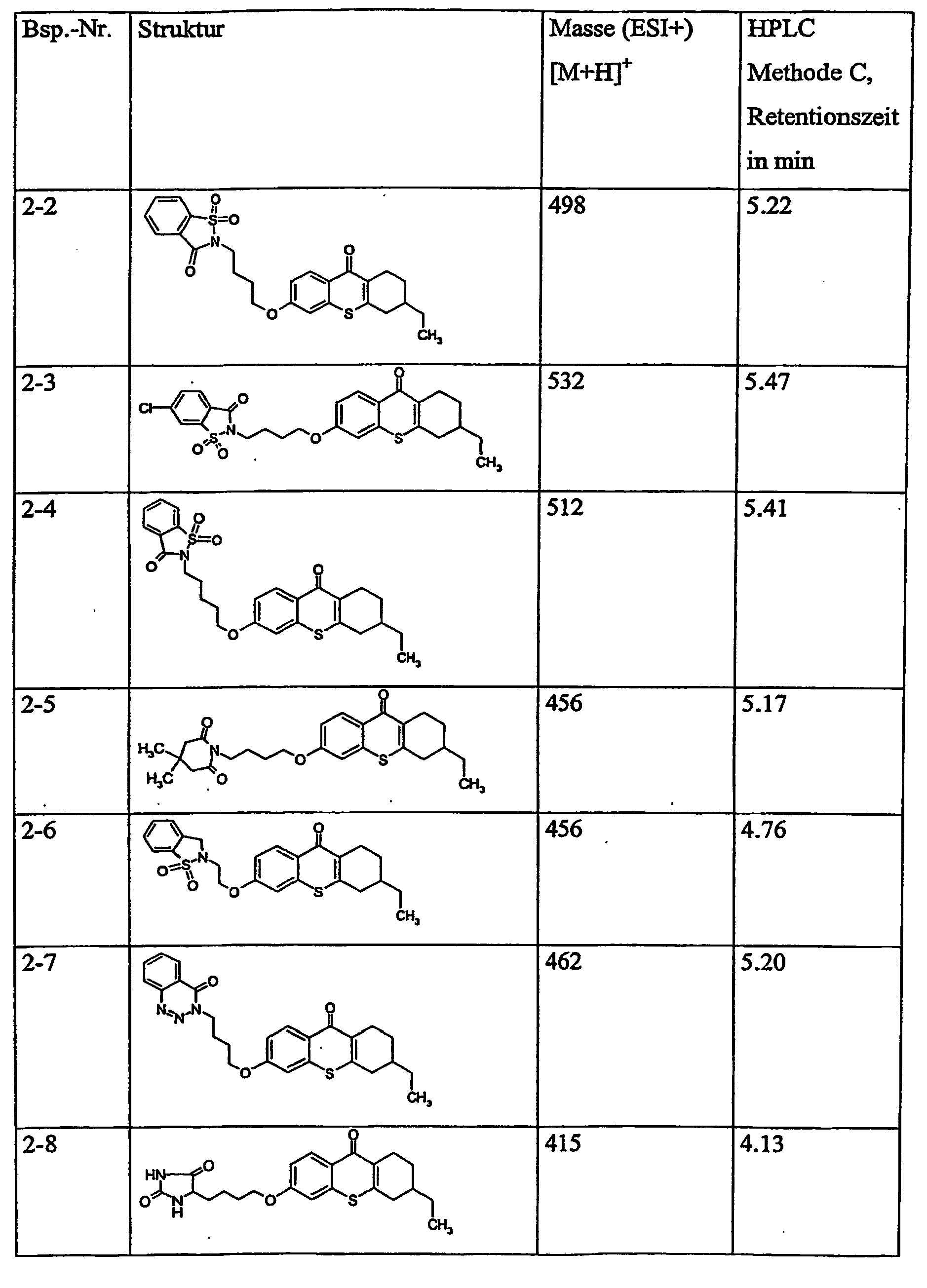

### Beispiel 2-9

### (3-Ethyl-9-oxo-2,3,4,5-tetrahydro-1H-thioxanthen-6-yl)-2-(1-naphthyl)ethansulfonat

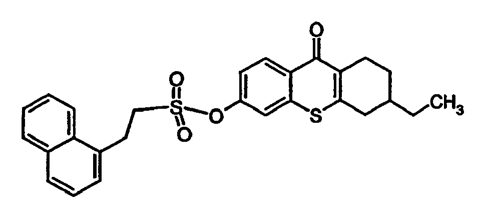

Die Lösung von 50 mg (0.19 mmol) 3-Ethyl-6-hydroxy-1,2,3,4-tetrahydro-9H-thioxanthen-9-on in 1 ml Dichlormethan wird mit 31 mg (0.1 mmol) Tetrabutylammmoniumbromid und 86 mg 45 % wässrige Natronlauge versetzt . Nach 10 Minuten Rühren bei Raumtemperatur werden 59 mg (0.23 mmol) 2-Naphthylethansulfonsäurechlorid zugegeben. Nach 1.5 Stunden Rühren bei Raumtemperatur wird mit Dichlormethan verdünnt und die organische Phase mit Wasser gewaschen. Filtration über Kieselgel, Einengen und Chromatographie des Rohproduktes (Kieselgel, Toluol/Ethylacetat 10/1) ergibt die Titelverbindung (37 %)
HPLC: 97 %, Retentionszeit 5.92 min (Methode A),
MS (ESI): 479 (M+H)⁺

In Analogie zur Vorschrift des Beispiels 2-9 werden erhalten:

### Beispiel 2-10

### 3-Ethyl-9-oxo-2,3,4,9-tetrahydro-1H-thioxanthen-6-yl dimethylsulfamat

Aus Dimethylamidosulfonylchlorid; Ausbeute: 57 %
HPLC: 100 %, Retentionszeit 5.23 min (Methode A),
MS (ESI): 368 (M+H)⁺

### Beispiel 2-11

### N-(3,3-Dimethyl-9-oxo-2,3,4,9-tetrahydro-1H-thioxanthen-6-yl)methansulfonamid

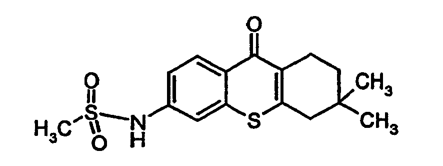

Die Lösung von 75 mg (0.3 mmol) 6-Amino-3,3-dixriethyl-1,2,3,4-tetrahydro-9H-thioxanthen-9-on und 4 mg 4-Dimethylaminopyridin in 1.5 ml Dichlormethan wird bei 0°C mit 0.12 ml Pyridin und 0.03 ml (0.35 mmol) Methansulfonsäurechlorid versetzt. Nach 3 h Rühren bei Raumtemperatur wird mit Dichlormethan verdünnt und wässrig aufgearbeitet (1N Salzsäure, Wasser, gesättigte Kochsalzlösung). Nach Trocknen über Natriumsulfat wird die organische Phase eingeengt. Der verbleibende Feststoff wird aus Ethylacetat umkristallisiert. Die Kristalle werden mit wenig Pentan nachgewaschen und im Vakuum getrocknet.
Ausbeute: 28 mg (29 %) farblose Kristalle.
Schmp. 272°C
HPLC: 100 %, Retentionszeit 4.61 min (Methode A),
MS (ESI): 338 (M+H)⁺
In Analogie zur Vorschrift des Beispiels 2-11 werden erhalten:

### Beispiel 2-12

### N-(3,3-Dimethyl-9-oxo-2,3,4,9-tetrahydro-1H-thioxanthen-6-yl)benzolsulfonamid

Aus Benzolsulfonsäurechlorid; Ausbeute: 73 % farblose Kristalle.
Schmp. 231°C
HPLC: 100 %, Retentionszeit 5.01 min (Methode A),
MS (ESI): 400 (M+H)⁺

### Beispiel 2-13

### N-(3,3-Dimethyl-9-oxo-2,3,4,9-tetrahydro-1H-thioxanthen-6-yl)-4-methylbenzolsulfonamid

Aus 4-Methylbenzolsulfonsäurechlorid; Ausbeute: 60 % farblose Kristalle.
Schmp. 279°C
HPLC: 100%, Retentionszeit 5.18 min (Methode A),
MS (ESI): 414 (M+H)⁺

### Beispiel 2-14

### 3,3-Dimethyl-9-oxo-2,3,4,9-tetrahydro-1H-thioxanthen-6-yl-[2-(trifluormethoxy)-phenyl]methansulfonat

Aus 2-(Trifluormethoxy)phenyl]Methansulfonsäurechlorid; Ausbeute: 55 % farblose Kristalle.
Schmp. 249°C
HPLC: 100%, Retentionszeit 5.32 min (Methode A),
MS (ESI): 498 (M+H)⁺

### Beispiel 2-15

### N-(3,3-Dimethyl-9-oxo-2,3,4,9-tetrahydro-1H-thioxanthen-6-yl)-isopropyl-sulfonamid

Aus Isopropylsulfonsäurechlorid (dreifache Menge); Ausbeute: 14 % farblose Kristalle.
Schmp. 263°C
HPLC: 100%, Retentionszeit 4.85 min (Methode A),
MS (ESI): 366 (M+H)⁺

### Beispiel 2-16

### N-(3,3-Dimethyl-9-oxo-2,3,4,9-tetrahydro-1H-thioxanthen-6-yl)-N-methylbenzolsulfonamid

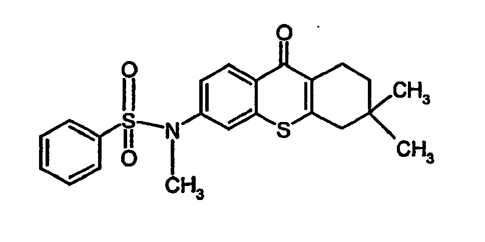

Die Lösung von 41 mg (0.1 mmol) N-(3,3-Dimethyl-9-oxo-2,3,4,9-tetrahydro-1H-thioxanthen-6-yl)benzolsulfonamid in 1 ml Tetrahydrofuran wird mit 7.4 ml 40% Natriumhydrid in Paraffinöl versetzt und 1 Stunde bei Raumtemperatur unter Argon gerührt. Es werden 16 mg (0.11 mmol) Methyliodid zugegeben. Die Mischung wird über Nacht bei 60°C gerührt. Anschließend wird noch einmal die gleiche Menge Methyliodid zugesetzt. Nach weiteren 5 Stunden bei 60°C wird wässrig aufgearbeitet (Ethylacetat, Wasser, ges. Kochsalzlösung, Trocknen über Natriumsulfat). Das Rohprodukt wird chromatographisch gereinigt (Kieselgel, Toluol/Ethylacetat 15:1). Ausbeute: 24 mg (53 %).
HPLC: 92%, Retentionszeit 5.28 min (Methode A),
MS (ESI): 414 (M+H)⁺

### Beispiel 2-17

### N-(3-Ethyl-9-oxo-2,3,4,9-tetrahydro-1H-thioxanthen-6-yl)-N-methylbenzolsulfonamid

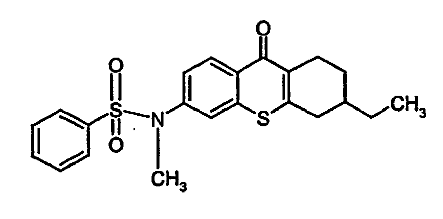

Die Lösung von 41 mg (0.1 mmol) N-(3-Ethyl-9-oxo-2,3,4,9-tetrahydro-1H-thioxanthen-6-yl)benzolsulfonamid in 2 ml Dimethylformamid wird mit 22 mg Methyliodid, 3 mg Tetrabutylammoniumhydrogensulfat und 1 g Kaliumcarbonat versetzt und 3 Tage bei Raumtemperatur gerührt. Wässrige Aufarbeitung (Ethylacetat, Wasser, ges. Kochsalzlösung, Trocknen über Natriumsulfat) ergibt ein Rohprodukt, das chromatographisch gereinigt wird (Kieselgel, Toluol/Ethylacetat 10:1).
Ausbeute: 27 mg (64 %).
HPLC: 9 8%, Retentionszeit 5.44 min (Methode A),
MS (DCI NH3): 414 (M+H)⁺

### Beispiel 2-18

### N-(Cyclopropylmethyl)-N-(3-ethyl-9-oxo-2,3,4,9-tetrahydro-1H-thioxanthen-6-yl)-benzolsulfonamid

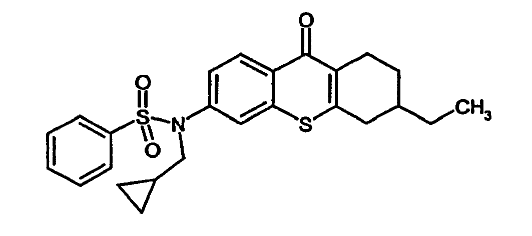

126 mg 60% Natriumhydrid werden unter Argon mit Pentan von Mineralöl befreit. Das gewaschene Natriumhydrid wird mit 7 ml Dimethylformamid versetzt, dann werden 1.05 g N-(3-Ethyl-9-oxo-2,3,4,9-tetrahydro-1H-thioxanthen-6-yl)benzolsulfonamid zugegeben. Die Mischung wird über Nacht bei Raumtemperatur gerührt. Die Lösung wird mit Dimethylformamid auf 10.5 ml Gesamtvolumen aufgefüllt.

0.5 ml vorstehender Lösung (=0.125 mmol Natriumsalz des Sulfonamids) werden mit einer Lösung von 25 mg (0.19 mmol) Cyclopropylmethylbromid in 0.5 ml Dimethylformamid versetzt und über Nacht bei 80°C geschüttelt. Nach Abkühlen wird mit 130 mg PS-Thiophenol (Fa. Argonaut, 1.41 mmol/g) versetzt und 1h bei Raumtemperatur geschüttelt. Waschen mit Dimethylformamid und Einengen des Fitrats im Hochvakuum ergibt ein Rohprodukt, das durch HPLC (Reverse Phase, Acetonitril/Wasser) gereinigt wird.
Ausbeute: 7 mg (13 %).
HPLC: 98 %, Retentionszeit 3.78 min (Methode D),
MS (ESI): 454 (M+H)⁺

In Analogie zur Vorschrift des Beispiel 2-18 werden erhalten:

### Beispiel 2-19

### N-(3-Ethyl-9-oxo-2,3,4,9-tetrahydro-1H-thioxanthen-6-yl)-N-(2-methoxyethyl)-benzolsulfonamid

Aus 2-Methoxyethylbromid; Ausbeute: 41 %
HPLC: 94 %, Retentionszeit 3.54 min (Methode D),
MS (ESI): 458 (M+H)⁺

### Beispiel 2-20

### N-(3-Ethyl-9-oxo-2,3,4,9-tetrahydro-1H-thioxanthen-6-yl)-N-(phenylsulfonyl)glycin-methylester

Aus Bromessigsäuremethylester, Ausbeute: 54 %
HPLC: 100 %, Retentionszeit 3.43 min (Methode D),
MS (ESI): 472 (M+H)⁺

### Beispiel 2-21

### N-(3-Ethyl-9-oxo-2,3,4,9-tetrahydro-1H-thioxanthen-6-yl)-N-(2-phenyl-ethyl)-benzolsulfonamid

Aus 2-Phenylethylbromid; Ausbeute: 20 %
HPLC: 97 %, Retentionszeit 3.94 min (Methode D),
MS (ESI): 504 (M+H)⁺

### Beispiel 2-22

### N-(3-Ethyl-9-oxo-2,3,4,9-tetrahydro-1H-thioxanthen-6-yl)-N-propyl-benzolsulfonamid

Aus 1-Propylbromid; Ausbeute: 22 %
HPLC: 99 %, Retentionszeit 3.81 min (Methode D),
MS (ESI): 442 (M+H)⁺

### Beispiel 2-23

### N-(3-Ethyl-9-oxo-2,3,4,9-tetrahydro-1H-thioxanthen-6-yl)-N-ethyl-benzolsulfonamid

Aus Ethylbromid; Ausbeute: 14 %
HPLC: 96 %, Retentionszeit 3.67 min (Methode D),
MS (ESI): 428 (M+H)⁺

### Beispiel 2-24

### 3,3-Dimethyl-6-(1H-pyrrol-1-yl)-1,2,3,4-tetrahydro-9H-thioxanthen-9-on

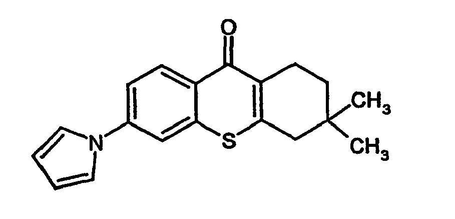

Die Lösung von 136 mg (0.53 mmol) 6-Amino-3,3-dimethyl-1,2,3,4-tetrahydro-9H-thioxanthen-9-on in 1.5 ml Eisessig wird mit 83 mg (0.63 mmol) 2,5-Dimethoxytetrahydrofuran versetzt und auf 120°C erhitzt. Dünnschichtchromatographisch ist nach kurzer Zeit kein Edukt mehr nachweisbar. Die Reaktionsmischung wird auf Raumtemperatur abgekühlt, mit Ethylacetat verdünnt und wässrig aufgearbeitet (Wasser, Natriumhydrogencarbonat, Wasser, gesättigte Kochsalzlösung). Das nach Trocknen und Einengen erhaltene Rohprodukt wird durch Säulenchromatographie (Kieselgel, Cyclohexan/Ethylacetat Gemische) gereinigt. Der erhaltene Feststoff wird mit Cyclohexan digeriert. Ausbeute: 69 mg (42%) farblose Kristalle.
Schmp. 169°C
HPLC: 100 %, Retentionszeit 5.34 min (Methode A),
MS (DCI, NH₃): 310 (M+H)⁺

In Analogie zur Vorschrift des Beispiels 2-24 erhält man

### Beispiel 2-25

### 3-Ethyl-6-(1H-pyrrol-1-yl)-1,2,3,4-tetrahydro-9H-thioxanthen-9-on

Aus 6-Amino-3-ethyl-1,2,3,4-tetrahydro-9H-thioxanthen-9-on; Ausbeute: 28 % farblose Kristalle.
Schmp. 145°C
HPLC: 99 %, Retentionszeit 5.49 min (Methode A),
MS (DCI, NH₃): 310 (M+H)⁺

### Ausführungsbeispiele 3

Die Bestimmung der Retentionszeit der Herstellungsbeispiele mit HPLC erfolgte unter folgenden Bedingungen.

Säule: Chromasil C18 60*2; Injektionsvolumen 1.00 µl; Fluss: 0,75 ml/min; Eluent: A= 5 ml HClO₄/l H₂O, B= CH₃CN; Gradient [t(min): A/B]: 0,5: 98/2; 4,5: 10/90; 6,5: 10/90; 6,7: 98/2; 7,5: 98:2.

### LC-MS Methode 1:

| | | | | | | |
|---|---|---|---|---|---|---|
| Methode: | MHZ 2Q | | | | | |
| Version-Nr.: | 3 | | | | | |
| Gerätetyp MS: | Micromass Quattro LCZ | | | | | |
| | Ionisierung: | | ESI positiv /negativ | | | |
| Gerätetyp HPLC: | HP 1100 | | | | | |
| | UV-Detektor DAD: | | 208-400nm | | | |
| | Ofentemp.: | | 40°C | | | |
| Säule: | Symmetry C 18 | | | | | |
| | 50 mm x 2,1 mm 3,5 µm | | | | | |
| Lieferfirma: | Waters | | | | | |

| Gradient: | Time | A:% | B:% | C:% | D:% | Flow |
|---|---|---|---|---|---|---|
| | 0, 00 | 10, 0 | 90, 0 | -- | -- | 0, 50 |
| | 4, 00 | 90, 0 | 10, 0 | -- | -- | 0, 50 |
| | 6, 00 | 90, 0 | 10, 0 | -- | -- | 0, 50 |
| | 6, 10 | 10, 0 | 90, 0 | -- | -- | 1, 00 |
| | 7, 50 | 10, 0 | 90, 0 | -- | -- | 0, 50 |
| A: | CH3CN + 0,1 %Ameisensäure | | | | | |
| B: | H2O + 0,1 %Ameisensäure | | | | | |
| C: | -- | | | | | |
| D: | -- | | | | | |

### LC-MS Methode 2:

| | | | | | | |
|---|---|---|---|---|---|---|
| Methode: | MHZ 2P | | | | | |
| Version-Nr.: | 3 | | | | | |
| Gerätetyp MS: | Micromass Platform LCZ | | | | | |
| | Ionisierung: | ESI positiv /negativ | | | | |
| Gerätetyp HPLC: | HP 1100 | | | | | |
| | UV-Detektor DAD : | 208-400 nm | | | | |
| | Ofentemp.: | 40°C | | | | |
| Säule: | Symmetry C 18 | | | | | |
| | 50 mm x 2,1 mm 3,5 µm | | | | | |
| Lieferfirma: | Waters | | | | | |

| Gradient: | Time | A:% | B:% | C:% | D:% | Flow |
|---|---|---|---|---|---|---|
| | 0, 00 | 10, 0 | 90, 0 | -- | -- | 0, 50 |
| | 4, 00 | 90, 0 | 10, 0 | -- | -- | 0, 50 |
| | 6, 00 | 90, 0 | 10, 0 | -- | -- | 0, 50 |
| | 6, 10 | 10, 0 | 90,0 | -- | -- | 1,00 |
| | 7, 50 | 10, 0 | 90, 0 | -- | -- | 0, 50 |
| A: | CH3CN + 0,1%Ameisensäure | | | | | |
| B: | H2O + 0,1%Ameisensäure | | | | | |
| C: | -- | | | | | |
| D: | -- | | | | | |

### LC-MS Methode 3

Säule: Symmetry C18 150*2,1; Injektionsvolumen 2.00 µl; Eluent: A= CH₃CN, B= 0,3 g HCl (30%)/l H₂O; Gradient [t(min): A/B]: 0: 10/90 Fluss: 0,9 ml/min; 3,0: 90/10 Fluss: 1,2 ml/min; 6,0: 90/10; Fluss: 1,2 ml/min.

### Beispiel 3-1

### 3-Ethyl-N-(2-methylbutyl)-9-oxo-2,3,4,9-tetrahydro-1H-thioxanthen-6-carbonsäureamid

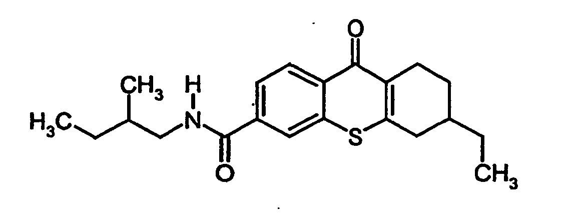

Unter Argon werden 0,70 g (2,43 mmol) 3-Ethyl-9-oxo-2,3,4,9-tetrahydro-1H-thioxanthen-6-carbonsäure und 3,22 g (7,28 mmol) 1-Benzotriazolyloxy-tris-(dimethylamino)-phosphoniumhexafluorophosphat in 44 ml THF 15 min bei 25°C gerührt. Es werden zunächst 0,85 ml (6,07 mmol) Triethylamin und nach weiteren 15 min 1,73 ml (14.6 mmol) 2-Methylbutylamin zu der Lösung getropft, die dann 24 h bei 25°C gerührt wird. Nach Verdünnen mit Dichlormethan wird mit Zitronensäure (5%-ig in Wasser) versetzt. Die Phasen werden getrennt und die organische Phase wird mit wässriger Natriumhydrogencarbonat-Lösung gewaschen. Nach Trocknen der vereinigten organischen Phasen über Natriumsulfat und Filtration wird das Lösungsmittel unter vermindertem Druck abdestilliert. Der Rückstand wird durch Chromatographie an Kieselgel (0,04-0,063 mm) mit Dichlormethan/Methanol 20:1 als Laufmittel vorgereinigt. Anschließend wird durch eine zweite Chromatographie an Kieselgel (0,04-0,063 mm) mit Cyclohexan/Essigsäureethylester 2:1 als Laufmittel sauberes Carbonsäureamid erhalten.
Ausbeute: 655 mg (75,5%)
R_{f} (CH₂Cl₂/MeOH 20/1) = 0,52
MS (DCI): 358 (M+H)
HPLC, Retentionszeit = 5,23 min
¹H-NMR (200 MHz, CDCl₃): δ = 0,96 (t, 3 H), 0,99 (d, 3 H), 1,00 (t, 3 H), 1,13-1,53 (m, 5 H), 1,63-1,80 (m, 2 H), 1,97-2,14 (m, 1 H), 2,34-2,63 (m, 2 H), 2,67-2,83 (m, 1 H), 2,84-3,02 (m, 1 H), 3,23-3,52 (m, 2 H), 6,14-6,28 (m, 1 H), 7,70 (dd, 1 H), 8,00 (d, 1 H), 8,54 (d, 1 H).

Nach dem für Beispiel 3-1 beschriebenen Verfahren wurden die Carbonsäureamide der Tabelle 1 erhalten.

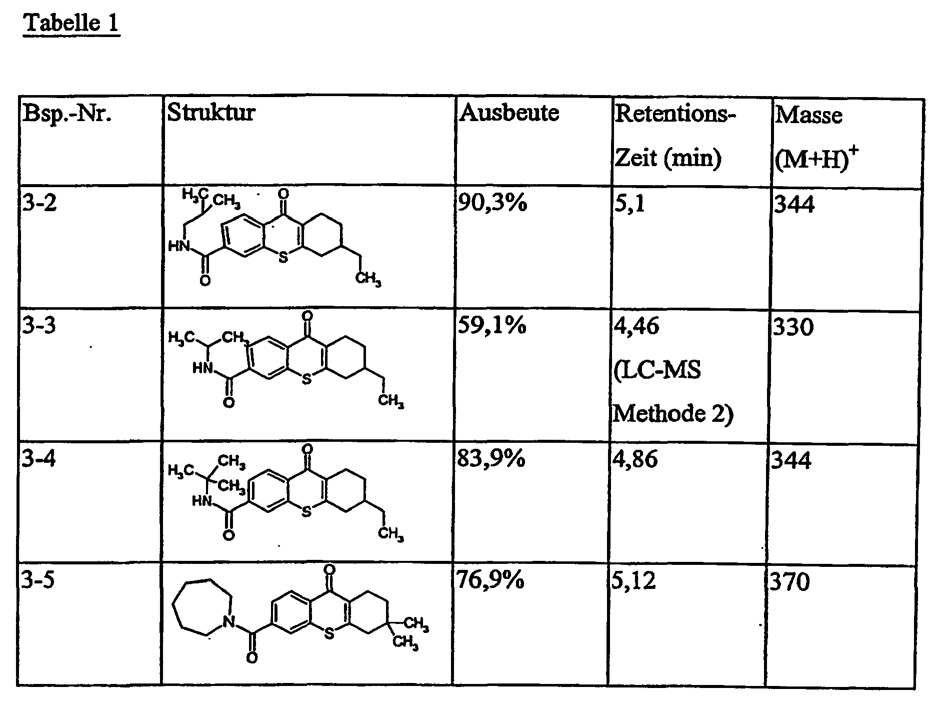

### Beispiel 3-8

### 6-(1-Azepanylcarbonyl)-3-ethyl-1,2,3,4-tetrahydro-9H-thioxanthen-9-on

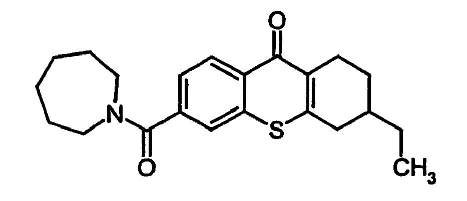

Unter Argon werden 50,0 mg (0,17 mmol) 3-Ethyl-9-oxo-2,3,4,9-tetrahydro-1H-thioxanthen-6-carbonsäure, 246 mg (0,23 mmol) PS-Carbodümid (0.94 mmol/g) und 26,6 mg (0,20 mmol) 1-Hydroxy-1H-benzotriazol in 3 ml Dichlormethan 30 min bei 25°C geschüttelt. Dann werden 13 µl (0,12 mmol) Azepan hinzugesetzt und die erhaltene Suspension 24 h bei 25°C geschüttelt. Es wird mit 165 mg (0,57 mmol) PS-Trisamin (3,5 mmol/g) versetzt und weitere 8 h geschüttelt. Nach Filtration wird die organische Phase mit wässriger Natriumcarbonat-Lösung versetzt und es wird über eine Extrelut NT1-Kartusche filtriert. Die Reinigung des Rohproduktes erfolgt durch Chromatographie an Kieselgel (0,04-0,063 nm) mit Cyclohexan/Essigsäureethylester 3:1 als Laufmittel.
Ausbeute: 41,6 mg (97,4%)
R_{f} (Cyclohexan/Essigsäureethylester 3:1) = 0,77
MS (EI): 370 (M+H)
HPLC, Retentionszeit = 4,98 min
¹H-NMR (200 MHz, CDCl₃): δ = 0,99 (t, 3 H), 1,20-1,75 (m, 11 H), 1,79-1,94 (m, 1 H), 1,97-2,12 (m, 1 H), 2,33-2,64 (m, 2 H), 2,65-2,81 (m, 1 H), 2,84-3,02 (m, 1 H), 3,26-3,40 (m, 2 H), 3,70 (dd, 2 H), 7,43 (dd, 1H), 7,52 (d, 1H), 8,52 (d, 1H).

### Beispiel 3-9

### 3-Ethyl-N-isopropyl-N-methyl-9-oxo-2,3,4,9-tetrahydro-1H-thioxanthen-6-carbonsäureamid

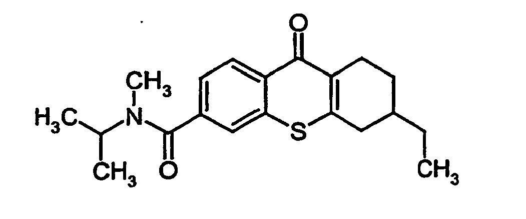

Unter Argon werden 50,0 mg (0,17 mmol) 3-Ethyl-9-oxo-2,3,4,9-tetrahydro-1H-thioxanthen-6-carbonsäure, 246 mg (0,23 mmol) PS-Carbodiimid (0.94 mmol/g) und 26,6 mg (0,20 mmol) 1-Hydroxy-1H-benzotriazol in 2 ml Dichlormethan 10 min bei 25°C geschüttelt. Nach Zugabe von 12 µl (0,12 mmol) N-Isopropyl-N-methylamin wird die erhaltene Suspension 24 h bei 25°C geschüttelt. Es wird mit 165 mg (0,57 mmol) PS-Trisamin (3,5 mmol/g) versetzt und weitere 16 h geschüttelt. Nach Filtration wird die organische Phase mit wässriger Natriumcarbonat-Lösung versetzt und es wird über eine Extrelut NT1-Kartusche filtriert. Abdestillation des Lösungsmittels unter vermindertem Druck und Chromatographie des Rückstandes an Kieselgel (0,04-0,063 mm) mit Cyclohexan/Essigsäureethylester 2:1 als Laufmittel liefert das gewünschte Produkt.
Ausbeute: 19,3 mg (48,6 %)
MS (EI): 344 (M+H)
HPLC, Retentionszeit = 4,76 min
¹H-NMR (200 MHz, CDCl₃): δ = 0,99 (t, 3 H), 1,16, (d, 3 H), 1,24 (d, 3 H), 1,33-1,52 (m, 3 H), 1,61-1,83 (m, 1 H), 1,96-2,13 (m, 1 H), 2,33-2,64 (m, 2 H), 2,65-3,02 (m, 5 H), 3,77-3,97 und 4,86-5,08 (m, 1 H), 7,42 (br. d, 1 H), 7,52 (br. s, 1 H), 8,52 (d, 1H).

Nach dem für Beispiel 3-8 und 3-9 beschriebenen Verfahren wurden die Carbonsäureamide der Tabelle 2 erhalten.

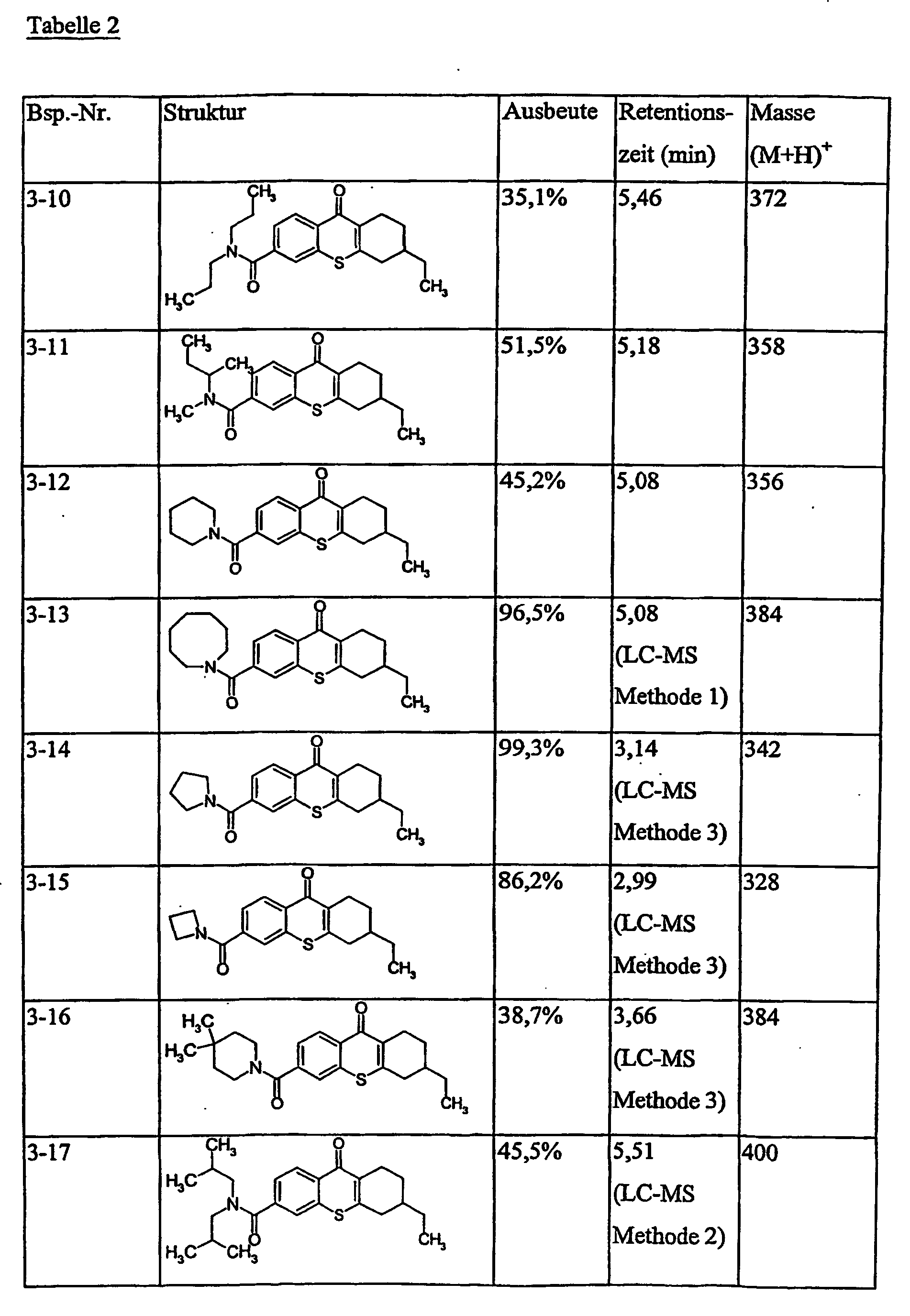

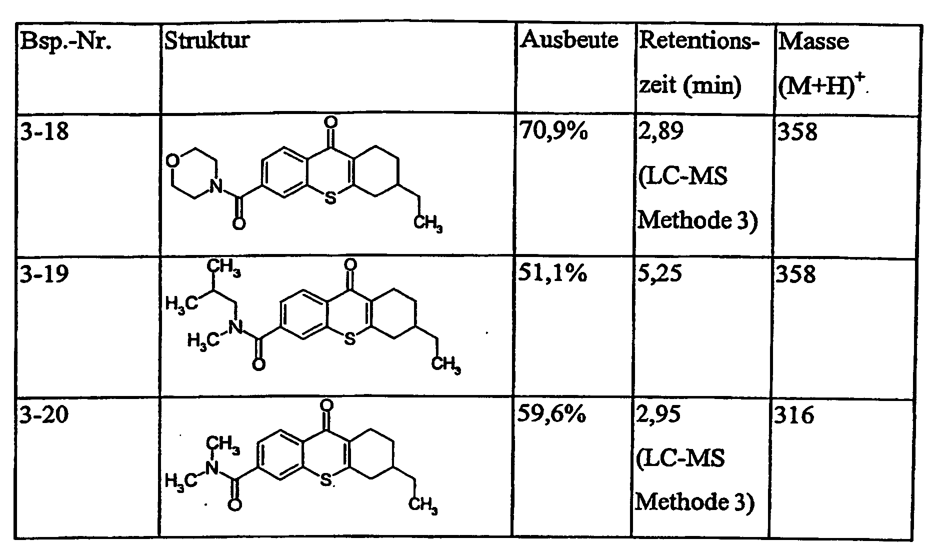

### Beispiel 3-21

### 3-Ethyl-6-[(2-methoxyethyl)(methyl)amino]-1,2,3,4-tetrahydro-9H-thio-xanthen-9-on

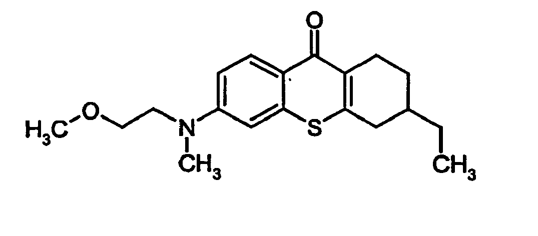

In einem ausgeheizten Kolben werden 1020 mg (3,16 mmol) 6-Brom-3-ethyl-1,2,3,4-tetrahydro-9H-thioxanthen-9-on mit 28,9 mg (0,03 mmol) Tris(dibenzylidenaceton)-dipalladium, 58,9 mg (0,09 mmol) (+/-)-2,2-Bis(diphenylphosphino)-1,1-binaphthyl und 425 mg (4,42 mmol) Natrium-tert.-butylat versetzt. Der Kolben wird evakuiert und anschließend 10 min mit Argon gespült. Es werden 0,38 ml (3,79 mmol) N-(2-Methoxyethyl)methylamin und 5 ml Toluol hinzugefügt und die erhaltene Suspension wird 3 h bei 80°C gerührt. Das Reaktionsgemisch wird durch Chromatographie an Kieselgel (0,04-0,063 mm) mit Cyclohexan/Essigsäureethylester 5:1/4:1/3:1/2:1 als Laufmittel gereinigt.
Ausbeute: 477 mg (45,6 %)
R_{f} (Cyclohexan/Essigsäureethylester 3:1) = 0,13
MS (EI): 332 (M+H)
HPLC, Retentionszeit = 5,05 min
¹H-NMR (200 MHz, CDCl₃): δ = 0,98 (t, 3 H), 1,26-1,49 (m, 3 H), 1,59-1,79 (m, 1 H), 1,93-2,09 (m, 1H), 2,24-2,55 (m, 2 H), 2,56-2,72 (m, 1H), 2,81-2,98 (m, 1 H), 3,08 (s, 3 H), 3,36 (s, 3 H), 3,56-3,62 (m, 4 H), 6,57 (d, 1 H), 6,85 (dd, 1 H), 8,31 (d, 1 H).

### Beispiel 3-22

### 3-Ethyl-6-(1-piperidinyl)-1,2,3,4-tetrahydro-9H-thioxanthen-9-on

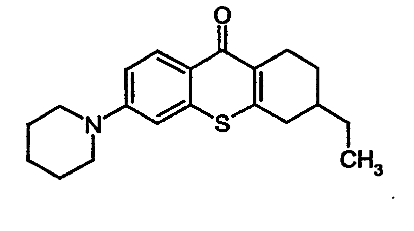

In einem ausgeheizten Kolben werden 1000 mg (3,09 mmol) 6-Brom-3-ethyl-1,2,3,4-tetrahydro-9H-thioxanthen-9-on mit 28,3 mg (0,03 mmol) Tris(dibenzylidenaceton)-dipalladium, 58,9 mg (0,09 mmol) (+/-)-2,2-Bis(diphenylphosphino)-1,1-binaphthyl und 416 mg (4,33 mmol) Natrium-tert.-butylat versetzt. Der Kolben wird evakuiert und anschließend 10 min mit Argon gespült. Es werden 0,37 ml (3,71 mmol) Piperidin und 20 ml Toluol hinzugefügt und die erhaltene Suspension wird 3 h bei 80°C gerührt. Verdünnen mit 10 ml Diethylether, Filtration, Entfernen des Lösungsmittels und anschließende Umkristallisation des Rückstandes aus tert.-Butylmethylether liefert einen Teil des gewünschten Produktes. Der größere Anteil wird durch Chromatographie des Filtrates der Umkristallisation an Kieselgel (0,04-0,063 mm) mit Cyclohexan/Essigsäureethylester 7:1/5:1 als Laufmittel erhalten.
Ausbeute: 719 mg (69,6 %)
R_{f} (Cyclohexan/Essigsäureethylester 3:1) = 0,40
MS (DCI): 328 (M+H)
HPLC, Retentionszeit = 5,43 min
¹H-NMR (200 MHz, CDCl₃): δ = 0,98 (t, 3 H), 1,22-1,49 (m, 3 H), 1,59-1,79 (m, 7 H), 1,83-2,09 (m, 1 H), 2,25-2,55 (m, 2 H), 2,56-2,73 (m, 1 H), 2,81-2,99 (m, 1 H), 3,26-3,43 (m, 4 H), 6,75 (d, 1 H), 7,02 (dd, 1 H), 8,32 (d, 1 H).

### Beispiel 3-23

### 3-Ethyl-7-fluor-6-(1-piperidinyl)-1,2,3,4-tetrahydro-9H-thioxanthen-9-on

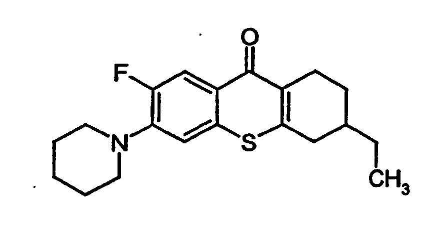

In einem ausgeheizten Kolben werden 1600 mg (5,39 mmol) 6-Chlor-3-ethyl-7-fluoro-1,2,3,4-tetrahydro-9H-thioxanthen-9-on mit 309,9 mg (0,54 mmol) Tris(dibenzyliden-aceton)di-palladium, 321,7 mg (0,09 mmol) 2-(Di-t-butylphosphino)biphenyl und 777,1 mg (8,09 mmol) Natrium-tert.-butylat versetzt. Der Kolben wird evakuiert und anschließend 10 min mit Argon gespült. Es werden 0,37 ml (3,71 mmol) Piperidin und 10 ml Toluol (entgast) hinzugefügt und die erhaltene Suspension wird 16 h bei 100°C gerührt. Da noch keine vollständige Umsetzung stattgefunden hatte, wird mit weiteren 30,9 mg Katalysator, 32,1 mg Ligand, 77,7 mg Base und 0,04 ml Piperidin versetzt und 24 h bei 100°C gerührt. Das Reaktionsgemisch wird mit 3 ml Essigsäureethylester versetzt, über eine Extrelut-Kartusche filtriert und anschließend vom Lösungsmittel befreit. Die Reinigung des Rückstandes erfolgt durch Chromatographie an Kieselgel (0,04-0,063 mm) mit Cyclohexan/Essigsäureethylester 15:1/10:1 als Laufmittel.
Ausbeute: 545 mg (29,2 %)
R_{f} (Cyclohexan/Diethylether 10:1) = 0,17
MS (EI): 346 (M+H)
HPLC, Retentionszeit = 5,80 min
¹H-NMR (200 MHz, CDCl₃): δ = 0,95 (t, 3 H), 1,29-1,50 (m, 3 H), 1,57-1,83 (m, 7 H), 1,85-2,10 (m, 1 H), 2,29-2,59 (m, 2 H), 2,60-2,75 (m, 1 H), 2,81-2,98 (m, 1 H), 3,18 (dd, 4 H), 6,87 (d, 1 H), 8,07 (d, 1 H).

### Beispiel 3-24

### 6-(1,5-Dioxa 9-azaspiro[5.5]undec-9-yl)-3-ethyl-1,2,3,4-tetrahydro-9H-thio-xanthen-9-on

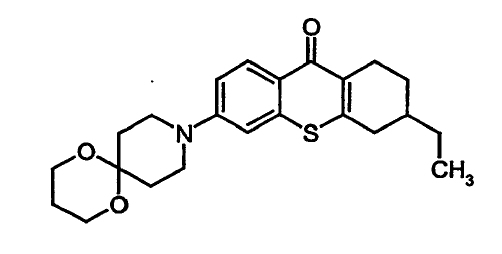

In einem ausgeheizten Kolben werden 100 mg (0,31 mmol) 6-Brom-3-ethyl-1,2,3,4-tetrahydro-9H-thioxanthen-9-on mit 2,8 mg (0,003 mmol) Tris(dibenzylidenaceton)-dipalladium, 5,9 mg (0,009 mmol) (+/-)-2,2-Bis(diphenylphosphino)-1,1-binaphthyl und 41,6 mg (0,43 mmol) Natrium-tert: butylat versetzt. Der Kolben wird evakuiert und anschließend 10 min mit Argon gespült. Es werden 59,6 mg (0,37 mmol) 1,5-Dioxa-9-azaspiro[5.5]undecan und 1 ml Toluol hinzugefügt und die erhaltene Suspension wird 5 h bei 80°C gerührt. Reinigung des Reaktionsgemisches durch Chromatographie an Kieselgel (0,04-0,063 mm) mit Cyclohexan/Essigsäureethylester 5:1/3:1/1:1 als Laufmittel liefert das gesuchte Produkt.
Ausbeute: 83 mg (67,5 %)
R_{f} (Cyclohexan/Essigsäureethylester 1:1) = 0,38
MS (LC-MS): 400 (M+H)
HPLC, Retentionszeit = 5,03 min (LC-MS Methode 1)
¹H-NMR (200 MHz, CDCl₃): δ = 0,98 (t, 3 H), 1,27-1,49 (m, 3 H), 1,64-1,84 (m, 3 H), 1,93-2,07 (m, 5 H), 2,26-2,57 (m, 2 H), 2,58-2,73 (m, 1H), 2,81-2,98 (m, 1 H), 3,43 (dd, 4 H), 3,95 (dd, 4 H), 6,77 (d, 1 H), 7,03 (dd, 1 H), 8,33 (d, 1H).

Nach den für die Beispiele 3-21 bis 3-24 beschriebenen Verfahren wurden die Verbindungen der Tabelle 3 erhalten.

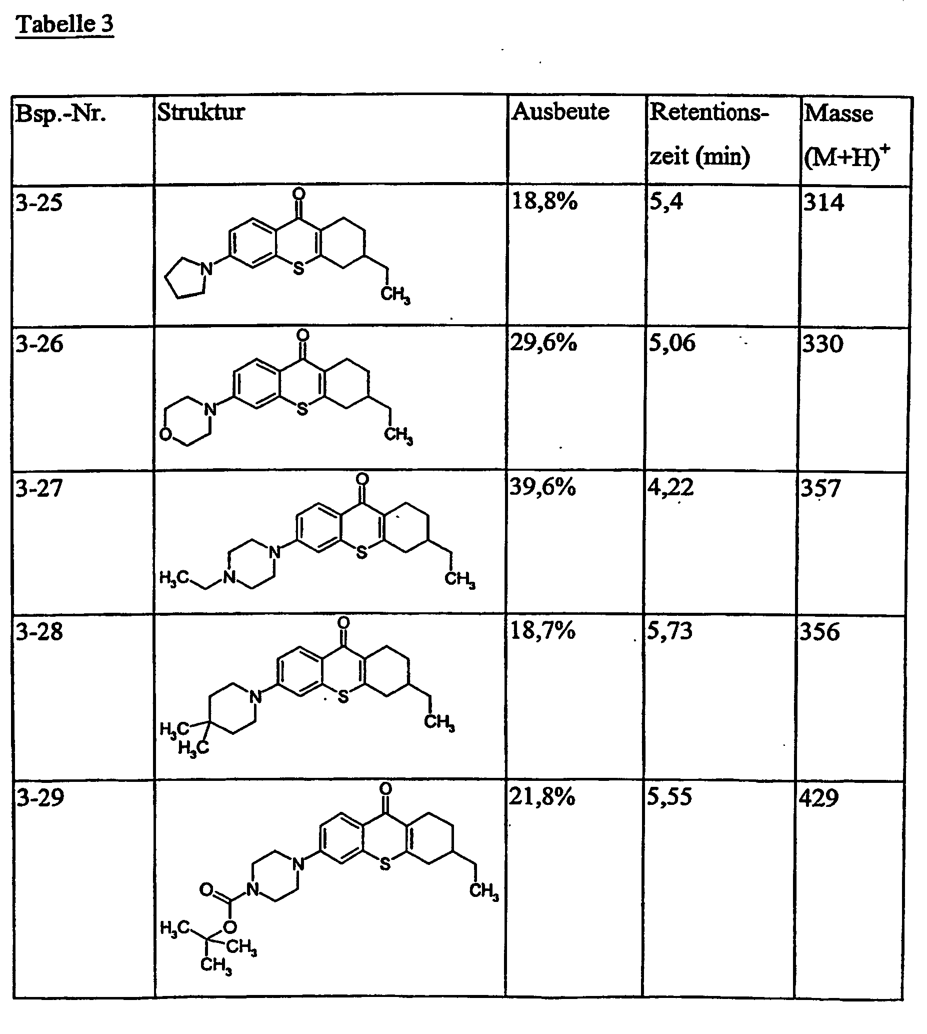

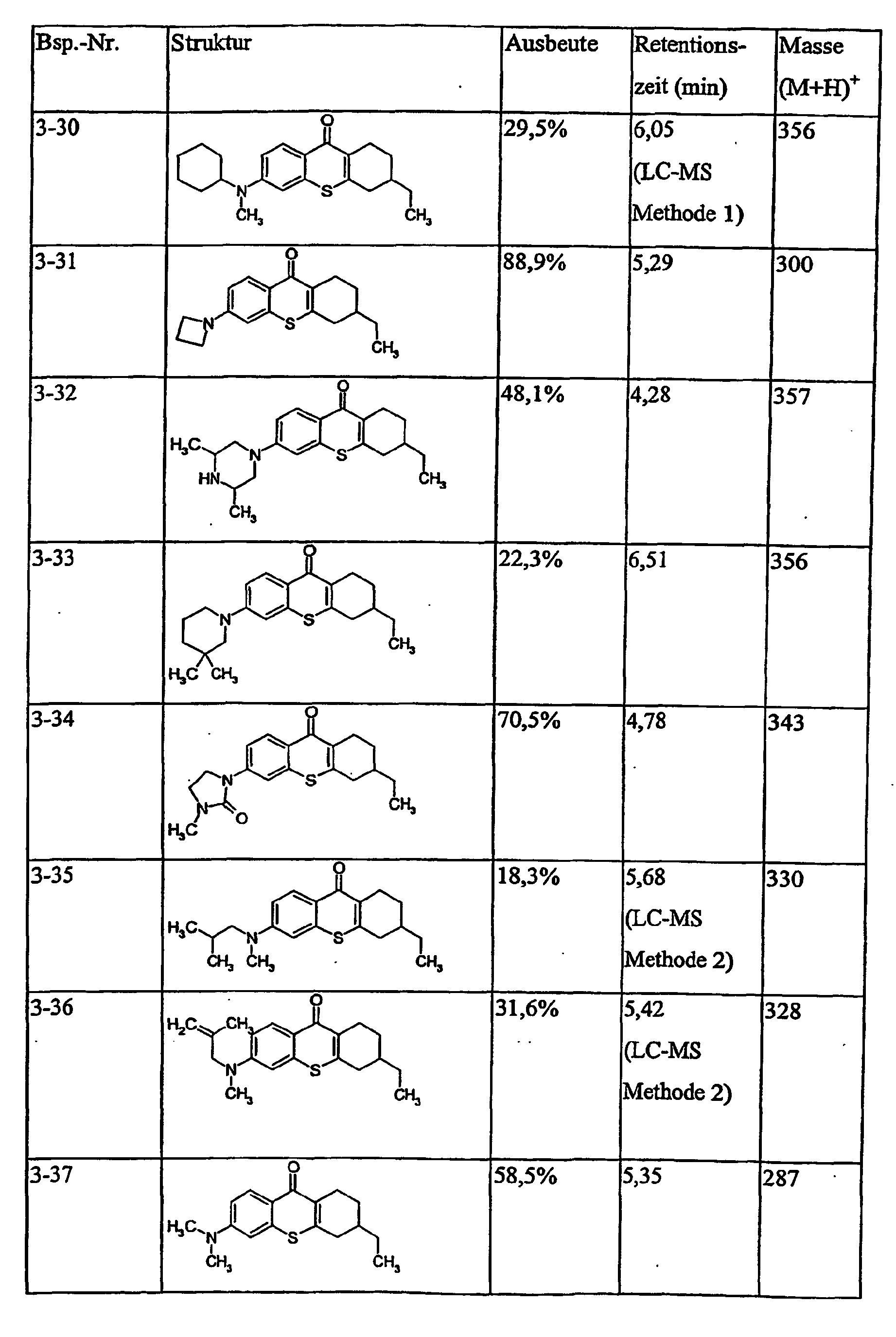

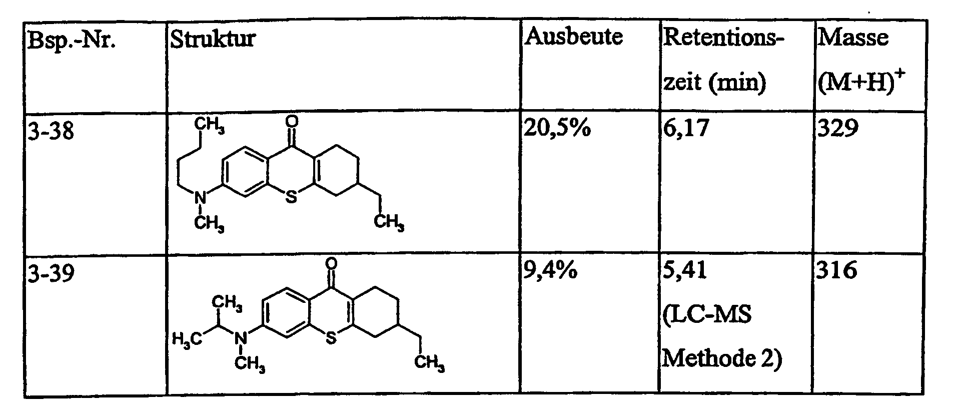

### Beispiel 3-40

### 1-(3-Ethyl-9-oxo-2,3,4,9-tetrahydro-1H-thioxanthen-6-yl)-4-piperidinon

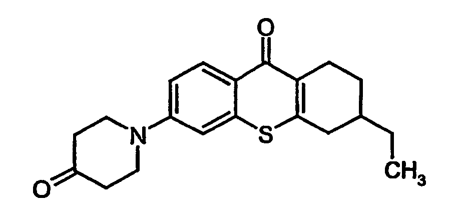

Bei 0°C werden 0,50 ml Triflouressigsäure (40% in H₂O) zu einer Lösung von 65 mg (0,16 mmol) 6-(1,5-Dioxa-9-azaspiro[5.5]undec-9-yl)-3-ethyl-1,2,3,4-tetrahydro-9H-thioxanthen-9-on in 5 ml Dichlormethan getropft. Es wird 48 h bei 25°C gerührt und anschließend mit 10 ml Natronlauge (1 N) versetzt. Nach Extraktion der organischen Phase mit Dichlormethan werden die vereinigten organischen Phasen über Natriumsulfat getrocknet. Filtration, Entfernen des Lösungsmittels unter schwachem Vakuum und Chromatographie des Rückstandes an Kieselgel (0,04-0,063 mm) mit Cyclohexan/Diethylether 1:4 als Laufmittel ergibt das gewünschte Produkt.
Ausbeute: 27 mg (48,6 %)
R_{f} (Cyclohexan/Diethylether 1:5) = 0,17
MS (EI): 342 (M+H)
HPLC, Retentionszeit = 4,69 min
¹H-NMR (200 MHz, CDCl₃): δ = 0,99 (t, 3 H), 1,30-1,51 (m, 3 H), 1,59-1,81 (m, 1 H), 1,95-2,12 (m, 1 H), 2,26-2,78 (m, 3 H), überlagert von 2,60 (dd, 4 H), 2,81-2,99 (m, 1 H), 3,77 (dd, 4 H), 6,81 (d, 1 H), 7,05 (dd, 1 H), 8,39 (d, 1 H).

### Beispiel 3-41

### 3-Ethyl-6-[methyl(phenyl)amino]-1,2,3,4-tetrahydro-9H-thioxanthen-9-on

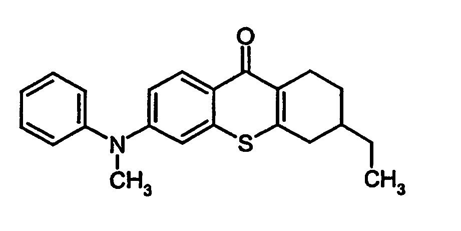

In einem ausgeheizten Kolben werden 41,7 mg (0,13 mmol) 6-Brom-3-ethyl-1,2,3,4-tetrahydro-9H-thioxanthen-9-on mit 2,9 mg (0,005 mmol) Tris(dibenzylidenaceton)-dipalladium, 3,1 mg (0,01 mmol) 2-(Di-t-butylphosphino)biphenyl und 17,3 mg (0,18 mmol) Natrium-tert.-butylat versetzt. Der Kolben wird evakuiert und anschließend mit Argon gespült. Es werden 0,02 ml (0,15 mmol) N-Methylanilin und 0,3 ml Toluol hinzugefügt und die erhaltene Suspension wird 24 h bei 80°C gerührt. Das Reaktionsgemisch wird mit 10 ml Essigsäureethylester verdünnt, über Celite filtriert und anschließend durch Präparative-HPLC (RP-C18, Acetonitril/H₂O-Gradient) gereinigt.
Ausbeute: 32,1 mg (71,2 %)
R_{f} (Cyclohexan/Essigsäureethylester 5:1) = 0,28
MS (EI): 350 (M+H)
HPLC, Retentionszeit = 5,89 min
¹H-NMR (200 MHz, CDCl₃): δ = 0,98 (t, 3 H), 1,22-1,49 (m, 3 H), 1,59-1,78 (m, 1 H), 1,93-2,09 (m, 1 H), 2,25-2,72 (m, 3 H), 2,81-2,98 (m, 1H), 3,38 (s, 3 H), 6,70 (d, 1 H), 6,86 (dd, 1 H), 7,18-7,31 (m, 3 H), 7,36-7,48 (m, 2 H), 8,26 (d, 1 H).

Nach dem für Beispiel 3-41 beschriebenen Verfahren wurden die Verbindungen der Tabelle 4 erhalten.

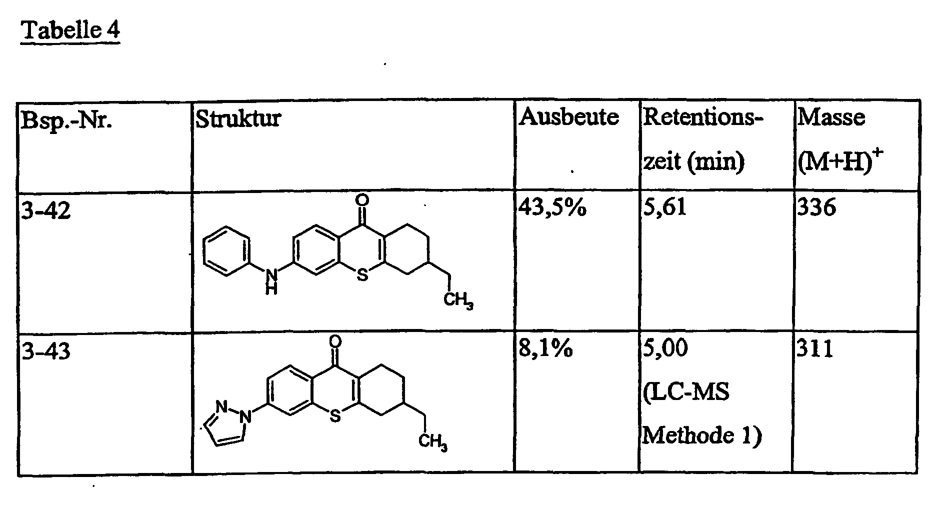

### Beispiel 3-44

### 3-[(3-Ethyl-9-oxo-2,3,4,9-tetrahydro-1H-thioxanthen-6-yl)oxy]-4-methyl-benzonitril

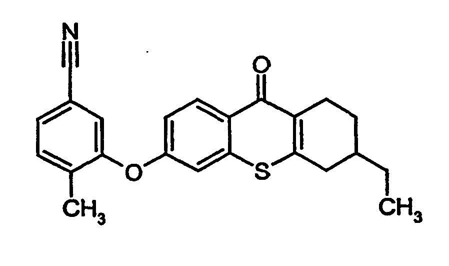

In einem ausgeheizten Kolben werden 60 mg (0,19 mmol) 6-Brom-3-ethyl-1,2,3,4-tetrahydro-9H-thioxanthen-9-on mit 6,8 mg (0,007 mmol) Tris(dibenzylidenaceton)-dipalladium, 3,4 mg (0,006 mmol) 2-(Di-t-butylphosphino)biphenyl und 78,8 mg (0,37 mmol) Kaliumphosphat versetzt. Der Kolben wird evakuiert und anschließend mit Argon gespült. Nach Zusatz von 30 mg (0,22 mmol) 2-Hydroxy-4-methylbenzonitril und 1,0 ml Toluol (entgast) wird die erhaltene Suspension 24 h bei 100°C gerührt. Das Reaktionsgemisch wird mit 3 ml Dichlormethan verdünnt, mit 0,5 ml Natronlauge (1 N) versetzt und anschließend über eine NT1-Extrelutkartusche filtriert. Entfernen des Lösungsmittels unter vermindertem Druck und Chromatographie des Rückstandes an Kieselgel (0,04-0,063 mm) mit Cyclohexan/Diethylether 5:1 als Laufinittel führt zu dem gewünschten Produkt.
Ausbeute: 29,1 mg (41,8 %)
R_{f} (Cyclohexan/Essigsäureethylester 5:1) = 0,33
MS (EI): 376 (M+H)
HPLC, Retentionszeit = 5,78 min
¹H-NMR (200 MHz, CDCl₃): δ = 0,99 (t, 3 H), 1,21-1,50 (m, 3 H), 1,60-1,79 (m, 1 H), 1,96-2,12 (m, 1H), 2,30 (s, 3 H), 2,34-2,77 (m, 3 H), 2,82-3,00 (m, 1H), 6,88 (d, 1 H), 7,03 (dd, 1 H), 7,20-7,31 (m, 1H, überlagert von CHCl₃-Signal), 7,35 (br. d, 1 H), 7,46 (dd, 1H), 8,50 (d, 1 H).

Nach dem für Beispiel 3-44 beschriebenen Verfahren wurden die Verbindungen der Tabelle 5 erhalten.

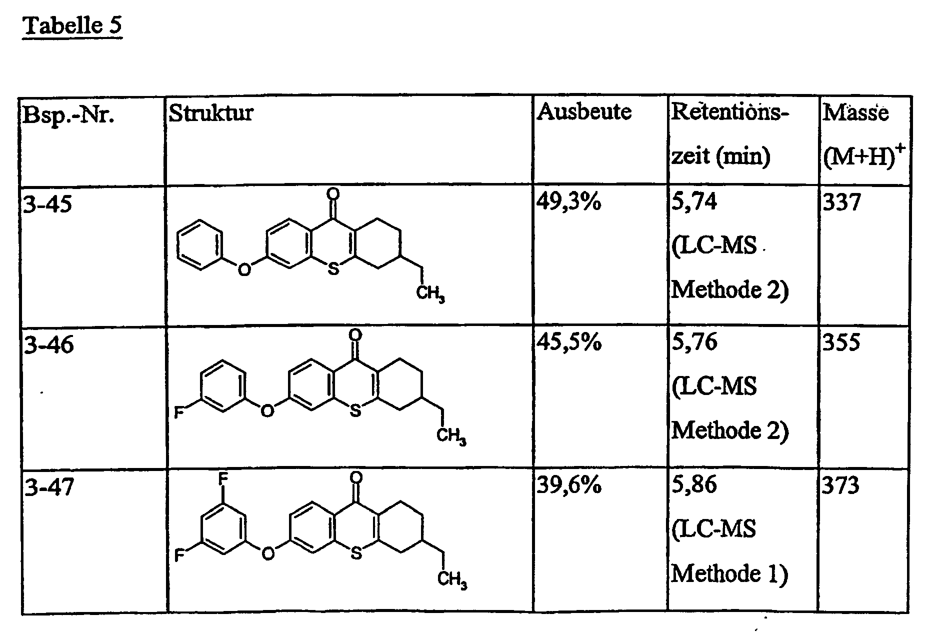

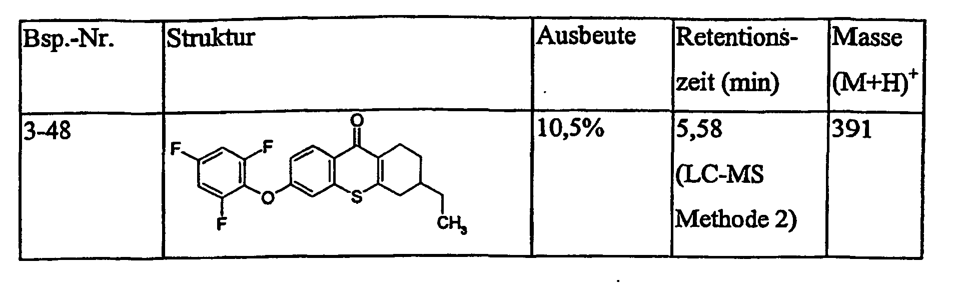

### Beispiel 3-49

### N-(3-Ethyl-9-oxo-2,3,4,9-tetrahydro-1H-thioxanthen-6-yl)propanamid

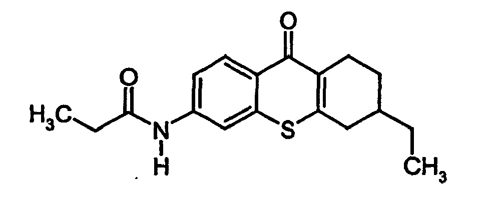

Zu einer Suspension von 70 mg (0,27 mmol) 6-Amino-3-ethyl-1,2,3,4-tetrahydro-9H-thioxanthen-9-on in 3,5 ml Dichlormethan werden 30 µl (0,30 mmol) Propionsäurechlorid getropft. Nach 10 min wird mit 40 µl (0,30 mmol) Triethylamin versetzt und es wird 4 h unter Rückfluss gerührt. Nach Abkühlen wird die Reaktionslösung in 10 ml Eiswasser gegeben und nach Auftauen wird ein Teil des Produktes als Feststoff abfiltriert. Dieser wird durch Umkristallisation aus Acetonitril gereinigt. Das Filtrat wird mit ges. wässriger Natriumcarbonat-Lösung neutralisiert. Nach Extraktion der wässrigen Phase mit Dichlormethan und Entfernen des Lösungsmittels wird der erhaltene Feststoff aus Acetonitril umkristallisiert.
Ausbeute: 71 mg (78,4%)
R_{f} (Cyclohexan/Essigsäureethylester 1:2 = 0,58
MS (EI): 315 (M)
HPLC, Retentionszeit = 4,89 min
¹H-NMR (200 MHz, DMSO-d₆): δ = 0,94 (t, 3 H), 1,09 (t, 3 H), 1,21-1,46 (m, 3 H), 1,51-1,75 (m, 1 H), 1,87-2,03 (m, 1 H), 2,26-2,47 (m, 4 H), 2,62-2,82 (m, 2 H), 7,53 (dd, 1 H), 8,17 (d, 1 H), 8,23 (d, 1H), 10,4 (s, 1 H).

Nach dem für Beispiel 3-49 beschriebenen Verfahren wurden die Verbindungen der Tabelle 6 erhalten.

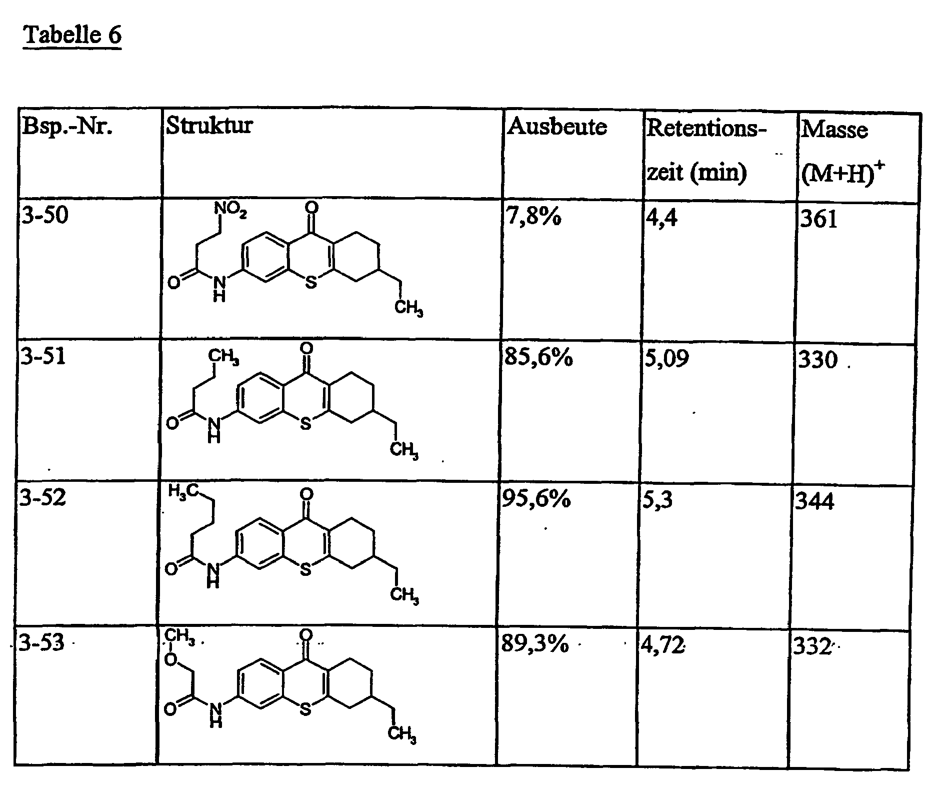

Nach dem für Beispiel 1-10 beschriebenen Verfahren wurden die Verbindungen der Tabelle 7 erhalten:

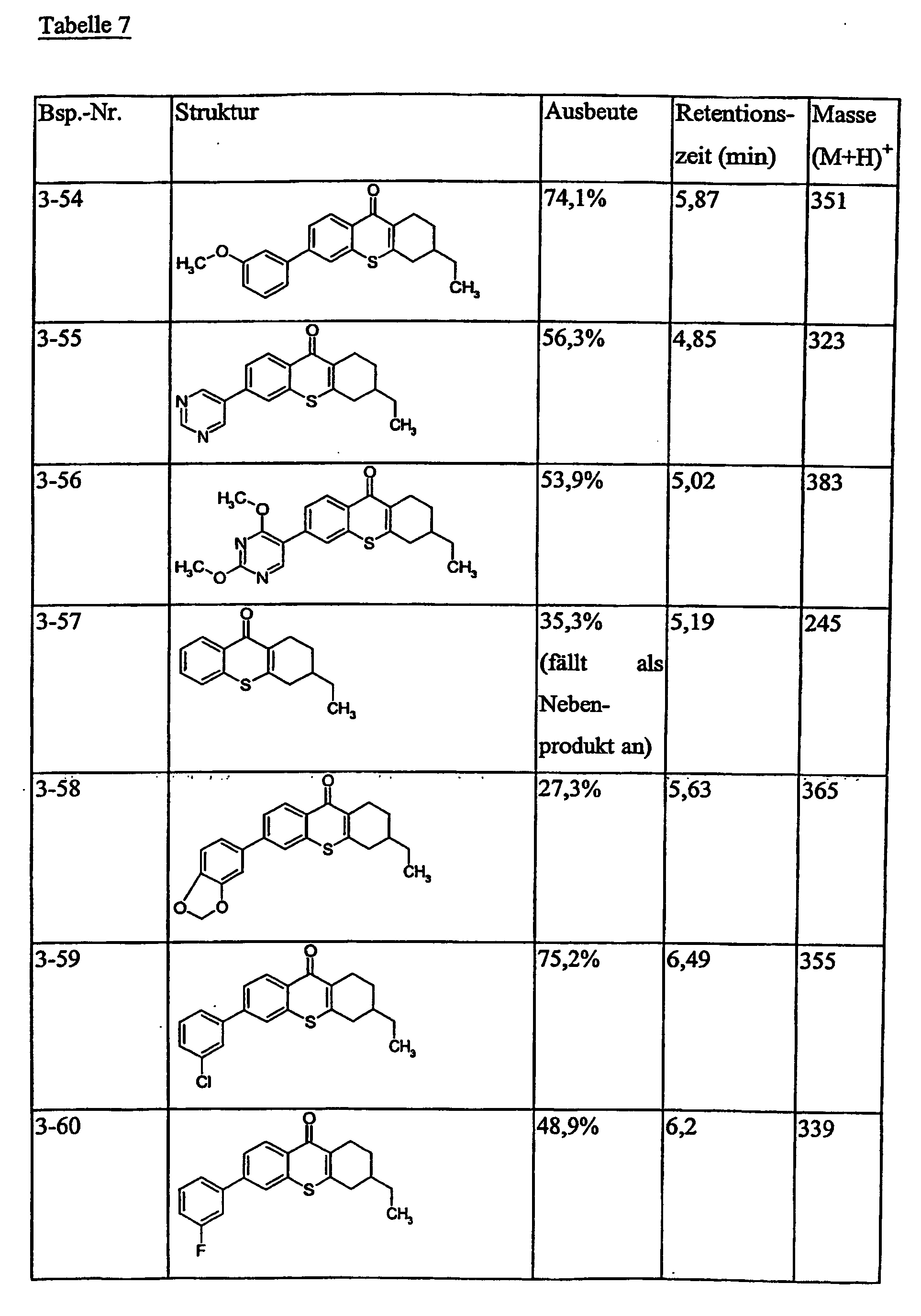

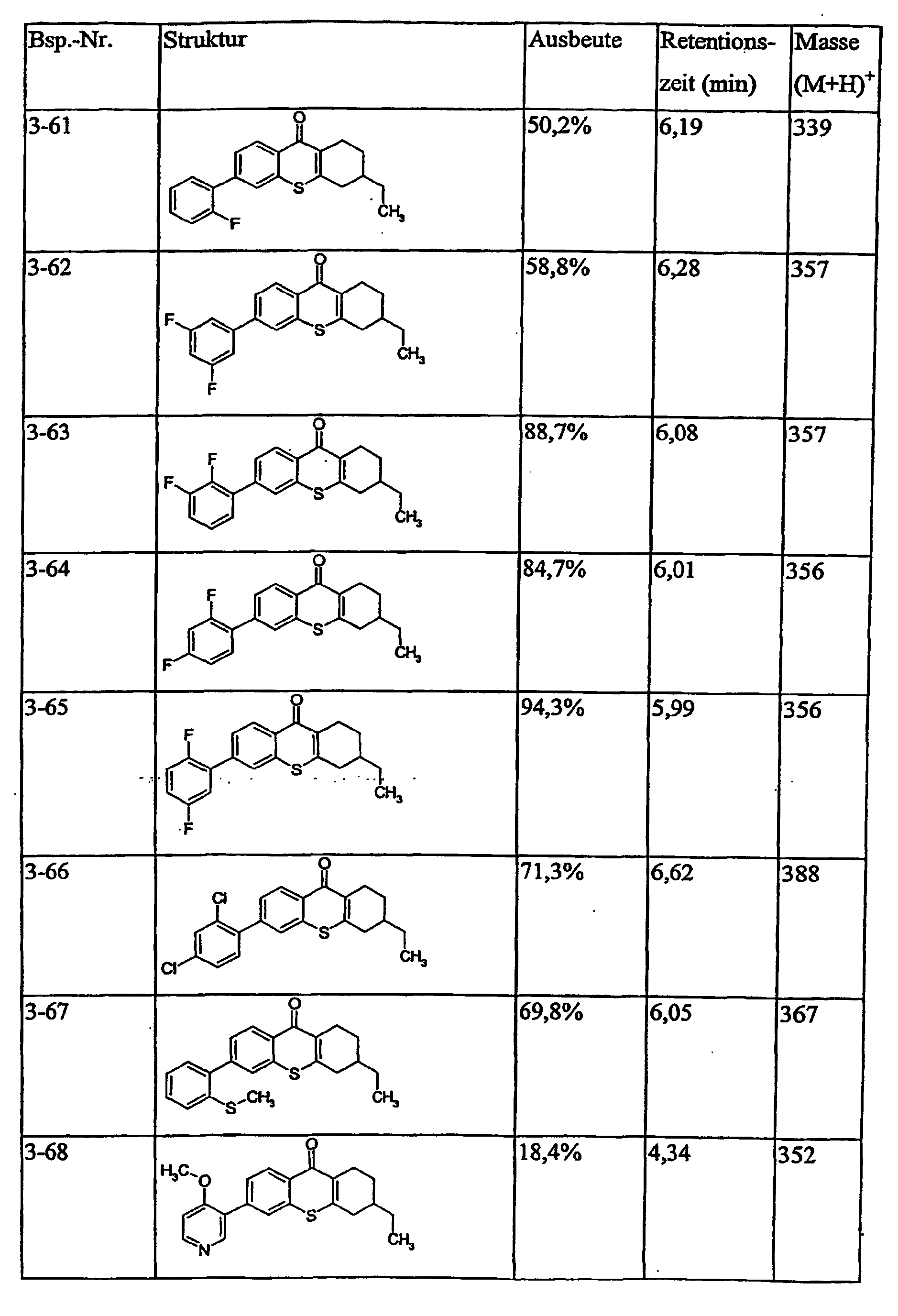

### Beispiel 3-69

### N,3,3-Trimethyl-9-oxo-N-(2,2,2-trifluorethyl)-2,3,4,9-tetrahydro-1H-thioxanthen-6-carbon-säureamid

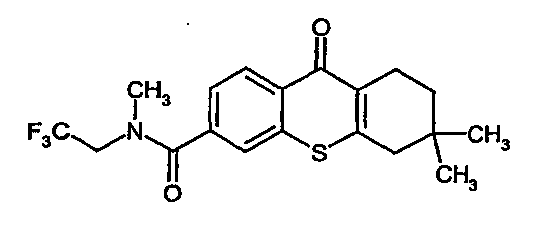

Unter Argon werden 0,70 g (2,43 mmol) 3,3-Dimethyl-9-oxo-2,3,4,9-tetrahydro-1H-thioxanthen-6-carbonsäure und 3,22 g (7,28 mmol) 1-Benzotriazolyloxy-tris-(dimethyl-amino)-phosphoniumhexafluorophosphat in 14 ml THF 15 min bei 25°C gerührt. Es werden zunächst 0,85 ml (6,07 mmol) Triethylamin und nach weiteren 15 min 1,10 g (9,71 mmol) N-(2-Trifluorethyl)methylamin zu der Lösung getropft, die dann 24 h bei 25°C gerührt wird Nach Verdünnen mit Dichlormethan wird mit Zitronensäure (5%-ig in Wasser) versetzt. Die Phasen werden getrennt und die organische Phase wird mit wäßriger Natriumhydrogencarbonat-Lösung gewaschen. Nach Trocknen der vereinigten organischen Phasen über Natriumsulfat und Filtration wird das Lösungsmittel unter vermindertem Druck abdestilliert. Der Rückstand wird durch Präparative-HPLC (RP-C18, Acetonitril/Wasser-Gradient) gereinigt.
Ausbeute: 838 mg (88,8%)
R_{f} (Cyclohexan/ Essigsäureethylester 2/1) = 0,20
MS (DCI): 384 (M+H)
HPLC, Retentionszeit = 4,86 min
¹H-NMR (200 MHz, CDCl₃): δ = 1,04 (s, 6 H), 1,64 (t, 2 H), 2,49 (br. s, 2 H), 2,72 (t, 2 H), 3,11 (br. s, 3 H), 3,66-4,35 (m, 2 H), 7,47 (d 1 H), 7,58 (s, 1 H), 8,56 (d, 1 H).

### Beispiel 3-70

### 7-Fluor-N-isobutyl-N,3,3-trimethyl-9-oxo-2,3,4,9-tetrahydro-1H-thioxanthen-6-carbon-säureamid

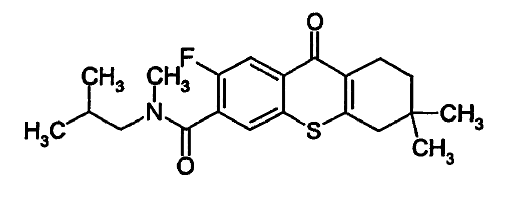

Unter Argon werden 0,43 g (1,40 mmol) 5-Fluor-3,3-dimethyl-9-oxo-2,3,4,9-tetrahydro-1H-thioxanthen-6-carbonsäure und 1,86 g (4,21 mmol) 1-Benzotriazolyloxy-tris-(dimethyl-amino)-phosphoniumhexafluomphosphat in 7 ml THF 15 min bei 25°C gerührt. Es werden zunächst 0,49 ml (3,51 mmol) Triethylamin und nach weiteren 15 min 0,49 g (5,61 mmol) N-Methylisobutylamin zu der Lösung getropft, die dann 24 h bei 25°C gerührt wird. Nach Verdünnen mit Dichlormethan wird mit Zitronensäure (5%-ig in Wasser) versetzt. Die Phasen werden getrennt und die organische Phase wird mit wäßriger Natriumhydrogencarbonat-Lösung gewaschen. Nach Trocknen der vereinigten organischen Phasen über Natriumsulfat und Filtration wird das Lösungsmittel unter vermindertem Druck abdestilliert. Der Rückstand wird durch durch Präparative-HPLC (RP-C18, Acetonitril/Wasser-Gradient) gereinigt.
Ausbeute: 541,4 mg (85,6%)
R_{f} (Cyclohexan/ Essigsäureethylester 2/1) = 0,45
MS (EI): 376 (M)
HPLC, Retentionszeit = 5,17 min
¹H-NMR (200 MHz, CDCl₃): δ = 0,76 (d, 3 H), 1,00 (d, 3 H), 1,04 (s, 6 H), 1,64 (t, 2 H), 1,84-2,19 (m, 1 H), 2,49 (br. s, 2 H), 2,72 (t, 2 H), 2,89 und 3,10 (2 s, 3 H), 3,00 und 3,41 (2 d, 2 H), 7,53 (dd, 1 H), 8,21 (dd, 1H).

In Analogie zu den für die Beispiele 3-1, 3-6, 3-7, 3-69 und 3-70 beschriebenen Verfahren werden die Carbonsäureamide der Tabelle 8 erhalten.

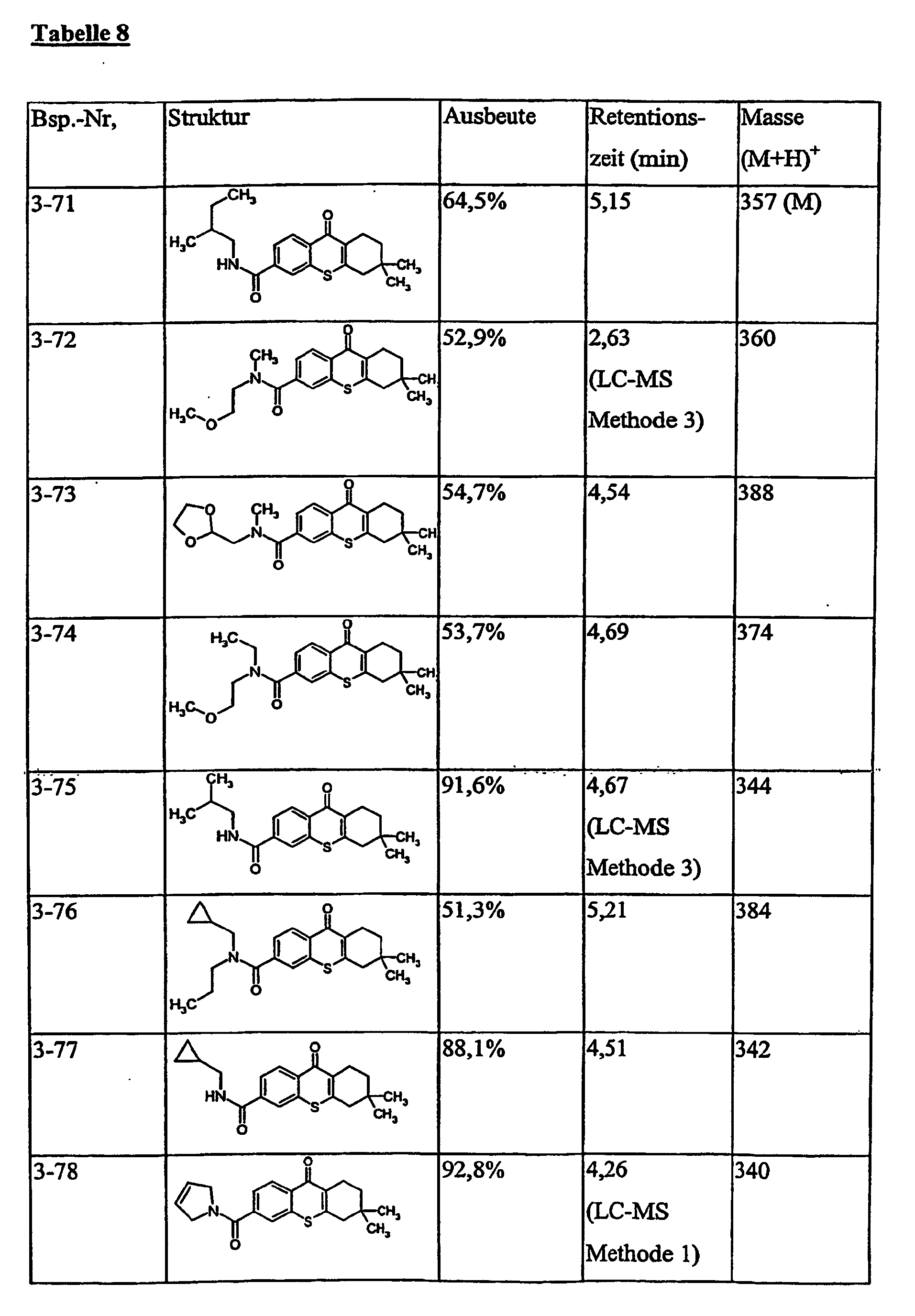

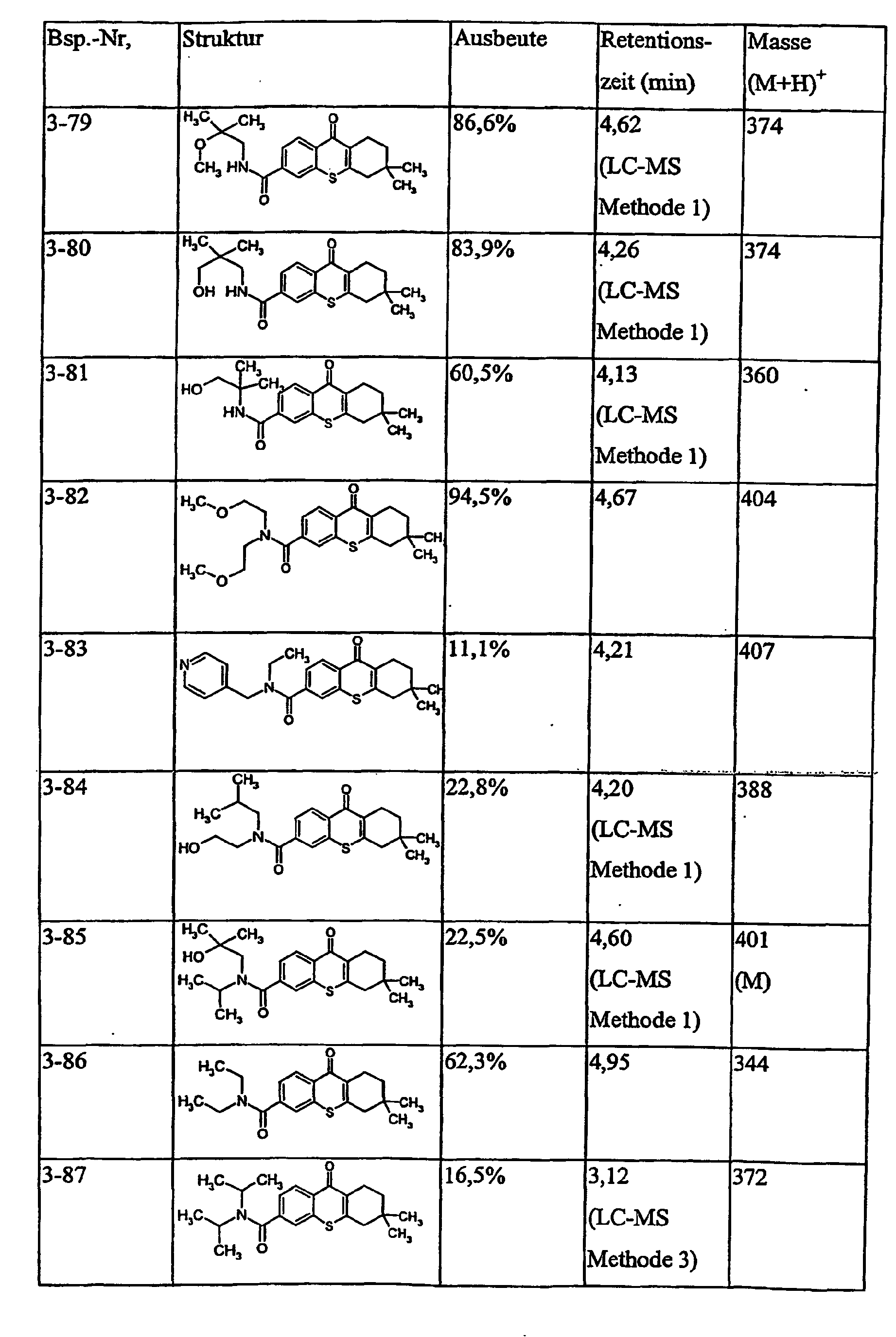

In Analogie zu den für die Beispiele 3-8 und 3-9 beschriebenen Verfahren wurden die Carbonsäureamide der Tabelle 9 erhalten.

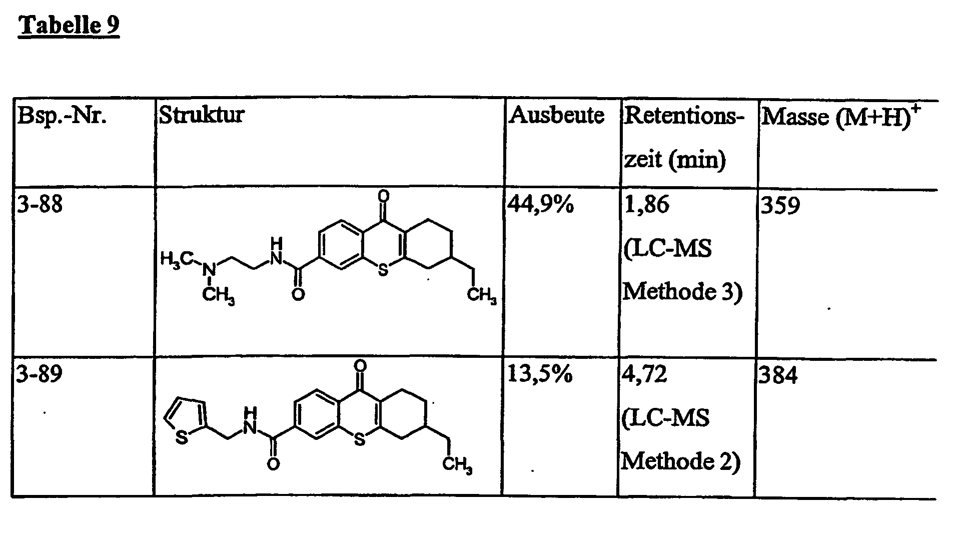

### Beispiel 3-90

### N-Ethyl-N-(2-hydroxy-2-methylpropyl)-3,3-dimethyl-9-oxo-2,3,4,9-tetrahydro-1H-thio-xanthen-6-carbonsäureamid

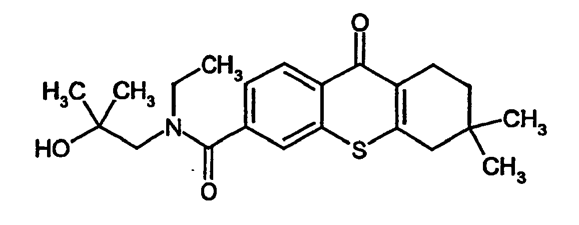

Unter Rühren werden zu einer Suspension von 60 mg (0,21 mmol) 3,3-Dimethyl-9-oxo-2,3,4,9-tetrahydro-1H-thioxanthen-6-carbonsäure in 10 ml Dichlormethan 50 µl (0,46 mmol) N-Methylmorpholin, 35 mg (0,23 mmol) N-Ethylamino-2-methyl-2-propanol-Hydrochlorid und 34 mg (0,25 mmol) 1-Hydroxy-1H-benzotriazol-Hydrat gegeben. Es wird auf 0°C gekühlt und anschließend mit 48 mg (0,25 mmol) N-(3-Dimethylaminopropyl)-N-ethylcarbodiimid Hydrochlorid versetzt. Das Kühlbad wird entfernt und es wird 24 h bei 25°C gerührt. Das Lösungsmittel wird unter vermindertem Druck abdestilliert und der erhaltene Rückstand wird durch Präparative-HPLC (RP-C18, Acetonitril/Wasser-Gradient) gereinigt. Anschließend wird durch eine zweite Chromatographie des so erhaltenen vorgereinigten Produktes an Kieselgel (0,04-0,063 mm) (Dichlormathan/Methanol 30:1, 20:1, 10:1, 5:1, 1:1 als Laufmittel) sauberes Carbonsäureamid erhalten.
Ausbeute: 33,2 mg (41,2%)
R_{f} (CH₂Cl₂/MeOH 10/1) = 0,55
LC-MS (Methode 1): 388 (M+H)
LC-MS, Retentionszeit = 4,10 min
¹H-NMR (200 MHz, CDCl₃): δ = 1,04 (s, 6 H), 1,08 (t, 3 H), 1,33 (s, 6 H), 1,64 (t, 2 H), 2,49 (s, 2 H), 2,73 (t, 2 H), 3,37 (q, 2 H), 3,58 (s, 2 H), 4,06 (s, 1 H), 7,47 (dd, 1 H), 7,54 (br. s, 1 H), 8,55 (d, 1 H).

In Analogie zu dem für Beispiel 3-90 beschriebenen Verfahren werden die Carbonsäureamide der Tabelle 10 erhalten.

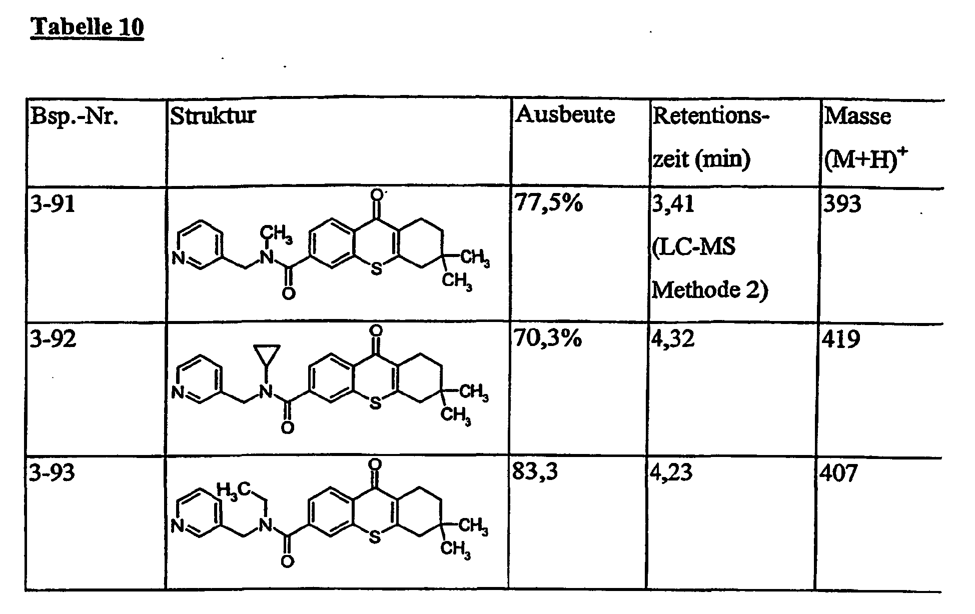

### Beispiel 3-94

### N-Ethyl-N-isobutyl-3,3-dimethyl-9-oxo-2,3,4,9-tetrahydro-1H-thioxanthen-6-carbonsäure-amid

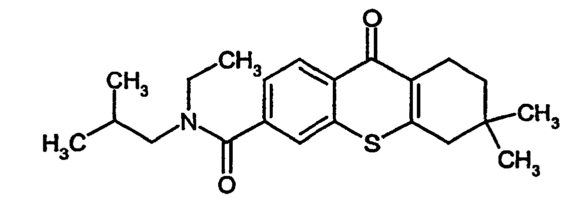

Unter Argon werden bei 0°C 0,127 g (5,03 mmol) Natriumhydrid zu einer Lösung von 0,576 g (1,68 mmol) N-Isobutyl-3,3-dimethyl-9-oxo-2,3,4,9-tetrahydro-1H-thioxanthen-6-carbon-säureamid in 19 ml THF gegeben und es wird 15 min bei 0°C gerührt. Dann wird mit 0,67 ml (8,4 mmol) Iodethan versetzt und das Kühlbad entfernt. Nach 2 h Rühren bei 25°C wird vorsichtig mit 2 ml Wasser versetzt, dann mit Essigsäureethylester verdünnt und anschließend wird über eine Extrelut NT3-Kartusche filtriert. Das Lösungsmittel wird unter vermindertem Druck abdestilliert und der erhaltene Rückstand wird durch Präparative-HPLC (RP-C18, Acetonitril/Wasser-Gradient) gereinigt.
Ausbeute: 275,9 mg (43,6%)
R_{f} (Cyclohexan/ Essigsäureethylester 3/1) = 0,23
LC-MS (Methode 2): 371 (M+H)
HPLC, Retentionszeit = 5,00 min

### Beispiel 3-95

### N-(Cyclopropylmethyl)-N-ethyl-3,3-dimethyl-9-oxo-2,3,4,9-tetrahydro-1H-thioxanthen-6-carbonsäureamid

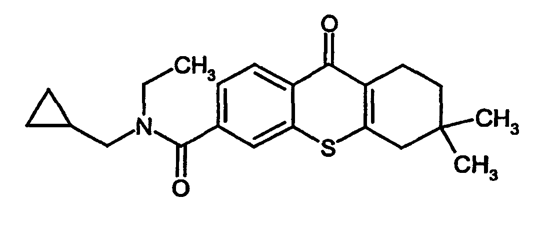

Unter Argon werden bei 0°C 32,4 mg (1,28 mmol) Natriumhydrid zu einer Lösung von 146 mg (0,43 mmol) N-(Cyclopropylmethyl)-3,3-dimethyl-9-oxo-2,3,4,9-tetrahydro-1H-thiaxanthen-6-carbonsäureamid in 2,8 ml THF gegeben und es wird 15 min bei 0°C gerührt. Dann wird mit 0,17 ml (2,1 mmol) Iodethan versetzt und das Kühlbad entfernt. Nach 1 h Rühren bei 25°C wird vorsichtig mit 2 ml Wasser versetzt, dann mit Essigsäureethylester verdünnt und anschließend wird über eine Extrelut NT3-Kartusche filtriert. Das Lösungsmittel wird unter vermindertem Druck abdestilliert und der erhaltene Rückstand wird durch Präparative-HPLC (RP-C18, Acetonitril/Wasser-Gradient) gereinigt.
Ausbeute: 275,9 mg (43,6%)
R_{f} (Cyclohexan/ Essigsäureethylester 1/1) = 0,55
LC-MS (Methode 3): 370 (M+H)
LC-MS, Retentionszeit = 3,02 min

In Analogie zu den für die Beispiele 3-21 bis 3-24 beschriebenen Verfahren werden die Verbindungen der Tabelle 11 erhalten.

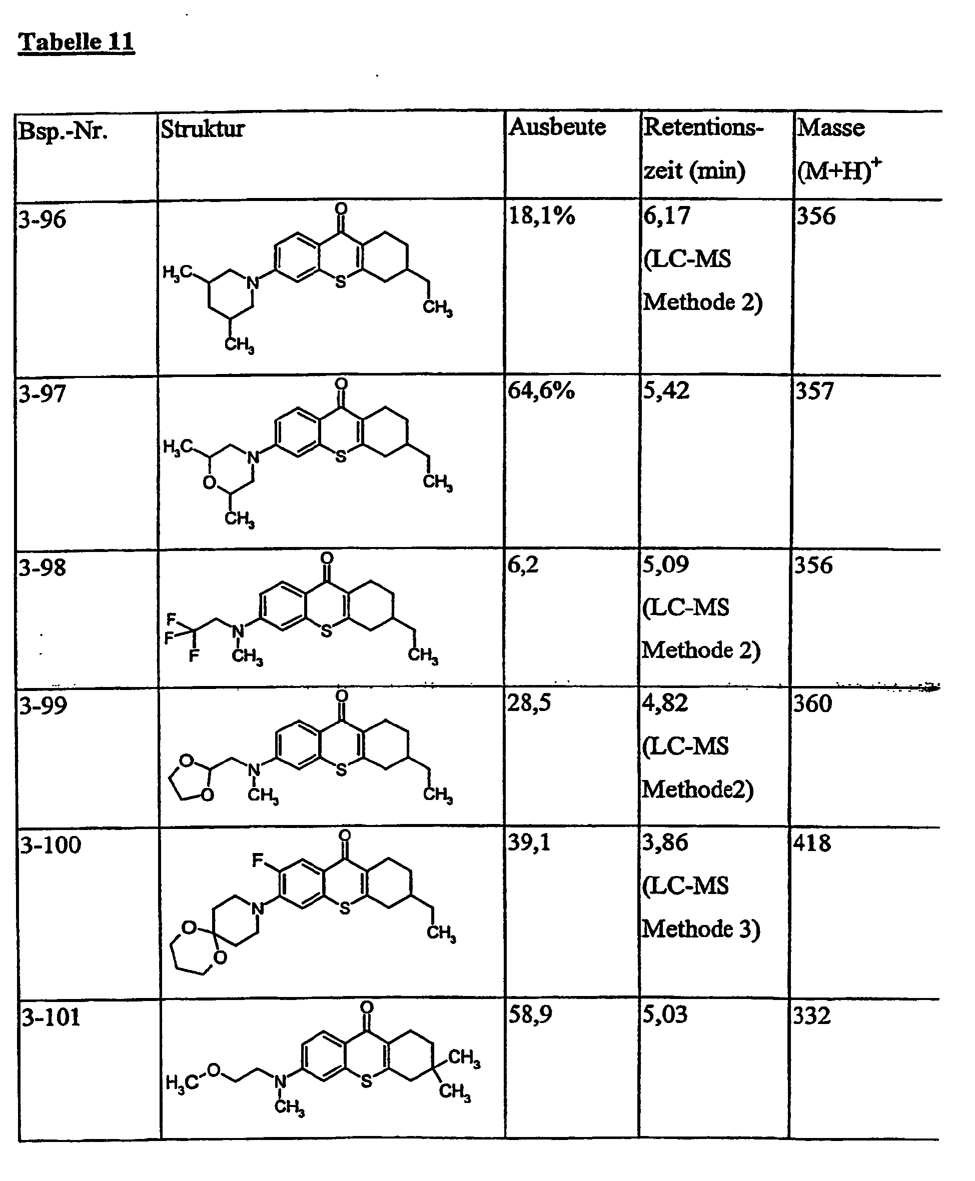

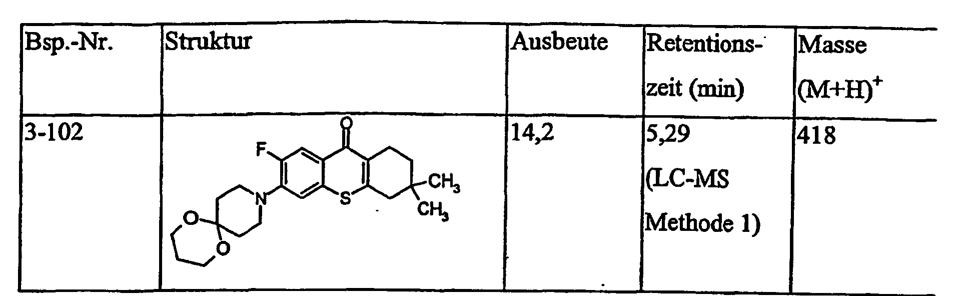

In Analogie zu dem für Beispiel 3-40 beschriebenen Verfahren werden die Verbindungen der Tabelle 12 aus den entsprechenden Ketalen erhalten.

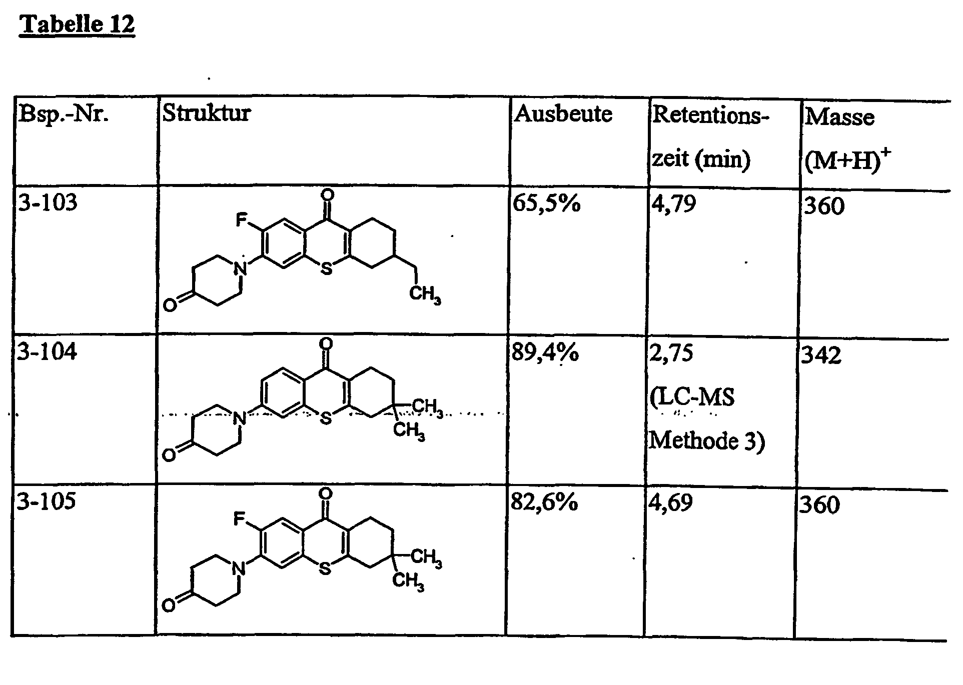

### Beispiel 3-106

### N-(3,3-Dimethyl-9-oxo-2,3,4,9-tetrahydro-1H-thioxanthen-6-yl)propanamid

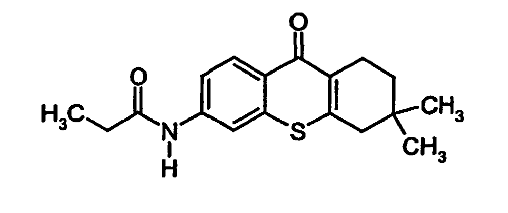

Zu einer Suspension von 1,50 g (5,78 mmol) 6-Amino-3,3-dimethyl-1,2,3,4-tetra hydro-9H-thioxanthen-9-on in 75 ml Dichlormethan werden 0,55 ml (6,36 mmol) Propionsäurechlorid getropft. Nach 10 min wird mit 0,89 ml (6,36 mmol) Triethylamin versetzt und es wird 1 h unter Rückfluß gerührt. Es werden 50 µl (0,6 mmol) Propionsäurechlorid und 80 µl (0,6 mmol) Triethylamin nachgesetzt und anschließend weitere 3 h bei Rückfluß gerührt. Nach Abkühlen wird die Reaktionslösung in 40 ml Eiswasser gegeben und nach Auftauen wird ein Teil des Produktes als Feststoff abfiltriert. Das Filtrat wird in 30 ml Dichlormethan aufgenommen und in 20 ml Eiswasser gegeben. Nach Auftauen wird weiteres Produkt als Feststoff abfiltriert
Ausbeute: 1,337 g (73,3%)
R_{f} (Cyclohexan/Essigsäureethylester 1:2 = 0,64
MS (EI): 316 (M+H)
HPLC, Retentionszeit = 4,73 min
¹H-NMR (200 MHz, DMSO-d₆): δ = 0,98 (s, 6 H), 1,09 (t, 3 H), 1,56 (t, 2 H), 2,39 (q, 2 H), 2,46-2,59 (m, 4 H), 7,54 (dd, 1 H), 8,17 (d, 1 H), 8,24 (d, 1 H), 10,35 (s, 1 H).

### Beispiel 3-107

### N-(3,3-Dimethyl-9-oxo-2,3,4,9-tetrahydro-1H-thioxanthen-6-yl)-N,N-dimethylglycinamid

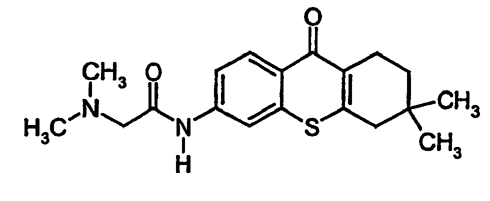

Unter Argon wird 0,476 g (4,62 mmol) N,N-Dimethylglycin zu einer Lösung von 1,10 g (4,20 mmol) 6-Amino-3,3-dimethyl-1,2,3,4-tetrahydro-9H-thioxanthen-9-on und 2,39 g (6,30 mmol) *o*-(7-Azobenzotriazol-1-yl)-N,N,N',N'-tetramethyluroniumhexafluorophosphat in 11 ml N,N-Dimethylformamid gegeben. Es wird 9 h bei 60°C gerührt. Nach Abkühlen wird das Reaktionsgemisch durch Präparative-HPLC (RP-C18, Acetonitril/Wasser-Gradient) gereinigt.
Ausbeute: 71 mg (92,8%)
R_{f} (Dichlormethan/Methanol 20:1 = 0,35
MS (EI): 345 (M+H)
HPLC, Retentionszeit = 4,02 min
¹H-NMR (200 MHz, DMSO-d₆): δ = 0,98 (s, 6 H), 1,57 (t, 2 H), 2,39-2,61 (m, 4 H), 2,88 (s, 6 H), 4,17 (s, 2 H), 7,59 (dd, 1 H), 8,10 (d, 1 H), 8,32 (d, 1 H), 10,9 (s, 1 H).

In Analogie zu dem für Beispiel 3-107 beschriebenen Verfahren werden die Verbindungen der Tabelle 13 erhalten.

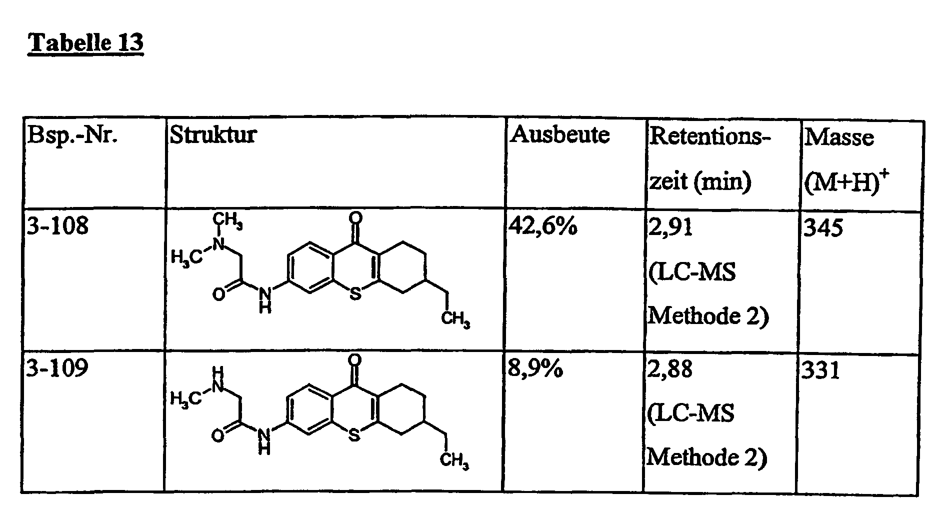

### Ausgangsverbindungen IV

### Beispiel IV-1

### N-(3-Ethyl-9-oxo-2,3,4,9-tetrahydro-1H-thioxanthen-6-yl)-1-butansulfonsäureamid

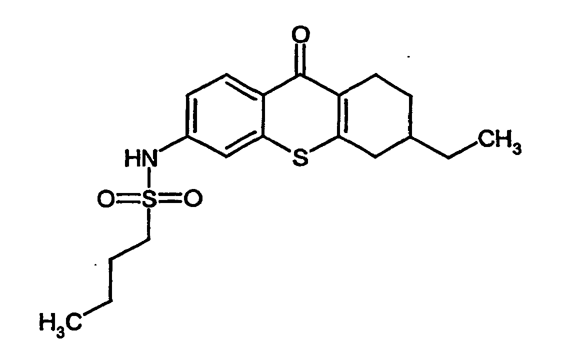

Zu einer Lösung von 75 mg (0,29 mmol) 6-Amino-3-ethyl-1,2,3,4-tethydro-9*H-*thioxanthen-9-on und 55 mg (0,35 mmol) Butansulfonylchlorid in 2 ml Methylenchlorid gibt man 120 µl (1,45 mmol) Pyridin sowie eine Spatelspitze 4-Dimethylaminopyridin. Man lässt über Nacht bei Raumtemperatur rühren. Anschließend wird das Reaktionsgemisch mit 30 ml Methylenchlorid verdünnt und nachfolgend mit Wasser, 1N Salzsäure und gesättigter Natriumchloridlösung gewaschen. Die organische Phase wird über Natriumsulfat getrocknet und eingeengt. Der erhaltene Rückstand wird aus Ethylacetat umkristallisiert. Man erhält 15mg (0,04 mmol, 13 % Ausbeute) des Produktes als hellgelben, mikrokristallinen Feststoff.
R_{f}-Wert: 0,23 (Cyclohexan/Ethylacetat 2:1).
¹H-NMR (300 MHz, CDCl₃, δ/ppm): 8,45 (d, 1H), 7,40 (d, 1H), 7,14 (dd, 1H), 6,69 (s, 1H), 3,16 (m, 2H), 2,91 (m, 1H), 2,61 (dd, 1H), 2,58-2,34 (m, 2H), 2,02 (m, 1H), 1,82 (m, 2H), 1,71 (m, 1H), 1,48-1,32 (m, 5H), 0,99 (t, 3H), 0,89 (t, 3H).
MS (ESI): 380,2 ([M+H]⁺).

### Beispiel IV-2

### N-(3-Ethyl-9-oxo-2,3,4,9-tetrahydro-1H-thioxanthen-6-yl)-3-pyridinsulfonsäureamid

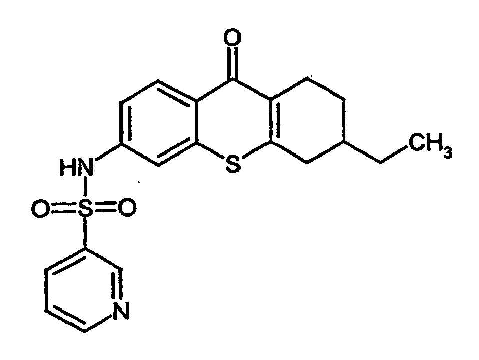

Analog zur Synthese von *N*-(3-Ethyl-9-oxo-2,3,4,9-tetrahydro-1*H*-thioxanthen-6-yl)-1-butansulfonsäureamid aus 75 mg (0,29 mmol) 6-Amino-3-ethyl-1,2,3,4-tetrahydro-9*H*-thioxanthen-9-on und 75 mg (0,35 mmol) 3-Pyridylsulfonsäurechlorid erhält man 60 mg (0,15 mmol, 51 % Ausbeute) des Produktes als blassgelben, mikrokristallinen Feststoff.
R_{f}-Wert: 0,21 (Cyclohexan/Ethylacetat 2:1).
MS (ESI): 401,1 ([M+H]⁺).

### Beispiel IV-3

### N-(3-Ethyl-9-oxo-2,3,4,9-tetrahydro-1H-thioxanthen-6-yl)-3-methylbenzen-sulfonsäureamid

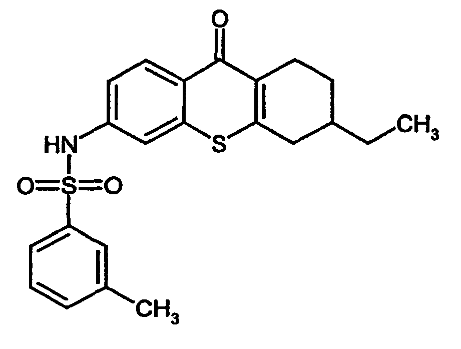

Analog zur Synthese von *N*-(3-Ethyl-9-oxo-2,3,4,9-tetrahydro-1*H*-thioxanthen-6-yl)-1-butansulfonsäureamid aus 100 mg (0,39 mmol) 6-Amino-3-ethyl-1 ,3,4-tetrahydro-9*H*-thioxanthen-9-on und 87 mg (0,46 mmol) *m*-Tolylsulfonsäurechlorid erhält man 14 mg (0,03 mmol, 9 % Ausbeute) des Produktes als farblosen, mikrokristallinen Feststoff.
R_{f}-Wert: 0,24 (Cyclohexan/Ethylacetat 2:1).
MS (ESI): 414 ([M+H]⁺).

### Beispiel IV-4

### 2-Cyano-N-(3-ethyl-9-oxo-2,3,4,9-tetrahydro-1H-thioxanthen-6-yl)-benzol-sulfonsäureamid

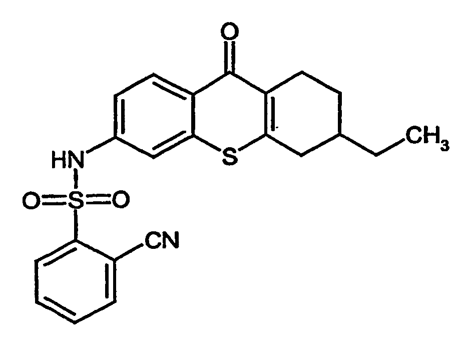

Analog zur Synthese von *N*-(3-Ethyl-9-oxo-2,3,4,9-tetrahydro-1*H*-thioxanthen-6-yl)-1-butansulfonsäureamid aus 75 mg (0,29 mmol) 6-Amino-3-ethyl-1,2,3,4-tetrahydro-9*H*-thioxanthen-9-on und 71 mg (0,35 mmol) 2-Cyanophenylsulfonsäurechlorid erhält man 60 mg (0,14 mmol, 49% Ausbeute) des Produktes als hellgelben, mikrokristallinen Feststoff.
R_{f}-Wert: 0,18 (Cyclohexan/Ethylacetat 2:1).
MS (ESI): 425,0 ([M+H]⁺).

### Beispiel IV-5

### 3-Cyano-N-(3-ethyl-9-oxo-2,3,4,9-tetrahydro-1H-thioxanthen-6-yl)-benzol-sulfonsäureamid

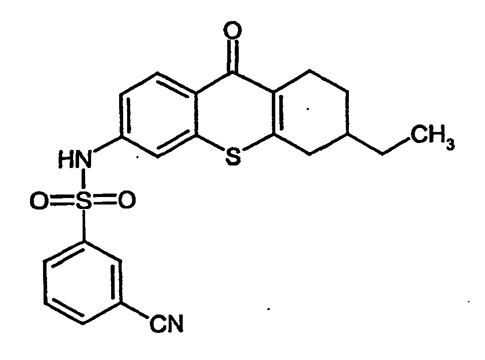

Analog zur Synthese von *N*-(3-Ethyl-9-oxo-2,3,4,9-tetrahydro-1*H*-thioxanthen-6-yl)-1-butansulfonsäureamid aus 75 mg (0,29 mmol) 6-Amino-3-ethyl-1,2,3,4-tetrahydro-9*H*-thioxanthen-9-on und 71 mg (0,35 mmol) 3-Cyanophenylsulfonsäurechlorid erhält man 20 mg (0,05 mmol, 16 % Ausbeute) des Produktes als hellgelben, mikrokristallinen Feststoff.
R_{f}-Wert: 0,18 (Cyclohexan/Ethylacetat 2:1).
MS (ESI): 425,0 ([M+H]⁺).

### Beispiel IV-6

### 4-Cyano-N-(3-ethyl-9-oxo-2,3,4,9-tetrahydro-1H-thioxanthen-6-yl)-benzolsulfonsäureamid

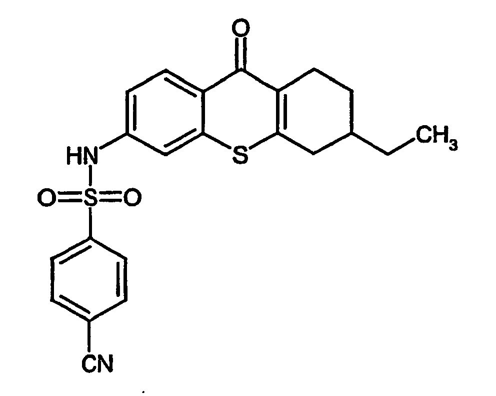

Analog zur Synthese von *N*-(3-Ethyl-9-oxo-2,3,4,9-tetrahydro-1*H*-thioxanthen-6-yl)-1-butansulfonsäureamid aus 100 mg (0,39 mmol) 6-Amino-3-ethyl-1,2,3,4-tetrahydro-9*H*-thioxanthen-9-on und 93 mg (0,46 mmol) 4-Cyanophenylsulfonsäurechlorid erhält man 140 mg (0,33 mmol, 85 % Ausbeute) des Produktes als hellgelben, mikrokristallinen Feststoff.
R_{f}-Wert: 0,18 (Cyclohexan/Ethylacetat 2:1).
MS (ESI): 425,0 ([M+H]⁺).

### Beispiel IV-7

### N-(3-Ethyl-9-oxo-2,3,4,9-tetrahydro-1H-thioxanthen-6-yl)-1-propansulfonsäureamid

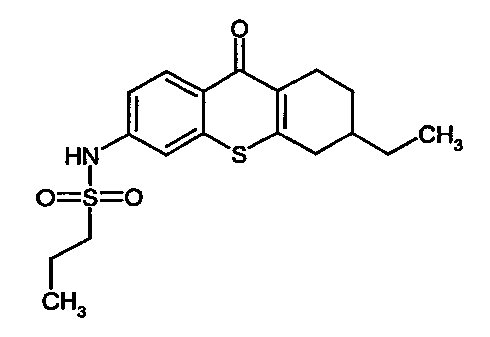

Analog zur Synthese von *N*-(3-Ethyl-9-oxo-2,3,4,9-tetrahydro-1H-thioxanthen-6-yl)-1-butansulfonsäureamid aus 75 mg (0,29 mmol) 6-Amino-3-ethyl-1,2,3,4-tetrahydro-9*H*-thioxanthen-9-on und 50 mg (0,35 mmol) *n*-Propylsulfonsäurechlorid erhält man 25 mg (0,07 mmol, 23 % Ausbeute) des Produktes als gelben, mikrokristallinen Feststoff.
R_{f}-Wert: 0,19 (Cyclohexan/Ethylacetat 2:1).
¹H-NMR (300 MHz, DMSO-d₆, δ/ppm): 10.49 (s, 1H), 8,25 (d, 1H), 7,38 (d, 1H), 7,33 (dd, 1H) 3,24 (t, 2H), 2,70 (m, 2H), 2,41 (t, 2H), 2,11 (t, 2H), 1,95 (m, 1H), 1,67 (m, 2H), 1,37 (m, 2H), 1,11 (1, 3H), 0,93 (t, 3H).
MS (ESI): 366,2 ([M+H]⁺).

### Beispiel IV -8

### N-(3-Ethyl-9-oxo-2,3,4,9-tetrahydro-1H-thioxanthen-6-yl)-cyclopropan-sulfonsäureamid

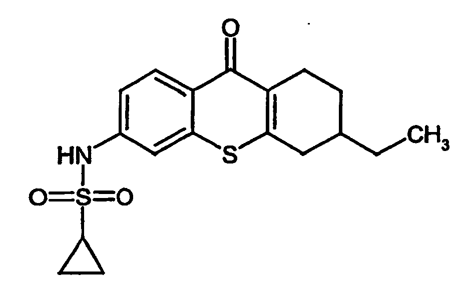

Analog zur Synthese von *N*-(3-Ethyl-9-oxo-2,3,4,9-tetrahydro-1*H* thioxanthen-6-yl)-1-butansulfonsäureamid aus 150 mg (0,58 mmol) 6-Amino-3-ethyl-1,2,3,4-tetrahydro-9*H*-thioxanthen-9-on und 97 mg (0,69 mmol) Cyclopropylsulfonsäurechlorid erhält man 71 mg (0,19 mmol, 34 % Ausbeute) des Produktes als farblosen, mikrokristallinen Feststoff.
R_{f}-Wert: 0,25 (Cyclohexan/Ethylacetat 2:1).
MS (ESI): 364 ([M+H]⁺).

### Beispiel IV-9

### N-(3-Ethyl-9-oxo-2,3,4,9-tetrahydro-1H-thioxanthen-6-yl)-4-methoxybenzensulfonsäureamid

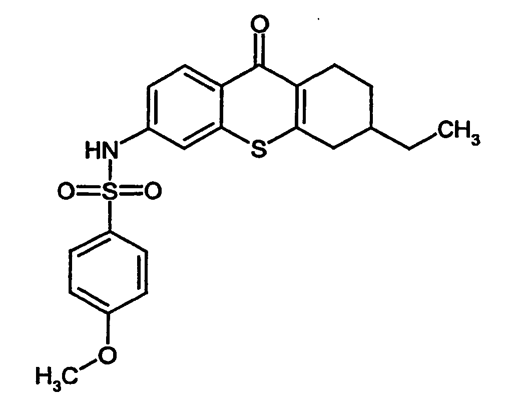

Analog zur Synthese von *N*-(3-Ethyl-9-oxo-2,3,4,9-tetrahydro-1H-thioxanthen-6-yl)-1-butansulfonsäureamid aus 100 mg (0,39 mmol) 6-Amino-3-ethyl-1,2,3,4-tetrahydro-9*H* thioxanthen-9-on und 95 mg (0,46 mmol) 4-Methoxyphenylsulfonsäurechlorid erhält man 110 mg (0,26 mmol, 66 % Ausbeute) des Produktes als blassgelben, mikrokristallinen Feststoff.
R_{f}-Wert: 0,15 (Ethylacetat/Cyclohexan 2:1).
MS (ESI): 430 ([M+H]⁺).

### Beispiel IV-10

### 3-Chlor-N-(3-ethyl-9-oxo-2,3,4,9-tetrahydro-1H-thioxanthen-6-yl)benzolsulfonsäureamid

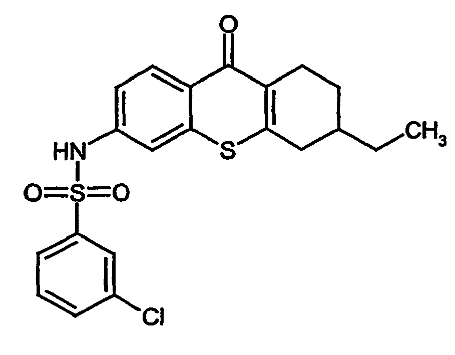

Analog zur Synthese von *N*-(3-Ethyl-9-oxo-2,3,4,9-tetrahydro-1*H*-thioxanthen-6-yl)-1-butansulfonsäureamid aus 100 mg (0,39 mmol) 6-Amino-3-ethyl-1,2,3,4-tetrahydro-9*H*-thioxanthen-9-on und 89 mg (0,42 mmol) 3-Chlorophenylsulfonsäurechlorid erhält man 30 mg (0,07 mmol, 18 % Ausbeute) des Produktes als farblosen, mikrokristallinen Feststoff.
R_{f}-Wert: 0,33 (Ethylacetat/Cyclohexan 2:1).
MS (ESI): 434 ([M+H]⁺).

### Beispiel IV-11

### N-(3-Ethyl-9-oxo-2,3,4,9-tetrahydro-1H-thioxanthen-6-yl)-2-methylbenzolsulfonsäureamid

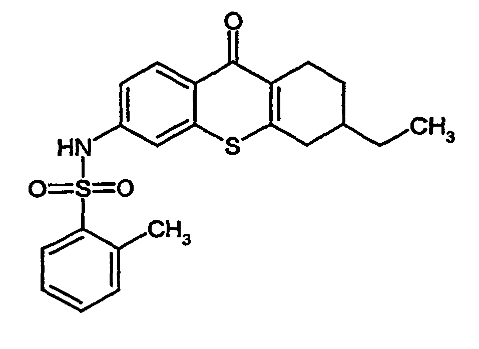

Analog zur Synthese von *N*-(3-Ethyl-9-oxo-2,3,4,9-tetrahydro-1*H*-thioxanthen-6-yl)-1-butansulfonsäureamid aus 100 mg (0,39 mmol) 6-Amino-3-ethyl-1,2,3,4-tetrahydro-9*H*-thioxanthen-9-on und 88 mg (0,46 mmol) *o*-Tolylsulfonsäurechlorid erhält man 36 mg (0,09 mmol, 23 % Ausbeute) des Produktes als hellgelben, mikrokristallinen Feststoff.
R_{f}-Wert: 0,16 (Ethylacetat/Cyclohexan 2:1).
MS (ESI): 414,1 ([M+H]⁺).

### Beispiel IV-12

### N-(3-Ethyl-9-oxo-2,3,4,9-tetrahydro-1H-thioxanthen-6-yl)-2,4-dimethylbenzolsulfonsäureamid

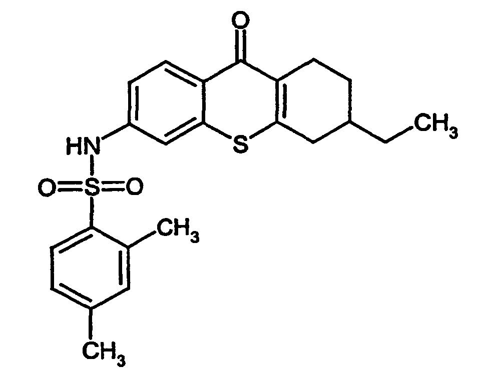

Analog zur Synthese von *N*-(3-Ethyl-9-oxo-2,3,4,9-tetrahydro-1*H*-thioxanthen-6-yl)-1-butansulfonsäureamid aus 100 mg (0,39 mmol) 6-Amino-3-ethyl-1,2,3,4-tetrahydro-9*H*-thioxanthen-9-on und 94 mg (0,46 mmol) 2,4-Dimethylphenylsulfonsäurechlorid erhält man 80 mg (0,19 mmol, 48 % Ausbeute) des Produktes als gelben, mikrokristallinen Feststoff.
R_{f}-Wert: 0,20 (Ethylacetat/Cyclohexan 2:1).
MS (ESI): 428 ([M+H]⁺).

### Beispiel IV-13

### N-(3-Ethyl-9-oxo-2,3,4,9-tetrahydro-1H-thioxanthen-6-yl)-3-nitrobenzensulfonsäureamid

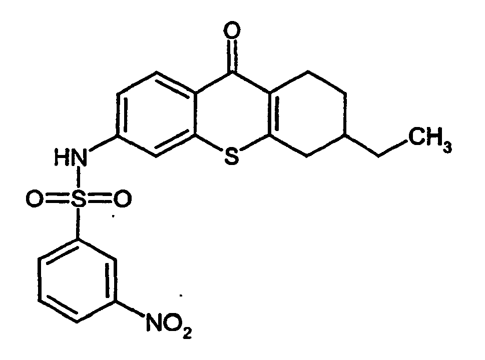

Analog zur Synthese von *N*-(3-Ethyl-9-oxo-2,3,4,9-tetrahydro-1*H* thioxanthen-6-yl)-1-butansulfonsäureamid aus 150 mg (0,58 mmol) 6-Amino-3-ethyl-1,2,3,4-tetrahydro-9*H*-thioxanthen-9-on und 153 mg (0,69 mmol) 3 Nitrophenylsulfonsäurechlorid erhält man 70 mg (0,15 mmol, 27 % Ausbeute) des Produktes als farblosen, mikrokristallinen Feststoff.
R_{f}-Wert: 0,07 (Ethylacetat/Cyclohexan 2: 1).
MS (ESI): 444,9 ([M+H]⁺).

### Beispiel IV-14

### 5-Chlor-N-(3-ethyl-9-oxo-2,3,4,9-tetrahydro-1H-thioxanthen-6-yl)-2-thiophen-sulfonsäureamid

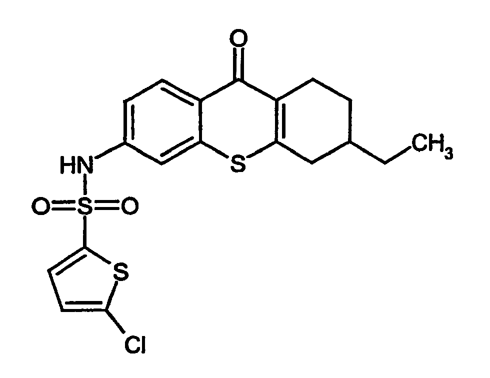

Analog zur Synthese von *N*-(3-Ethyl-9-oxo-2,3,4,9-tetrahydro-1*H*-thioxanthen-6-yl)-1-butansulfonsäureamid aus 150 mg (0,58 mmol) 6-Amino-3-ethyl-1,2,3,4-tetrahydro-9*H*-thioxanthen-9-on und 150 mg (0,69 mmol) 5-Chlorothiophensnlfonsäurechlorid erhält man 105 mg (0,24 mmol, 41 % Ausbeute) des Produktes als gelben, mikrokristallinen Feststoff.
R_{f}-Wert: 0,33 (Ethylacetat/Cyclohexan 2:1).
MS (ESI): 440 ([M+H]⁺).

### Beispiel IV-15

### N-(3-Ethyl-9-oxo-2,3,4,9-tetrahydro-1H-thioxanthen-6-yl)-2-naphthalensulfonsäureamid

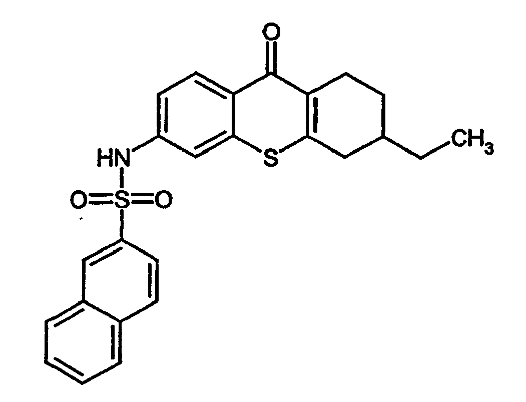

Analog zur Synthese von *N*-(3-Ethyl-9-oxo-2,3,4,9-tetrahydro-1*H*-thioxanthen-6-yl)-1-butansulfonsäureamid aus 150 mg (0,58 mmol) 6-Amino-3-ethyl-1,2,3,4-tetrahydro-9*H*-thioxanthen-9-on und 156 mg (0,69 mmol) 2-Naphthalensulfonsäurechlorid erhält man 73 mg (0,16 mmol, 28 % Ausbeute) des Produktes als gelben, mikrokristallinen Feststoff.
R_{f}-Wert: 0,41 (Ethylacetat/Cyclohexan 2: 1).
MS (ESI): 450 ([M+H]⁺).

### Beispiel IV-16

### N-(3-Ethyl-9-oxo-2,3,4,9-tetrahydro-1H-thioxanthen-6-yl)-2-thiophensulfonsäureamid

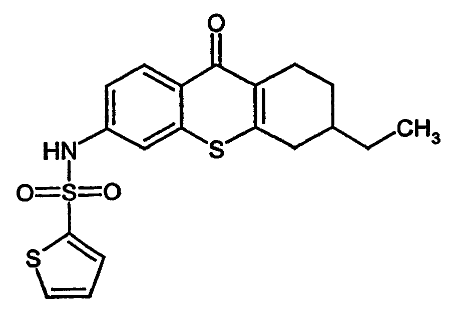

Analog zur Synthese von *N*-(3-Ethyl-9-oxo-2,3,4,9-tetrahydro-1*H*-thioxanthen-6-yl)-1-butansulfonsäureamid aus 150 mg (0,58 mmol) 6-Amino-3-ethyl-1,2,3,4-tetrahydro-9*H*-thioxanthen-9-on und 126 mg (0,69 mmol) 2-Thiophensulfonsäurechlorid erhält man 123 mg (0,30 mmol, 52 % Ausbeute) des Produktes als hellgelben Feststoff.
R_{f}-Wert: 0,36 (Ethylacetat/Cyclohexan2:1).
MS (ESI): 406 ([M+H]⁺).

### Beispiel IV-17

### 2-(1,3-Dioxo-1,3-dihydro-2H-isoindol-2-yl)-N-(3-ethyl-9-oxo-2,3,4,9-tetrahydro-1H thioxanthen-6-yl)ethansulfonsäureamid

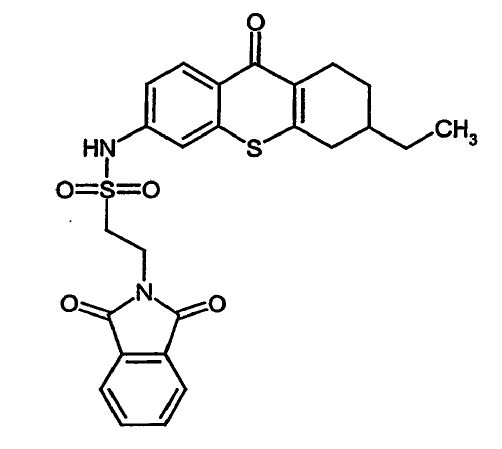

Analog zur Synthese von *N*-(3-Ethyl-9-oxo-2,3,4,9-tetrahydm-1*H*-thioxanthen-6-yl)-1-butansulfonsäureamid aus 150 mg (0,58 mmol) 6-Amino-3-ethyl-1,2,3,4-tetra hydro-9*H*-thioxanthen-9-on und 188 mg (0,69 mmol) 2-(1,3-Dioxo-1,3-dihydro-2*H-*isomdol-2-yl)ethausulfosäurechlorid erhält man 125 mg (0,25 mmol; 42% Ausbeute) des Produktes als hellgelben Feststoff.
R_{f}- Wert: 0,32 (Ethylacetat/Cyclohexan 2:1).
MS (ESI): 497,1 ([M+H]⁺).

### Ausführungsbeispiele 4

### Beispiel 4-1

### 6-Amino-3-ethyl-1,2,3,4-tetrahydro-9H thioxanthen-9-on

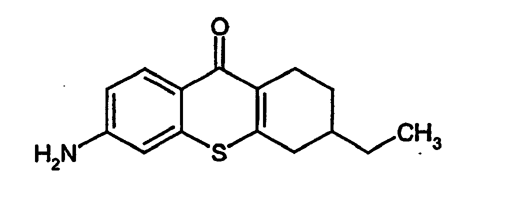

426 g Polyphosphorsäure werden unter Argon 15 Minuten bei Raumtemperatur gerührt. Anschließend erwärmt man für 5 Minuten auf ca. 150°C, lässt abkühlen und gibt vorsichtig 15,0 g (120 mmol) 3-Aminothiophenol und 21,6 g (109 mmol) 4-Ethyl-2-oxocyclohexanecarbonsäureethylester hinzu. Man rührt 2 Stunden bei 90°C und lässt auf Raumtemperatur abkühlen. Die erhaltene rote Mischung wird mit 430 ml Eiswasser versetzt, 30 Minuten nachgerührt und zehnmal mit Ethylacetat ausgeschüttelt. Die vereinigten organischen Phasen werden nachfolgend mit Wasser, gesättigter Natriumhydrogencarbonatlösung, Wasser und gesättigter Natriumchloridlösung gewaschen und über Natriumsulfat getrocknet. Nach dem Entfernen des Lösungsmittels wird das Rohprodukt mit Methylenchlorid versetzt, wobei ein Teil des Produktes (5,46 g) als gelber Feststoff ausfällt. Das Filtrat wird säulenchromatographisch gereinigt (Kieselgel, Cyclohexan/Etyhlacetat 40:1). Die Produktfraktion (6,18 g) wird eingeengt und am Vakuum getrocknet. Es werden insgesamt 11,64 g (44,9 mmol, 41 % Ausbeute) eines gelben Feststoffes erhalten.
R_{f}-Wert: 0,17 (Cyclohexan/Ethylacetat 2:1).
¹H-NMR (300 MHz, DMSO-d₆, δ/ppm): 7,98 (d, 1H), 6,72 (dd, 1H), 6,60 (d, 1H), 6,12 (s, 2H), 2,66 (m, 2H), 2,32 (m, 2H), 1,91 (m, 1H), 1,62 (m, 1H), 1,34 (m, 3H), 0,93 (t, 3H).
MS (EI): 259 (M⁺).

### Beispiel 4-2

### 3-Ethyl-6-(ethylamino)-1 ,2,3,4-tetrahydro-9H-thioxanthen-9-on

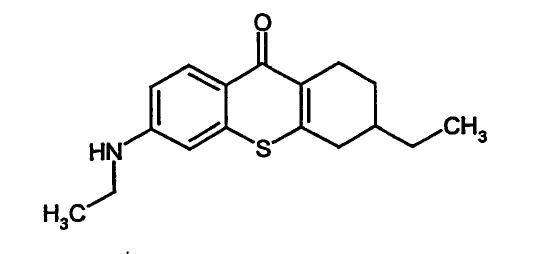

Eine Lösung von 1,5 g (5,8 mmol) 6-Amino-3-ethyl-1,2,3,4-tetrahydro-9H thioxanthen-9-on in 40 ml Eisessig wird mit 2,19 mg (57,8 mmol) Natriumborhydrid versetzt und 2 Stunden bei Raumtemperatur gerührt. Anschließend wird die Lösung mit 200 ml Wasser verdünnt und mit 2N Natronlauge bis zum pH-Wert von 9 versetzt. Man extrahiert mit Methylenchlorid, trocknet über Natriumsulfat und engt dann bis zur Trockne ein. Das Rohprodukt wird säulenchromatographisch gereinigt (Kieselgel, Methylenchlorid/Methanol 600:1-100:1). Die Produktfraktion wird eingeengt und am Vakuum getrocknet. Es werden 1,01 g (3,51 mmol, 60 % Ausbeute) eines blassgelben Feststoffes erhalten. Als Nebenprodukt wird 6-(Diethylamino)-3-ethyl-1,2,3,4-tetrahydro-9*H*-thioxanthen-9-on erhalten.
R_{f}- Wert: 0,74 (Methylenchlorid/Methanol 20:1).
¹H-NMR (300 MHz, DMSO-d₆, δ/ppm): 7,99 (d, 1H), 6,76 (dd, 1H), 6,65 (t, 1H), 6,56 (d, 1H), 3,12 (dq, 2H), 2,67 (m, 2H), 2,34 (m, 2H), 1,92 (m, 1H), 1,60 (m, 1H), 1,32 (m, 3H), 1,18 (t, 3H), 0,94 (t, 3H).
MS (EI): 287 (M⁺).

### Beispiel 4-3

### 6-(Diethylamino)-3-ethyl-1,2,3,4-tetrahydro-9H-thioxanthen-9-on

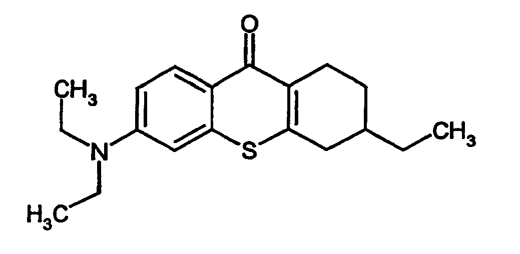

6-(-Diethylamino)-3-ethyl-1,2,3,4-tetrahydro-9*H*-thioxanthen-9-on wird als Nebenprodukt bei der Synthese von 3-Ethyl-6-(ethylamino)-1,2,3,4-tetrahydro-9*H*-thioxanthen-9-on erhalten. Es werden 373 mg (1,18 mmol, 20 % Ausbeute) eines blassgelben Feststoffes isoliert.
R_{f}- Wert: 0,37 (Cyclohexan/Ethylacetat 20:1).
¹H-NMR (300 MHz, DMSO-d₆, δ/ppm): 8,07 (d, 1H), 6,89 (dd, 1H), 6,68 (d, 1H), 3,42 (q, 4H), 2,67 (m, 2H), 2,34 (m, 2H), 1,92 (m, 1H), 1,60 (m, 1H), 1,32 (m, 3H), 1,12 (t, 6H), 0,94 (t, 3H).
MS (EI): 315 (M⁺).

### Beispiel 4-4

### N-(3-Ethyl-9-oxo-2,3,4,9-tetrahydro-1H-thioxanthen-6-yl)-N-methyl-1-butansulfonsäureamid

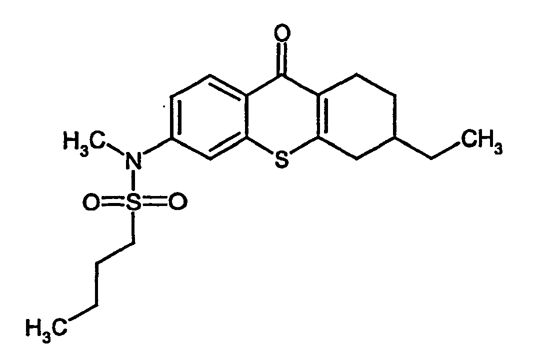

Zu einer Lösung von 30 mg (0,08 mmol) *N*-(3-Ethyl-9-oxo-2,3,4,9-tetrahydro-1*H-*thioxanthen-6-yl)-1-butansulfonsäureamid in 2 ml Aceton gibt man 45 mg (0,32 mmol) Methyliodid und 109 mg (0,79 mmol) Kaliumcarbonat. Man lässt über Nacht unter Rückfluss rühren. Anschließend wird das Reaktionsgemisch bis zur Trockne eingeengt und der Rückstand zwischen Methylenchlorid und Wasser verteilt. Die organische Phase wird über Natriumsulfat getrocknet und eingeengt. Der erhaltene Rückstand wird mittels präparativer HPLC (Säule: Kromasil 120 ODS-4 HE, 10 µm, 250 x 20 mm; Eluent: Acetonitril/Wasser; Fluss: 25 ml/min; UV-Detektion bei 210 nm) aufgetrennt. Man erhält 9 mg (0,02 mmol, 29 % Ausbeute) des Produktes als farblosen, kristallinen Feststoff.
R_{f}-Wert: 0,44 (Cyclohexan/Ethylacetat 2:1).
MS (ESI): 394 ([M+H]⁺).

### Beispiel 4-5

### N-(3-Ethyl-9-oxo-2,3,4,9-tet-ahydro-1H-thioxanthen-6-yl)-N-methyl-3-pyridinsulfonsäureamid

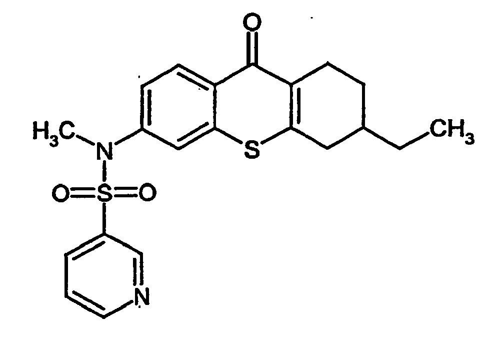

Analog zur Synthese von *N*-(3-Ethyl-9-oxo-2,3,4,9-tetrahydro-1*H*-thioxanthen-6-yl)-*N*-methyl-1-butansulfonsäureamid aus 30 mg (0,07 mmol) *N*-(3-Ethyl-9-oxo-2,3,4,9-tetrahydro-1*H*-thioxanthen-6-yl)3-pyridinsulfonsäureamid, 39 mg (0,28 mmol) Methyliodid und 97 mg (0,70 mmol) Kaliumcarbonat erhält man 7 mg (0,02 mmol, 21 % Ausbeute) des Produktes als hellgelben, mikrokristallinen Feststoff.
R_{f}-Wert: 0,38 (Cyclohexan/Ethylacetat 2:1).
MS (ESI): 415,2 ([M+H]⁺).

### Beispiel 4-6

### N-(3-Ethyl-9-oxo-2,3,4,9-tetrahydro-1H-thioxanthen-6-yl)-N,3-dimethylbenzolsulfonsäureamid

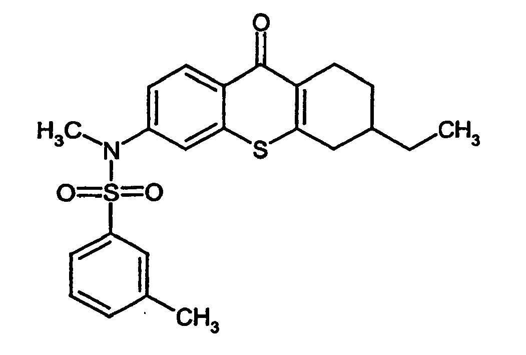

Analog zur Synthese von *N*-(3-Ethyl-9-oxo-2,3,4,9-tetrahydro-1*H*-thioxanthen-6-yl)-*N-*methyl-1-butansulfonsäureamid aus 50 mg (0,12 mmol) *N*-(3-Ethyl-9-oxo-2,3,4,9-tetrahydro-1*H*-thioxanthen-6-yl)-3-methylbenzolsulfonsäureamid, 68 mg (0,48 mmol) Methyliodid und 167 mg (1,21 mmol) Kaliumcarbonat erhält man 24 mg (0,06 mmol, 47 % Ausbeute) des Produktes als farblosen, mikrokristallinen Feststoff.
R_{f}-Wert: 0,46 (Cyclohexan/Ethylacetat 20:1).
MS (ESI): 428,1 ([M+H]⁺).

### Beispiel 4-7

### 2-Cyano-N-(3-ethyl-9-oxo-2,3,4,9-tetrahydro-1H-thioxanthen-6-yl)-N-methylbenzolsulfonsäureamid

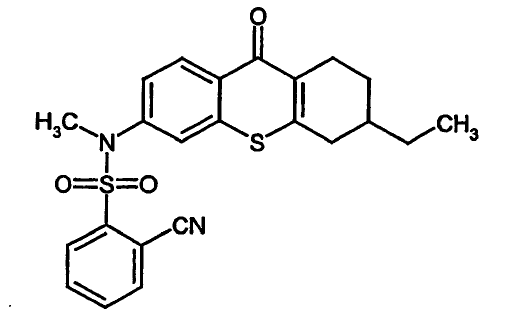

Analog zur Synthese von *N*-(3-Ethyl-9-oxo-2,3,4,9-tetrahydro-1*H*-thioxanthen-6-yl)-*N*-methyl-1-butansulfonsäureamid aus 50 mg (0,12 mmol) 2-Cyano-*N*-(3-ethyl-9-oxo-2,3,4,9-tetrahydro-1*H*-thioxanthen-6-yl)-benzolsulfonsäureamid, 67 mg (0,47 mmol) Methyliodid und 98 mg (0,71 mmol) Kaliumcarbonat erhält man 39 mg (0,09 mmol, 75 % Ausbeute) des Produktes als farblosen, mikrokristallinen Feststoff.
R_{f}-Wert: 0,43 (Cyclohexan/Ethylacetat 2:1).
¹H-NMR (300 MHz, CDCl₃, δ/ppm): 8,38 (d, 1H), 7,72 (m, 2H), 7,65 (m, 2H), 7,50 (d, 1H), 7,13 (dd, 1H), 3,48 (s, 3H), 2,89 (m, 1H), 2,70 (dd, 1H), 2,55-2,35 (m, 2H), 2,03 (m, 1H), 1,70 (m, 1H), 1,48-1,36 (m, 3H), 0,99 (t, 3H).
MS (ESI): 439,2 ([M+H]⁺).

### Beispiel 4-8

### 3-Cyano-N-(3-ethyl-9-oxo-2,3,4,9-tetrahydro-1H-thioxanthen-6-yl)-N-methylbenzolsulfonsäureamid

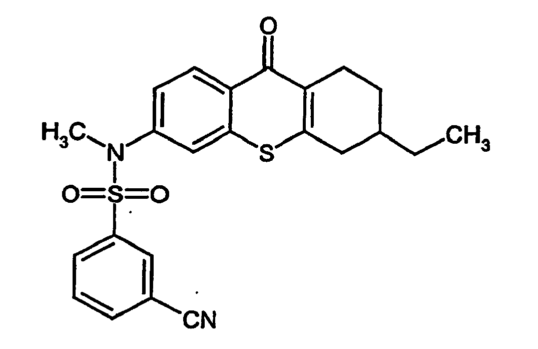

Analog zur Synthese von *N*-(3-Ethyl-9-oxo-2,3,4,9-tetrahydro-1*H*-thioxanthen-6-yl)-*N*-methyl-1-butansulfonsäureamid aus 50 mg (0,12 mmol) 3-Cyano-*N*-(3-ethyl-9-oxo-2,3,4,9-tetrahydro-1*H*-thioxanthen-6-yl)-benzolsulfonsäureamid, 67 mg (0,47 mmol) Methyliodid und 98 mg (0,71 mmol) Kaliumcarbonat erhält man 27 mg (0,06 mmol, 52 % Ausbeute) des Produktes als farblosen, mikrokristallinen Feststoff.
R_{f}-Wert: 0,43 (Cyclohexan/Ethylacetat 2:1).
¹H-NMR (300 MHz, CDCl₃, δ/ppm): 8,41 (d, 1H), 7,90 (m, 2H), 7,66 (m, 1H), 7,60 (d, 1H), 7,38 (d, 1H), 7,08 (dd, 1H), 3,27 (s, 3H), 2,91 (m, 1H), 2,72 (dd, 1H), 2,60-2,35 (m, 2H), 2,05 (m, 1H), 1,71 (m, 1H), 1,47-1,34 (m, 3H), 0,99 (t, 3H).
MS (ESI): 439,1 ([M+H]⁺).

### Beispiel 4-9

### 4-Cyano-N-(3-ethyl-9-oxo-2,3,4,9-tetrahydro-1H-thioxanthen-6-yl)-N-methylbenzolsulfonsäureamid

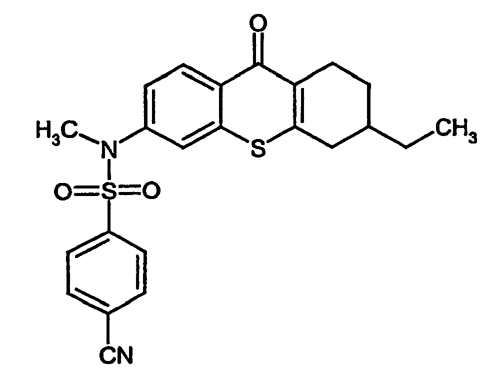

Analog zur Synthese von *N*-(3-Ethyl-9-oxo-2,3,4,9-tetrahydro-1*H*-thioxanthen-6-yl)-*N*-methyl-1-butansulfonsäureamid aus 50 mg (0,12 mmol) 4-Cyano-*N*-(3-ethyl-9-oxo-2,3,4,9-tetrahydro-1*H*-thioxanthen-6-yl)-benzolsulfonsäureamid, 67 mg (0,47 mmol) Methyliodid und 163 mg (1,18 mmol) Kaliumcarbonat erhält man 31 mg (0,07 mmol, 60 % Ausbeute) des Produktes als farblosen, amorphen Feststoff.
R_{f}-Wert: 0,41 (Cyclohexan/Ethylacetat 2:1).
¹H-NMR (300 MHz, CDCl₃, δ/ppm): 8,42 (d, 1H), 7,75 (d, 2H), 7,64 (d, 2H), 7,36 (d, 1H), 7,11 (dd, 1H), 3,27 (s, 3H), 2,91 (m, 1H), 2,72 (dd, 1H), 2,59-2,36 (m, 2H), 2,06 (m, 1H), 1,71 (m, 1H), 1,48-1,37 (m, 3H), 1,00 (t, 3H).
MS (ESI): 439 ([M+H]⁺).

### Beispiel 4-10

### N-(3-Ethyl-9-oxo-2,3,4,9-tetrahydro-1H-thioxanthen-6-yl)-N-methyl-1-propansulfonsäureamid

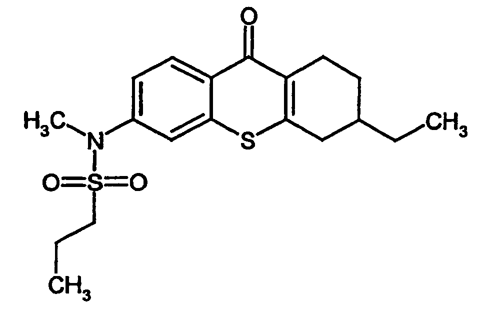

Analog zur Synthese von *N*-(3-Ethyl-9-oxo-2,3,4,9-tetrahydro-1*H*-thioxanthen-6-yl)-*N-*methyl-1-butansulfonsäureamid aus 50 mg (0,14 mmol) *N*-(3-Ethyl-9-oxo-2,3,4,9-tetrahydro-1*H*-thioxanthen-6-yl)-1-propansulfonsäureamid, 78 mg (0,55 mmol) Methyliodid und 190 mg (1,38 mmol) Kaliumcarbonat erhält man 8 mg (0,02 mmol, 15 % Ausbeute) des Produktes als farblosen, mikrokristallinen Feststoff.
R_{f}-Wert: 0,29 (Cyclohexan/Ethylacetat 2:1).
¹H-NMR (300 MHz, CDCl₃, δ/ppm): 8,48 (d, 1H), 7,56 (d, 1H), 7,45 (dd, 1H), 3,40 (s, 3H), 3,00 (m, 2H), 2,91 (m, 1H), 2,71 (dd, 1H), 2,58-2,35 (m, 2H), 2,06 (m, 1H), 1,82 (m, 2H), 1,71 (m, 1H), 1,48-1,36 (m, 3H), 1,02 (t, 3H), 0,99 (t, 3H).
MS (ESI): 380 ([M+H]⁺).

### Beispiel 4-11

### N-(3-Ethyl-9-oxo-2,3,4,9-tetrahydro-1H-thioxanthen-6-yl)-N-methylcyclopropansulfonsäureamid

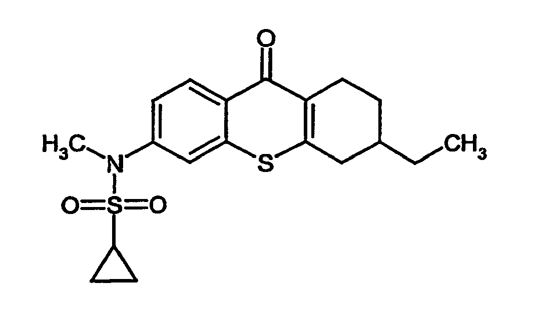

Analog zur Synthese von *N*-(3-Ethyl-9-oxo-2,3,4,9-tetrahydro-1*H*-thioxanthen-6-yl)-*N*-methyl-1-butansulfonsäureamid aus 50 mg (0,14 mmol) *N*-(3-Ethyl-9-oxo-2,3,4,9-tetrahydro-1*H*-thioxanthen-6-yl)-cyclopropansulfonsäureamid, 78 mg (0,55 mmol) Methyliodid und 190mg (1,38 mmol) Kaliumcarbonat erhält man 18 mg (0,05 mmol, 35 % Ausbeute) des Produktes als farblosen, amorphen Feststoff.
R_{f}-Wert: 0,31 (Cyclohexan/Ethylacetat 2:1).
MS (ESI): 378 ([M+H]⁺).

### Beispiel 4-12

### N-(3-Ethyl-9-oxo-2,3,4,9-tetrahydro-1H-thioxanthen-6-yl)-4-methoxy-N-methylbenzolsulfonsäureamid

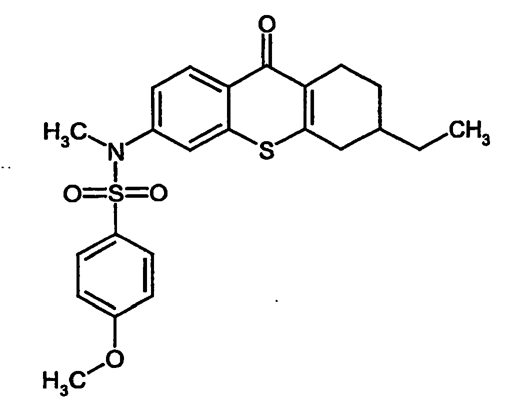

Analog zur Synthese von *N*-(3-Ethyl-9-oxo-2,3,4,9-tetrahydro-1*H*-thioxanthen-6-yl)-*N*-methyl-1-butansulfonsäureamid aus 50 mg (0,12 mmol) *N*-(3-Ethyl-9-oxo-2,3,4,9-tetrahydro-1*H*-thioxanthen-6-yl)-4-methoxybenzolsulfonsäureamid, 67 mg (0,47 mmol) Methyliodid und 160 mg (1,16 mmol) Kaliumcarbonat erhält man 14 mg (0,03 mmol, 27 % Ausbeute) des Produktes als farblosen, amorphen Feststoff.
R_{f}- Wert: 0,45 (Cyclohexan/Ethylacetat 2:1).
¹H-NMR (300 MHz, CDCl₃, δ/ppm): 8,44 (d, 1H), 7,57 (t, 1H), 7,42 (m, 3H), 7,12 (dd, 1H), 3,26 (s, 3H), 2,92 (m, 1H), 2,72 (dd, 1H), 2,58-2,37 (m, 2H), 2,07 (m, 1H), 1,72 (m, 1H), 1,48-1,37 (m, 3H), 1,01 (t, 3H).
MS (ESI): 443,9 ([M+H]⁺).

### Beispiel 4-13

### 3-Chlor-N-(3-ethyl-9-oxo-2,3,4,9-tetrahydro-1H-thioxanthen-6-yl)-N-methyl-benzolsulfonsäureamid

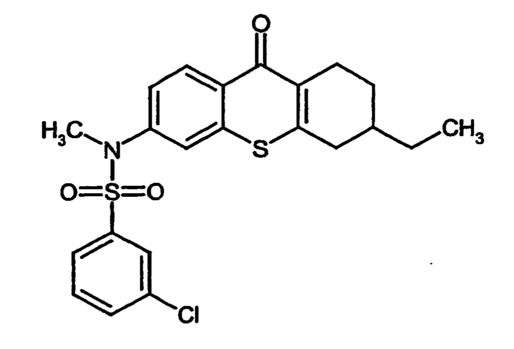

Analog zur Synthese von *N*-(3-Ethyl-9-oxo-2,3,4,9-tetrahydro-1*H*-thioxanthen-6-yl)-*N*-methyl-1-butansulfonsäureamid aus 50 mg (0,12 mmol) 3-Chlor-*N*-(3-ethyl-9-oxo-2,3,4,9-tetrahydro-1*H*-thioxanthen-6-yl)benzolsulfonsäureamid, 65 mg (0,46 mmol) Methyliodid und 159 mg (1,15 mmol) Kaliumcarbonat erhält man 11 mg (0,02 mmol, 21 % Ausbeute) des Produktes als hellgelben, mikrokristallinen Feststoff.
R_{f}-Wert: 0,41 (Cyclohexan/Ethylacetat 2:1).
MS (ESI): 448,0 ([M+H]⁺).

### Beispiel 4-14

### N-(3-Ethyl-9-oxo-2,3,4,9-tetrahydro-1H-thioxanthen-6-yl)-N,2-dimethyl-benzolsulfonsäureamid

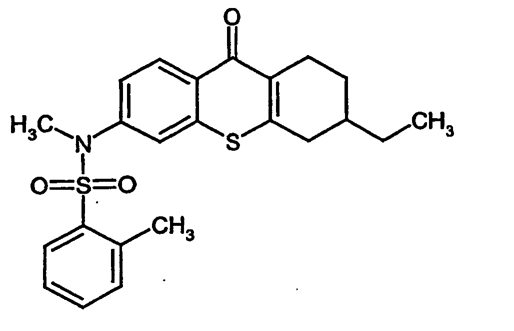

Analog zur Synthese von *N*-(3-Ethyl-9-oxo-2,3,4,9-tetrahydro-1*H*-thioxanthen-6-yl)-*N*-methyl-1-butansulfonsäureamid aus 40 mg (0,10 mmol) *N*-(3-Ethyl-9-oxo-2,3,4,9-tetrahydro-1*H* thioxanthen-6-yl)-2-methylbenzolsulfonsäureamid, 55 mg (0,39 mmol) Methyliodid und 134 mg (0,97 mmol) Kaliumcarbonat erhält man 27 mg (0,06 mmol, 65 % Ausbeute) des Produktes als farblosen, amorphen Feststoff.
R_{f}-Wert: 0,48 (Cyclohexan/Ethylacetat 2:1).
MS (ESI): 428,1 ([M+H]⁺).

### Beispiel 4-15

### N-(3-Ethyl-9-oxo-2,3,4,9-tetrahydro-1H-thioxanthen-6-yl)-N,2,4-trimethylbenzolsulfon-säureamid

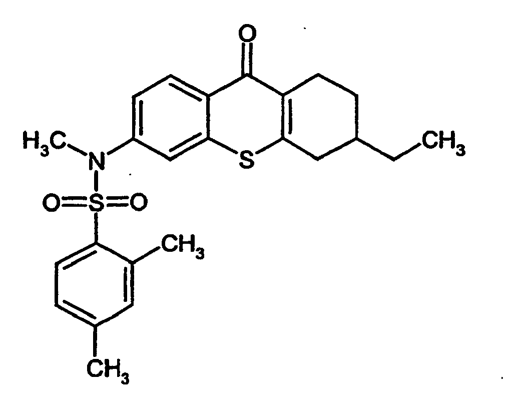

Analog zur Synthese von *N*-(3-Ethyl-9-oxo-2,3,4,9-tetrahydro-1*H*-thioxanthen-6-yl)-*N*-methyl-1-butansulfonsäureamid aus 50 mg (0,12 mmol) *N*-(3-Ethyl-9-oxo-2,3,4,9-tetrahydro-1*H*-thioxanthen-6-yl)-2,4-dimethylbenzolsulfonsäureamid, 67 mg (0,47 mmol) Methyliodid und 162 mg (1,17 mmol) Kaliumcarbonat erhält man 37 mg (0,08 mmol, 71 % Ausbeute) des Produktes als farblosen, amorphen Feststoff.
R_{f}-Wert: 0,46 (Cyclohexan/Ethylacetat 2:1).
MS (ESI): 442,4 ([M+H]⁺).

### Beispiel 4-16

### N-(3-Ethyl-9-oxo-2,3,4,9-tetrahydro-1H-thioxanthen-6-yl)-N-methyl-3-nitro-benzolsulfonsäureamid

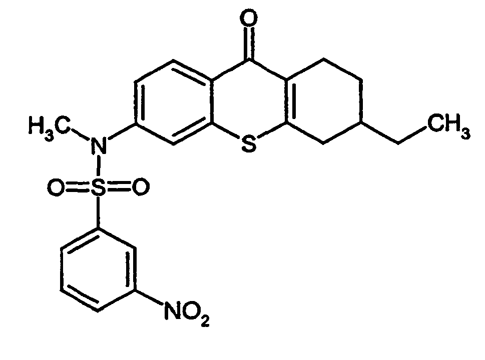

Analog zur Synthese von *N*-(3-Ethyl-9-oxo-2,3,4,9-tetrahydro-1*H*-thioxanthen-6-yl)-*N*-methyl-1-butansulfonsäureamid aus 40 mg (0,09 mmol) *N*-(3-Ethyl-9-oxo-2,3,4,9-tetrahydro-1*H*-thioxanthen-6-yl)-3-nitrobenzolsulfonsäureamid, 51 mg (0,36 mmol) Methyliodid und 124 mg (0,90 mmol) Kaliumcarbonat erhält man 20 mg (0,04 mmol, 48 % Ausbeute) des Produktes als farblosen, amorphen Feststoff.
R_{f}-Wert: 0,48 -(Cyclohexan/Ethylacetat 2:1).
MS (ESI): 459,2 ([M+H]⁺).

### Beispiel 4-17

### 5-Chloro-N-(3-ethyl-9-oxo-2,3,4,9-tetrahydro-1H-thioxanthen-6-yl)-N-Methyl-2-thiophensulfonsäureamid

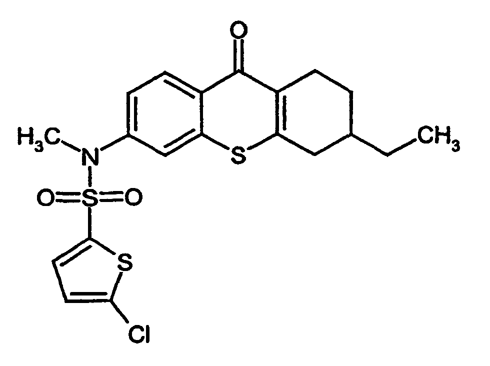

Analog zur Synthese von *N*-(3-Ethyl-9-oxo-2,3,4,9-tetrahydro-1*H*-thioxanthen-6-yl)-*N*-methyl-1-butansulfonsäureamid aus 80 mg (0,18 mmol) 5-Chlor-*N*-(3-ethyl-9-oxo-2,3,4,9-tetrahydro-1*H*-thioxanthen-6-yl)-2-thiophensulfonsäureamid, 104 mg (0,73 mmol) Methyliodid und 252 mg (1,82 mmol) Kaliumcarbonat erhält man 81 mg (0,18 mmol, 98 % Ausbeute) des Produktes als blassgelben, mikrokristallinen Feststoff.
R_{f}-Wert: 0,51 (Cyclohexan/Ethylacetat 2:1).
MS (ESI): 454 ([M+H]⁺).

### Beispiel 4-18

### 3-Ethyl-6-(2-pyridinyl)-1,2,3,4-tetrahydro-9H-thioxanthen-9-on

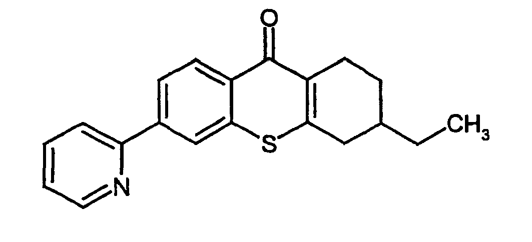

Unter einer Argonatmosphäre werden 150 mg (0,46 mmol) 6-Brom-3-ethyl-1,2,3,4-tetrahydro-9H-thioxanthen-9-on in 3 ml Dimethylformamid vorgelegt. Zu dieser Lösung gibt man nachfolgend 141 mg (0,56 mmol) Bis(pinacolato)diboron, 137 mg (1,39 mmol) Kaliumacetat und 10 mg (0,01 mmol) Dichloro[bis(diphenylphosphino)ferrocenyl]palladium(II) als Katalysator und rührt 4h bei 70°C. Anschließend gibt man 88 mg (0,56 mmol) 2-Brompyridin, weitere 10 mg Katalysator gelöst in 1 ml Dimethylformamid und 1 ml 2M Natriumcarbonatlösung zu dieser Lösung und rührt über Nacht bei 70°C. Nach Abkühlen wird die Lösung zwischen Ethylacetat und Wasser verteilt, die organische Phase mit Wasser gewaschen und über Natriumsulfat getrocknet. Der nach Einengen erhaltene Rückstand wird mittels präparativer HPLC (Säule: Kromasil 120 ODS-4 HE, 10 µm, 250 x 20 mm; Eluent: Acetonitril/Wasser, Fluss: 25 ml/min; UV-Detektion bei 210 nm) aufgetrennt. Man erhält 21 mg (0,06 mmol, 14 % Ausbeute) des Produktes als farblosen, kristallinen Feststoff.
R_{f}-Wert: 0,62 (Cyclohexan/Ethylacetat 2:1).
MS (EI): 321 (M⁺).

### Beispiel 4-19

### 3-Ethyl-6-(2-pyrimidinyl)-1,2,3,4-tetrahydro-9H-thioxanthen-9-on

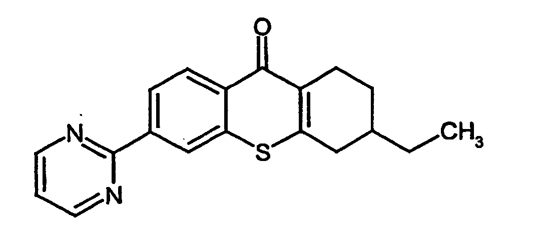

Analog zur Synthese von 3-Ethyl-6-(2-pyridinyl)-1,2,3,4-tetrahydro-9*H*-thioxanthen-9-on aus 150 mg (0,46 mmol) 6-Brom-3-ethyl-1,2,3,4-tetrahydro-9*H*-thioxanthen-9-on, 141 mg (0,56 mmol) Bis(pinacolato)diboron, 137 mg (1,39 mmol) Kaliumacetat und 88 mg (0,56 mmol) 2-Brompyrimidin erhält man 8 mg (0,02 mmol, 5 % Ausbeute) des Produktes als farblosen, mikrokristallinen Feststoff.
R_{f}-Wert: 0,41 (Cyclohexan/Ethylacetat 2:1).
MS (ESI): 323,2 ([M+H]⁺).

### Beispiel 4-20

### 2-(3-Ethyl-9-oxo-2,3,4,9-tetrahydro-1H-thioxanthen-6-yl)benzonitril

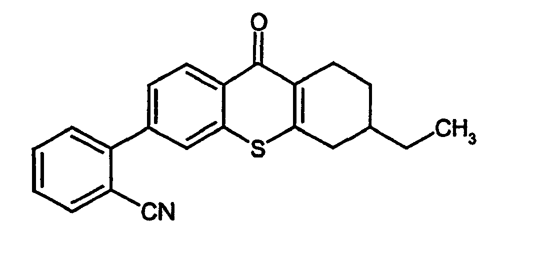

Analog zur Synthese von 3-Ethyl-6-(2-pyridinyl)-1,2,3,4-tetrahydro-9*H*-thioxanthen-9-on aus 150 mg (0,82 mmol) 2-Brombenzonitril, 251 mg (0,99 mmol) Bis(pinacolato)diboron, 243 mg (2,47 mmol) Kaliumacetat, und 320 mg (0,99 mmol) 6-Brom-3-ethyl-1,2,3,4-tetrahydro-9*H*-thioxanthen-9-on erhält man 72 mg (0,21 mmol, 25 % Ausbeute) des Produktes als farblosen, mikrokristallinen Feststoff.
R_{f}-Wert: 0,42 (Cyclohexan/Ethylacetat 2:1).
¹H-NMR (300 MHz, DMSO-d₆, δ/ppm): 8,42 (d, 1H), 8,06 (d, 1H), 8,03 (d, 1H), 7,86 (m, 1H), 7,70 (m, 3H), 2,70 (m, 2H), 2,49 (m, 2H, überlagert durch DMSO-Signal), 1,99 (m, 1H), 1,70 (m, 1H), 1,48-1,28 (m, 3H), 0,95 (t, 3H).
MS (EI): 345 (M⁺).

### Beispiel 4-21

### 3-Ethyl-6-(6-methyl-3-pyridinyl)-1,2,3,4-tetrahydro-9H-thioxanthen-9-on

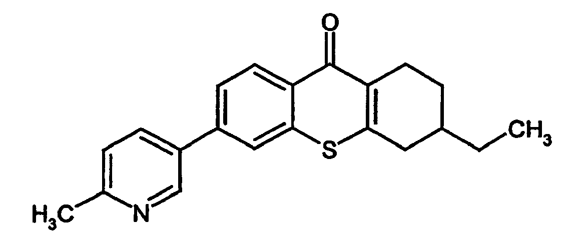

Analog zur Synthese von 3-Ethyl-6-(2 pyridinyl)-1,2,3,4-tetrahydro-9*H*-thioxanthen 9-on aus 80 mg (0,25 mmol) 6-Brom-3-ethyl-1,2,3,4-tetrahydro-9*H*-thioxanthen-9-on, 76 mg (0,30 mmol) Bis(pinacolato)diboron, 73 mg (0,74 mmol) Kaliumacetat und 52 mg (0,30 mmol) 2-Brom-5-methylpyridin erhält man 19 mg (0,06 mmol, 23 % Ausbeute) des Produktes als blassgelben, amorphen Feststoff.
R_{f}-Wert: 0,34 (Cyclohexan/Ethylacetat 2:1).
¹H-NMR (300 MHz, DMSO-d₆, δ/ppm): 8,58 (d, 1H), 8,45 (d, 1H), 8,41 (d, 1H), 8,25 (dd, 1H), 8,08 (d, 1H), 7,78 (dd, 1H), 2,69 (m, 2H), 2,44 (m, 2H, überlagert durch DMSO-Signal), 2,38 (s, 3H), 1,98 (m, 1H), 1,68 .(m, 1H), 1,46-1,25 (m, 3H), 0,94 (t, 3H).
MS (EI): 335 (M⁺).

### Beispiel 4-22

### 3-Ethyl-6-(6-chlor-3-pyridinyl)-1,2,3,4-tetrahydro-9H thioxanthen-9-on

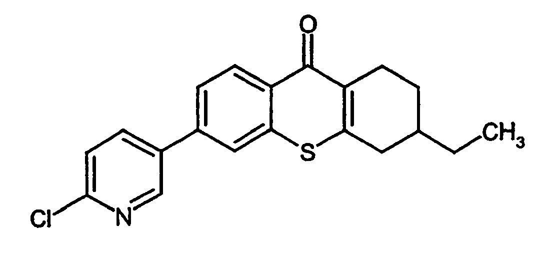

Analog zur Synthese von 3-Ethyl-6-(2-pyridinyl)-1,2,3,4-tetrahydro-9*H*-thioxanthen-9-on aus 100 mg (0,31 mmol) 6-Brom-3-ethyl-1,2,3,4-tetrahydro-9*H*-thioxanthen-9-on, 86 mg (0,34 mmol) Bis(pinacolato)diboron, 91 mg (0,93 mmol) Kaliumacetat und 71 mg (0,37 mmol) 2-Brom-5-chlorpyridin erhält man 10 mg (0,03 mmol, 9 % Ausbeute) des Produktes als farblosen, amorphen Feststoff.
R_{f}-Wert: 0,38 (Cyclohexan/Ethylacetat 2:1).
MS (ESI): 356 ([M+H]⁺).

### Beispiel 4-23

### 3-Ethyl-6-(2-methoxyphenyl)-1,2,3,4-tetrabydro-9H-thioxanthen-9-on

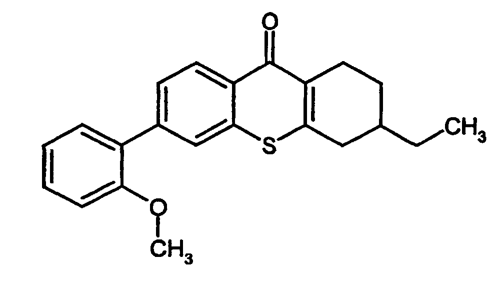

Unter einer Argonatmosphäre werden 100 mg (0,31 mmol) 6-Brom-3-ethyl-1,2,3,4-tetrahydro-9*H*-thioxanthen-9-on in 3 ml Dimethoxyethan vorgelegt. Zu dieser Lösung gibt man nachfolgend 56 mg (0,37 mmol) 2-Methoxyphenylboronsäure, 10 mg (0,01 mmol) Dichloro[bis(triphenylphosphino)]palladium(II) als Katalysator und 0,34 ml 2M Natriumcarbonatlösung und rührt 2h bei 90°C. Nach Abkühlen wird die Lösung über Silica abfiltriert und mit Ethylacetat nachgewaschen. Der nach Einengen erhaltene Rückstand wird säulenchromatographisch gereinigt (Kieselgel, Methylenchlorid-Methylenchlorid/Methanol 800:1-20:1). Man erhält 90 mg (0,26 mmol, 83 % Ausbeute) des Produktes als farblosen, kristallinen Feststoff.
R_{f}-Wert: 0,60 (Cyclohexan/Ethylacetat 2:1).
¹H-NMR (300 MHz, CDCl₃, δ/ppm): 8,50 (d, 1H), 7,65 (m, 2R), 7,36 (m, 2H), 7,07 (d, 1H), 7,01 (d, 1H), 3,82 (s, 3H), 2,93 (dt, 1H), 2,71 (dd, 1H), 2,62-2,36 (m, 2H), 2,06 (m, 1H), 1,70 (m, 1H), 1,48-1,36 (m, 3H), 0,99 (t, 3H).
MS (EI): 350 (M⁺).

### Beispiel 4-24

### 4-(3-Ethyl-9-oxo-2,3,4,9-tetrahydro-1H-thioxanthen-6-yl)benzaldehyd

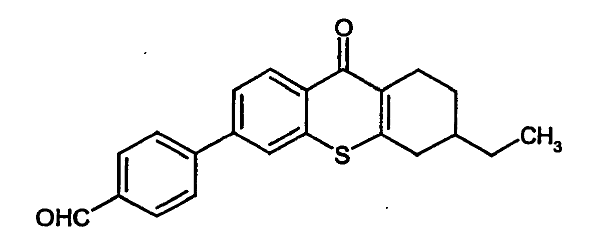

Analog zur Synthese von 3-Ethyl-6-(2-methoxyphenyl)-1,2,3,4-tetrahydro-9*H*-thioxanthen-9-on aus 100 mg (0,31 mmol) 6-Brom-3-ethyl-1,2,3,4-tetrahydro-9*H*-thioxanthen-9-on und 56 mg (0,37 mmol) 4-Formylphenylboronsäure erhält man 81 mg (0,23 mmol, 75 % Ausbeute) des Produktes als farblosen, kristallinen Feststoff.
R_{f}-Wert: 0,52 (Cyclohexan/Ethylacetat 2:1).
¹H-NMR (300 MHz, CDCl₃, δ/ppm): 10,10 (s, 1H), 8,60 (d, 1H), 8,00 (d, 2H), 7,82 (d, 2H), 7,75 (d, 1H), 7,72 (dd, 1H), 2,96 (dt, 1H), 2,77 (dd, 1H), 2,67-2,36 (m, 2H), 2,08 (m, 1H), 1,73 (m, 1H), 1,50-1,32 (m, 3H), 1,03 (t, 3H).
MS (ESI): 349,2 ([M+H]⁺).

### Beispiel 4-25

### 3-(3-Ethyl-9-oxo-2,3,4,9-tetrahydro-1H-thioxanthen-6-yl)benzaldehyd

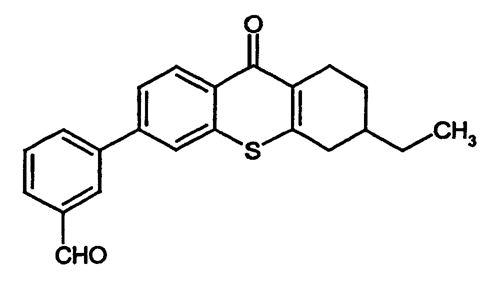

Analog zur Synthese von 3-Ethyl-6-(2-methoxyphenyl)-1,2,3,4-tetrahydro-9*H*-thioxanthen-9-on aus 100 mg (0,31 mmol) 6-Brom-3-ethyl-1,2,3,4-tetrahydro-9*H-*thioxanthen-9-on und 56 mg (0,37 mmol) 3-Formylphenylboronsäure erhält man 72 mg (0,21 mmol, 67 % Ausbeute) des Produktes als farblosen, kristallinen Feststoff.
R_{f}-Wert: 0,54 (Cyclohexan/Ethylacetat 2:1).
¹H-NMR (300 MHz, CDCl₃, δ/ppm): 10,12 (s, 1H), 8,59 (d, 1H), 8,16 (m, 1H), 7,92 (m, 2H), 7,75-7,63 (m, 3H), 2,96 (dt, 1H), 2,75 (dd, 1H), 2,62-2,39 (m, 2H), 2,06 (m; 1H), 1,72 (m, 1H), 1,50-1,37 (m, 3H), 1,00 (t, 3H).
MS (ESI): 349,2 ([M+H]⁺).

### Beispiel 4-26

### 5-(3-Ethyl-9-oxo-2,3,4,9-tetrahydro-1H-thioxanthen-6-yl)-2-thiophen-carbaldehyd

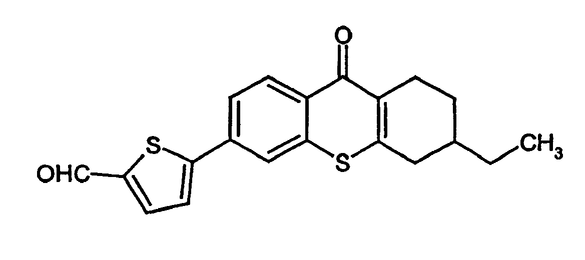

Analog zur Synthese von 3-Ethyl-6-(2-methoxyphenyl)-1,2,3,4-tetrahydro-9*H-*thioxanthen-9-on aus 300 mg (0,93 mmol) 6-Brom-3-ethyl-1,2,3,4-tetrahydro-9*H-*thioxanthen-9-on und 173 mg (1,11 mmol) 5-Formyl-2-thienylboronsäure erhält man 252 mg (0,71 mmol, 76 % Ausbeute) des Produktes als hellgelben, kristallinen Feststoff.
R_{f}-Wert: 0,38 (Cyclohexan/Ethylacetat 2:1).
¹H-NMR (300 MHz, CDCl₃, δ/ppm): 9,94 (s, 1H), 8,55 (d, 1H), 7,79 (d, 1H), 7,77 (d, 1H), 7,74 (dd, 1H), 7,54 (d, 1H), 2,94 (dt, 1H), 2,74 (dd, 1H), 2,62-2,37 (m, 2H), 2,07 (m, 1H), 1,73 (m, 1H), 1,49-1,30 (m, 3H), 1,01 (t, 3H).
MS (ESI): 354,9 ([M+H]⁺).

### Beispiel 4-27

### 3-Ethyl-6-(4-hydroxyphenyl)-1,2,3,4-tetrahydro-9H-thioxanthen-9-on

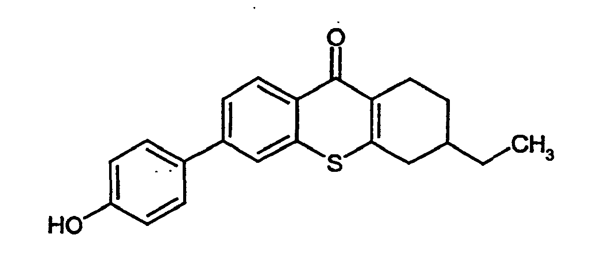

Unter einer Argonatmosphäre werden 100 mg (0,31 mmol) 3-Ethyl-6-(4-methoxyphenyl)-1,2,3,4-tetrahydro-9*H*-thioxanthen-9-on in 20 ml Methylenchlorid vorgelegt. Zu dieser Lösung gibt man unter Eiskühlung langsam 1,43 ml einer 1M Bortribromidlösung in Methylenchlorid und rührt 3h bei Raumtemperatur. Anschließend wird die Lösung unter Eiskühlung vorsichtig mit 3 ml Methanol versetzt. Der nach Einengen erhaltene Rückstand wird in Methylenchlorid gelöst und mit Wasser gewaschen. Man erhält nach Trocknung der organischen Phase über Natriumsulfat und Einengen 90 mg (0,27 mmol, 94 % Ausbeute) des Produktes als blassgelben, amorphen Feststoff.
R_{f}-Wert: 0,20 (Cyclohexan/Ethylacetat 2:1).
¹H-NMR (300 MHz, CDCl₃, δ/ppm): 8,52 (d, 1H), 7,68 (dd, 1H), 7,64 (d, 1H), 7,58 (d, 2H), 6,95 (d, 2H), 5,06 (s, 1H), 2,96 (dt, 1H), 2,74 (dd, 1H), 2,65-2,35 (m, 2H), 2,07 (m, 1H), 1,72 (m, 1H), 1,51-1,36 (m, 3H), 1,00 (t, 3H).
MS (EI): 336 (M⁺).

### Beispiel 4-28

### 3-Ethyl-6-(3-hydroxyphenyl)-1,2,3,4-tetrahydro-9H-thioxanthen-9-on

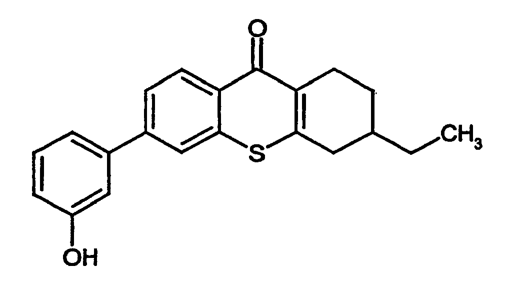

Unter einer Argonatmosphäre werden 510 mg (1,46 mmol) 3-Ethyl-6-(3-methoxyphenyl)-1,2,3,4-tetrahydro-9*H*-thioxanthen-9-on in 40 ml Methylenchlorid vorgelegt. Zu dieser Lösung gibt man unter Eiskühlung langsam 5 ml einer 1M Bortribromidlösung in Methylenchlorid und rührt 3h bei Raumtemperatur. Anschließend wird die Lösung unter Eiskühlung vorsichtig mit 8 ml Methanol versetzt. Der nach Einengen erhaltene Rückstand wird in Methylenchlorid gelöst und mit Wasser gewaschen. Man erhält nach Trocknung der organischen Phase über Natriumsulfat und Einengen 440 mg (1,30 mmol, 90 % Ausbeute) des Produktes als blassgelben, amorphen Feststoff.
R_{f}-Wert: 0,24 (Cyclohexan/Ethylacetat 2:1).
¹H-NMR (300 MHz, CDCl₃, δ/ppm): 8,49 (d, 1H), 7,69 (d, 1H), 7,66 (dd, 1H), 7,27 (d, 1H), 7,13 (m, 2H), 6,89 (m, 1H), 4,87 (s, 1H), 2,92 (dt, 1H), 2,76 (dd, 1H), 2,60-2,38 (m, 2H), 2,08 (m, 1H), 1,73 (m, 1H), 1,53-1,25 (m, 3H), 1,02 (t, 3H).
MS (ESI): 337,3 (M⁺).

### Beispiel 4-29

### 3-Ethyl-6-[4-(4-morpholinylmethyl)phenyl]-1,2,3,4-tetrahydro-9H-thioxanthen-9-on

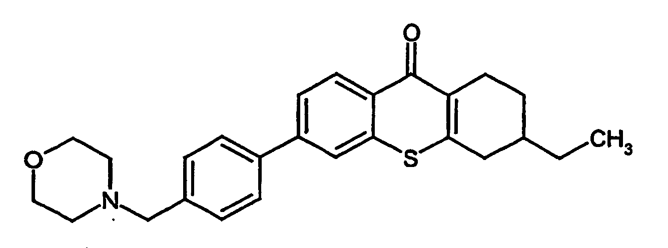

Unter einer Argonatmosphäre werden 30 mg (0,09 mmol) 4-(3-Ethyl-9-oxo-2,3,4,9-tetrahydro-1*H*-thioxanthen-6-yl)benzaldehyd in 5 ml 1,2-Dichlorethan gelöst und unter Rühren mit 8 mg (0,09 mmol) Morpholin und 28 mg (0,13 mmol) Natrium-(triacetoxyborhydrid) versetzt. Nach 1h gibt man zu dieser Lösung 5 µl Eisessig und lässt über Nacht rühren. Anschließend verteilt man diese Lösung zwischen Methylenchlorid und gesättigter Natriumhydrogencarbonatlösung, wäscht die organische. Phase mit Wasser und trocknet diese über Natriumsulfat. Nach Einengung erhält man 32 mg (0,08 mmol, 85 % Ausbeute) des Produktes als farblosen, amorphen Feststoff.
R_{f}-Wert: 0,19 (Cyclohexan/Ethylacetat 2:1).
¹H-NMR (300 MHz, CDCl₃, δ/ppm): 8,56 (d, 1H), 7,68 (m, 2H), 7,62 (d, 2H), 7,44 (d, 2H), 3,72 (t, 4H), 3,57 (s, 2H), 2,95 (dt, 1H), 2,76 (dd, 1H), 2,66-2,35 (m+t, 6H), 2,07 (m, 1H), 1,72 (m, 1H), 1,52-1,36 (m, 3H), 1,01 (t, 3H).
MS (ESI): 420,4 ([M+H]⁺).

### Beispiel 4-30

### N-(3-Ethyl-9-oxo-2,3,4,9-tetrahydro-1H-thioxanthen-6-yl)-N-methyl-2-naphthalensulfonsäureamid

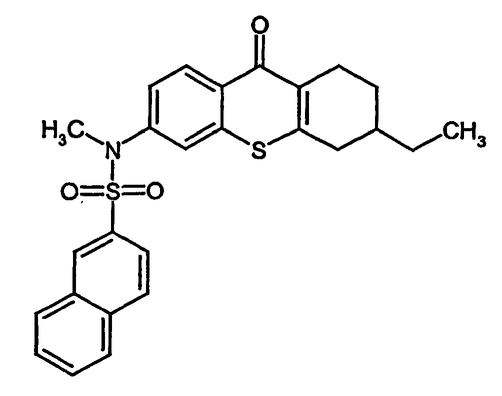

Analog zur Synthese von *N*-(3-Ethyl-9-oxo-2,3,4,9-tetrahydro-1*H*-thioxanthen-6-yl)-*N*-methyl-1-butansulfonsäureamid aus 58 mg (0,13 mmol) *N*-(3-Ethyl-9-oxo-2,3,4,9-tetrahydro-1*H*-thioxanthen-6-yl)-2-naphthalensulfonsäureamid, 75 mg (0,53 mmol) Methyliodid und 183 mg (1,33 mmol) Kaliumcarbonat erhält man 10 mg (0,02 mmol, 15 % Ausbeute) des Produktes als blaßgelben; mikrokristallinen Feststoff.
R_{f}-Wert: 0,56 (Cyclohexan/Ethylacetat 2:1).
MS (ESI): 464,5 ([M+H]⁺).

### Beispiel 4-31

### N-(3-ethyl-9-oxo-2,3,4,9-tetrahydro-1H-thioxanthen-6-yl)-N-Methyl-2-thiophensulfonsäureamid

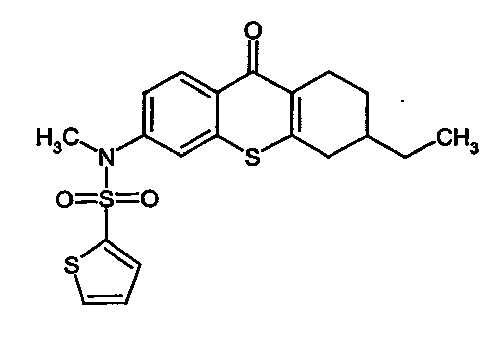

Analog zur Synthese von *N*-(3-Ethyl-9-oxo-2,3,4,9-tetrahydro-1*H*-thioxanthen-6-yl)-*N*-methyl-1-butansulfonsäureamid aus 28 mg (0,07 mmol) N-(3-Ethyl-9-oxo-2,3,4,9-tetrahydro-1*H*-thioxanthen-6-yl)-2-thiophensulfonsäureamid, 40 mg (0,28 mmol) Methyliodid und 95 mg (0,69 mmol) Kaliumcarbonat erhält man 23 mg (0,05 mmol, 82 % Ausbeute) des Produktes als blaßgelben, mikrokristallinen Feststoff.
R_{f}-Wert: 0,53 (Cyclohexan/Ethylacetat 2:1).
MS (ESI): 420,4 ([M+H]⁺).

### Beispiel 4-32

### 2-(1,3-Dioxo-1-,3-dihydro-2H-isoindol-2-yl)-N-(3-ethyl-9-oxo-2,3,4,9-tetrahydro-1H thioxanthen-6-yl)-N-methylsulfonsäureamid

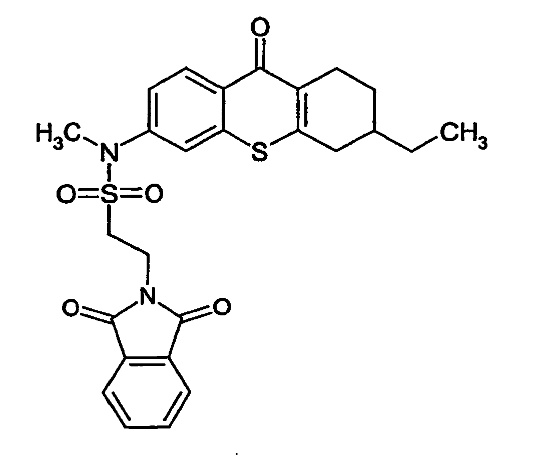

Analog zur Synthese von *N*-(3-Ethyl-9-oxo-2,3,4,9-tetrahydro-1*H*-thioxanthen-6-yl)-*N*-methyl-1-butansulfonsäureamid aus 39 mg (0,08 mmol) 2-(1,3-Dioxo-1,3-dihydro-2*H*-isoindol-2-yl)*-N*-(3-ethyl-9-oxo-2,3,4,9-tetrahydro-1*H*-thioxanthen-6-yl)ethansulfonsäure-amid, 48 mg (0,34 mmol) Methyliodid und 117mg (0,85 mmol) Kaliumcarbonat erhält man 15 mg (0,03 mmol, 35 % Ausbeute) des Produktes als blaßgelben Feststoff.
R_{f}-Wert: 0,41 (Cyclohexan/Ethylacetat 2:1).
MS (ESI): 511,2 ([M+H]⁺).

### Beispiel 4-33

### 3,3-Dimethyl-6-(4-methyl-3-pyridinyl)-1,2,3,4-tetrahydro-9H-thioxanthen-9-on

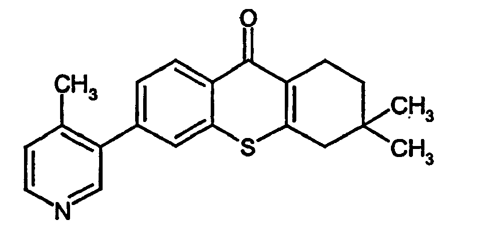

Analog zur Synthese von 3-Ethyl-6-(2-pyridinyl)-1,2,3,4-tetrahydro-9*H*-thioxanthen-9-on aus 150 mg (0,46 mmol) 6-Brom-3,3-dimethyl-1,2,3,4-tetrahydro-9H-thioxanthen-9-on, 142 mg (0,56 mmol) Bis(pinacolato)diboron, 136 mg (1,39 mmol) Kaliumacetat und 103 mg (0,60 mmol) 3-Brom-4-methylpyridin erhält man 49 mg (0,15 mmol, 31 % Ausbeute) des Produktes als farblosen, kristallinen Feststoff.
R_{f}-Wert: 0,48 (Cyclohexan/Ethylacetat 2:1).
MS (ESI): 336 ([M+H]⁺).

### Beispiel 4-34

### 6-(6-Methoxy-3-pyridinyl)-3,3-dimethyl-1,2,3,4-tetrahydro-9H-thioxanthen-9-on

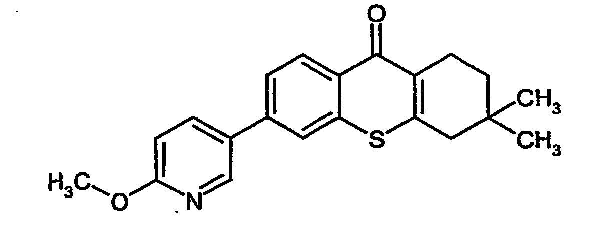

Analog zur Synthese von 3-Ethyl-6-(2-pyridinyl)-1,2,3,4-tetrahydro-9*H*-thioxanthen-9-on aus 150 mg (0,46 mmol) 6-Brom-3,3-dimethyl-1,2,3,4-tetrahydro-9H-thioxanthen-9-on, 142 mg (0,56 mmol) Bis(pinacolato)diboron, 136 mg (1,39 mmol) Kaliumacetat und 113 mg (0,60 mmol) 3-Brom-6-methoxypyridin erhält man 68 mg (0,19 mmol, 42 % Ausbeute) des Produktes als blassgelben, kristallinen Feststoff.
R_{f}-Wert: 0,42 (Cyclohexan/Ethylacetat 2:1).
¹H-NMR (300 MHz, CDCl₃, δ/ppm): 8,58 (d, 1H), 8,45 (d, 1H), 7,84 (dd, 1H), 7,63 (m, 2H), 6,86 (d, 1H), 4,01 (s, 3H), 2,73 (t, 2H), 2,49 (s, 2H), 1,64 (t, 2H), 1,06 (s, 6H).
MS (ESI): 352 ([M+H]⁺).

### Beispiel 4-35

### 6-(4-Methoxy-5-pyrimidinyl)-3,3-dimethyl-1,2,3,4-tetrahydro-9H-thioxanthen-9-on

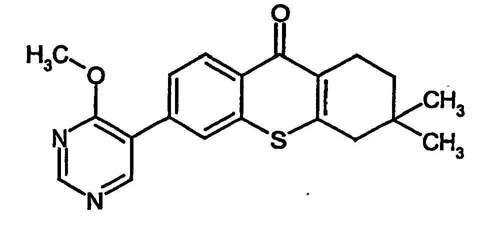

Analog zur Synthese von 3-Ethyl-6-(2-pyridinyl)-1,2,3,4-tetrahydro-9*H*-thioxanthen-9-on aus 132 mg (0,41 mmol) 6-Brom-3,3-dimethyl-1,2,3,4-tetrahydro-9H-thioxanthen-9-on, 124 mg (0,49 mmol) Bis(pinacolato)diboron, 119 mg (1,22 mmol) Kaliumacetat und 100 mg (0,53 mmol) 3-Brom-4-methoxypyrimidin erhält man 62 mg (0,18 mmol, 43 % Ausbeute) des Produktes als blassgelben, kristallinen Feststoff.
R_{f}-Wert: 0,22 (Cyclohexan/Ethylacetat 2:1).
¹H-NMR (300 MHz, CDCl₃, δ/ppm): 8,80 (s, 1H), 8,58 (d, 1H), 8,53 (s, 1H), 7,69 (d, 1H), 7,63 (dd, 1H), 4,07 (s, 3H), 2,73 (t, 2H), 2,49 (s, 2H), 1,63 (t, 2H), 1,04 (s, 6H).
MS (ESI): 353 ([M+H]⁺).

### Beispiel 4-36

### 3,3-Dimethyl-6-(5-methyl-3-pyridinyl)-1,2,3,4-tetrahydro-9H-thioxanthen-9-on

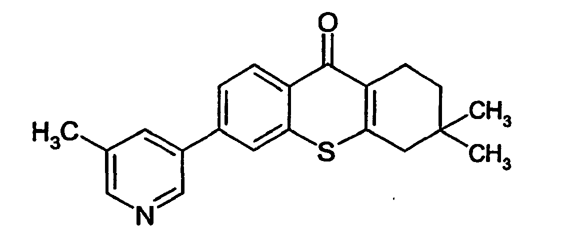

Analog zur Synthese von 3-Ethyl-6-(2-pyridinyl)-1,2,3,4-tetrahydro-9*H*-thioxanthen-9-on aus 150 mg (0,46 mmol) 6-Brom-3,3-dimethyl-1,2,3,4-tetrahydro-9H-thioxanthen-9-on, 142 mg (0,56 mmol) Bis(pinacolato)diboron, 136 mg (1,39 mmol) Kaliumacetat und 102 mg (0,60 mmol) 3-Brom-5-methylpyridin erhält man 37 mg (0,11 mmol, 24 % Ausbeute) des Produktes als farblosen, kristallinen Feststoff.
R_{f}-Wert: 0,39 (Cyclohexan/Ethylacetat 2:1).
MS (ESI): 336 ([M+H]⁺).

### Beispiel 4-37

### 6-(5-Acetyl-3-pyridinyl)-3,3-dimethyl-1,2,3,4-tetrahydro-9H-thioxanthen-9-on

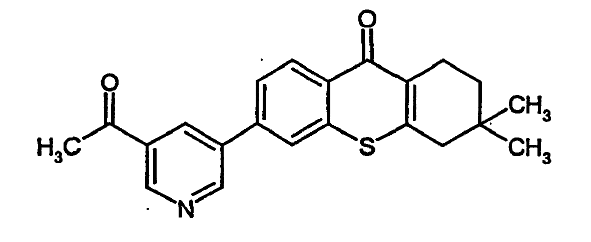

Analog zur Synthese von 3-Ethyl-6-(2-pyridinyl)-1,2,3,4-tetrahydro-9*H*-thioxanthen-9-on aus 150 mg (0,46 mmol) 6-Brom-3,3-dimethyl-1,2,3,4-tetrahydro-9H-thioxanthen-9-on, 142 mg (0,56 mmol) Bis(pinacolato)diboron, 136 mg (1,39 mmol) Kaliumacetat und 120 mg (0,60 mmol) 3-Brom-5-acetylpyridin erhält man 34 mg (0,09 mmol, 20 % Ausbeute) des Produktes als farblosen, kristallinen Feststoff.
R_{f}-Wert: 0,42 (Cyclohexan/Ethylacetat 2:1).
¹H-NMR (300 MHz, CDCl₃, δ/ppm): 9,19 (d, 1H), 9,05 (d, 1H), 8,62 (d, 1H), 8,47 (t, 1H), 7,71 (m, 2H), 2,77 (t, 2H), 2,72 (s, 3H), 2,51 (s, 2H), 1,65 (t, 2H), 1,06 (s, 6M.
MS (FS1): 364 ([M+H]⁺).

### Beispiel 4-38

### 5-(3,3-Dimethyl-9-oxo-2,3,4,9-tetrahydro-1H-thioxanthen-6-yl)nicotinsäuremethylester

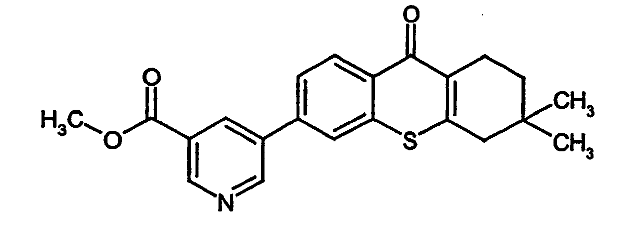

Analog zur Synthese von 3-Ethyl-6-(2-pyridinyl)-1,2,3,4-tetrahydro-9*H*-thioxanthen-9-on aus 150 mg (0,46 mmol) 6-Brom-3,3-dimethyl-1,2,3,4-tetrahydro-9H-thioxanthen-9-on, 142 mg (0,56 mmol) Bis(pinacolato)diboron, 136 mg (1,39 mmol) Kaliumacetat und 130 mg (0,60 mmol) 3-Bromnicotinsäuremethylester erhält man 104 mg (0,27 mmol, 59 % Ausbeute) des Produktes als farblosen, kristallinen Feststoff.
R_{f}-Wert: 0,40 (Cyclohexan/Ethylacetat 2:1).
¹H-NMR (300 MHz, CDCl₃, δ/ppm): 9,26 (d, 1H), 9,05 (d, 1H), 8,62 (d, 1H), 8,58 (t, 1H), 7,72 (m, 2H), 4,01 (s, 3H), 2,75 (t, 2H), 2,51 (s, 2H), 1,66 (t, 2H), 1,05 (s, 6H).
MS (ESI): 380 ([M+H]⁺).

### Beispiel 4-39

### 6-(5,6-Dünethyl-3-pyridinyl)-3,3-dimethyl-1,2,3,4-tetrahydro-9H-thioxanthen-9-on

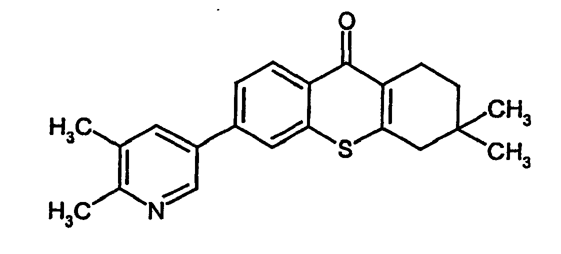

Analog zur Synthese von 3-Ethyl-6-(2-pyridinyl)-1,2,3,4-tetrahydro-9*H*-thioxanthen-9-on aus 150 mg (0,46 mmol) 6-Brom-3,3-dimethyl-1,2,3,4-tetrahydro-9H-thioxanthen-9-on, 142 mg (0,56 mmol) Bis(pinacolato)diboron, 136 mg (1,39 mmol) Kaliumacetat und 112 mg (0,60 mmol) 3-Brom-5,6-dimethylpyridin erhält man 61 mg (0,17 mmol, 38% Ausbeute) des Produktes als farblosen, kristallinen Feststoff.
R_{f-}Wert: 0,37 (Cyclohexan/Ethylacetat 2:1).
MS (ESI): 349 ([M]⁺).

### Beispiel 4-40

### 6-(4,6-Dimethoxy-5-pyrimidinyl)-3,3-dimethyl-1,2,3,4-tetrahydro-9H-thioxanthen-9-on

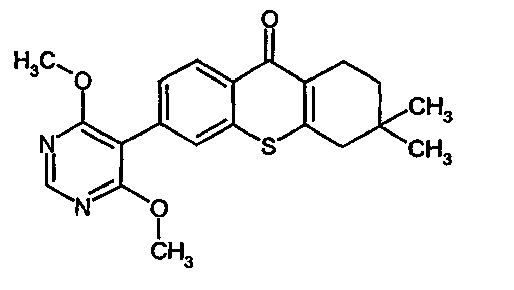

Analog zur Synthese von 3-Ethyl-6-(2-pyridinyl)-1,2,3,4-tetrahydro-9*H*-thioxanthen-9-on aus 150 mg (0,46 mmol) 6-Brom-3,3-dimethyl-1,2,3,4-tetrahydro-9H-thioxanthen-9-on, 142 mg (0,56 mmol) Bis(pinacolato)diboron, 136 mg (1,39 mmol) Kaliumacetat und 131 mg (0,60 mmol) 3-Brom-4,6-dimethoxypyrimidin erhält man 104 mg (0,27 mmol, 59% Ausbeute) des Produktes als farblosen, kristallinen Feststoff.
R_{f}-Wert: 0,25 (Cyclohexan/Ethylacetat 2:1).
¹H-NMR (300 MHz, CDCl₃, δ/ppm): 8,52 (d, 1H), 8,46 (s, 1H), 7,56 (d, 1H), 7,50 (dd, 1H), 3,95 (s, 6H), 2,73 (t, 2H), 2,48 (s, 2H), 1,63 (t, 2H), 1,03 (s, 6H).
MS (ESI): 383 ([M+H]⁺).

### Beispiel 4-41

### 3-(3-Ethyl-9-oxo-2,3,4,9-tetrahydro-1H-thioxanthen-6-yl)-2-thiophenecarbaldehyd

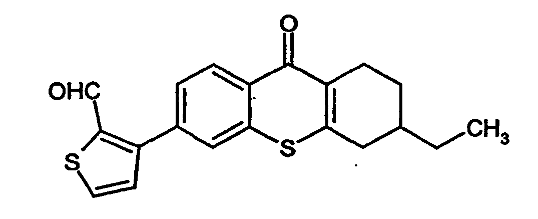

Analog zur Synthese von 3-Ethyl-6-(2-methoxyphenyl)-1,2,3,4-tetrahydro-9*H-*thioxanthen-9-on aus 300 mg (0,93 mmol) 6-Brom-3-ethyl-1,2,3,4-tetrahydro-9*H-*thioxanthen-9-on und 173 mg (1,11 mmol) 2-Formylthiophen-3-boronsäure erhält man 210 mg (0,59 mmol, 63 % Ausbeute) des Produktes als hellgelben, kristallinen Feststoff.
R_{f}-Wert: 0,41 (Cyclohexan/Ethylacetat 2:1).
MS (ESI): 355,2 ([M+H]⁺).

### Beispiel 4-42

### 3,3-Dimethyl-6-(5-pyrimidinyl)-1,2,3,4-tetrahydro-9H-thioxanthen-9-on

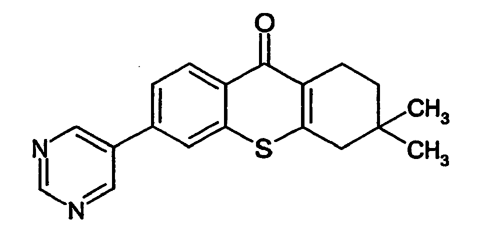

Analog zur Synthese von 3-Ethyl-6-(2-methoxyphenyl)-1,2,3,4-tetrahydro-9*H* thioxanthen-9-on aus 1,00 g (3,09 mmol) 6-Brom-3,3-dimethyl-1,2,3,4-tetrahydro-9H-thioxanthen-9-on und 1,02 g (4,95 mmol) 5-(4,4,5,5-Tetramethyl-1,3,2-dioxaborolan-2-yl)pyrimidin unter zusätzlicher Verwendung von Kaliumphosphat als Base und Dichlor[bis(diphenyl-phosphino)ferrocenyl]palladium(II) als Katalysator erhält man 0,9 g (2,79 mmol, 90 % Ausbeute) des Produktes als farblosen, kristallinen Feststoff.
R_{f}-Wert: 0,18 (Cyclohexan/Ethylacetat 2:1).
¹H-NMR (300 MHz, CDCl₃, δ/ppm): 9,29 (s, 1H), 9,02 (s, 2H), 8,65 (d, 1H), 7,69 (m, 2H), 2,75 (t, 2H), 2,51 (s, 2H), 1,66 (t, 2H), 1,05 (s, 6H).
MS (ESI): 323 ([M+H]⁺).

### Beispiel 4-43

### 6-(4-Methoxy-3-pyridinyl)-3,3-dimethyl-1,2,3,4-tetrahydro-9H-thioxanthen-9-on

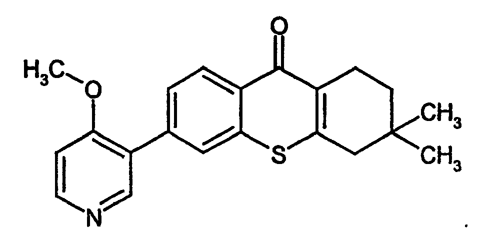

Analog zur Synthese von 3-Ethyl-6-(2-methoxyphenyl)-1,2,3,4-tetrahydro-9*H-*thioxanthen-9-on aus 240 mg (0,75 mmol) 6-Brom-3,3-dimethyl-1,2,3,4-tetrahydro-9H-thioxanthen-9-on und 150 mg (0,98 mmol) 4-Methoxy-3-pyridinboronsäure erhält man 60 mg (0,17 mmol, 22 % Ausbeute) des Produktes als farblosen Feststoff.
R_{f}-Wert: 0,38 (Cyclohexan/Ethylacetat 2:1).
MS (ESI): 351 ([M]⁺).

### Beispiel 4-44

### 3-Ethyl-6-[5-(4-morpholinylmethyl)-2-thienyl]-1,2,3,4-tetrahydro-9H thioxanthen-9-on

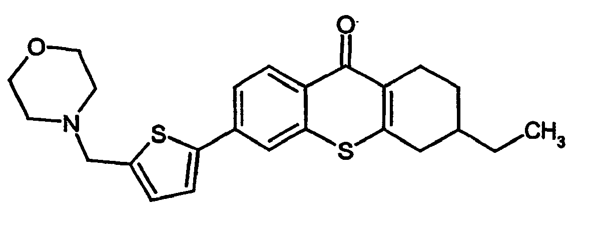

Analog zur Synthese von 3-Ethyl-6-[4-(4-morpholinylmethyl)phenyl]-1,2,3,4-tetrahydm-9*H*-thioxanthen-9-on aus 71 mg (0,20 mmol) 5-(3-Ethyl-9-oxo-2,3,4,9-tetrahydro-1*H*-thioxanthen-6-yl)-2-thiophenecarbaldehyd, 19 mg (0,22 mmol) Morpholin, 63 mg (0,30 mmol) Natrium(triacetoxyborhydrid) und 20 µl Eisessig erhält man 13 mg (0,03 mmol, 15 % Ausbeute) des Produktes als farblosen Feststoff
R_{f}-Wert: 0,21 (Cyclohexan/Ethylacetat 2:1).
MS (ESI): 425 ([M]⁺).

## Patentansprüche

1. Verbindungen der allgemeinen Formel (I), 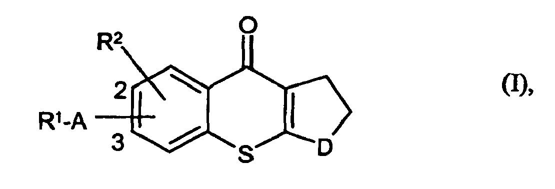 in welcher
der Rest R¹-A- an einer der Positionen 2 oder 3 des Thiochromenon-Rings sitzt,
R¹ für (C₆-C₁₀)-Aryl oder 5- bis 10-gliedriges Heteroaryl steht, wobei Aryl und Heteroaryl gegebenenfalls gleich oder verschieden durch Reste ausgewählt aus der Gruppe Halogen, Formyl, Carbamoyl, Cyano, Hydroxy, Trifluormethoxy, Nitro, -NR³R⁴, Tetrazolyl, (C₁-C₆)-Alkoxycarbonyl sowie gegebenenfalls durch Hydroxy, Morpholinyl, (C₁-C₆)-Acyloxy oder Halogen substituiertes (C₁-C₆)-Alkyl, (C₁-C₆)-Alkoxy, (C₁-C₆)-Acyl und (C₁-C₆)-Alkylthio substituiert sind,
worin R³ und R⁴ unabhängig voneinander Wasserstoff, (C₁-C₆)-Alkyl oder (C₁₋C₆)-Acyl bedeuten,
für 3- bis 12-gliedriges Carbocyclyl oder 4- bis 12-gliedriges Heterocyclyl steht, wobei Carbocyclyl und Heterocyclyl gegebenenfalls gleich oder verschieden durch Reste ausgewählt aus der Gruppe (C₁-C₆)-Alkyl, (C₁-C₆)-Alkoxy, (C₁-C₆)-Acyl, (C₁-C₆)-Alkoxycarbonyl oder Oxo substituiert sind,
und
A für eine Bindung oder eine Gruppe der Formel O, S, NR⁶, CO, SO, SO₂, SO₂₋O, CO-NR⁷, SO₂-NR⁸, O-SO₂, NR⁹-CO, NR¹⁰-SO₂, NR¹¹-SO₂-O, NR¹²-SO₂₋NR¹³ oder NR¹⁴-CO-NR¹⁵ steht,
worin R⁶, R⁷, R⁸, R⁹, R¹⁰ R¹¹, R¹², R¹³, R¹⁴ und R¹⁵ (C₃-C₈)-Cycloalkyl oder gegebenenfalls ungesättigtes (C₁-C₆)-Alkyl, das gegebenenfalls durch Hydroxy, Phenyl, (C₁-C₆)-Alkoxy, (C₁-C₆)-Alkoxycarbonyl oder (C₃-C₈)-Cycloalkyl substituiert ist, bedeuten, wobei Phenyl seinerseits durch Halogen oder (C₁-C₄)-Alkyl substituiert sein kann, oder
worin R⁶, R⁷, R⁹, R¹⁰, R¹¹, R¹², R¹⁴ und R¹⁵ Wasserstoff bedeuten,
oder
R¹ für eine Gruppe der Formel R⁵-E- steht,
worin
E gegebenenfalls ungesättigtes (C₁-C₁₀)-Alkandiyl bedeutet, und
R⁵ Wasserstoff, Carbamoyl, Halogen, Hydroxy, Nitro, Trifluoromethyl, Amino, Mono-(C₁-C₆)-Alkylamino, Di-(C₁-C₆)-Alkylamino, (C₁-C₆)-Alkoxy, (C₆₋C₁₀)-Aryl, 5- bis 10-gliedriges Heteroaryl oder 4- bis 10-gliedriges, gegebenenfalls mit Oxo und/oder (C₁-C₆)-Alkyl substituiertes, gegebenenfalls benzokondensiertes Heterocyclyl bedeutet, wobei Aryl, Heteroaryl und Benzo ihrerseits durch Reste ausgewählt aus der Gruppe Halogen, Cyano, Trifluormethyl, Trifluormethoxy, Nitro und (C₁-C₆)-Alkyl substituiert sein können,
und
A für S, NR⁶, CO, SO, SO₂, SO₂-O, CO-NR⁷, SO₂-NR⁸, O-SO₂, NR⁹-CO, NR¹⁰⁻SO₂, NR¹¹-SO₂-O, NR¹²-SO₂-NR¹³ oder NR¹⁴-CO-NR¹⁵ steht,
worin R⁶, R⁷, R⁸, R⁹, R¹⁰, R¹¹, R¹² , R¹³, R¹⁴ und R¹⁵ (C₃-C₈)-Cycloalkyl oder gegebenenfalls ungesättigtes (C₁-C₆)-Alkyl, das gegebenenfalls durch Hydroxy, Phenyl, (C₁-C₆)-Alkoxy, (C₁-C₆)-Alkoxycarbonyl oder (C₃-C₈)-
Cycloalkyl substituiert ist, bedeuten, wobei Phenyl seinerseits durch Halogen oder (C₁-C₄)-Alkyl substituiert sein kann, oder
worin R⁶, R⁷, R⁹, R¹⁰, R¹¹, R¹², R¹⁴ und R¹⁵ Wasserstoff bedeuten,
oder
der Rest R¹-A- für Wasserstoff oder Amino steht,
R² für Wasserstoff, Halogen oder (C₁-C₆)-Alkyl oder (C₁-C₆)-Alkoxy steht, wobei Alkyl und Alkoxy gegebenfalls bis zu zweifach gleich oder verschieden durch Resten ausgewählt aus der Gruppe Hydroxy, (C₁-C₆)-Alkoxy, Mono- und Di-(C₁-C₆)-alkylamino substituiert sind, und
D für einen gegebenenfalls mit Fluor substituierten, zweibindigen Kohlenwasserstoffrest mit 3 bis 10 Kohlenstoffatomen steht,
und deren Salze, Hydrate und/oder Solvate,
mit der Ausnahme von 2-Chloro-6,7,8,9,10, 10a-hexahydrocyclohepta[b]thiochromen-11(5aH)-on.

2. Verbindungen der allgemeinen Formel (I) nach Anspruch 1, wobei
der Rest R¹-A- an der Position 3 des Thiochromenon-Rings sitzt,
R¹ für (C₆-C₁₀)-Aryl oder 5- bis 10-gliedriges Heteroaryl steht, wobei Aryl und Heteroaryl gegebenenfalls gleich oder verschieden bis zu zweifach durch Reste ausgewählt aus der Gruppe Halogen, Formyl, Cyano, Hydroxy, Hydroxymethyl, (C₁-C₆)-Alkyl, substituiert sind, für 4- bis 10-gliedriges Heterocyclyl steht, wobei Heterocyclyl gegebenenfalls gleich oder verschieden durch Reste ausgewählt aus der Gruppe (C₁-C₆)-Alkyl, (C₁-C₆)-Alkoxy, (C₁-C₆)-Alkoxycarbonyl oder Oxo substituiert sind,
A für eine Bindung oder eine Gruppe der Formel NR⁶, CO-NR⁷, SO₂₋NR⁸ oder NR⁹-CO, steht,
worin R⁶, R⁷, R⁸ und R⁹ gegebenenfalls ungesättigtes (C₁-C₆)-Alkyl, das gegebenenfalls bis zu zweifach gleich oder verschieden durch Hydroxy oder Methoxy substituiert ist, bedeuten, oder
worin R⁶, R⁷ und R⁹ Wasserstoff bedeuten,
R² für Wasserstoff steht, und
D für eine Gruppe der Formel (CH₂)ₘ-CR¹⁶R¹⁷-(CH₂)ₙ steht,
worin
die Gesamtzahl der Kohlenstoffatome 3 bis 10 beträgt,
m und n gleich oder verschieden sind und eine natürliche Zahl aus der Reihe 0 bis 6 bedeuten,
und
R¹⁶ und R¹⁷ gleich oder verschieden sind und Wasserstoff oder (C₁₋C₆)-Alkyl, das gegebenenfalls gleich oder verschieden mit (C₃₋C₅)-Cycloalkyl oder Halogen substituiert ist, bedeuten,
oder
CR¹⁶R¹⁷ (C₃-C₆)-Cycloalkan-1,1-diyl bedeutet,
und deren Salze, Hydrate und/oder Solvate.

3. Verbindungen der allgemeinen Formel (I) nach Anspruch 1, wobei
der Rest R¹-A- an der Position 3 des Thiochromenon-Rings sitzt,
R¹ für Phenyl oder 5- bis 6-gliedriges Heteroaryl steht, wobei Phenyl und Heteroaryl gegebenenfalls gleich oder verschieden bis zu zweifach durch Reste ausgewählt aus der Gruppe Halogen, Cyano, (C₁-C₃)-Alkyl, substituiert sind,
für 5- bis 7-gliedriges Heterocyclyl steht, wobei Heterocyclyl gegebenenfalls gleich oder verschieden durch Reste ausgewählt aus der Gruppe (C₁-C₃)-Alkyl oder Oxo substituiert sind,
A für eine Bindung oder eine Gruppe der Formel NR⁶, SO₂-NR⁸ oder NR⁹-CO, steht,
worin R⁶, R⁸ und R⁹ gegebenenfalls ungesättigtes (C₁-C₃)-Alkyl, das gegebenenfalls bis zu zweifach gleich oder verschieden durch Hydroxy oder Methoxy substituiert ist, bedeuten, und worin R⁶, R⁸ und R⁹-Wasserstoffbedeuten,
R² für Wasserstoff steht, und
D für eine Gruppe der Formel (CH₂)ₘ-CR¹⁶R¹⁷-(CH₂)ₙ steht,
worin
die Gesamtzahl der Kohlenstoffatome 3 bis 6 beträgt,
m und n gleich oder verschieden sind und eine natürliche Zahl aus der Reihe 0 bis 2 bedeuten,
und
R¹⁶ und R¹⁷ gleich oder verschieden sind und Wasserstoff oder (C₁₋C₃)-Alkyl, bedeuten,
oder
CR¹⁶R¹⁷ -(C₃-C₆)-Cyclealkan-1,1-diyl bedeutet,
und deren Salze, Hydrate und/oder Solvate.

4. Verbindungen der allgemeinen Formel (I) nach Anspruch 1 zur Behandlung und/oder Prophylaxe von Krankheiten.

5. Arzneimittel enthaltend mindestens eine der Verbindungen der allgemeinen Formel (I) nach einem der Ansprüche 1 bis 3 in Zusammenmischung mit mindestens einem pharmazeutisch verträglichen, im wesentlichen nichtgiftigen Träger oder Exzipienten.

6. Verwendung von Verbindungen der allgemeinen Formel (I) nach einem der Ansprüche 1 bis 3 zur Herstellung eines Arzneimittels zur Behandlung und/oder Prophylaxe von Schmerzzuständen und/oder neurodegenerativen Erkrankungen.

## Claims

1. Compounds of the formula (I), 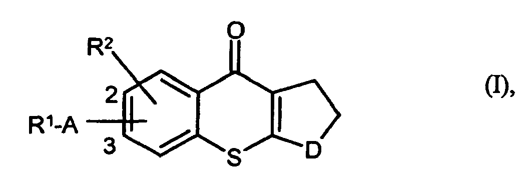 in which
the radical R¹-A- is located at either of positions 2 or 3 of the thiochromenone ring,
R¹ is (C₆-C₁₀)-aryl or 5- to 10-membered heteroaryl, where aryl and heteroaryl are optionally substituted identically or differently by radicals selected from the group of halogen, formyl, carbamoyl, cyano, hydroxyl, trifluoromethoxy, nitro, -NR³R⁴, tetrazolyl, (C₁-C₆)-alkoxycarbonyl and optionally hydroxyl-, morpholinyl-, (C₁-C₆)-acyloxy- or halogen-substituted (C₁-C₆)-alkyl, (C₁-C₆)-alkoxy, (C₁₋C₆)-acyl and (C₁-C₆)-alkylthio,
in which R³ and R⁴ are, independently of one another, hydrogen, (C₁-C₆)-alkyl or (C₁-C₆)-acyl,
is 3- to 12-membered carbocyclyl or 4- to 12-membered heterocyclyl, where carbocyclyl and heterocyclyl are optionally substituted identically or differently by radicals selected from the group of (C₁- C₆)-alkyl, (C₁-C₆)-alkoxy, (C₁-C₆)-acyl, (C₁-C₆)-alkoxycarbonyl or oxo,
and
A is a bond or a group of the formula O, S, NR⁶, CO, SO, SO₂, SO₂-O, CO-NR⁷, SO₂-NR⁸, O-SO₂, NR⁹-CO, NR¹⁰-SO₂, NR¹¹-SO₂-O, NR¹²⁻SO₂-NR¹³ or NR¹⁴-CO-NR¹⁵,
in which R⁶, R⁷, R⁸, R⁹, R¹⁰, R¹¹, R¹², R¹³, R¹⁴ and R¹⁵ are (C₃-C₈)-cycloalkyl or optionally unsaturated (C₁-C₆)-alkyl which is optionally substituted by hydroxyl, phenyl, (C₁-C₆)-alkoxy, (C₁-C₆)-alkoxycarbonyl or (C₃-C₈)-cycloalkyl, where phenyl in turn may be substituted by halogen or (C₁-C₄)-alkyl, or
in which R⁶, R⁷, R⁹, R¹⁰, R¹¹, R¹², R¹⁴ and R¹⁵ are hydrogen,
R¹ is a group of the formula R⁵-E-,
in which
E is optionally unsaturated (C₁-C₁₀)-alkanediyl, and
R⁵ is hydrogen, carbamoyl, halogen, hydroxyl, nitro, trifluoromethyl, amino, mono-(C₁-C₆)-alkylamino, di-(C₁-C₆)-alkylamino, (C₁-C₆)-alkoxy, (C₆-C₁₀)-aryl, 5- to 10-membered heteroaryl or 4- to 10-membered, optionally oxo- and/or (C₁-C₆)-alkyl-substituted, optionally benzo-fused heterocyclyl, where aryl, heteroaryl and benzo in turn may be substituted by radicals selected from the group of halogen, cyano, trifluoromethyl, trifluoromethoxy, nitro and (C₁-C₆)-alkyl,
and
A is S, NR⁶, CO, SO, SO₂, SO₂-O CO-NR⁷, SO₂-NR⁸, O-SO₂, NR⁹-CO, NR¹⁰-SO₂, NR¹¹-SO₂-O, NR¹²-SO₂-NR¹³ or NR¹⁴-CO-NR¹⁵,
in which R⁶, R⁷, R⁸, R⁹, R¹⁰, R¹¹, R¹², R¹³, R¹⁴ and R¹⁵ are (C₃-C₈)-cycloalkyl or optionally unsaturated (C₁-C₆)-alkyl which is optionally substituted by hydroxyl, phenyl, (C₁-C₆)-alkoxy, (C₁-C₆)-alkoxycarbonyl or (C₃-C₈)-cycloalkyl, where phenyl in turn may be substituted by halogen or (C₁-C₄)-alkyl, or
in which R⁶, R⁷, R⁹, R¹⁰, R¹¹, R¹², R¹⁴ and R¹⁵ are hydrogen,
or
the radical R¹-A- is hydrogen or amino,
R² is hydrogen, halogen or (C₁-C₆)-alkyl or (C₁-C₆)-alkoxy, where alkyl and alkoxy are optionally substituted up to twice identically or differently by radicals selected from the group of hydroxyl, (C₁-C₆)-alkoxy, mono- and di-(C₁-C₆)-alkylamino, and
D is an optionally fluorine-substituted, divalent hydrocarbon radical having 3 to 10 carbon atoms,
and the salts, hydrates and/or solvates thereof,
with the exception of 2-chloro-6,7,8,9,10,10a-hexahydrocyclohepta[b]thiochromen-11(5aH)-one.

2. Compounds of the formula (I) according to Claim 1, where
the radical R¹-A- is located at position 3 of the thiochromenone ring,
R¹ is (C₆-C₁₀)-aryl or 5- to 10-membered heteroaryl, where aryl and heteroaryl are optionally substituted identically or differently up to twice by radicals selected from the group of halogen, formyl, cyano, hydroxyl, hydroxymethyl, (C₁-C₆)-alkyl,
is 4- to 10-membered heterocyclyl, where heterocyclyl are optionally substituted identically or differently by radicals selected from the group of (C₁-C₆)-alkyl, (C₁-C₆)-alkoxy, (C₁-C₆)-alkoxycarbonyl or oxo,
A is a bond or a group of the formula NR⁶, CO-NR⁷, SO₂-NR⁸ or NR⁹⁻CO,
in which R⁶, R⁷, R⁸ and R⁹ are optionally unsaturated (C₁-C₆)-alkyl which is optionally substituted up to twice, identically or differently, by hydroxyl or methoxy, or
in which R⁶, R⁷ and R⁹ are hydrogen,
R² is hydrogen, and
D is a group of the formula (CH₂)ₘ-CR¹⁶R¹⁷-(CH₂)ₙ,
in which
the total number of carbon atoms is 3 to 10,
m and n are identical or different and are a natural number from the series 0 to 6,
and
R¹⁶ and R¹⁷ are identical or different and are hydrogen or (C₁-C₆)-alkyl which is optionally substituted identically or differently by (C₃₋C₅)-cycloalkyl or halogen,
or
CR¹⁶R¹⁷ is (C₃-C₆)-cycloalkane-1,1-diyl,
and the salts, hydrates and/or solvates thereof.

3. Compounds of the formula (I) according to Claim 1, where
the radical R¹-A- is located at position 3 of the thiochromenone ring,
R¹ is phenyl or 5- to 6-membered heteroaryl, where phenyl and heteroaryl are optionally substituted identically or differently up to twice by radicals selected from the group of halogen, cyano, (C₁-C₃)-alkyl,
is 5- to 7-membered heterocyclyl, where heterocyclyl are optionally substituted identically or differently by radicals selected from the group of (C₁-C₃)-alkyl or Oxo,
A is a bond or a group of the formula NR⁶, SO₂-NR⁸ or NR⁹-CO,
in which R⁶, R⁸ and R⁹ are optionally unsaturated (C₁-C₃)-alkyl which is optionally substituted up to twice, identically or differently, by hydroxyl or methoxy, and
in which R⁶, R⁸ and R⁹ are hydrogen,
R² is hydrogen, and
D is a group of the formula (CH₂)ₘ-CR¹⁶R¹⁷-(CH₂)ₙ,
in which
the total number of carbon atoms is 3 to 6,
m and n are identical or different and are a natural number from the series 0 to 2,
and
R¹⁶ and R¹⁷ are identical or different and are hydrogen or (C₁-C₃)-alkyl,
or
CR¹⁶R¹⁷ is (C₃-C₆)-cycloalkane-1,1-diyl,
and the salts, hydrates and/or solvates thereof.

4. Compounds of the formula (I) according to Claim 1 for the treatment and/or prophylaxis of diseases.

5. Medicament comprising at least one of the compounds of the formula (I) according to any of Claims 1 to 3 mixed with at least one pharmaceutically acceptable, essentially non-toxic carrier or excipient.

6. Use of compounds of the formula (I) according to any of Claims 1 to 3 for producing a medicament for the treatment and/or prophylaxis of states of pain and/or neurodegenerative disorders.

## Revendications

1. Composés de formule générale (I) 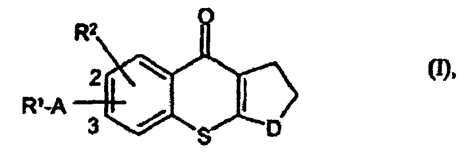 dans laquelle
le reste R¹-A- occupe l'une des positions 2 ou 3 du noyau de thiochroménone,
R¹ représente un reste aryle en C₆ à C₁₀ ou un reste hétéroaryle pentagonal à décagonal, le reste aryle et le reste hétéroaryle étant éventuellement substitués par des restes identiques ou différents choisis dans le groupe halogéno, formyle, carbamoyle, cyano, hydroxy, trifluorométhoxy, nitro, -NR³R⁴, tétrazolyle, (alkoxy en C₁ à C₆) carbonyle ainsi que, le cas échéant, alkyle en C₁ à C₆, alkoxy en C₁ à C₆, acyle en C₁ à C₆ et alkylthio en C₁ à C₆ portant éventuellement un substituant hydroxy, morpholinyle, acyloxy en C₁ à C₆ ou halogéno,
où R³ et R⁴ représentent indépendamment l'un de l'autre l'hydrogène, un reste alkyle en C₁ à C₆ ou acyle en C₁ à C₆,
un reste carbocyclyle triangulaire à dodécagonal ou un reste hétérocyclyle tétragonal à dodécagonal, les restes carbocyclyle et hétérocyclyle étant éventuellement substitués par des restes, identiques ou différents, choisis dans le groupe des restes alkyle en C₁ à C₆, alkoxy en C₁ à C₆, acyle en C₁ à C₆, (alkoxy en C₁ à C₆)carbonyle ou oxo,
et
A désigne une liaison ou représente un groupe de formule O, S, NR⁶, CO, SO, SO₂, SO₂-O, CO-NR⁷, SO₂-NR⁸, O-SO₂, NR⁹-CO, NR¹⁰-SO₂, NR¹¹-SO₂-O, NR¹²-SO₂-NR¹³ ou NR¹⁴-CO-NR¹⁵,
où R⁶, R⁷, R⁸, R⁹, R¹⁰, R¹¹, R¹², R¹³, R¹⁴ et R¹⁵ représentent un reste cycloalkyle en C₃ à C₈ ou un reste alkyle en C₁ à C₆ éventuellement insaturé, qui porte éventuellement un substituant hydroxy, phényle, alkoxy en C₁ à C₆, (alkoxy en C₁ à C₆)carbonyle ou cycloalkyle en C₃ à C₈, le reste phényle pouvant lui-même être substitué par un radical halogéno ou alkyle en C₁ à C₄, ou bien
R⁶, R⁷, R⁹, R¹⁰, R¹¹, R¹², R¹⁴ et R¹⁵ représentent l'hydrogène,
ou bien
R¹ désigne un groupe de formule R⁵-E-,
dans laquelle
E est un reste alcanediyle en C₁ à C₁₀ éventuellement insaturé, et
R⁵ représente l'hydrogène, un groupe carbamoyle, un halogène, un groupe hydroxy, nitro, un reste trifluorométhyle, un groupe amino, un reste mono(alkyle en C₁ à C₆)amino, di(alkyle en C₁ à C₆)amino, alkoxy en C₁ à C₆, aryle en C₆ à C₁₀, hétéroaryle pentagonal à décagonal ou hétérocyclyle tétragonal à décagonal portant éventuellement un substituant oxo et/ou alkyle en C₁ à C₆ et éventuellement condensé au benzène, les restes aryle, hétéroaryle et benzo pouvant eux-mêmes être substitués par des restes choisis dans le groupe des restes halogéno, cyano, trifluorométhyle, trifluorométhoxy, nitro et alkyle en C₁ à C₆,
et
A représente S, NR⁶, CO, SO, SO₂, SO₂-O, CO-NR⁷, SO₂-NR⁸, O-SO₂, NR⁹-CO, NR¹⁰-SO₂, NR¹¹-SO₂-O, NR¹²-SO₂-NR¹³ ou NR¹⁴⁻CO-NR¹⁵ ,
où R⁶, R⁷, R⁸, R⁹, R¹⁰, R¹¹, R¹², R¹³, R¹⁴ et R¹⁵ représentent un reste cycloalkyle en C₃ à C₈ ou un reste alkyle en C₁ à C₆ éventuellement insaturé, qui porte éventuellement un substituant hydroxy, phényle, alkoxy en C₁ à C₆, (alkoxy en C₁ à C₆) carbonyle ou cycloalkyle en C₃ à C₈, le reste phényle pouvant lui- même être porté par un substituant halogéno ou alkyle en C₁ à C₄, ou bien
R⁶, R⁷, R⁹, R¹⁰, R¹¹, R¹², R¹⁴ et R¹⁵ représentent l'hydrogène,
ou bien
le reste R¹-A- représente l'hydrogène ou un groupe amino,
R² représente l'hydrogène, un halogène, un reste alkyle en C₁ à C₆ ou alkoxy en C₁ à C₆, le reste alkyle et le reste alkoxy étant éventuellement substitués jusqu'à deux fois identiques ou différentes par des restes choisis dans le groupe des restes hydroxy, alkoxy en C₁ à C₆, mono- et di (alkyle en C₁ à C₆) amino, et
D désigne un reste hydrocarboné de trois à dix atomes de carbone, à deux liaisons, éventuellement substitué par du fluor,
et leurs sels, leurs hydrates et/ou leurs produits de solvatation,
excepté la 2-chloro-6,7,8,9,10,10a-hexahydrocyclohepta[b]-thiochromène-11(5aH)-one.

2. Composés de formule générale (I) suivant la revendication 1, formule dans laquelle
le reste R¹-A- occupe la position 3 du noyau de thiochroménone,
R¹ est un reste aryle en C₆ à C₁₀ ou un reste hétéroaryle pentagonal à décagonal, le reste aryle et le reste hétéroaryle étant éventuellement substitués jusqu'à deux fois par des restes, identiques ou différents, choisis dans le groupe des restes halogéno, formyle, cyano, hydroxy, hydroxyméthyle, alkyle en C₁ à C₆, un reste hétérocyclyle pentagonal à décagonal, le reste hétérocyclyle étant éventuellement substitué par des restes, identiques ou différents, choisis dans le groupe des restes alkyle en C₁ à C₆, alkoxy en C₁ à C₆, (alkoxy en C₁ à C₆)carbonyle ou oxo,
A désigne une liaison ou un groupe de formule NR⁶, CO-NR⁷, SO₂-NR⁸ ou NR⁹-CO,
où R⁶, R⁷, R⁸ et R⁹ désignent un reste alkyle en C₁ à C₆, éventuellement insaturé, qui porte le cas échéant jusqu'à deux substituants, identiques ou différents, hydroxy ou méthoxy, ou bien
R⁶, R⁷ et R⁹ désignent l'hydrogène,
R² désigne l'hydrogène, et
D est un groupe de formule (CH₂)ₘ-CR¹⁶R¹⁷-(CH₂)ₙ,
dans lequel
le nombre total d'atomes de carbone va de 3 à 10,
m et n sont identiques ou différents et représentent un nombre entier de la série de 0 à 6,
et
R¹⁶ et R¹⁷ sont identiques ou différents et représentent l'hydrogène ou un reste alkyle en C₁ à C₆, qui est éventuellement substitué de façon identique ou différente par un radical cycloalkyle en C₃ à C₅ ou halogéno,
ou bien
CR¹⁶R¹⁷ désigne un reste cycloalcane-1,1-diyle en C₃ à C₆, et leurs sels, leurs hydrates et/ou leurs produits de solvatation.

3. Composés de formule générale (I) suivant la revendication 1, formule dans laquelle
le reste R¹-A- est en position 3 sur le noyau de thiochroménone,
R¹ est un reste phényle ou un reste hétéroaryle pentagonal ou hexagonal, le reste phényle et le reste hétéroaryle pouvant éventuellement être substitués jusqu'à deux fois par des restes choisis dans le groupe des restes halogéno, cyano, alkyle en C₁ à C₃,
un reste hétérocyclyle pentagonal à heptagonal, le reste hétérocyclyle étant éventuellement substitué par des restes identiques ou différents choisis dans le groupe des restes alkyle en C₁ à C₃ ou oxo,
A désigne une liaison ou représente un groupe de formule NR⁶, SO₂-NR⁸ ou NR₉-CO,
où R⁶, R⁸ et R⁹ désignent un reste alkyle en C₁ à C₃ éventuellement insaturé, qui porte le cas échéant jusqu'à deux substituants, identiques ou différents, hydroxy ou méthoxy, et
où R⁶, R⁸ et R⁹ représentent l'hydrogène,
R² représente l'hydrogène, et
D désigne un groupe de formule (CH₂)ₘ-CR¹⁶R¹⁷-(CH₂)ₙ, où
le nombre total d'atomes de carbone est de 3 à 6,
m et n sont identiques ou différents et représentent un nombre entier de la série 0 à 2,
et
R¹⁶ et R¹⁷ sont identiques ou différents et représentent l'hydrogène ou un reste alkyle en C₁ à C₃,
ou bien
CR¹⁶R¹⁷ désigne un groupe cycloalcane-1,1-diyle en C₃ à C₆,
et leurs sels, leurs hydrates et/ou leurs produits de solvatation.

4. Composés de formule générale (I) suivant la revendication 1, destinés au traitement et/ou à la prophylaxie de maladies.

5. Médicament contenant au moins l'un des composés de formule générale (I) suivant l'une des revendications 1 à 3, en mélange avec au moins un support ou excipient principalement non toxique, acceptable du point pharmaceutique.

6. utilisation de composés de formule générale (I) suivant l'une des revendications 1 à 3, pour la préparation d'un médicament destiné au traitement et/ou à la prophylaxie d'états douloureux et/ou de maladies neurodégénératives.
